# EUROPEAN PATENT SPECIFICATION

(11) **EP 2 899 203 B1**
(45) Date of publication and mention of the grant of the patent: **10.07.2019**
(21) Application number: 15154516.7
(22) Date of filing: 06.05.2009
(51) Int. Cl.: C07K 14/435, A61K 39/008, A61K 39/00, G01N 33/68, C12N 15/12, C07K 16/18, A61P 33/02

(54) **LEISHMANIA VACCINE USING SAND FLY SALIVARY IMMUNOGEN**
LEISHMANIA IMPFSTOFF UNTER VERWENDUNG VON SANDMÜCKEN SPEICHELIMMUNOGEN
VACCIN CONTRE LA LEISHMANIOSE UTILISANT UN IMMUNOGÈNE SALIVAIRE DE PHLÉBOTOME

(30) Priority: 08.05.2008 US 51635 P; 30.09.2008 US 101345 P
(43) Date of publication of application: 29.07.2015
(62) Divisional of application: 09743563.0
(73) Proprietor: Boehringer Ingelheim Animal Health USA Inc., Duluth, GA 30096 (US); GOV'T of The USA as represented by the Secretary of the Department of Health and Human Services, Rockville, MD 20852 (US)
(72) Inventor: Fischer, Laurent Bernard, 69110 Sainte Foy Les Lyon (FR); Valenzuela, Jesus G., Gaithersburg, MD 20878 (US); Ribeiro, Jose, Rockville, MD 20855 (US); Kamhawi, Shaden, Darwood, MD 20855 (US)
(74) Representative: D Young & Co LLP

(56) References cited:
- WO-A-2004/039958
- US-A1- 2006 051 364
- COLLIN NICOLAS ET AL: "Sand Fly Salivary Proteins Induce Strong Cellular Immunity in a Natural Reservoir of Visceral Leishmaniasis with Adverse Consequences for Leishmania", PLOS PATHOGENS, vol. 5, no. 5, May 2009 (2009-05), XP002548409,
- SCHUNK MICHAEL K ET AL: "Applications and optimization of immunization procedures", ILAR JOURNAL, vol. 46, no. 3, 2005, pages 241-257, ISSN: 1084-2020

### SEQUENCE LISTING

<110> Merial Limited
   The Government of the United States of America as represented by the Secretary of the Department of Health and Human Services
<120> Leishmania Vaccine Using Sand Fly Salivary Immunogen
<130> P042348EPA
<150> US 61/051,635
   <151> 2008-05-08
<150> US 61/101,345
   <151> 2008-09-30
<160> 94
<170> PatentIn version 3.5
<210> 1
   <211> 301
   <212> PRT
   <213> Lutzomyia longipalpis
<400> 1
   Met Asn Ser Ile Asn Phe Leu Ser Ile Val Gly Leu Ile Ser Phe Gly
   1               5                   10                  15
   Phe Ile Val Ala Val Lys Cys Asp Gly Asp Glu Tyr Phe Ile Gly Lys
               20                  25                  30
   Tyr Lys Glu Lys Asp Glu Thr Leu Phe Phe Ala Ser Tyr Gly Leu Lys
           35                  40                  45
   Arg Asp Pro Cys Gln Ile Val Leu Gly Tyr Lys Cys Ser Asn Asn Gln
       50                  55                  60
   Thr His Phe Val Leu Asn Phe Lys Thr Asn Lys Lys Ser Cys Ile Ser
   65                  70                  75                  80
   Ala Ile Lys Leu Thr Ser Tyr Pro Lys Ile Asn Gln Asn Ser Asp Leu
                   85                  90                  95
   Thr Lys Asn Leu Tyr Cys Gln Thr Gly Gly Ile Gly Thr Asp Asn Cys
               100                 105                 110
   Lys Leu Val Phe Lys Lys Arg Lys Arg Gln Ile Ala Ala Asn Ile Glu
           115                 120                 125
   Ile Tyr Gly Ile Pro Ala Lys Lys Cys Ser Phe Lys Asp Arg Tyr Ile
       130                 135                 140
   Gly Ala Asp Pro Leu His Val Asp Ser Tyr Gly Leu Pro Tyr Gln Phe
   145                 150                 155                 160
   Asp Gln Glu His Gly Trp Asn Val Glu Arg Tyr Asn Ile Phe Lys Asp
                   165                 170                 175
   Thr Arg Phe Ser Thr Glu Val Phe Tyr His Lys Asn Gly Leu Phe Asn
               180                 185                 190
   Thr Gln Ile Thr Tyr Leu Ala Glu Glu Asp Ser Phe Ser Glu Ala Arg
           195                 200                 205
   Glu Ile Thr Ala Lys Asp Ile Lys Lys Lys Phe Ser Ile Ile Leu Pro
       210                 215                 220
   Asn Glu Glu Tyr Lys Arg Ile Ser Phe Leu Asp Val Tyr Trp Phe Gln
   225                 230                 235                 240
   Glu Thr Met Arg Lys Lys Pro Lys Tyr Pro Tyr Ile His Tyr Asn Gly
                   245                 250                 255
   Glu Cys Ser Asn Glu Asn Lys Thr Cys Glu Leu Val Phe Asp Thr Asp
               260                 265                 270
   Glu Leu Met Thr Tyr Ala Leu Val Lys Val Phe Thr Asn Pro Glu Ser
           275                 280                 285
   Asp Gly Ser Arg Leu Lys Glu Glu Asp Leu Gly Arg Gly
       290                 295                 300
<210> 2
   <211> 906
   <212> DNA
   <213> Lutzomyia longipalpis
<400> 2
   atgaattcga ttaatttcct atcaatagtt ggtttaatca gttttggatt cattgttgca     60
   gtaaagtgtg atggtgatga atatttcatt ggaaaataca aagaaaaaga tgagacactg    120
   ttttttgcaa gctacggcct aaagagggat ccttgccaaa ttgtcttagg ctacaaatgc    180
   tcaaacaatc aaacccactt tgtgcttaat tttaaaacca ataagaaatc ctgcatatca    240
   gcaattaagc tgacttctta cccaaaaatc aatcaaaact cggatttaac taaaaatctc    300
   tactgccaaa ctggaggaat aggaacagat aactgcaaac ttgtcttcaa gaaacgtaaa    360
   agacaaatag cagctaatat tgaaatctac ggcattccag cgaagaaatg ttccttcaag    420
   gatcgttaca ttggagctga tccactccac gtcgattcct atgggcttcc gtatcagttt    480
   gatcaggaac atggatggaa tgtggaacga tataacattt tcaaagacac aagattttcc    540
   acagaagttt tctaccacaa aaatggttta tttaacaccc aaataactta tttggctgaa    600
   gaagattcct tctctgaagc tcgagagatt actgcgaagg atattaagaa gaagttttca    660
   attattttgc ccaatgaaga gtataagagg attagtttct tggacgttta ttggttccag    720
   gagactatgc gaaaaaagcc taaatatccc tacattcact acaatggaga atgcagcaat    780
   gagaataaaa cttgtgaact tgtctttgac accgatgaac taatgaccta cgcccttgtt    840
   aaagtcttta ctaatcctga gagtgatgga tctaggctca aagaagagga tttgggaaga    900
   ggataa                                                               906
<210> 3
   <211> 278
   <212> PRT
   <213> Lutzomyia longipalpis
<400> 3
   Asp Gly Asp Glu Tyr Phe Ile Gly Lys Tyr Lys Glu Lys Asp Glu Thr
   1               5                   10                  15
   Leu Phe Phe Ala Ser Tyr Gly Leu Lys Arg Asp Pro Cys Gln Ile Val
               20                  25                  30
   Leu Gly Tyr Lys Cys Ser Asn Asn Gln Thr His Phe Val Leu Asn Phe
           35                  40                  45
   Lys Thr Asn Lys Lys Ser Cys Ile Ser Ala Ile Lys Leu Thr Ser Tyr
       50                  55                  60
   Pro Lys Ile Asn Gln Asn Ser Asp Leu Thr Lys Asn Leu Tyr Cys Gln
   65                  70                  75                  80
   Thr Gly Gly Ile Gly Thr Asp Asn Cys Lys Leu Val Phe Lys Lys Arg
                   85                  90                  95
   Lys Arg Gln Ile Ala Ala Asn Ile Glu Ile Tyr Gly Ile Pro Ala Lys
               100                 105                 110
   Lys Cys Ser Phe Lys Asp Arg Tyr Ile Gly Ala Asp Pro Leu His Val
           115                 120                 125
   Asp Ser Tyr Gly Leu Pro Tyr Gln Phe Asp Gln Glu His Gly Trp Asn
       130                 135                 140
   Val Glu Arg Tyr Asn Ile Phe Lys Asp Thr Arg Phe Ser Thr Glu Val
   145                 150                 155                 160
   Phe Tyr His Lys Asn Gly Leu Phe Asn Thr Gln Ile Thr Tyr Leu Ala
                   165                 170                 175
   Glu Glu Asp Ser Phe Ser Glu Ala Arg Glu Ile Thr Ala Lys Asp Ile
               180                 185                 190
   Lys Lys Lys Phe Ser Ile Ile Leu Pro Asn Glu Glu Tyr Lys Arg Ile
           195                 200                 205
   Ser Phe Leu Asp Val Tyr Trp Phe Gln Glu Thr Met Arg Lys Lys Pro
       210                 215                 220
   Lys Tyr Pro Tyr Ile His Tyr Asn Gly Glu Cys Ser Asn Glu Asn Lys
   225                 230                 235                 240
   Thr Cys Glu Leu Val Phe Asp Thr Asp Glu Leu Met Thr Tyr Ala Leu
                   245                 250                 255
   Val Lys Val Phe Thr Asn Pro Glu Ser Asp Gly Ser Arg Leu Lys Glu
               260                 265                 270
   Glu Asp Leu Gly Arg Gly
           275
<210> 4
   <211> 837
   <212> DNA
   <213> Lutzomyia longipalpis
<400> 4
   gatggtgatg aatatttcat tggaaaatac aaagaaaaag atgagacact gttttttgca      60
   agctacggcc taaagaggga tccttgccaa attgtcttag gctacaaatg ctcaaacaat     120
   caaacccact ttgtgcttaa ttttaaaacc aataagaaat cctgcatatc agcaattaag     180
   ctgacttctt acccaaaaat caatcaaaac tcggatttaa ctaaaaatct ctactgccaa     240
   actggaggaa taggaacaga taactgcaaa cttgtcttca agaaacgtaa aagacaaata     300
   gcagctaata ttgaaatcta cggcattcca gcgaagaaat gttccttcaa ggatcgttac     360
   attggagctg atccactcca cgtcgattcc tatgggcttc cgtatcagtt tgatcaggaa     420
   catggatgga atgtggaacg atataacatt ttcaaagaca caagattttc cacagaagtt     480
   ttctaccaca aaaatggttt atttaacacc caaataactt atttggctga agaagattcc     540
   ttctctgaag ctcgagagat tactgcgaag gatattaaga agaagttttc aattattttg     600
   cccaatgaag agtataagag gattagtttc ttggacgttt attggttcca ggagactatg     660
   cgaaaaaagc ctaaatatcc ctacattcac tacaatggag aatgcagcaa tgagaataaa     720
   acttgtgaac ttgtctttga caccgatgaa ctaatgacct acgcccttgt taaagtcttt     780
   actaatcctg agagtgatgg atctaggctc aaagaagagg atttgggaag aggataa        837
<210> 5
   <211> 412
   <212> PRT
   <213> Lutzomyia longipalpis
<400> 5
   Met Arg Phe Phe Phe Val Phe Leu Ala Ile Val Leu Phe Gln Gly Ile
   1               5                   10                  15
   His Gly Ala Tyr Val Glu Ile Gly Tyr Ser Leu Arg Asn Ile Thr Phe
               20                  25                  30
   Asp Gly Leu Asp Thr Asp Asp Tyr Asn Pro Lys Phe Asn Ile Pro Thr
           35                  40                  45
   Gly Leu Ala Val Asp Pro Glu Gly Tyr Arg Leu Phe Ile Ala Ile Pro
       50                  55                  60
   Arg Arg Lys Pro Lys Val Pro Tyr Thr Val Ala Glu Leu Asn Met Val
   65                  70                  75                  80
   Met Asn Pro Gly Phe Pro Val Glu Arg Ala Pro Ser Phe Glu Lys Phe
                   85                  90                  95
   Lys Lys Phe Asn Gly Glu Gly Lys Lys Asp Leu Val Asn Val Tyr Gln
               100                 105                 110
   Pro Val Ile Asp Asp Cys Arg Arg Leu Trp Val Leu Asp Ile Gly Lys
           115                 120                 125
   Val Glu Tyr Thr Gly Gly Asp Ala Asp Gln Tyr Pro Lys Gly Lys Pro
       130                 135                 140
   Thr Leu Ile Ala Tyr Asp Leu Lys Lys Asp His Thr Pro Glu Ile His
   145                 150                 155                 160
   Arg Phe Glu Ile Pro Asp Asp Leu Tyr Ser Ser Gln Val Glu Phe Gly
                   165                 170                 175
   Gly Phe Ala Val Asp Val Val Asn Thr Lys Gly Asp Cys Thr Glu Ser
               180                 185                 190
   Phe Val Tyr Leu Thr Asn Phe Lys Asp Asn Ser Leu Ile Val Tyr Asp
           195                 200                 205
   Glu Thr Gln Lys Lys Ala Trp Lys Phe Thr Asp Lys Thr Phe Glu Ala
       210                 215                 220
   Asp Lys Glu Ser Thr Phe Ser Tyr Ser Gly Glu Glu Gln Met Lys Tyr
   225                 230                 235                 240
   Lys Val Gly Leu Phe Gly Ile Ala Leu Gly Asp Arg Asp Glu Met Gly
                   245                 250                 255
   His Arg Pro Ala Cys Tyr Ile Ala Gly Ser Ser Thr Lys Val Tyr Ser
               260                 265                 270
   Val Asn Thr Lys Glu Leu Lys Thr Glu Asn Gly Gln Leu Asn Pro Gln
           275                 280                 285
   Leu His Gly Asp Arg Gly Lys Tyr Thr Asp Ala Ile Ala Leu Ala Tyr
       290                 295                 300
   Asp Pro Glu His Lys Val Leu Tyr Phe Ala Glu Ser Asp Ser Arg Gln
   305                 310                 315                 320
   Val Ser Cys Trp Asn Val Asn Met Glu Leu Lys Pro Asp Asn Thr Asp
                   325                 330                 335
   Val Ile Phe Ser Ser Ala Arg Phe Thr Phe Gly Thr Asp Ile Leu Val
               340                 345                 350
   Asp Ser Lys Gly Met Leu Trp Ile Met Ala Asn Gly His Pro Pro Val
           355                 360                 365
   Glu Asp Gln Glu Lys Ile Trp Lys Met Arg Phe Val Asn Arg Lys Ile
       370                 375                 380
   Arg Ile Met Lys Val Asp Thr Glu Arg Val Phe Lys Tyr Ser Arg Cys
   385                 390                 395                 400
   Asn Pro Asn Tyr Lys Pro Pro Lys Glu Ile Glu Val
                   405                 410
<210> 6
   <211> 1239
   <212> DNA
   <213> Lutzomyia longipalpis
<400> 6
   atgaggttct tctttgtttt ccttgccatc gtcctttttc aagggatcca cggagcttat      60
   gtggaaatag gatattctct gagaaatatt acattcgatg gattggatac agatgactac     120
   aatccaaagt tcaacattcc aacgggtttg gcagttgatc ccgaaggata taggctcttc     180
   atagccatcc caaggagaaa gccaaaggtt ccctacactg tggctgaact gaatatggtc     240
   atgaatcccg gatttcccgt cgagagagct ccgagctttg agaaattcaa aaaattcaat     300
   ggcgagggca aaaaggatct tgttaatgtg tatcagccag tcattgatga ttgtcgtcgt     360
   ctttgggtgc ttgacattgg gaaggtggaa tacaccggtg gtgatgctga tcaatatccc     420
   aaaggaaagc ctaccctaat tgcctacgac ctcaagaagg atcatactcc ggaaattcat     480
   cgatttgaaa ttccagacga tctctatagc tcacaagttg aatttggtgg atttgccgtt     540
   gatgttgtta acacgaaagg agactgtacg gagtcatttg tctacctgac caatttcaag     600
   gataactctc taattgtcta cgatgagaca caaaagaaag cttggaaatt cacagataaa     660
   acatttgaag ctgataagga atccacgttc tcctactcgg gagaggaaca aatgaagtac     720
   aaagtcggtc tttttgggat agctctgggt gatagggatg aaatggggca tcgtcctgcc     780
   tgctacatcg ctgggagtag caccaaagtc tacagtgtta acactaaaga actcaaaaca     840
   gagaatggtc agttaaatcc tcagcttcac ggtgatcgtg gaaagtacac agatgcaatt     900
   gccctagcct acgatcctga gcataaagtc ctctactttg ctgaatccga cagcaggcag     960
   gtgtcctgtt ggaatgtaaa tatggagcta aaaccagaca atacggatgt gatcttctct    1020
   agtgcccgtt ttacttttgg aacggatatt ttggttgata gcaagggaat gctgtggata    1080
   atggctaatg gacatccacc agtagaggat caagagaaga tttggaagat gagattcgta    1140
   aaccggaaga tccgtattat gaaagtggat acggaacgtg ttttcaaata ttcacgctgc    1200
   aatccaaatt ataagccccc aaaggaaatt gaagtttga                           1239
<210> 7
   <211> 394
   <212> PRT
   <213> Lutzomyia longipalpis
<400> 7
   Ala Tyr Val Glu Ile Gly Tyr Ser Leu Arg Asn Ile Thr Phe Asp Gly
   1               5                   10                  15
   Leu Asp Thr Asp Asp Tyr Asn Pro Lys Phe Asn Ile Pro Thr Gly Leu
               20                  25                  30
   Ala Val Asp Pro Glu Gly Tyr Arg Leu Phe Ile Ala Ile Pro Arg Arg
           35                  40                  45
   Lys Pro Lys Val Pro Tyr Thr Val Ala Glu Leu Asn Met Val Met Asn
       50                  55                  60
   Pro Gly Phe Pro Val Glu Arg Ala Pro Ser Phe Glu Lys Phe Lys Lys
   65                  70                  75                  80
   Phe Asn Gly Glu Gly Lys Lys Asp Leu Val Asn Val Tyr Gln Pro Val
                   85                  90                  95
   Ile Asp Asp Cys Arg Arg Leu Trp Val Leu Asp Ile Gly Lys Val Glu
               100                 105                 110
   Tyr Thr Gly Gly Asp Ala Asp Gln Tyr Pro Lys Gly Lys Pro Thr Leu
           115                 120                 125
   Ile Ala Tyr Asp Leu Lys Lys Asp His Thr Pro Glu Ile His Arg Phe
       130                 135                 140
   Glu Ile Pro Asp Asp Leu Tyr Ser Ser Gln Val Glu Phe Gly Gly Phe
   145                 150                 155                 160
   Ala Val Asp Val Val Asn Thr Lys Gly Asp Cys Thr Glu Ser Phe Val
                   165                 170                 175
   Tyr Leu Thr Asn Phe Lys Asp Asn Ser Leu Ile Val Tyr Asp Glu Thr
               180                 185                 190
   Gln Lys Lys Ala Trp Lys Phe Thr Asp Lys Thr Phe Glu Ala Asp Lys
           195                 200                 205
   Glu Ser Thr Phe Ser Tyr Ser Gly Glu Glu Gln Met Lys Tyr Lys Val
       210                 215                 220
   Gly Leu Phe Gly Ile Ala Leu Gly Asp Arg Asp Glu Met Gly His Arg
   225                 230                 235                 240
   Pro Ala Cys Tyr Ile Ala Gly Ser Ser Thr Lys Val Tyr Ser Val Asn
                   245                 250                 255
   Thr Lys Glu Leu Lys Thr Glu Asn Gly Gln Leu Asn Pro Gln Leu His
               260                 265                 270
   Gly Asp Arg Gly Lys Tyr Thr Asp Ala Ile Ala Leu Ala Tyr Asp Pro
           275                 280                 285
   Glu His Lys Val Leu Tyr Phe Ala Glu Ser Asp Ser Arg Gln Val Ser
       290                 295                 300
   Cys Trp Asn Val Asn Met Glu Leu Lys Pro Asp Asn Thr Asp Val Ile
   305                 310                 315                 320
   Phe Ser Ser Ala Arg Phe Thr Phe Gly Thr Asp Ile Leu Val Asp Ser
                   325                 330                 335
   Lys Gly Met Leu Trp Ile Met Ala Asn Gly His Pro Pro Val Glu Asp
               340                 345                 350
   Gln Glu Lys Ile Trp Lys Met Arg Phe Val Asn Arg Lys Ile Arg Ile
           355                 360                 365
   Met Lys Val Asp Thr Glu Arg Val Phe Lys Tyr Ser Arg Cys Asn Pro
       370                 375                 380
   Asn Tyr Lys Pro Pro Lys Glu Ile Glu Val
   385                 390
<210> 8
   <211> 1185
   <212> DNA
   <213> Lutzomyia longipalpis
<400> 8
   gcttatgtgg aaataggata ttctctgaga aatattacat tcgatggatt ggatacagat     60
   gactacaatc caaagttcaa cattccaacg ggtttggcag ttgatcccga aggatatagg    120
   ctcttcatag ccatcccaag gagaaagcca aaggttccct acactgtggc tgaactgaat    180
   atggtcatga atcccggatt tcccgtcgag agagctccga gctttgagaa attcaaaaaa    240
   ttcaatggcg agggcaaaaa ggatcttgtt aatgtgtatc agccagtcat tgatgattgt    300
   cgtcgtcttt gggtgcttga cattgggaag gtggaataca ccggtggtga tgctgatcaa    360
   tatcccaaag gaaagcctac cctaattgcc tacgacctca agaaggatca tactccggaa    420
   attcatcgat ttgaaattcc agacgatctc tatagctcac aagttgaatt tggtggattt    480
   gccgttgatg ttgttaacac gaaaggagac tgtacggagt catttgtcta cctgaccaat    540
   ttcaaggata actctctaat tgtctacgat gagacacaaa agaaagcttg gaaattcaca    600
   gataaaacat ttgaagctga taaggaatcc acgttctcct actcgggaga ggaacaaatg    660
   aagtacaaag tcggtctttt tgggatagct ctgggtgata gggatgaaat ggggcatcgt    720
   cctgcctgct acatcgctgg gagtagcacc aaagtctaca gtgttaacac taaagaactc    780
   aaaacagaga atggtcagtt aaatcctcag cttcacggtg atcgtggaaa gtacacagat    840
   gcaattgccc tagcctacga tcctgagcat aaagtcctct actttgctga atccgacagc    900
   aggcaggtgt cctgttggaa tgtaaatatg gagctaaaac cagacaatac ggatgtgatc    960
   ttctctagtg cccgttttac ttttggaacg gatattttgg ttgatagcaa gggaatgctg   1020
   tggataatgg ctaatggaca tccaccagta gaggatcaag agaagatttg gaagatgaga   1080
   ttcgtaaacc ggaagatccg tattatgaaa gtggatacgg aacgtgtttt caaatattca   1140
   cgctgcaatc caaattataa gcccccaaag gaaattgaag tttga                   1185
<210> 9
   <211> 6247
   <212> DNA
   <213> Artificial sequence
<220>
   <223> nucleic acid sequence of one strand of the plasmid pVR2001 LJM17
<400> 9
   aagggatcca gatctgctgt gccttctagt tgccagccat ctgttgtttg cccctccccc     60
   gtgccttcct tgaccctgga aggtgccact cccactgtcc tttcctaata aaatgaggaa    120
   attgcatcgc attgtctgag taggtgtcat tctattctgg ggggtggggt ggggcagcac    180
   agcaaggggg aggattggga agacaatagc aggcatgctg gggatgcggt gggctctatg    240
   ggtacccagg tgctgaagaa ttgacccggt tcctcctggg ccagaaagaa gcaggcacat    300
   ccccttctct gtgacacacc ctgtccacgc ccctggttct tagttccagc cccactcata    360
   ggacactcat agctcaggag ggctccgcct tcaatcccac ccgctaaagt acttggagcg    420
   gtctctccct ccctcatcag cccaccaaac caaacctagc ctccaagagt gggaagaaat    480
   taaagcaaga taggctatta agtgcagagg gagagaaaat gcctccaaca tgtgaggaag    540
   taatgagaga aatcatagaa tttcttccgc ttcctcgctc actgactcgc tgcgctcggt    600
   cgttcggctg cggcgagcgg tatcagctca ctcaaaggcg gtaatacggt tatccacaga    660
   atcaggggat aacgcaggaa agaacatgtg agcaaaaggc cagcaaaagg ccaggaaccg    720
   taaaaaggcc gcgttgctgg cgtttttcca taggctccgc ccccctgacg agcatcacaa    780
   aaatcgacgc tcaagtcaga ggtggcgaaa cccgacagga ctataaagat accaggcgtt    840
   tccccctgga agctccctcg tgcgctctcc tgttccgacc ctgccgctta ccggatacct    900
   gtccgccttt ctcccttcgg gaagcgtggc gctttctcaa tgctcacgct gtaggtatct    960
   cagttcggtg taggtcgttc gctccaagct gggctgtgtg cacgaacccc ccgttcagcc   1020
   cgaccgctgc gccttatccg gtaactatcg tcttgagtcc aacccggtaa gacacgactt   1080
   atcgccactg gcagcagcca ctggtaacag gattagcaga gcgaggtatg taggcggtgc   1140
   tacagagttc ttgaagtggt ggcctaacta cggctacact agaaggacag tatttggtat   1200
   ctgcgctctg ctgaagccag ttaccttcgg aaaaagagtt ggtagctctt gatccggcaa   1260
   acaaaccacc gctggtagcg gtggtttttt tgtttgcaag cagcagatta cgcgcagaaa   1320
   aaaaggatct caagaagatc ctttgatctt ttctacgggg tctgacgctc agtggaacga   1380
   aaactcacgt taagggattt tggtcatgag attatcaaaa aggatcttca cctagatcct   1440
   tttaaattaa aaatgaagtt ttaaatcaat ctaaagtata tatgagtaaa cttggtctga   1500
   cagttaccaa tgcttaatca gtgaggcacc tatctcagcg atctgtctat ttcgttcatc   1560
   catagttgcc tgactccggg gggggggggc gctgaggtct gcctcgtgaa gaaggtgttg   1620
   ctgactcata ccaggcctga atcgccccat catccagcca gaaagtgagg gagccacggt   1680
   tgatgagagc tttgttgtag gtggaccagt tggtgatttt gaacttttgc tttgccacgg   1740
   aacggtctgc gttgtcggga agatgcgtga tctgatcctt caactcagca aaagttcgat   1800
   ttattcaaca aagccgccgt cccgtcaagt cagcgtaatg ctctgccagt gttacaacca   1860
   attaaccaat tctgattaga aaaactcatc gagcatcaaa tgaaactgca atttattcat   1920
   atcaggatta tcaataccat atttttgaaa aagccgtttc tgtaatgaag gagaaaactc   1980
   accgaggcag ttccatagga tggcaagatc ctggtatcgg tctgcgattc cgactcgtcc   2040
   aacatcaata caacctatta atttcccctc gtcaaaaata aggttatcaa gtgagaaatc   2100
   accatgagtg acgactgaat ccggtgagaa tggcaaaagc ttatgcattt ctttccagac 2160
   ttgttcaaca ggccagccat tacgctcgtc atcaaaatca ctcgcatcaa ccaaaccgtt 2220
   attcattcgt gattgcgcct gagcgagacg aaatacgcga tcgctgttaa aaggacaatt 2280
   acaaacagga atcgaatgca accggcgcag gaacactgcc agcgcatcaa caatattttc 2340
   acctgaatca ggatattctt ctaatacctg gaatgctgtt ttcccgggga tcgcagtggt 2400
   gagtaaccat gcatcatcag gagtacggat aaaatgcttg atggtcggaa gaggcataaa 2460
   ttccgtcagc cagtttagtc tgaccatctc atctgtaaca tcattggcaa cgctaccttt 2520
   gccatgtttc agaaacaact ctggcgcatc gggcttccca tacaatcgat agattgtcgc 2580
   acctgattgc ccgacattat cgcgagccca tttataccca tataaatcag catccatgtt 2640
   ggaatttaat cgcggcctcg agcaagacgt ttcccgttga atatggctca taacacccct 2700
   tgtattactg tttatgtaag cagacagttt tattgttcat gatgatatat ttttatcttg 2760
   tgcaatgtaa catcagagat tttgagacac aacgtggctt tccccccccc cccattattg 2820
   aagcatttat cagggttatt gtctcatgag cggatacata tttgaatgta tttagaaaaa 2880
   taaacaaata ggggttccgc gcacatttcc ccgaaaagtg ccacctgacg tctaagaaac 2940
   cattattatc atgacattaa cctataaaaa taggcgtatc acgaggccct ttcgtctcgc 3000
   gcgtttcggt gatgacggtg aaaacctctg acacatgcag ctcccggaga cggtcacagc 3060
   ttgtctgtaa gcggatgccg ggagcagaca agcccgtcag ggcgcgtcag cgggtgttgg 3120
   cgggtgtcgg ggctggctta actatgcggc atcagagcag attgtactga gagtgcacca 3180
   tatgcggtgt gaaataccgc acagatgcgt aaggagaaaa taccgcatca gattggctat 3240
   tggccattgc atacgttgta tccatatcat aatatgtaca tttatattgg ctcatgtcca 3300
   acattaccgc catgttgaca ttgattattg actagttatt aatagtaatc aattacgggg 3360
   tcattagttc atagcccata tatggagttc cgcgttacat aacttacggt aaatggcccg 3420
   cctggctgac cgcccaacga cccccgccca ttgacgtcaa taatgacgta tgttcccata 3480
   gtaacgccaa tagggacttt ccattgacgt caatgggtgg agtatttacg gtaaactgcc 3540
   cacttggcag tacatcaagt gtatcatatg ccaagtacgc cccctattga cgtcaatgac 3600
   ggtaaatggc ccgcctggca ttatgcccag tacatgacct tatgggactt tcctacttgg 3660
   cagtacatct acgtattagt catcgctatt accatggtga tgcggttttg gcagtacatc 3720
   aatgggcgtg gatagcggtt tgactcacgg ggatttccaa gtctccaccc cattgacgtc 3780
   aatgggagtt tgttttggca ccaaaatcaa cgggactttc caaaatgtcg taacaactcc 3840
   gccccattga cgcaaatggg cggtaggcgt gtacggtggg aggtctatat aagcagagct 3900
   cgtttagtga accgtcagat cgcctggaga cgccatccac gctgttttga cctccataga 3960
   agacaccggg accgatccag cctccgcggc cgggaacggt gcattggaac gcggattccc 4020
   cgtgccaaga gtgacgtaag taccgcctat agagtctata ggcccacccc cttggcttct 4080
   tatgcatgct atactgtttt tggcttgggg tctatacacc cccgcttcct catgttatag 4140
   gtgatggtat agcttagcct ataggtgtgg gttattgacc attattgacc actcccctat 4200
   tggtgacgat actttccatt actaatccat aacatggctc tttgccacaa ctctctttat 4260
   tggctatatg ccaatacact gtccttcaga gactgacacg gactctgtat ttttacagga 4320
   tggggtctca tttattattt acaaattcac atatacaaca ccaccgtccc cagtgcccgc 4380
   agtttttatt aaacataacg tgggatctcc acgcgaatct cgggtacgtg ttccggacat 4440
   gggctcttct ccggtagcgg cggagcttct acatccgagc cctgctccca tgcctccagc 4500
   gactcatggt cgctcggcag ctccttgctc ctaacagtgg aggccagact taggcacagc 4560
   acgatgccca ccaccaccag tgtgccgcac aaggccgtgg cggtagggta tgtgtctgaa 4620
   aatgagctcg gggagcgggc ttgcaccgct gacgcatttg gaagacttaa ggcagcggca 4680
   gaagaagatg caggcagctg agttgttgtg ttctgataag agtcagaggt aactcccgtt 4740
   gcggtgctgt taacggtgga gggcagtgta gtctgagcag tactcgttgc tgccgcgcgc 4800
   gccaccagac ataatagctg acagactaac agactgttcc tttccatggg tcttttctca 4860
   cgtcaccgtc gtcgaccaga gctgagatcc tacaggagtc cagggctgga gagaaaacct 4920
   ctgcgaggaa agggaaggag caagccgtga atttaaggga cgctgtgaag caatcatgga 4980
   tgcaatgaag agagggctct gctgtgtgct gctgctgtgt ggagcagtct tcgtttcgcc 5040
   cagcggtacc ggatccaccc ttgcttatgt ggaaatagga tattctctga gaaatattac 5100
   attcgatgga ttggatacag atgactacaa tccaaagttc aacattccaa cgggtttggc 5160
   agttgatccc gaaggatata ggctcttcat agccatccca aggagaaagc caaaggttcc 5220
   ctacactgtg gctgaactga atatggtcat gaatcccgga tttcccgtcg agagagctcc 5280
   gagctttgag aaattcaaaa aattcaatgg cgagggcaaa aaggatcttg ttaatgtgta 5340
   tcagccagtc attgatgatt gtcgtcgtct ttgggtgctt gacattggga aggtggaata 5400
   caccggtggt gatgctgatc aatatcccaa aggaaagcct accctaattg cctacgacct 5460
   caagaaggat catactccgg aaattcatcg atttgaaatt ccagacgatc tctatagctc 5520
   acaagttgaa tttggtggat ttgccgttga tgttgttaac acgaaaggag actgtacgga 5580
   gtcatttgtc tacctgacca atttcaagga taactctcta attgtctacg atgagacaca 5640
   aaagaaagct tggaaattca cagataaaac atttgaagct gataaggaat ccacgttctc 5700
   ctactcggga gaggaacaaa tgaagtacaa agtcggtctt tttgggatag ctctgggtga 5760
   tagggatgaa atggggcatc gtcctgcctg ctacatcgct gggagtagca ccaaagtcta 5820
   cagtgttaac actaaagaac tcaaaacaga gaatggtcag ttaaatcctc agcttcacgg 5880
   tgatcgtgga aagtacacag atgcaattgc cctagcctac gatcctgagc ataaagtcct   5940
   ctactttgct gaatccgaca gcaggcaggt gtcctgttgg aatgtaaata tggagctaaa   6000
   accagacaat acggatgtga tcttctctag tgcccgtttt acttttggaa cggatatttt   6060
   ggttgatagc aagggaatgc tgtggataat ggctaatgga catccaccag tagaggatca   6120
   agagaagatt tggaagatga gattcgtaaa ccggaagatc cgtattatga aagtggatac   6180
   ggaacgtgtt ttcaaatatt cacgctgcaa tccaaattat aagcccccaa aggaaattga   6240
   agtttga                                                             6247
<210> 10
   <211> 5899
   <212> DNA
   <213> Artificial sequence
<220>
   <223> nucleic acid sequence of one strand of the plasmid pVR2001 LJL143
<400> 10
   aagggatcca gatctgctgt gccttctagt tgccagccat ctgttgtttg cccctccccc     60
   gtgccttcct tgaccctgga aggtgccact cccactgtcc tttcctaata aaatgaggaa    120
   attgcatcgc attgtctgag taggtgtcat tctattctgg ggggtggggt ggggcagcac    180
   agcaaggggg aggattggga agacaatagc aggcatgctg gggatgcggt gggctctatg    240
   ggtacccagg tgctgaagaa ttgacccggt tcctcctggg ccagaaagaa gcaggcacat    300
   ccccttctct gtgacacacc ctgtccacgc ccctggttct tagttccagc cccactcata    360
   ggacactcat agctcaggag ggctccgcct tcaatcccac ccgctaaagt acttggagcg    420
   gtctctccct ccctcatcag cccaccaaac caaacctagc ctccaagagt gggaagaaat    480
   taaagcaaga taggctatta agtgcagagg gagagaaaat gcctccaaca tgtgaggaag    540
   taatgagaga aatcatagaa tttcttccgc ttcctcgctc actgactcgc tgcgctcggt    600
   cgttcggctg cggcgagcgg tatcagctca ctcaaaggcg gtaatacggt tatccacaga    660
   atcaggggat aacgcaggaa agaacatgtg agcaaaaggc cagcaaaagg ccaggaaccg    720
   taaaaaggcc gcgttgctgg cgtttttcca taggctccgc ccccctgacg agcatcacaa    780
   aaatcgacgc tcaagtcaga ggtggcgaaa cccgacagga ctataaagat accaggcgtt    840
   tccccctgga agctccctcg tgcgctctcc tgttccgacc ctgccgctta ccggatacct    900
   gtccgccttt ctcccttcgg gaagcgtggc gctttctcaa tgctcacgct gtaggtatct    960
   cagttcggtg taggtcgttc gctccaagct gggctgtgtg cacgaacccc ccgttcagcc   1020
   cgaccgctgc gccttatccg gtaactatcg tcttgagtcc aacccggtaa gacacgactt   1080
   atcgccactg gcagcagcca ctggtaacag gattagcaga gcgaggtatg taggcggtgc   1140
   tacagagttc ttgaagtggt ggcctaacta cggctacact agaaggacag tatttggtat 1200
   ctgcgctctg ctgaagccag ttaccttcgg aaaaagagtt ggtagctctt gatccggcaa 1260
   acaaaccacc gctggtagcg gtggtttttt tgtttgcaag cagcagatta cgcgcagaaa 1320
   aaaaggatct caagaagatc ctttgatctt ttctacgggg tctgacgctc agtggaacga 1380
   aaactcacgt taagggattt tggtcatgag attatcaaaa aggatcttca cctagatcct 1440
   tttaaattaa aaatgaagtt ttaaatcaat ctaaagtata tatgagtaaa cttggtctga 1500
   cagttaccaa tgcttaatca gtgaggcacc tatctcagcg atctgtctat ttcgttcatc 1560
   catagttgcc tgactccggg gggggggggc gctgaggtct gcctcgtgaa gaaggtgttg 1620
   ctgactcata ccaggcctga atcgccccat catccagcca gaaagtgagg gagccacggt 1680
   tgatgagagc tttgttgtag gtggaccagt tggtgatttt gaacttttgc tttgccacgg 1740
   aacggtctgc gttgtcggga agatgcgtga tctgatcctt caactcagca aaagttcgat 1800
   ttattcaaca aagccgccgt cccgtcaagt cagcgtaatg ctctgccagt gttacaacca 1860
   attaaccaat tctgattaga aaaactcatc gagcatcaaa tgaaactgca atttattcat 1920
   atcaggatta tcaataccat atttttgaaa aagccgtttc tgtaatgaag gagaaaactc 1980
   accgaggcag ttccatagga tggcaagatc ctggtatcgg tctgcgattc cgactcgtcc 2040
   aacatcaata caacctatta atttcccctc gtcaaaaata aggttatcaa gtgagaaatc 2100
   accatgagtg acgactgaat ccggtgagaa tggcaaaagc ttatgcattt ctttccagac 2160
   ttgttcaaca ggccagccat tacgctcgtc atcaaaatca ctcgcatcaa ccaaaccgtt 2220
   attcattcgt gattgcgcct gagcgagacg aaatacgcga tcgctgttaa aaggacaatt 2280
   acaaacagga atcgaatgca accggcgcag gaacactgcc agcgcatcaa caatattttc 2340
   acctgaatca ggatattctt ctaatacctg gaatgctgtt ttcccgggga tcgcagtggt 2400
   gagtaaccat gcatcatcag gagtacggat aaaatgcttg atggtcggaa gaggcataaa 2460
   ttccgtcagc cagtttagtc tgaccatctc atctgtaaca tcattggcaa cgctaccttt 2520
   gccatgtttc agaaacaact ctggcgcatc gggcttccca tacaatcgat agattgtcgc 2580
   acctgattgc ccgacattat cgcgagccca tttataccca tataaatcag catccatgtt 2640
   ggaatttaat cgcggcctcg agcaagacgt ttcccgttga atatggctca taacacccct 2700
   tgtattactg tttatgtaag cagacagttt tattgttcat gatgatatat ttttatcttg 2760
   tgcaatgtaa catcagagat tttgagacac aacgtggctt tccccccccc cccattattg 2820
   aagcatttat cagggttatt gtctcatgag cggatacata tttgaatgta tttagaaaaa 2880
   taaacaaata ggggttccgc gcacatttcc ccgaaaagtg ccacctgacg tctaagaaac 2940
   cattattatc atgacattaa cctataaaaa taggcgtatc acgaggccct ttcgtctcgc 3000
   gcgtttcggt gatgacggtg aaaacctctg acacatgcag ctcccggaga cggtcacagc 3060
   ttgtctgtaa gcggatgccg ggagcagaca agcccgtcag ggcgcgtcag cgggtgttgg 3120
   cgggtgtcgg ggctggctta actatgcggc atcagagcag attgtactga gagtgcacca 3180
   tatgcggtgt gaaataccgc acagatgcgt aaggagaaaa taccgcatca gattggctat 3240
   tggccattgc atacgttgta tccatatcat aatatgtaca tttatattgg ctcatgtcca 3300
   acattaccgc catgttgaca ttgattattg actagttatt aatagtaatc aattacgggg 3360
   tcattagttc atagcccata tatggagttc cgcgttacat aacttacggt aaatggcccg 3420
   cctggctgac cgcccaacga cccccgccca ttgacgtcaa taatgacgta tgttcccata 3480
   gtaacgccaa tagggacttt ccattgacgt caatgggtgg agtatttacg gtaaactgcc 3540
   cacttggcag tacatcaagt gtatcatatg ccaagtacgc cccctattga cgtcaatgac 3600
   ggtaaatggc ccgcctggca ttatgcccag tacatgacct tatgggactt tcctacttgg 3660
   cagtacatct acgtattagt catcgctatt accatggtga tgcggttttg gcagtacatc 3720
   aatgggcgtg gatagcggtt tgactcacgg ggatttccaa gtctccaccc cattgacgtc 3780
   aatgggagtt tgttttggca ccaaaatcaa cgggactttc caaaatgtcg taacaactcc 3840
   gccccattga cgcaaatggg cggtaggcgt gtacggtggg aggtctatat aagcagagct 3900
   cgtttagtga accgtcagat cgcctggaga cgccatccac gctgttttga cctccataga 3960
   agacaccggg accgatccag cctccgcggc cgggaacggt gcattggaac gcggattccc 4020
   cgtgccaaga gtgacgtaag taccgcctat agagtctata ggcccacccc cttggcttct 4080
   tatgcatgct atactgtttt tggcttgggg tctatacacc cccgcttcct catgttatag 4140
   gtgatggtat agcttagcct ataggtgtgg gttattgacc attattgacc actcccctat 4200
   tggtgacgat actttccatt actaatccat aacatggctc tttgccacaa ctctctttat 4260
   tggctatatg ccaatacact gtccttcaga gactgacacg gactctgtat ttttacagga 4320
   tggggtctca tttattattt acaaattcac atatacaaca ccaccgtccc cagtgcccgc 4380
   agtttttatt aaacataacg tgggatctcc acgcgaatct cgggtacgtg ttccggacat 4440
   gggctcttct ccggtagcgg cggagcttct acatccgagc cctgctccca tgcctccagc 4500
   gactcatggt cgctcggcag ctccttgctc ctaacagtgg aggccagact taggcacagc 4560
   acgatgccca ccaccaccag tgtgccgcac aaggccgtgg cggtagggta tgtgtctgaa 4620
   aatgagctcg gggagcgggc ttgcaccgct gacgcatttg gaagacttaa ggcagcggca 4680
   gaagaagatg caggcagctg agttgttgtg ttctgataag agtcagaggt aactcccgtt 4740
   gcggtgctgt taacggtgga gggcagtgta gtctgagcag tactcgttgc tgccgcgcgc 4800
   gccaccagac ataatagctg acagactaac agactgttcc tttccatggg tcttttctca 4860
   cgtcaccgtc gtcgaccaga gctgagatcc tacaggagtc cagggctgga gagaaaacct 4920
   ctgcgaggaa agggaaggag caagccgtga atttaaggga cgctgtgaag caatcatgga    4980
   tgcaatgaag agagggctct gctgtgtgct gctgctgtgt ggagcagtct tcgtttcgcc    5040
   cagcggtacc ggatccaccc ttgatggtga tgaatatttc attggaaaat acaaagaaaa    5100
   agatgagaca ctgttttttg caagctacgg cctaaagagg gatccttgcc aaattgtctt    5160
   aggctacaaa tgctcaaaca atcaaaccca ctttgtgctt aattttaaaa ccaataagaa    5220
   atcctgcata tcagcaatta agctgacttc ttacccaaaa atcaatcaaa actcggattt    5280
   aactaaaaat ctctactgcc aaactggagg aataggaaca gataactgca aacttgtctt    5340
   caagaaacgt aaaagacaaa tagcagctaa tattgaaatc tacggcattc cagcgaagaa    5400
   atgttccttc aaggatcgtt acattggagc tgatccactc cacgtcgatt cctatgggct    5460
   tccgtatcag tttgatcagg aacatggatg gaatgtggaa cgatataaca ttttcaaaga    5520
   cacaagattt tccacagaag ttttctacca caaaaatggt ttatttaaca cccaaataac    5580
   ttatttggct gaagaagatt ccttctctga agctcgagag attactgcga aggatattaa    5640
   gaagaagttt tcaattattt tgcccaatga agagtataag aggattagtt tcttggacgt    5700
   ttattggttc caggagacta tgcgaaaaaa gcctaaatat ccctacattc actacaatgg    5760
   agaatgcagc aatgagaata aaacttgtga acttgtcttt gacaccgatg aactaatgac    5820
   ctacgccctt gttaaagtct ttactaatcc tgagagtgat ggatctaggc tcaaagaaga    5880
   ggatttggga agaggataa                                                 5899
<210> 11
   <211> 301
   <212> PRT
   <213> Lutzomyia longipalpis
<400> 11
   Met Asn Ser Ile Asn Phe Leu Ser Ile Val Gly Leu Ile Ser Phe Gly
   1               5                   10                  15
   Phe Ile Val Ala Val Lys Cys Asp Gly Asp Glu Tyr Phe Ile Gly Lys
               20                  25                  30
   Tyr Lys Glu Lys Asp Glu Thr Leu Phe Phe Ala Ser Tyr Gly Leu Lys
           35                  40                  45
   Arg Asp Pro Cys Gln Ile Val Leu Gly Tyr Lys Cys Ser Asn Asn Gln
       50                  55                  60
   Thr His Phe Val Leu Asn Phe Lys Thr Asn Lys Lys Ser Cys Ile Ser
   65                  70                  75                  80
   Ala Ile Lys Leu Thr Ser Tyr Pro Lys Ile Asn Gln Asn Ser Asp Leu
                   85                  90                  95
   Thr Arg Asn Leu Tyr Cys Gln Thr Gly Gly Ile Gly Thr Asp Asn Cys
               100                 105                 110
   Lys Leu Val Phe Lys Lys Arg Lys Arg Gln Ile Ala Ala Asn Ile Glu
           115                 120                 125
   Ile Tyr Gly Ile Pro Ala Lys Lys Cys Ser Phe Lys Asp Arg Tyr Ile
       130                 135                 140
   Gly Ala Asp Pro Leu His Val Asp Ser Tyr Gly Leu Ser Tyr Gln Phe
   145                 150                 155                 160
   Asp Gln Glu His Gly Trp Asn Leu Glu Arg Asn Asn Ile Phe Lys Asp
                   165                 170                 175
   Thr Arg Phe Ser Thr Glu Val Phe Tyr His Lys Asn Gly Leu Phe Asn
               180                 185                 190
   Thr Gln Ile Thr Tyr Leu Ala Glu Glu Asp Ser Phe Ser Glu Ala Arg
           195                 200                 205
   Glu Ile Thr Ala Lys Asp Ile Lys Lys Lys Phe Ser Ile Ile Leu Pro
       210                 215                 220
   Asn Glu Glu Tyr Lys Arg Ile Ser Phe Leu Asp Val Tyr Trp Phe Gln
   225                 230                 235                 240
   Glu Thr Met Arg Lys Lys Pro Lys Tyr Pro Tyr Ile His Tyr Asn Gly
                   245                 250                 255
   Glu Cys Ser Asn Glu Asn Lys Thr Cys Glu Leu Val Phe Asp Thr Asp
               260                 265                 270
   Glu Leu Met Thr Tyr Ala Leu Val Lys Val Phe Thr Asn Pro Glu Ser
           275                 280                 285
   Asp Gly Ser Arg Leu Lys Glu Glu Asp Leu Gly Arg Gly
       290                 295                 300
<210> 12
   <211> 906
   <212> DNA
   <213> Lutzomyia longipalpis
<400> 12
   atgaattcga ttaatttcct atcaatagtt ggtttaatca gttttggatt cattgttgca      60
   gtaaagtgtg atggtgatga atatttcatt ggaaaataca aagaaaaaga tgagacactg     120
   ttttttgcaa gctacggcct aaagagggat ccttgccaga ttgtcttagg ctacaaatgc     180
   tcaaacaatc aaacccactt tgtgcttaat tttaaaacca ataagaaatc ctgcatatca     240
   gcaattaagc tgacttctta cccaaaaatc aatcaaaact cggatttaac tagaaatctc     300
   tactgccaaa ctggaggaat aggaacagat aactgcaaac ttgtcttcaa gaaacgtaaa     360
   agacaaatag cagctaatat tgaaatctac ggcattccag cgaagaaatg ttccttcaag     420
   gatcgttaca ttggagctga tccactccac gtcgattcct atgggctttc gtatcagttt     480
   gatcaggaac atggatggaa tttggaacga aataacattt tcaaagacac aagattttcc     540
   acagaagttt tctaccacaa aaatggttta tttaacaccc aaataactta tttggctgaa     600
   gaagattcct tctctgaagc tcgagagatt actgcgaagg atattaagaa gaagttttca     660
   attattttgc ccaatgaaga gtataagagg attagtttct tggacgttta ttggttccag     720
   gagactatgc gaaaaaagcc taaatatccc tacattcact acaatggaga atgcagcaat     780
   gagaataaaa cttgtgaact tgtctttgac accgatgaac taatgaccta cgcccttgtt     840
   aaagtcttta ctaatcctga gagtgatgga tctaggctca aagaagagga tttgggaaga     900
   ggataa                                                                906
<210> 13
   <211> 278
   <212> PRT
   <213> Lutzomyia longipalpis
<400> 13
   Asp Gly Asp Glu Tyr Phe Ile Gly Lys Tyr Lys Glu Lys Asp Glu Thr
   1               5                   10                  15
   Leu Phe Phe Ala Ser Tyr Gly Leu Lys Arg Asp Pro Cys Gln Ile Val
               20                  25                  30
   Leu Gly Tyr Lys Cys Ser Asn Asn Gln Thr His Phe Val Leu Asn Phe
           35                  40                  45
   Lys Thr Asn Lys Lys Ser Cys Ile Ser Ala Ile Lys Leu Thr Ser Tyr
       50                  55                  60
   Pro Lys Ile Asn Gln Asn Ser Asp Leu Thr Arg Asn Leu Tyr Cys Gln
   65                  70                  75                  80
   Thr Gly Gly Ile Gly Thr Asp Asn Cys Lys Leu Val Phe Lys Lys Arg
                   85                  90                  95
   Lys Arg Gln Ile Ala Ala Asn Ile Glu Ile Tyr Gly Ile Pro Ala Lys
               100                 105                 110
   Lys Cys Ser Phe Lys Asp Arg Tyr Ile Gly Ala Asp Pro Leu His Val
           115                 120                 125
   Asp Ser Tyr Gly Leu Ser Tyr Gln Phe Asp Gln Glu His Gly Trp Asn
       130                 135                 140
   Leu Glu Arg Asn Asn Ile Phe Lys Asp Thr Arg Phe Ser Thr Glu Val
   145                 150                 155                 160
   Phe Tyr His Lys Asn Gly Leu Phe Asn Thr Gln Ile Thr Tyr Leu Ala
                   165                 170                 175
   Glu Glu Asp Ser Phe Ser Glu Ala Arg Glu Ile Thr Ala Lys Asp Ile
               180                 185                 190
   Lys Lys Lys Phe Ser Ile Ile Leu Pro Asn Glu Glu Tyr Lys Arg Ile
           195                 200                 205
   Ser Phe Leu Asp Val Tyr Trp Phe Gln Glu Thr Met Arg Lys Lys Pro
       210                 215                 220
   Lys Tyr Pro Tyr Ile His Tyr Asn Gly Glu Cys Ser Asn Glu Asn Lys
   225                 230                 235                 240
   Thr Cys Glu Leu Val Phe Asp Thr Asp Glu Leu Met Thr Tyr Ala Leu
                   245                 250                 255
   Val Lys Val Phe Thr Asn Pro Glu Ser Asp Gly Ser Arg Leu Lys Glu
               260                 265                 270
   Glu Asp Leu Gly Arg Gly
           275
<210> 14
   <211> 837
   <212> DNA
   <213> Lutzomyia longipalpis
<400> 14
   gatggtgatg aatatttcat tggaaaatac aaagaaaaag atgagacact gttttttgca     60
   agctacggcc taaagaggga tccttgccag attgtcttag gctacaaatg ctcaaacaat    120
   caaacccact ttgtgcttaa ttttaaaacc aataagaaat cctgcatatc agcaattaag    180
   ctgacttctt acccaaaaat caatcaaaac tcggatttaa ctagaaatct ctactgccaa    240
   actggaggaa taggaacaga taactgcaaa cttgtcttca agaaacgtaa aagacaaata    300
   gcagctaata ttgaaatcta cggcattcca gcgaagaaat gttccttcaa ggatcgttac    360
   attggagctg atccactcca cgtcgattcc tatgggcttt cgtatcagtt tgatcaggaa    420
   catggatgga atttggaacg aaataacatt ttcaaagaca caagattttc cacagaagtt    480
   ttctaccaca aaaatggttt atttaacacc caaataactt atttggctga agaagattcc    540
   ttctctgaag ctcgagagat tactgcgaag gatattaaga agaagttttc aattattttg    600
   cccaatgaag agtataagag gattagtttc ttggacgttt attggttcca ggagactatg    660
   cgaaaaaagc ctaaatatcc ctacattcac tacaatggag aatgcagcaa tgagaataaa    720
   acttgtgaac ttgtctttga caccgatgaa ctaatgacct acgcccttgt taaagtcttt    780
   actaatcctg agagtgatgg atctaggctc aaagaagagg atttgggaag aggataa       837
<210> 15
   <211> 412
   <212> PRT
   <213> Lutzomyia longipalpis
<400> 15
   Met Arg Phe Phe Phe Val Phe Leu Ala Ile Val Leu Phe Gln Gly Ile
   1               5                   10                  15
   His Gly Ala Tyr Val Glu Ile Gly Tyr Ser Leu Arg Asn Ile Thr Phe
               20                  25                  30
   Asp Gly Leu Asp Thr Asp Asp Tyr Asn Pro Lys Phe Asn Ile Pro Thr
           35                  40                  45
   Gly Leu Ala Val Asp Pro Glu Gly Tyr Arg Leu Phe Ile Ala Ile Pro
       50                  55                  60
   Arg Arg Lys Pro Lys Val Pro Tyr Thr Val Ala Glu Leu Asn Met Val
   65                  70                  75                  80
   Met Asn Pro Gly Phe Pro Val Glu Arg Ala Pro Ser Phe Glu Lys Phe
                   85                  90                  95
   Lys Lys Phe Asn Gly Glu Gly Lys Lys Asp Leu Val Asn Val Tyr Gln
               100                 105                 110
   Pro Val Ile Asp Asp Cys Arg Arg Leu Trp Val Leu Asp Ile Gly Lys
           115                 120                 125
   Val Glu Tyr Thr Gly Gly Asp Ala Asp Gln Tyr Pro Lys Gly Lys Pro
       130                 135                 140
   Thr Leu Ile Ala Tyr Asp Leu Lys Lys Asp His Thr Pro Glu Ile His
   145                 150                 155                 160
   Arg Phe Glu Ile Pro Asp Asp Leu Tyr Ser Ser Gln Val Glu Phe Gly
                   165                 170                 175
   Gly Phe Ala Val Asp Val Val Asn Thr Lys Gly Asp Cys Thr Glu Ser
               180                 185                 190
   Phe Val Tyr Leu Thr Asn Phe Lys Asp Asn Ser Leu Ile Val Tyr Asp
           195                 200                 205
   Glu Thr Gln Lys Lys Ala Trp Lys Phe Thr Asp Lys Thr Phe Glu Ala
       210                 215                 220
   Asp Lys Glu Ser Thr Phe Ser Tyr Ser Gly Glu Glu Gln Met Lys Tyr
   225                 230                 235                 240
   Lys Val Gly Leu Phe Gly Ile Ala Leu Gly Asp Arg Asp Glu Met Gly
                   245                 250                 255
   His Arg Pro Ala Tyr Tyr Ile Ala Gly Ser Ser Thr Lys Val Tyr Ser
               260                 265                 270
   Val Asn Thr Lys Glu Leu Lys Thr Glu Asn Gly Gln Leu Asn Pro Gln
           275                 280                 285
   Leu His Gly Asp Arg Gly Lys Tyr Thr Asp Ala Ile Ala Leu Ala His
       290                 295                 300
   Asp Pro Glu His Lys Val Leu Tyr Phe Ala Glu Ser Asp Ser Arg Gln
   305                 310                 315                 320
   Val Ser Cys Trp Asn Val Asp Met Glu Leu Lys Pro Asp Asn Thr Asp
                   325                 330                 335
   Val Ile Phe Ser Ser Ala Arg Phe Thr Phe Gly Thr Asp Ile Leu Val
               340                 345                 350
   Asp Ser Lys Gly Met Leu Trp Ile Met Ala Asn Gly His Pro Pro Val
           355                 360                 365
   Glu Asp Gln Glu Lys Ile Trp Lys Met Arg Phe Val Asn Arg Lys Ile
       370                 375                 380
   Ser Ile Met Lys Val Asp Thr Glu Arg Val Phe Lys Tyr Ser Arg Cys
   385 390 395 400
   Asn Pro Asn Tyr Lys Pro Pro Lys Glu Ile Glu Val
   405 410
<210> 16
   <211> 1239
   <212> DNA
   <213> Lutzomyia longipalpis
<400> 16
   atgaggttct tctttgtttt ccttgccatc gtcctttttc aagggatcca cggagcttat      60
   gtggaaatag gatattctct gagaaatatt acattcgatg gattggatac agatgactac     120
   aatccaaagt tcaacattcc aacgggtttg gcagttgatc ccgaaggata taggctcttc     180
   atagccatcc caaggagaaa gccaaaggtt ccctacactg tggctgaact gaatatggtc     240
   atgaatcccg gatttcccgt cgagagagct ccgagctttg agaaattcaa aaaattcaat     300
   ggcgagggca aaaaggatct tgttaatgtg tatcagccag tcattgatga ttgtcgtcgt     360
   ctttgggtgc ttgacattgg gaaggtggaa tacaccggtg gtgatgctga tcaatatccc     420
   aaaggaaagc ctaccctaat tgcctacgac ctcaagaagg atcatactcc ggaaattcat     480
   cgatttgaaa ttccagacga tctctatagc tcacaagttg aatttggtgg atttgccgtt     540
   gatgttgtta acacgaaagg agactgtacg gagtcatttg tctacctgac caatttcaag     600
   gataactctc taattgtcta cgatgagaca caaaagaaag cttggaaatt tacagataaa     660
   acatttgaag ctgataagga atccacgttc tcctactcgg gagaggaaca aatgaagtac     720
   aaagttggtc tttttgggat agctctgggt gatagggatg aaatggggca tcgtcctgcc     780
   tactatatcg ctgggagtag caccaaagtc tacagtgtta acactaaaga actcaaaaca     840
   gagaatggtc agttaaatcc tcagcttcac ggtgatcgtg gaaagtacac ggatgcaatt     900
   gccctagccc acgatcctga gcataaagtc ctctactttg ctgaatccga cagcaggcag     960
   gtgtcctgtt ggaatgtaga tatggagcta aaaccagaca atacggatgt gatcttctct    1020
   agtgcccgtt ttacttttgg aacggatatt ttggttgata gcaagggaat gctgtggata    1080
   atggctaatg gacatccacc agtagaggat caagagaaga tttggaagat gagattcgta    1140
   aaccggaaga tcagtattat gaaagtggat acggaacgtg tattcaaata ttcacgctgc    1200
   aatccaaatt ataagccccc gaaagaaatt gaagtttga                            1239
<210> 17
   <211> 394
   <212> PRT
   <213> Lutzomyia longipalpis
<400> 17
   Ala Tyr Val Glu Ile Gly Tyr Ser Leu Arg Asn Ile Thr Phe Asp Gly
   1               5                   10                  15
   Leu Asp Thr Asp Asp Tyr Asn Pro Lys Phe Asn Ile Pro Thr Gly Leu
               20                  25                  30
   Ala Val Asp Pro Glu Gly Tyr Arg Leu Phe Ile Ala Ile Pro Arg Arg
           35                  40                  45
   Lys Pro Lys Val Pro Tyr Thr Val Ala Glu Leu Asn Met Val Met Asn
       50                  55                  60
   Pro Gly Phe Pro Val Glu Arg Ala Pro Ser Phe Glu Lys Phe Lys Lys
   65                  70                  75                  80
   Phe Asn Gly Glu Gly Lys Lys Asp Leu Val Asn Val Tyr Gln Pro Val
                   85                  90                  95
   Ile Asp Asp Cys Arg Arg Leu Trp Val Leu Asp Ile Gly Lys Val Glu
               100                 105                 110
   Tyr Thr Gly Gly Asp Ala Asp Gln Tyr Pro Lys Gly Lys Pro Thr Leu
           115                 120                 125
   Ile Ala Tyr Asp Leu Lys Lys Asp His Thr Pro Glu Ile His Arg Phe
       130                 135                 140
   Glu Ile Pro Asp Asp Leu Tyr Ser Ser Gln Val Glu Phe Gly Gly Phe
   145                 150                 155                 160
   Ala Val Asp Val Val Asn Thr Lys Gly Asp Cys Thr Glu Ser Phe Val
                   165                 170                 175
   Tyr Leu Thr Asn Phe Lys Asp Asn Ser Leu Ile Val Tyr Asp Glu Thr
               180                 185                 190
   Gln Lys Lys Ala Trp Lys Phe Thr Asp Lys Thr Phe Glu Ala Asp Lys
           195                 200                 205
   Glu Ser Thr Phe Ser Tyr Ser Gly Glu Glu Gln Met Lys Tyr Lys Val
       210                 215                 220
   Gly Leu Phe Gly Ile Ala Leu Gly Asp Arg Asp Glu Met Gly His Arg
   225                 230                 235                 240
   Pro Ala Tyr Tyr Ile Ala Gly Ser Ser Thr Lys Val Tyr Ser Val Asn
                   245                 250                 255
   Thr Lys Glu Leu Lys Thr Glu Asn Gly Gln Leu Asn Pro Gln Leu His
               260                 265                 270
   Gly Asp Arg Gly Lys Tyr Thr Asp Ala Ile Ala Leu Ala His Asp Pro
           275                 280                 285
   Glu His Lys Val Leu Tyr Phe Ala Glu Ser Asp Ser Arg Gln Val Ser
       290                 295                 300
   Cys Trp Asn Val Asp Met Glu Leu Lys Pro Asp Asn Thr Asp Val Ile
   305                 310                 315                 320
   Phe Ser Ser Ala Arg Phe Thr Phe Gly Thr Asp Ile Leu Val Asp Ser
                   325                 330                 335
   Lys Gly Met Leu Trp Ile Met Ala Asn Gly His Pro Pro Val Glu Asp
               340                 345                 350
   Gln Glu Lys Ile Trp Lys Met Arg Phe Val Asn Arg Lys Ile Ser Ile
           355                 360                 365
   Met Lys Val Asp Thr Glu Arg Val Phe Lys Tyr Ser Arg Cys Asn Pro
       370                 375                 380
   Asn Tyr Lys Pro Pro Lys Glu Ile Glu Val
   385                 390
<210> 18
   <211> 1185
   <212> DNA
   <213> Lutzomyia longipalpis
<400> 18
   gcttatgtgg aaataggata ttctctgaga aatattacat tcgatggatt ggatacagat     60
   gactacaatc caaagttcaa cattccaacg ggtttggcag ttgatcccga aggatatagg    120
   ctcttcatag ccatcccaag gagaaagcca aaggttccct acactgtggc tgaactgaat    180
   atggtcatga atcccggatt tcccgtcgag agagctccga gctttgagaa attcaaaaaa    240
   ttcaatggcg agggcaaaaa ggatcttgtt aatgtgtatc agccagtcat tgatgattgt    300
   cgtcgtcttt gggtgcttga cattgggaag gtggaataca ccggtggtga tgctgatcaa    360
   tatcccaaag gaaagcctac cctaattgcc tacgacctca agaaggatca tactccggaa    420
   attcatcgat ttgaaattcc agacgatctc tatagctcac aagttgaatt tggtggattt    480
   gccgttgatg ttgttaacac gaaaggagac tgtacggagt catttgtcta cctgaccaat    540
   ttcaaggata actctctaat tgtctacgat gagacacaaa agaaagcttg gaaatttaca    600
   gataaaacat ttgaagctga taaggaatcc acgttctcct actcgggaga ggaacaaatg    660
   aagtacaaag ttggtctttt tgggatagct ctgggtgata gggatgaaat ggggcatcgt    720
   cctgcctact atatcgctgg gagtagcacc aaagtctaca gtgttaacac taaagaactc    780
   aaaacagaga atggtcagtt aaatcctcag cttcacggtg atcgtggaaa gtacacggat    840
   gcaattgccc tagcccacga tcctgagcat aaagtcctct actttgctga atccgacagc    900
   aggcaggtgt cctgttggaa tgtagatatg gagctaaaac cagacaatac ggatgtgatc    960
   ttctctagtg cccgttttac ttttggaacg gatattttgg ttgatagcaa gggaatgctg   1020
   tggataatgg ctaatggaca tccaccagta gaggatcaag agaagatttg gaagatgaga   1080
   ttcgtaaacc ggaagatcag tattatgaaa gtggatacgg aacgtgtatt caaatattca   1140
   cgctgcaatc caaattataa gcccccgaaa gaaattgaag tttga                   1185
<210> 19
   <211> 6247
   <212> DNA
   <213> Artificial sequence
<220>
   <223> nucleic acid sequence of one strand of the plasmid pNBO002
<400> 19
   ttggctattg gccattgcat acgttgtatc catatcataa tatgtacatt tatattggct 60
   catgtccaac attaccgcca tgttgacatt gattattgac tagttattaa tagtaatcaa 120
   ttacggggtc attagttcat agcccatata tggagttccg cgttacataa cttacggtaa 180
   atggcccgcc tggctgaccg cccaacgacc cccgcccatt gacgtcaata atgacgtatg 240
   ttcccatagt aacgccaata gggactttcc attgacgtca atgggtggag tatttacggt 300
   aaactgccca cttggcagta catcaagtgt atcatatgcc aagtacgccc cctattgacg 360
   tcaatgacgg taaatggccc gcctggcatt atgcccagta catgacctta tgggactttc 420
   ctacttggca gtacatctac gtattagtca tcgctattac catggtgatg cggttttggc 480
   agtacatcaa tgggcgtgga tagcggtttg actcacgggg atttccaagt ctccacccca 540
   ttgacgtcaa tgggagtttg ttttggcacc aaaatcaacg ggactttcca aaatgtcgta 600
   acaactccgc cccattgacg caaatgggcg gtaggcgtgt acggtgggag gtctatataa 660
   gcagagctcg tttagtgaac cgtcagatcg cctggagacg ccatccacgc tgttttgacc 720
   tccatagaag acaccgggac cgatccagcc tccgcggccg ggaacggtgc attggaacgc 780
   ggattccccg tgccaagagt gacgtaagta ccgcctatag agtctatagg cccaccccct 840
   tggcttctta tgcatgctat actgtttttg gcttggggtc tatacacccc cgcttcctca 900
   tgttataggt gatggtatag cttagcctat aggtgtgggt tattgaccat tattgaccac 960
   tcccctattg gtgacgatac tttccattac taatccataa catggctctt tgccacaact 1020
   ctctttattg gctatatgcc aatacactgt ccttcagaga ctgacacgga ctctgtattt 1080
   ttacaggatg gggtctcatt tattatttac aaattcacat atacaacacc accgtcccca 1140
   gtgcccgcag tttttattaa acataacgtg ggatctccac gcgaatctcg ggtacgtgtt 1200
   ccggacatgg gctcttctcc ggtagcggcg gagcttctac atccgagccc tgctcccatg 1260
   cctccagcga ctcatggtcg ctcggcagct ccttgctcct aacagtggag gccagactta 1320
   ggcacagcac gatgcccacc accaccagtg tgccgcacaa ggccgtggcg gtagggtatg 1380
   tgtctgaaaa tgagctcggg gagcgggctt gcaccgctga cgcatttgga agacttaagg 1440
   cagcggcaga agaagatgca ggcagctgag ttgttgtgtt ctgataagag tcagaggtaa 1500
   ctcccgttgc ggtgctgtta acggtggagg gcagtgtagt ctgagcagta ctcgttgctg 1560
   ccgcgcgcgc caccagacat aatagctgac agactaacag actgttcctt tccatgggtc 1620
   ttttctcacg tcaccgtcgt cgaccagagc tgagatccta caggagtcca gggctggaga 1680
   gaaaacctct gcgaggaaag ggaaggagca agccgtgaat ttaagggacg ctgtgaagca 1740
   atcatggatg caatgaagag agggctctgc tgtgtgctgc tgctgtgtgg agcagtcttc 1800
   gtttcgccca gcggtaccgg atccaccctt gcttatgtgg aaataggata ttctctgaga 1860
   aatattacat tcgatggatt ggatacagat gactacaatc caaagttcaa cattccaacg 1920
   ggtttggcag ttgatcccga aggatatagg ctcttcatag ccatcccaag gagaaagcca 1980
   aaggttccct acactgtggc tgaactgaat atggtcatga atcccggatt tcccgtcgag 2040
   agagctccga gctttgagaa attcaaaaaa ttcaatggcg agggcaaaaa ggatcttgtt 2100
   aatgtgtatc agccagtcat tgatgattgt cgtcgtcttt gggtgcttga cattgggaag 2160
   gtggaataca ccggtggtga tgctgatcaa tatcccaaag gaaagcctac cctaattgcc 2220
   tacgacctca agaaggatca tactccggaa attcatcgat ttgaaattcc agacgatctc 2280
   tatagctcac aagttgaatt tggtggattt gccgttgatg ttgttaacac gaaaggagac 2340
   tgtacggagt catttgtcta cctgaccaat ttcaaggata actctctaat tgtctacgat 2400
   gagacacaaa agaaagcttg gaaatttaca gataaaacat ttgaagctga taaggaatcc 2460
   acgttctcct actcgggaga ggaacaaatg aagtacaaag ttggtctttt tgggatagct 2520
   ctgggtgata gggatgaaat ggggcatcgt cctgcctact atatcgctgg gagtagcacc 2580
   aaagtctaca gtgttaacac taaagaactc aaaacagaga atggtcagtt aaatcctcag 2640
   cttcacggtg atcgtggaaa gtacacggat gcaattgccc tagcccacga tcctgagcat 2700
   aaagtcctct actttgctga atccgacagc aggcaggtgt cctgttggaa tgtagatatg 2760
   gagctaaaac cagacaatac ggatgtgatc ttctctagtg cccgttttac ttttggaacg 2820
   gatattttgg ttgatagcaa gggaatgctg tggataatgg ctaatggaca tccaccagta 2880
   gaggatcaag agaagatttg gaagatgaga ttcgtaaacc ggaagatcag tattatgaaa 2940
   gtggatacgg aacgtgtatt caaatattca cgctgcaatc caaattataa gcccccgaaa 3000
   gaaattgaag tttgaaaggg atccagatct gctgtgcctt ctagttgcca gccatctgtt 3060
   gtttgcccct cccccgtgcc ttccttgacc ctggaaggtg ccactcccac tgtcctttcc 3120
   taataaaatg aggaaattgc atcgcattgt ctgagtaggt gtcattctat tctggggggt 3180
   ggggtggggc agcacagcaa gggggaggat tgggaagaca atagcaggca tgctggggat 3240
   gcggtgggct ctatgggtac ccaggtgctg aagaattgac ccggttcctc ctgggccaga 3300
   aagaagcagg cacatcccct tctctgtgac acaccctgtc cacgcccctg gttcttagtt 3360
   ccagccccac tcataggaca ctcatagctc aggagggctc cgccttcaat cccacccgct 3420
   aaagtacttg gagcggtctc tccctccctc atcagcccac caaaccaaac ctagcctcca 3480
   agagtgggaa gaaattaaag caagataggc tattaagtgc agagggagag aaaatgcctc 3540
   caacatgtga ggaagtaatg agagaaatca tagaatttct tccgcttcct cgctcactga 3600
   ctcgctgcgc tcggtcgttc ggctgcggcg agcggtatca gctcactcaa aggcggtaat 3660
   acggttatcc acagaatcag gggataacgc aggaaagaac atgtgagcaa aaggccagca 3720
   aaaggccagg aaccgtaaaa aggccgcgtt gctggcgttt ttccataggc tccgcccccc 3780
   tgacgagcat cacaaaaatc gacgctcaag tcagaggtgg cgaaacccga caggactata 3840
   aagataccag gcgtttcccc ctggaagctc cctcgtgcgc tctcctgttc cgaccctgcc 3900
   gcttaccgga tacctgtccg cctttctccc ttcgggaagc gtggcgcttt ctcaatgctc 3960
   acgctgtagg tatctcagtt cggtgtaggt cgttcgctcc aagctgggct gtgtgcacga 4020
   accccccgtt cagcccgacc gctgcgcctt atccggtaac tatcgtcttg agtccaaccc 4080
   ggtaagacac gacttatcgc cactggcagc agccactggt aacaggatta gcagagcgag 4140
   gtatgtaggc ggtgctacag agttcttgaa gtggtggcct aactacggct acactagaag 4200
   gacagtattt ggtatctgcg ctctgctgaa gccagttacc ttcggaaaaa gagttggtag 4260
   ctcttgatcc ggcaaacaaa ccaccgctgg tagcggtggt ttttttgttt gcaagcagca 4320
   gattacgcgc agaaaaaaag gatctcaaga agatcctttg atcttttcta cggggtctga 4380
   cgctcagtgg aacgaaaact cacgttaagg gattttggtc atgagattat caaaaaggat 4440
   cttcacctag atccttttaa attaaaaatg aagttttaaa tcaatctaaa gtatatatga 4500
   gtaaacttgg tctgacagtt accaatgctt aatcagtgag gcacctatct cagcgatctg 4560
   tctatttcgt tcatccatag ttgcctgact ccgggggggg ggggcgctga ggtctgcctc 4620
   gtgaagaagg tgttgctgac tcataccagg cctgaatcgc cccatcatcc agccagaaag 4680
   tgagggagcc acggttgatg agagctttgt tgtaggtgga ccagttggtg attttgaact 4740
   tttgctttgc cacggaacgg tctgcgttgt cgggaagatg cgtgatctga tccttcaact 4800
   cagcaaaagt tcgatttatt caacaaagcc gccgtcccgt caagtcagcg taatgctctg 4860
   ccagtgttac aaccaattaa ccaattctga ttagaaaaac tcatcgagca tcaaatgaaa 4920
   ctgcaattta ttcatatcag gattatcaat accatatttt tgaaaaagcc gtttctgtaa 4980
   tgaaggagaa aactcaccga ggcagttcca taggatggca agatcctggt atcggtctgc 5040
   gattccgact cgtccaacat caatacaacc tattaatttc ccctcgtcaa aaataaggtt 5100
   atcaagtgag aaatcaccat gagtgacgac tgaatccggt gagaatggca aaagcttatg 5160
   catttctttc cagacttgtt caacaggcca gccattacgc tcgtcatcaa aatcactcgc 5220
   atcaaccaaa ccgttattca ttcgtgattg cgcctgagcg agacgaaata cgcgatcgct 5280
   gttaaaagga caattacaaa caggaatcga atgcaaccgg cgcaggaaca ctgccagcgc 5340
   atcaacaata ttttcacctg aatcaggata ttcttctaat acctggaatg ctgttttccc 5400
   ggggatcgca gtggtgagta accatgcatc atcaggagta cggataaaat gcttgatggt 5460
   cggaagaggc ataaattccg tcagccagtt tagtctgacc atctcatctg taacatcatt 5520
   ggcaacgcta cctttgccat gtttcagaaa caactctggc gcatcgggct tcccatacaa 5580
   tcgatagatt gtcgcacctg attgcccgac attatcgcga gcccatttat acccatataa 5640
   atcagcatcc atgttggaat ttaatcgcgg cctcgagcaa gacgtttccc gttgaatatg 5700
   gctcataaca ccccttgtat tactgtttat gtaagcagac agttttattg ttcatgatga 5760
   tatattttta tcttgtgcaa tgtaacatca gagattttga gacacaacgt ggctttcccc 5820
   ccccccccat tattgaagca tttatcaggg ttattgtctc atgagcggat acatatttga 5880
   atgtatttag aaaaataaac aaataggggt tccgcgcaca tttccccgaa aagtgccacc 5940
   tgacgtctaa gaaaccatta ttatcatgac attaacctat aaaaataggc gtatcacgag 6000
   gccctttcgt ctcgcgcgtt tcggtgatga cggtgaaaac ctctgacaca tgcagctccc 6060
   ggagacggtc acagcttgtc tgtaagcgga tgccgggagc agacaagccc gtcagggcgc 6120
   gtcagcgggt gttggcgggt gtcggggctg gcttaactat gcggcatcag agcagattgt 6180
   actgagagtg caccatatgc ggtgtgaaat accgcacaga tgcgtaagga gaaaataccg 6240
   catcaga 6247
<210> 20
   <211> 5899
   <212> DNA
   <213> Artificial sequence
<220>
   <223> nucleic acid sequence of one strand of the plasmid pNBO003
<400> 20
   ttggctattg gccattgcat acgttgtatc catatcataa tatgtacatt tatattggct     60
   catgtccaac attaccgcca tgttgacatt gattattgac tagttattaa tagtaatcaa    120
   ttacggggtc attagttcat agcccatata tggagttccg cgttacataa cttacggtaa    180
   atggcccgcc tggctgaccg cccaacgacc cccgcccatt gacgtcaata atgacgtatg    240
   ttcccatagt aacgccaata gggactttcc attgacgtca atgggtggag tatttacggt    300
   aaactgccca cttggcagta catcaagtgt atcatatgcc aagtacgccc cctattgacg    360
   tcaatgacgg taaatggccc gcctggcatt atgcccagta catgacctta tgggactttc    420
   ctacttggca gtacatctac gtattagtca tcgctattac catggtgatg cggttttggc    480
   agtacatcaa tgggcgtgga tagcggtttg actcacgggg atttccaagt ctccacccca    540
   ttgacgtcaa tgggagtttg ttttggcacc aaaatcaacg ggactttcca aaatgtcgta    600
   acaactccgc cccattgacg caaatgggcg gtaggcgtgt acggtgggag gtctatataa    660
   gcagagctcg tttagtgaac cgtcagatcg cctggagacg ccatccacgc tgttttgacc    720
   tccatagaag acaccgggac cgatccagcc tccgcggccg ggaacggtgc attggaacgc 780
   ggattccccg tgccaagagt gacgtaagta ccgcctatag agtctatagg cccaccccct 840
   tggcttctta tgcatgctat actgtttttg gcttggggtc tatacacccc cgcttcctca 900
   tgttataggt gatggtatag cttagcctat aggtgtgggt tattgaccat tattgaccac 960
   tcccctattg gtgacgatac tttccattac taatccataa catggctctt tgccacaact 1020
   ctctttattg gctatatgcc aatacactgt ccttcagaga ctgacacgga ctctgtattt 1080
   ttacaggatg gggtctcatt tattatttac aaattcacat atacaacacc accgtcccca 1140
   gtgcccgcag tttttattaa acataacgtg ggatctccac gcgaatctcg ggtacgtgtt 1200
   ccggacatgg gctcttctcc ggtagcggcg gagcttctac atccgagccc tgctcccatg 1260
   cctccagcga ctcatggtcg ctcggcagct ccttgctcct aacagtggag gccagactta 1320
   ggcacagcac gatgcccacc accaccagtg tgccgcacaa ggccgtggcg gtagggtatg 1380
   tgtctgaaaa tgagctcggg gagcgggctt gcaccgctga cgcatttgga agacttaagg 1440
   cagcggcaga agaagatgca ggcagctgag ttgttgtgtt ctgataagag tcagaggtaa 1500
   ctcccgttgc ggtgctgtta acggtggagg gcagtgtagt ctgagcagta ctcgttgctg 1560
   ccgcgcgcgc caccagacat aatagctgac agactaacag actgttcctt tccatgggtc 1620
   ttttctcacg tcaccgtcgt cgaccagagc tgagatccta caggagtcca gggctggaga 1680
   gaaaacctct gcgaggaaag ggaaggagca agccgtgaat ttaagggacg ctgtgaagca 1740
   atcatggatg caatgaagag agggctctgc tgtgtgctgc tgctgtgtgg agcagtcttc 1800
   gtttcgccca gcggtaccgg atccaccctt gatggtgatg aatatttcat tggaaaatac 1860
   aaagaaaaag atgagacact gttttttgca agctacggcc taaagaggga tccttgccag 1920
   attgtcttag gctacaaatg ctcaaacaat caaacccact ttgtgcttaa ttttaaaacc 1980
   aataagaaat cctgcatatc agcaattaag ctgacttctt acccaaaaat caatcaaaac 2040
   tcggatttaa ctagaaatct ctactgccaa actggaggaa taggaacaga taactgcaaa 2100
   cttgtcttca agaaacgtaa aagacaaata gcagctaata ttgaaatcta cggcattcca 2160
   gcgaagaaat gttccttcaa ggatcgttac attggagctg atccactcca cgtcgattcc 2220
   tatgggcttt cgtatcagtt tgatcaggaa catggatgga atttggaacg aaataacatt 2280
   ttcaaagaca caagattttc cacagaagtt ttctaccaca aaaatggttt atttaacacc 2340
   caaataactt atttggctga agaagattcc ttctctgaag ctcgagagat tactgcgaag 2400
   gatattaaga agaagttttc aattattttg cccaatgaag agtataagag gattagtttc 2460
   ttggacgttt attggttcca ggagactatg cgaaaaaagc ctaaatatcc ctacattcac 2520
   tacaatggag aatgcagcaa tgagaataaa acttgtgaac ttgtctttga caccgatgaa 2580
   ctaatgacct acgcccttgt taaagtcttt actaatcctg agagtgatgg atctaggctc 2640
   aaagaagagg atttgggaag aggataaaag ggatccagat ctgctgtgcc ttctagttgc 2700
   cagccatctg ttgtttgccc ctcccccgtg ccttccttga ccctggaagg tgccactccc 2760
   actgtccttt cctaataaaa tgaggaaatt gcatcgcatt gtctgagtag gtgtcattct 2820
   attctggggg gtggggtggg gcagcacagc aagggggagg attgggaaga caatagcagg 2880
   catgctgggg atgcggtggg ctctatgggt acccaggtgc tgaagaattg acccggttcc 2940
   tcctgggcca gaaagaagca ggcacatccc cttctctgtg acacaccctg tccacgcccc 3000
   tggttcttag ttccagcccc actcatagga cactcatagc tcaggagggc tccgccttca 3060
   atcccacccg ctaaagtact tggagcggtc tctccctccc tcatcagccc accaaaccaa 3120
   acctagcctc caagagtggg aagaaattaa agcaagatag gctattaagt gcagagggag 3180
   agaaaatgcc tccaacatgt gaggaagtaa tgagagaaat catagaattt cttccgcttc 3240
   ctcgctcact gactcgctgc gctcggtcgt tcggctgcgg cgagcggtat cagctcactc 3300
   aaaggcggta atacggttat ccacagaatc aggggataac gcaggaaaga acatgtgagc 3360
   aaaaggccag caaaaggcca ggaaccgtaa aaaggccgcg ttgctggcgt ttttccatag 3420
   gctccgcccc cctgacgagc atcacaaaaa tcgacgctca agtcagaggt ggcgaaaccc 3480
   gacaggacta taaagatacc aggcgtttcc ccctggaagc tccctcgtgc gctctcctgt 3540
   tccgaccctg ccgcttaccg gatacctgtc cgcctttctc ccttcgggaa gcgtggcgct 3600
   ttctcaatgc tcacgctgta ggtatctcag ttcggtgtag gtcgttcgct ccaagctggg 3660
   ctgtgtgcac gaaccccccg ttcagcccga ccgctgcgcc ttatccggta actatcgtct 3720
   tgagtccaac ccggtaagac acgacttatc gccactggca gcagccactg gtaacaggat 3780
   tagcagagcg aggtatgtag gcggtgctac agagttcttg aagtggtggc ctaactacgg 3840
   ctacactaga aggacagtat ttggtatctg cgctctgctg aagccagtta ccttcggaaa 3900
   aagagttggt agctcttgat ccggcaaaca aaccaccgct ggtagcggtg gtttttttgt 3960
   ttgcaagcag cagattacgc gcagaaaaaa aggatctcaa gaagatcctt tgatcttttc 4020
   tacggggtct gacgctcagt ggaacgaaaa ctcacgttaa gggattttgg tcatgagatt 4080
   atcaaaaagg atcttcacct agatcctttt aaattaaaaa tgaagtttta aatcaatcta 4140
   aagtatatat gagtaaactt ggtctgacag ttaccaatgc ttaatcagtg aggcacctat 4200
   ctcagcgatc tgtctatttc gttcatccat agttgcctga ctccgggggg ggggggcgct 4260
   gaggtctgcc tcgtgaagaa ggtgttgctg actcatacca ggcctgaatc gccccatcat 4320
   ccagccagaa agtgagggag ccacggttga tgagagcttt gttgtaggtg gaccagttgg 4380
   tgattttgaa cttttgcttt gccacggaac ggtctgcgtt gtcgggaaga tgcgtgatct 4440
   gatccttcaa ctcagcaaaa gttcgattta ttcaacaaag ccgccgtccc gtcaagtcag 4500
   cgtaatgctc tgccagtgtt acaaccaatt aaccaattct gattagaaaa actcatcgag 4560
   catcaaatga aactgcaatt tattcatatc aggattatca ataccatatt tttgaaaaag 4620
   ccgtttctgt aatgaaggag aaaactcacc gaggcagttc cataggatgg caagatcctg 4680
   gtatcggtct gcgattccga ctcgtccaac atcaatacaa cctattaatt tcccctcgtc 4740
   aaaaataagg ttatcaagtg agaaatcacc atgagtgacg actgaatccg gtgagaatgg 4800
   caaaagctta tgcatttctt tccagacttg ttcaacaggc cagccattac gctcgtcatc 4860
   aaaatcactc gcatcaacca aaccgttatt cattcgtgat tgcgcctgag cgagacgaaa 4920
   tacgcgatcg ctgttaaaag gacaattaca aacaggaatc gaatgcaacc ggcgcaggaa 4980
   cactgccagc gcatcaacaa tattttcacc tgaatcagga tattcttcta atacctggaa 5040
   tgctgttttc ccggggatcg cagtggtgag taaccatgca tcatcaggag tacggataaa 5100
   atgcttgatg gtcggaagag gcataaattc cgtcagccag tttagtctga ccatctcatc 5160
   tgtaacatca ttggcaacgc tacctttgcc atgtttcaga aacaactctg gcgcatcggg 5220
   cttcccatac aatcgataga ttgtcgcacc tgattgcccg acattatcgc gagcccattt 5280
   atacccatat aaatcagcat ccatgttgga atttaatcgc ggcctcgagc aagacgtttc 5340
   ccgttgaata tggctcataa caccccttgt attactgttt atgtaagcag acagttttat 5400
   tgttcatgat gatatatttt tatcttgtgc aatgtaacat cagagatttt gagacacaac 5460
   gtggctttcc cccccccccc attattgaag catttatcag ggttattgtc tcatgagcgg 5520
   atacatattt gaatgtattt agaaaaataa acaaataggg gttccgcgca catttccccg 5580
   aaaagtgcca cctgacgtct aagaaaccat tattatcatg acattaacct ataaaaatag 5640
   gcgtatcacg aggccctttc gtctcgcgcg tttcggtgat gacggtgaaa acctctgaca 5700
   catgcagctc ccggagacgg tcacagcttg tctgtaagcg gatgccggga gcagacaagc 5760
   ccgtcagggc gcgtcagcgg gtgttggcgg gtgtcggggc tggcttaact atgcggcatc 5820
   agagcagatt gtactgagag tgcaccatat gcggtgtgaa ataccgcaca gatgcgtaag 5880
   gagaaaatac cgcatcaga 5899
<210> 21
   <211> 1239
   <212> DNA
   <213> Artificial sequence
<220>
   <223> codon-optimized nucleic acid sequence for mammalian expression (unprocessed LJM17 protein from Lutzomyia longipalpis)
<400> 21
   tcacacttcg atttctttgg ggggcttgta gttggggttg caccggctgt acttgaacac      60
   ccgctcggtg tccaccttca tgatccggat cttccggttc acgaaccgca tcttccagat     120
   cttttcctgg tcctccacgg gggggtggcc gttggccatg atccacagca tgcccttgct     180
   gtccaccagg atgtcggtgc cgaaggtgaa ccgggcgctg ctgaagatca cgtcggtgtt     240
   gtcgggcttc agttccatgt tcacgttcca gcaggacacc tgccggctgt cgctctcggc     300
   gaagtacagc accttgtgct cggggtcgta ggccagggca atggcgtcgg tgtacttgcc     360
   ccggtcgccg tgcagctggg ggttcagctg gccgttctcg gttttcagct ctttggtatt     420
   cacgctgtac accttggtgc tgctgccggc gatgtagcag gcgggcctgt ggcccatctc     480
   gtcccggtcg cccagggcga tgccgaacag gcccactttg tacttcatct gttcctcgcc     540
   gctgtagctg aaggtgctct ctttgtcggc ctcgaaggtc ttgtcggtga acttccaggc     600
   cttcttctgg gtctcgtcgt acacgatcag gctgttgtcc ttgaagttgg tcaggtacac     660
   gaagctctcg gtgcagtcgc ccttggtgtt caccacgtcc acggcaaagc cgccgaactc     720
   cacctggctg ctgtacaggt cgtcggggat ctcgaaccgg tggatctcgg gggtgtggtc     780
   cttcttcagg tcgtaggcga tcagggtggg cttgcccttg gggtactggt cggcgtcgcc     840
   gcctgtgtac tccaccttgc cgatgtccag cacccacagc cgcctgcagt cgtcgatcac     900
   gggctggtac acgttcacca ggtctttctt gccctcgccg ttaaacttct tgaacttctc     960
   gaagctgggg gccctctcca cggggaagcc ggggttcatc accatgttca gctcggccac    1020
   ggtgtagggc accttgggct tccgcctggg gatggcgatg aacagccggt agccctcggg    1080
   gtccacggcc aggccggtgg ggatgttgaa cttggggttg tagtcgtcgg tgtccaggcc    1140
   gtcgaaggtg atgttccgca ggctgtagcc gatctccacg taggcgccgt ggatgccctg    1200
   gaacagcacg atggccagga acacgaagaa gaaccgcat                           1239
<210> 22
   <211> 906
   <212> DNA
   <213> Artificial sequence
<220>
   <223> codon-optimized nucleic acid sequence for mammalian expression (unprocessed LJL143 protein from Lutzomyia longipalpis)
<400> 22
   atgaacagca tcaactttct gagcatcgtg ggcctgatca gcttcggctt catcgtggcc      60
   gtgaagtgcg acggcgacga gtacttcatc ggcaagtaca aagagaagga cgagaccctg     120
   ttcttcgcca gctacggcct gaagcgggac ccctgccaga tcgtgctggg ctacaagtgc     180
   agcaacaacc agacccactt cgtgctgaac ttcaagacca acaagaagag ctgcatcagc     240
   gccatcaagc tgaccagcta ccccaagatc aaccagaaca gcgacctgac caagaacctg     300
   tactgccaga ccggcggcat cggcaccgac aactgcaagc tggtgttcaa gaagcggaag     360
   cggcagatcg ccgccaacat cgagatctac ggcatccccg ccaagaagtg cagcttcaag     420
   gaccggtaca tcggcgccga ccccctgcac gtggactcct acggcctgcc ctaccagttc     480
   gaccaggaac acggctggaa cgtcgagcgg tacaacatct tcaaggacac ccggttcagc     540
   accgaggtgt tctaccacaa gaacggcctg ttcaacaccc agatcaccta cctggccgaa     600
   gaggacagct tcagcgaggc ccgggagatc accgccaagg acatcaagaa gaagttcagc     660
   atcatcctgc ccaacgagga atacaagcgg atcagcttcc tggacgtgta ctggttccag     720
   gaaaccatgc ggaagaagcc caagtacccc tacatccact acaacggcga gtgctccaac     780
   gagaacaaga cctgcgaact ggtgttcgac accgacgagc tgatgaccta cgccctggtg     840
   aaggtgttca ccaaccccga gagcgacggc agccggctga aagaagagga cctgggcagg     900
   ggctga                                                                906
<210> 23
   <211> 271
   <212> PRT
   <213> Lutzomyia longipalpis
<400> 23
   Met Leu Gln Ile Lys His Leu Leu Ile Phe Val Gly Leu Leu Val Val
   1 5 10 15
   Val Asn Ala Gln Ser Asn Tyr Cys Lys Gln Glu Ser Cys Ser Ser Gly
   20 25 30
   Gly Val Glu Arg Pro His Ile Gly Cys Lys Asn Ser Gly Asp Phe Ser
   35 40 45
   Glu Thr Cys Ser Gly Asp Ala Glu Ile Val Lys Met Asp Lys Lys Lys
   50 55 60
   Gln Asn Leu Leu Val Lys Met His Asn Arg Leu Arg Asp Arg Phe Ala
   65 70 75 80
   Arg Gly Ala Val Pro Gly Phe Ala Pro Ala Ala Lys Met Pro Met Leu
   85 90 95
   Lys Trp Asn Asp Glu Leu Ala Lys Leu Ala Glu Tyr Asn Val Arg Thr
   100 105 110
   Cys Lys Phe Ala His Asp Lys Cys Arg Ala Ile Asp Val Cys Pro Tyr
   115 120 125
   Ala Gly Gln Asn Leu Ala Gln Met Met Ser Tyr Pro Thr His Arg Asp
   130 135 140
   Leu Asn Tyr Val Leu Lys Asn Leu Thr Arg Glu Trp Phe Trp Glu Tyr
   145                 150                 155                 160
   Arg Trp Ala Lys Gln Ser Gln Leu Asp Asn Tyr Val Gly Gly Pro Gly
                   165                 170                 175
   Lys Asp Asn Lys Gln Ile Gly His Phe Thr Ala Phe Val His Glu Lys
               180                 185                 190
   Thr Asp Lys Val Gly Cys Ala Ile Ala Arg Phe Thr Asn Glu His Asn
           195                 200                 205
   Phe Lys Glu Thr Leu Leu Ala Cys Asn Tyr Cys Tyr Thr Asn Met Met
       210                 215                 220
   Lys Glu Arg Ile Tyr Thr Gln Gly Lys Pro Cys Ser Gln Cys Gln Ser
   225                 230                 235                 240
   Lys Lys Cys Gly Pro Val Tyr Lys Asn Leu Cys Asp Pro Ser Glu Lys
                   245                 250                 255
   Val Asp Pro Thr Pro Asp Val Leu Lys Gln Trp Lys His Gly Lys
               260                 265                 270
<210> 24
   <211> 905
   <212> DNA
   <213> Lutzomyia longipalpis
<400> 24
   agttgtggag cttttggtca ttttacgtga tgttgcaaat taaacatctt ctgatttttg      60
   tgggattgct cgtggttgtt aatgcacaga gcaattactg caaacaggaa tcgtgctcat     120
   cgggaggtgt tgagagaccc catattgggt gcaaaaactc tggagatttt tccgaaactt     180
   gctccggaga tgcagaaatt gttaagatgg acaagaagaa gcagaacctc cttgtgaaaa     240
   tgcacaatcg cctgagagat agatttgctc gtggtgcagt gccaggtttt gcaccagctg     300
   cgaaaatgcc aatgcttaaa tggaacgatg aactggccaa attggcagag tacaacgtga     360
   gaacgtgcaa atttgcccac gataaatgcc gcgcaattga tgtctgcccc tatgctggac     420
   agaatctagc tcaaatgatg tcctatccta cccatcgaga tctaaactat gttcttaaga     480
   atctcacaag ggaatggttc tgggagtaca gatgggctaa gcaatctcag cttgataatt     540
   acgtgggtgg tcctgggaaa gacaacaaac aaattggaca tttcacagct tttgtgcatg     600
   agaaaacaga caaagttgga tgcgctatag ctcgatttac aaatgagcac aattttaagg     660
   agaccctcct agcttgcaac tactgctaca cgaatatgat gaaggagagg atctacacgc     720
   agggaaaacc ttgttcacag tgtcagagca aaaagtgtgg gccagtctac aagaacctgt     780
   gtgatccttc ggagaaggtt gatccaactc ctgatgtcct taagcaatgg aagcatggaa     840
   aatgattatt aagctcactt caaatgtttc caatccaaaa aaaaaaaaaa aaaaaaaaaa     900
   aaaaa                                                                 905
<210> 25
   <211> 159
   <212> PRT
   <213> Lutzomyia longipalpis
<400> 25
   Met Leu Leu Arg Ser Leu Phe Val Leu Phe Leu Ile Phe Leu Thr Phe
   1               5                   10                  15
   Cys Asn Ala Glu Glu Glu Leu Ile Glu Arg Lys Leu Thr Gly Lys Thr
               20                  25                  30
   Ile Tyr Ile Ser Thr Ile Lys Leu Pro Trp Phe Gln Ala Leu Asn His
           35                  40                  45
   Cys Val Lys Asn Gly Tyr Thr Met Val Ser Ile Lys Thr Phe Glu Glu
       50                  55                  60
   Asn Lys Glu Leu Leu Lys Glu Leu Lys Arg Val Ile Arg Thr Glu Asp
   65                  70                  75                  80
   Thr Gln Val Trp Ile Gly Gly Leu Lys His His Gln Phe Ala Asn Phe
                   85                  90                  95
   Arg Trp Val Ser Asp Gly Ser His Val Ala Thr Ala Ser Gly Tyr Thr
               100                 105                 110
   Asn Trp Ala Pro Gly Glu Pro Ala Asp Ser Phe Tyr Tyr Asp Gln Phe
           115                 120                 125
   Cys Met Ala Met Leu Phe Arg Lys Asp Gly Ala Pro Trp Asp Asp Leu
       130                 135                 140
   Asn Cys Trp Val Lys Asn Leu Phe Val Cys Glu Lys Arg Asp Asp
   145                 150                 155
<210> 26
   <211> 617
   <212> DNA
   <213> Lutzomyia longipalpis
<400> 26
   ttttgagaaa aacatttcct tgtgagttaa atagttggta aattaaatca agagaatgtt      60
   gcttcgttcc ttgtttgttc tttttctaat tttcttaaca ttctgcaacg ctgaggaaga     120
   acttattgag agaaagttaa caggaaaaac gatctatatc tcaacaataa agcttccgtg    180
   gttccaagct cttaatcatt gtgttaaaaa tggctacaca atggtgtcaa ttaagacatt    240
   tgaagagaat aaagaactcc ttaaagaact caaaagggtg attaggacag aagatacaca    300
   agtttggatt ggaggcctca aacatcatca atttgcaaac tttcgttggg taagcgatgg    360
   aagccacgta gcaacagctt cagggtacac caattgggcc ccaggggagc cagctgattc    420
   cttctattac gatcaatttt gcatggcgat gttgttcaga aaagacggcg ctccgtggga    480
   tgatttgaat tgttgggtta agaatctttt tgtttgtgag aaacgagatg attgagaggc    540
   tatttttgtt atctcaccgt tttgttgaat aaaaaagaag aagaaagaca aaaaaaaaaa    600
   aaaaaaaaaa aaaaaaa                                                  617
<210> 27
   <211> 304
   <212> PRT
   <213> Lutzomyia longipalpis
<400> 27
   Met Lys Leu Leu Gln Ile Ile Phe Ser Leu Phe Leu Val Phe Phe Pro
   1               5                   10                  15
   Thr Ser Asn Gly Ala Leu Thr Gly Asn Glu Ser Ala Ala Asn Ala Ala
               20                  25                  30
   Pro Leu Pro Val Val Leu Trp His Gly Met Gly Asp Ser Cys Cys Phe
           35                  40                  45
   Pro Phe Ser Leu Gly Ser Ile Lys Lys Leu Ile Glu Gln Gln Ile Pro
       50                  55                  60
   Gly Ile His Val Val Ser Leu Lys Ile Gly Lys Ser Leu Ile Glu Asp
   65                  70                  75                  80
   Tyr Glu Ser Gly Phe Phe Val His Pro Asp Lys Gln Ile Gln Glu Val
                   85                  90                  95
   Cys Glu Ser Leu Gln Asn Asp Leu Thr Leu Ala Asn Gly Phe Asn Ala
               100                 105                 110
   Ile Gly Phe Ser Gln Gly Ser Gln Phe Leu Arg Gly Leu Val Gln Arg
           115                 120                 125
   Cys Ser Ser Ile Gln Val Arg Asn Leu Ile Ser Ile Gly Gly Gln His
       130                 135                 140
   Gln Gly Val Phe Gly Leu Pro Tyr Cys Pro Ser Leu Ser Arg Lys Thr
   145                 150                 155                 160
   Cys Glu Tyr Phe Arg Lys Leu Leu Asn Tyr Ala Ala Tyr Glu Lys Trp
                   165                 170                 175
   Val Gln Lys Leu Leu Val Gln Ala Thr Tyr Trp His Asp Pro Leu Asn
               180                 185                 190
   Glu Asp Ala Tyr Arg Thr Gly Ser Thr Phe Leu Ala Asp Ile Asn Asn
           195                 200                 205
   Glu Arg Gln Ile Asn Asn Asp Tyr Ile Asn Asn Ile Arg Lys Leu Asn
       210                 215                 220
   Arg Phe Val Met Val Lys Phe Leu Asn Asp Ser Met Val Gln Pro Ile
   225                 230                 235                 240
   Glu Ser Ser Phe Phe Gly Phe Tyr Ala Pro Gly Thr Asp Thr Glu Val
                   245                 250                 255
   Leu Pro Leu Lys Gln Ser Lys Ile Tyr Leu Glu Asp Arg Leu Gly Leu
               260                 265                 270
   Gln Ser Val Pro Ile Asp Tyr Leu Glu Cys Gly Gly Asp His Leu Gln
           275                 280                 285
   Phe Thr Lys Glu Trp Phe Ile Lys Phe Ile Ile Pro Tyr Leu Lys Gln
       290                 295                 300
<210> 28
   <211> 1273
   <212> DNA
   <213> Lutzomyia longipalpis
<400> 28
   tacttcgtac tctcagaatt tcttacaagt tcctttttct cttaactttt aaagttttat      60
   ttaacaaaat tgctccattt tttcgttttc tgaatattct gttgaaattt tgattaatct     120
   attttatgtg cagtttttac taaaaatccc ttatcagcaa cccggtgtct acagttttgt     180
   cacgctcagt agcatcttca aggtggtaag aaaaaatgaa actcctgcaa atcatcttct     240
   ctctcttcct ggtctttttc ccgacctcaa atggggccct gaccggaaat gaaagtgcag     300
   caaatgcagc tcccttgcct gtcgtcctgt ggcacgggat gggcgattct tgctgctttc     360
   ccttcagttt gggaagcata aaaaaattaa ttgaacaaca aattcctggg attcatgttg     420
   ttagcctgaa aattggaaag tctctcattg aggactatga aagtggattt tttgttcatc     480
   cagacaagca aattcaggaa gtttgtgagt cacttcagaa cgatctaaca ctcgcaaatg     540
   gattcaatgc aattggattt tctcagggta gtcagttcct gcgaggtctt gtgcaacgat     600
   gttcttctat acaagtaagg aatctcattt ccattggagg acagcatcaa ggggtttttg     660
   gtctgcccta ttgtccttcg ttgagcagaa agacttgcga atactttaga aagctcctga     720
   attatgcagc ttatgaaaaa tgggtacaga aactcctagt tcaagccacc tactggcatg     780
   atcctctaaa tgaggatgca tatcggactg gaagcacttt ccttgctgat ataaataatg     840
   agagacaaat caataatgac tatattaata atattcggaa gctaaatcgt tttgtgatgg     900
   taaagttcct caacgacagc atggttcagc caattgaatc tagtttcttt ggattctacg     960
   ctccaggaac tgatacagaa gttctcccat taaaacaaag caagatttat ttggaagatc    1020
   gtttgggact tcaatcagta ccgatagatt atctagaatg cggaggagat catttgcaat    1080
   ttacaaaaga atggttcata aagtttatca taccctatct gaagcaataa gagctgcaat    1140
   gtaattgatt aaaaaatgtt aaccatttca ggatgattgg gtgacccctt aaaaatataa    1200
   atgaaaaaat atacaaaaga aataaatttt tatattgatc ccacaaaaaa aaaaaaaaaa    1260
   aaaaaaaaaa aaa                                                       1273
<210> 29
   <211> 102
   <212> PRT
   <213> Lutzomyia longipalpis
<400> 29
   Met Arg Asn Phe Ala Val Val Ser Leu Ala Val Ala Val Leu Leu Phe
   1               5                   10                  15
   Cys Ala Trp Pro Ile Asn Ala Glu Asp Asn Glu Glu Val Gly Lys Ala
               20                  25                  30
   Arg Glu Lys Arg Gly Leu Lys Asp Ala Met Glu His Phe Lys Asn Gly
           35                  40                  45
   Phe Lys Glu Leu Thr Lys Asp Phe Lys Leu Pro Ser Leu Pro Ser Leu
       50                  55                  60
   Pro Gly Phe Gly Lys Lys Pro Glu Ser Gly Ser Ser Glu Asp Ser Gly
   65                  70                  75                  80
   Asp Lys Thr Glu Asp Thr Ser Gly Ser Lys Asp Asp Gln Ser Lys Asp
                   85                  90                  95
   Asn Thr Val Glu Glu Ser
               100
<210> 30
   <211> 466
   <212> DNA
   <213> Lutzomyia longipalpis
<400> 30
   ggatcggcca ttatggccgg ggcagttaat cgccacaatt taataaaatg aggaactttg      60
   ctgtagtcag tttagccgtt gctgtcctgc tcttctgtgc atggcctata aatgcggaag     120
   ataatgaaga agttggaaag gcgagagaaa aaagaggctt aaaagacgca atggaacact     180
   tcaaaaatgg atttaaggag ctgacaaagg actttaaact tccaagcctt ccaagtcttc     240
   ctggatttgg taaaaagcct gaatctggaa gttctgaaga ttctggagat aaaactgagg     300
   ataccagtgg atctaaggac gaccaatcaa aggataatac ggtcgaagaa tcttaagaaa     360
   ggcgcaaata gctattttca aagtggcgaa tgtttctttc tttatctgaa ataaatattt     420
   ttaaaccttt cgaaaccaaa aaaaaaaaaa aaaaaaaaaa aaaaaa                    466
<210> 31
   <211> 247
   <212> PRT
   <213> Lutzomyia longipalpis
<400> 31
   Met Asn Phe Leu Leu Lys Ile Phe Ser Leu Leu Cys Leu Cys Gly Leu
   1               5                   10                  15
   Gly Tyr Ser Trp Gln Asp Val Arg Asn Ala Asp Gln Thr Leu Trp Ala
               20                  25                  30
   Tyr Arg Ser Cys Gln Lys Asn Pro Glu Asp Lys Asp His Val Pro Gln
           35                  40                  45
   Trp Arg Lys Phe Glu Leu Pro Asp Asp Glu Lys Thr His Cys Tyr Val
       50                  55                  60
   Lys Cys Val Trp Thr Arg Leu Gly Ala Tyr Asn Glu Asn Glu Asn Val
   65                  70                  75                  80
   Phe Lys Ile Asp Val Ile Thr Lys Gln Phe Asn Glu Arg Gly Leu Glu
                   85                  90                  95
   Val Pro Ala Gly Leu Asp Gln Glu Leu Gly Gly Ser Thr Asp Gly Thr
               100                 105                 110
   Cys Lys Ala Val Tyr Asp Lys Ser Met Lys Phe Phe Lys Ser His Phe
           115                 120                 125
   Met Asp Phe Arg Asn Ala Tyr Tyr Ala Thr Tyr Asp Gly Ser Asp Glu
       130                 135                 140
   Trp Phe Ser Lys Asn Pro Asp Val Lys Pro Lys Gly Thr Lys Val Ser
   145                 150                 155                 160
   Glu Tyr Cys Lys Asn Lys Asp Asp Gly Asp Cys Lys His Ser Cys Ser
                   165                 170                 175
   Met Tyr Tyr Tyr Arg Leu Ile Asp Glu Asp Asn Leu Val Ile Pro Phe
               180                 185                 190
   Ser Asn Leu Pro Asp Tyr Pro Glu Asp Lys Leu Glu Glu Cys Arg Asn
           195                 200                 205
   Glu Ala Lys Ser Ala Asn Glu Cys Lys Ser Ser Val Ile Tyr Gln Cys
       210                 215                 220
   Leu Glu Asn Ala Asp Lys Ser Ala Leu Asp Ala Ser Leu Asn Ile Leu
   225                 230                 235                 240
   Asp Glu Phe Ser Gly Arg Tyr
                   245
<210> 32
   <211> 955
   <212> DNA
   <213> Lutzomyia longipalpis
<400> 32
   acttaaagat ttttgtttaa gcaaaatgaa cttcttgttg aaaattttct ctttgctctg      60
   tctctgtgga ctggggtatt catggcagga tgtgagaaat gccgatcaaa ccctctgggc     120
   gtatagatcg tgccaaaaga atcctgaaga taaggatcac gtacctcaat ggaggaagtt     180
   cgaattaccc gacgatgaaa agactcattg ctacgtcaag tgcgtatgga cgcgtttggg     240
   agcttacaat gaaaatgaaa atgttttcaa aattgatgtc attactaagc aatttaatga     300
   acgtggccta gaagttccgg ctggacttga tcaagaattg ggtggttcta cagatggaac     360
   ttgcaaagca gtttacgata aatccatgaa gttcttcaaa tctcatttta tggactttag     420
   gaatgcttac tacgcaactt atgacggttc tgatgaatgg tttagcaaga accctgatgt     480
   aaaaccgaaa ggaacaaaag tttccgaata ctgcaaaaat aaagatgatg gagattgcaa     540
   acattcctgc agtatgtact actaccgctt aatcgatgaa gacaacttag ttattccgtt     600
   cagcaactta cctgactatc ccgaagataa gctcgaggaa tgcaggaatg aagccaagtc     660
   cgcaaatgag tgcaaatcat ctgttatcta tcagtgtttg gaaaatgcgg ataagtcagc     720
   tttagacgcg tctttgaata tactcgatga gttttctgga agatattaaa acaaactgga     780
   taaaaaactt aggccaacct atgattcgaa cttacgattt tgaacttgaa attcatgtgc     840
   tttaacctat tgtcccacta ggaagaaaaa tccatatttg gtgatgttaa actatttttg     900
   aacctcttca aaataaacaa ttttcaaaaa aaaaaaaaaa aaaaaaaaaa aaaaa          955
<210> 33
   <211> 325
   <212> PRT
   <213> Lutzomyia longipalpis
<400> 33
   Met Phe Leu Lys Trp Val Val Cys Ala Phe Ala Thr Val Phe Leu Val
   1               5                   10                  15
   Gly Val Ser Gln Ala Ala Pro Pro Gly Val Glu Trp Tyr His Phe Gly
               20                  25                  30
   Leu Ile Ala Asp Met Asp Lys Lys Ser Ile Ala Ser Asp Lys Thr Thr
           35                  40                  45
   Phe Asn Ser Val Leu Lys Ile Asp Glu Leu Arg His Asn Thr Lys Thr
       50                  55                  60
   Asp Gln Tyr Ile Tyr Val Arg Ser Arg Val Lys Lys Pro Val Ser Thr
   65                  70                  75                  80
   Arg Tyr Gly Phe Lys Gly Arg Gly Ala Glu Leu Ser Glu Ile Val Val
                   85                  90                  95
   Phe Asn Asn Lys Leu Tyr Thr Val Asp Asp Lys Ser Gly Ile Thr Phe
               100                 105                 110
   Arg Ile Thr Lys Asp Gly Lys Leu Phe Pro Trp Val Ile Leu Ala Asp
           115                 120                 125
   Ala Asp Gly Gln Arg Pro Asp Gly Phe Lys Gly Glu Trp Ala Thr Ile
       130                 135                 140
   Lys Asp Asp Thr Ile Tyr Val Gly Ser Thr Gly Met Leu Lys Phe Thr
   145                 150                 155                 160
   Ser Ser Leu Trp Val Lys Lys Ile Thr Lys Asp Gly Val Val Thr Ser
                   165                 170                 175
   His Asp Trp Thr Asp Lys Tyr Arg Lys Ile Leu Lys Ala Leu Asn Met
               180                 185                 190
   Pro Asn Gly Phe Val Trp His Glu Ala Val Thr Trp Ser Pro Phe Arg
               195                 200                 205
   Lys Gln Trp Val Phe Met Pro Arg Lys Cys Ser Arg His Pro Phe Ser
       210                 215                 220
   Gln Glu Leu Glu Glu Arg Thr Gly Cys Asn Lys Ile Val Thr Ala Asp
   225                 230                 235                 240
   Glu Asn Phe Asn Asp Ile Gln Val Ile His Ile Gln Asp Gln Pro Tyr
                   245                 250                 255
   Asn Leu Ala Ser Gly Phe Ser Ser Phe Arg Phe Ile Pro Gly Thr Lys
               260                 265                 270
   Asn Glu Arg Leu Leu Ala Leu Arg Thr Val Glu Gln Glu Asp Gln Val
           275                 280                 285
   Lys Thr Trp Ala Val Val Met Asp Met Lys Gly Thr Val Leu Met Tyr
       290                 295                 300
   Glu Lys Glu Leu Tyr Asp Glu Lys Phe Glu Gly Leu Ala Phe Phe Gly
   305                 310                 315                 320
   Gly Ile Lys Lys Asn
                   325
<210> 34
   <211> 1071
   <212> DNA
   <213> Lutzomyia longipalpis
<400> 34
   aaagagaagt agtgagaatg tttcttaagt gggttgtttg tgcttttgcg actgtcttcc      60
   ttgttggggt gagtcaggca gccccaccgg gggttgaatg gtatcacttt ggtctgattg     120
   ctgatatgga caaaaaatcc atcgcgagtg acaaaaccac ctttaacagc gtcctaaaga     180
   tcgatgaatt gcgccacaac acaaaaacgg atcaatacat ttatgtgcgt agtcgagtga     240
   agaagcccgt ttccacgagg tatgggttca aaggacgcgg tgcggaattg tcggaaattg     300
   ttgtcttcaa caataaactt tacacagttg atgataaatc tggaattacg ttccgcataa     360
   cgaaagacgg aaaactcttc ccgtgggtta ttctcgcaga tgccgatgga cagcgacccg     420
   atggctttaa gggtgaatgg gctacaatta aggatgatac aatctatgtt ggatctacgg     480
   ggatgctcaa gttcacttca tccctttggg tgaagaagat cacgaaagat ggcgttgtta     540
   cgagtcacga ttggactgat aaataccgaa agattctcaa agctctaaac atgccaaatg     600
   gttttgtctg gcatgaggct gttacgtggt ctccattcag gaagcaatgg gtcttcatgc     660
   cgagaaagtg ctcaaggcat cccttctcac aggaactcga agaacgcaca gggtgcaata     720
   aaatagtgac ggcagatgag aatttcaacg acattcaagt tattcacatt caagatcagc     780
   catataattt agcttctggt ttctcttcct tccgctttat tcctggtacg aaaaatgaaa     840
   gacttctcgc cttgaggaca gtagagcagg aagatcaggt taaaacttgg gctgtggtca     900
   tggatatgaa aggaacagtt ctgatgtacg aaaaggaact ttatgacgaa aaattcgaag     960
   gtttagcatt ctttggtggt attaaaaaga attaatttgt tccagaagct tttagatgaa    1020
   ataataaatt ttatttcatt ttaaaaaaaa aaaaaaaaaa aaaaaaaaaa a             1071
<210> 35
   <211> 160
   <212> PRT
   <213> Lutzomyia longipalpis
<400> 35
   Met Ala Leu Lys Phe Leu Pro Val Leu Leu Leu Ser Cys Phe Ala Met
   1               5                   10                  15
   Ser Thr Ala Leu Gln Val Thr Glu Lys Glu Leu Ser Asp Gly Lys Lys
               20                  25                  30
   Ile Phe Ile Ser Lys Val Glu Leu Asn Trp Phe Glu Ala Leu Asp Phe
           35                  40                  45
   Cys Ile His Arg Gly Leu Thr Leu Leu Ser Ile Lys Ser Ala Lys Glu
       50                  55                  60
   Asn Val Asp Val Thr Lys Ala Ile Arg Ala Glu Leu Asn Phe Asp Ser
   65                  70                  75                  80
   Lys Lys Leu Ala His Val Trp Thr Gly Gly Ile Arg His Ser Gln Asp
                   85                  90                  95
   Lys Tyr Phe Arg Trp Ile Asn Asp Gly Thr Lys Val Val Lys Arg Val
               100                 105                 110
   Tyr Thr Asn Trp Phe Thr Gly Glu Pro Asn Asn Gly Tyr Trp Lys Asp
           115                 120                 125
   Glu Phe Cys Leu Glu Ile Tyr Tyr Lys Thr Glu Glu Gly Lys Trp Asn
       130                 135                 140
   Asp Asp Lys Cys His Val Lys His His Phe Val Cys Gln Glu Lys Lys
   145                 150                 155                 160
<210> 36
   <211> 648
   <212> DNA
   <213> Lutzomyia longipalpis
<400> 36
   cgcggccgcg tcgaccgaca gaaggggtag tttgtagaga actttgagtt ctaaaggaaa     60
   ttctcaagaa gaaaatattc aaaagtaaag aatggcgttg aagtttcttc cggttctcct    120
   tctaagctgc ttcgcaatga gcacggcact acaagttact gagaaggaac tttctgatgg    180
   gaaaaagatc ttcatctcca aagttgagct aaactggttc gaagctcttg atttctgtat    240
   ccatcgtggt cttacgttgc tctcaattaa atccgccaag gaaaatgtag acgtaacaaa    300
   agcaattcgg gctgaattga attttgattc aaagaaattg gctcatgtgt ggactggagg    360
   tattcgccat agtcaagata agtatttccg ttggataaat gatggaacta aagttgttaa    420
   acgagtctac accaattggt tcactggaga accaaataat ggttactgga aggatgaatt    480
   ttgtctggaa atttactata aaaccgaaga agggaagtgg aatgatgata aatgtcacgt    540
   gaagcatcat tttgtatgtc aagaaaagaa ataaattgat tgattttgtt tgctgatttg    600
   cagttcagaa ttgaaaagcc aaaaaaaaaa aaaaaaaaaa aaaaaaaa                  648
<210> 37
   <211> 161
   <212> PRT
   <213> Lutzomyia longipalpis
<400> 37
   Met Ala Phe Ser Asn Thr Leu Phe Val Leu Phe Val Ser Phe Leu Thr
   1               5                   10                  15
   Phe Cys Gly Ala Asp Gln Thr Leu Ile Glu Lys Glu Leu Thr Gly Arg
               20                  25                  30
   Thr Val Tyr Ile Ser Lys Ile Lys Leu Asn Trp Asn Asp Ala Phe Asp
           35                  40                  45
   Tyr Cys Ile Arg Asn Gly Leu Thr Phe Ala Lys Ile Lys Ser Ala Glu
       50                  55                  60
   Glu Asn Thr Glu Leu Ser Glu Lys Leu Lys Thr Val Ile Arg Thr Glu
   65                  70                  75                  80
   Glu Phe Gln Val Trp Ile Gly Gly Ile Glu His His Gln Asp Ser Ser
                   85                  90                  95
   Phe Arg Trp Val Ser Asp Ser Gln Pro Ile Thr Asn Lys Leu Gly Tyr
               100                 105                 110
   Lys Tyr Thr Asn Trp Asn Thr Gly Glu Pro Thr Asn Tyr Gln Asn Asn
           115                 120                 125
   Glu Tyr Cys Leu Glu Ile Leu Phe Arg Lys Glu Asp Gly Lys Trp Asn
       130                 135                 140
   Asp Phe Pro Cys Ser Ala Arg His His Phe Val Cys Glu Lys Arg Thr
   145                 150                 155                 160
   Lys
<210> 38
   <211> 586
   <212> DNA
   <213> Lutzomyia longipalpis
<400> 38
   aatagatctt caaaacgtct aagaatggct ttcagcaaca ctttatttgt tctttttgtg      60
   agttttttaa cgttttgtgg cgctgatcag acacttattg agaaggaatt aaccggaaga     120
   actgtttata tctccaaaat taagctaaat tggaacgatg ccttcgatta ctgcatccgc     180
   aatggcctca cctttgctaa gattaaatca gctgaagaaa acaccgaact gagtgagaaa     240
   ctcaagacag tcattcgtac ggaggagttt caagtttgga ttggaggcat tgaacatcat     300
   caagacagtt ccttccgctg ggtaagcgac tcccaaccaa taaccaacaa attgggctac     360
   aaatacacaa actggaatac cggagagccc acaaattacc aaaacaacga atattgcttg     420
   gaaatattat tccggaagga agatggaaaa tggaatgatt ttccctgcag tgcaagacat     480
   cattttgttt gtgaaaaaag aacaaaataa aatgaagaaa atgtgatttt cctttggttg     540
   aagaataaaa ttctgttgaa aaaaaaaaaa aaaaaaaaaa aaaaaa                    586
<210> 39
   <211> 105
   <212> PRT
   <213> Lutzomyia longipalpis
<400> 39
   Met Gln Asn Phe Leu Leu Val Ser Leu Ala Leu Ala Ala Leu Met Leu
   1               5                   10                  15
   Cys Ala Glu Ala Lys Pro Tyr Asp Phe Pro Leu Tyr Gln Asp Leu Ile
               20                  25                  30
   Gln Gly Val Ile Gln Arg Glu Ser Gln Ala Glu Arg Glu Lys Arg Ser
           35                  40                  45
   Pro Asn Glu Asp Tyr Glu Lys Gln Phe Gly Asp Ile Val Asp Gln Ile
       50                  55                  60
   Lys Glu Ile Ser Phe Asn Val Met Lys Met Pro His Phe Gly Ser Ser
   65                  70                  75                  80
   Asp Asp Asn Arg Asp Asp Gly Glu Tyr Val Asp His His Tyr Gly Asp
                   85                  90                  95
   Glu Asp Asp Arg Asp Tyr Asp His Tyr
               100                 105
<210> 40
   <211> 457
   <212> DNA
   <213> Lutzomyia longipalpis
<400> 40
   atttagtttg tgtttaacaa aacaagaatg cagaacttcc ttttagtttc cttggcttta     60
   gctgccttaa tgctatgtgc cgaagcaaag ccgtacgatt ttccgcttta tcaggactta    120
   attcagggcg ttattcagcg cgaaagtcaa gctgagaggg agaagagaag ccccaatgag    180
   gactatgaga agcaatttgg ggatattgtt gatcaaatta aggaaattag tttcaatgtc    240
   atgaaaatgc cccattttgg aagctctgat gataatcgtg atgatggcga gtacgttgat    300
   catcattatg gtgacgaaga tgatcgtgat tatgatcatt actaaatact acttgctcct    360
   gctgaatgac ttgaaggaat catttttttg caaaaatatc catcaaatta ttgaattaat    420
   aaagttgcaa aaaaaaaaaa aaaaaaaaaa aaaaaaa                             457
<210> 41
   <211> 157
   <212> PRT
   <213> Lutzomyia longipalpis
<400> 41
   Met Lys Phe Tyr Ile Phe Gly Val Phe Leu Val Ser Phe Leu Ala Leu
   1               5                   10                  15
   Cys Asn Ala Glu Asp Tyr Asp Lys Val Lys Leu Thr Gly Arg Thr Val
               20                  25                  30
   Tyr Ile Ser Arg Ser Lys Ala Pro Trp Phe Thr Ala Leu Asp Asn Cys
           35                  40                  45
   Asn Arg Arg Phe Thr Phe Ala Met Ile Lys Ser Gln Lys Glu Asn Glu
       50                  55                  60
   Glu Leu Thr Asn Ala Leu Leu Ser Val Ile Lys Ser Asp Glu Glu Asn
   65                  70                  75                  80
   Val Trp Ile Gly Gly Leu Arg His Asp Leu Asp Asp Tyr Phe Arg Trp
                   85                  90                  95
   Ile Ser Phe Gly Thr Ala Leu Ser Lys Thr Ser Tyr Thr Asn Trp Ala
               100                 105                 110
   Pro Lys Glu Pro Thr Gly Arg Pro His Arg Thr Gln Asn Asp Glu Phe
           115                 120                 125
   Cys Met Gln Met Ser Phe Lys Asp Gly Gly Lys Trp Ser Asp Asn Thr
       130                 135                 140
   Cys Trp Arg Lys Arg Leu Tyr Val Cys Glu Lys Arg Asp
   145                 150                 155
<210> 42
   <211> 596
   <212> DNA
   <213> Lutzomyia longipalpis
<400> 42
   gtttaaggaa tttctttcat ctcagtcttc gattttcttt aaacaaataa tgaagtttta     60
   tatttttgga gttttcctgg tgagctttct tgcattatgc aatgctgagg attatgataa    120
   agtaaaactt actggaagaa ctgtttacat ctccagatca aaggctccgt ggttcacagc    180
   tttagacaat tgtaatcgtt tacgcttcac cttcgccatg atcaagtctc agaaggagaa    240
   tgaagagcta acaaatgcgc ttttaagtgt aattaaatct gacgaagaaa atgtttggat    300
   tggaggtctt aggcacgatc tggatgacta cttccgttgg attagttttg gaactgcatt    360
   gtcaaagact tcgtacacca attgggcccc aaaggaaccc acaggaaggc cccatagaac    420
   tcaaaatgat gaattctgca tgcaaatgtc tttcaaagat ggtggcaaat ggagtgataa    480
   cacctgttgg cgtaaacgtt tgtacgtttg tgaaaagcgt gattaaataa aggaacactg    540
   ccaatgaata ttgggcaatt tgagagaaat taaattaaaa aaaaaaaaaa aaaaaa        596
<210> 43
   <211> 295
   <212> PRT
   <213> Lutzomyia longipalpis
<400> 43
   Met Ile Lys Glu Val Phe Ser Leu Ala Leu Leu Val Ala Leu Ala Gln
   1               5                   10                  15
   Cys Ala Asn Glu Ile Pro Ile Asn Arg Gln Gly Lys Asp Tyr Pro Val
               20                  25                  30
   Pro Ile Ile Asp Pro Asn Lys Ser Ser Ser Asp Asp Tyr Phe Asp Asp
           35                  40                  45
   Arg Phe Tyr Pro Asp Ile Asp Asp Glu Gly Ile Ala Glu Ala Pro Lys
       50                  55                  60
   Asp Asn Arg Gly Lys Ser Arg Gly Gly Gly Ala Ala Gly Ala Arg Glu
   65                  70                  75                  80
   Gly Arg Leu Gly Thr Asn Gly Ala Lys Pro Gly Gln Gly Gly Thr Arg
                   85                  90                  95
   Pro Gly Gln Gly Gly Thr Arg Pro Gly Gln Gly Gly Thr Arg Pro Gly
               100                 105                 110
   Gln Gly Gly Thr Arg Pro Gly Gln Gly Gly Thr Arg Pro Gly Gln Gly
           115                 120                 125
   Arg Thr Lys Pro Ala Gln Gly Thr Thr Arg Pro Ala Gln Gly Thr Arg
       130                 135                 140
   Asn Pro Gly Ser Val Gly Thr Lys Glu Ala Gln Asp Ala Ser Lys Gln
   145                 150                 155                 160
   Gly Gln Gly Lys Arg Arg Pro Gly Gln Val Gly Gly Lys Arg Pro Gly
                   165                 170                 175
   Gln Ala Asn Ala Pro Asn Ala Gly Thr Arg Lys Gln Gln Lys Gly Ser
               180                 185                 190
   Arg Gly Val Gly Arg Pro Asp Leu Ser Arg Tyr Lys Asp Ala Pro Ala
           195                 200                 205
   Lys Phe Val Phe Lys Ser Pro Asp Phe Ser Gly Glu Gly Lys Thr Pro
       210                 215                 220
   Thr Val Asn Tyr Phe Arg Thr Lys Lys Lys Glu His Ile Val Thr Arg
   225                 230                 235                 240
   Gly Ser Pro Asn Asp Glu Phe Val Leu Glu Ile Leu Asp Gly Asp Pro
                   245                 250                 255
   Thr Gly Leu Gly Leu Lys Ser Glu Thr Ile Gly Lys Asp Thr Arg Leu
               260                 265                 270
   Val Leu Glu Asn Pro Asn Gly Asn Ser Ile Val Ala Arg Val Lys Ile
   275 280 285
   Tyr Lys Asn Gly Tyr Ser Gly
   290 295
<210> 44
   <211> 989
   <212> DNA
   <213> Lutzomyia longipalpis
<400> 44
   actaaagcgt ctcaccgaaa tcagggaaaa tgattaagga agttttctct ctggctctac 60
   ttgtggcctt ggcacagtgt gctaatgaaa tccctattaa tcgtcagggg aaagattatc 120
   cagttccgat cattgatcca aataaatcat cttcggatga ttatttcgat gatcgcttct 180
   accctgatat tgatgatgag ggcatagctg aggctcctaa ggataatagg ggaaaatccc 240
   gtggtggtgg tgcggctggc gcaagagaag gtaggttagg tacgaatggg gctaaaccgg 300
   gtcagggtgg aactagacca ggacagggtg gaactaggcc aggacagggt ggaactaggc 360
   caggtcaggg tggaactagg ccaggtcagg gtggaactag acctgggcaa ggtagaacta 420
   agcctgctca gggaactact aggccagctc agggaactag aaatccagga tcggttggta 480
   cgaaagaagc ccaggatgcg tcaaaacaag gtcaaggtaa aagaaggcca gggcaagttg 540
   gtggtaaaag accaggacaa gcaaatgctc ctaatgcagg cactagaaag caacagaaag 600
   gcagtagagg cgttggaagg cctgatctat cgcgctacaa agatgcccct gctaaattcg 660
   ttttcaaatc tcccgatttc agtggagaag gcaaaactcc aactgtaaat tactttagaa 720
   cgaagaagaa ggagcacatt gtgacccgtg gtagtcctaa tgatgaattt gttctggaga 780
   ttctcgatgg ggatccaact gggcttggac taaagagtga aaccataggc aaagatacgc 840
   gtttagtgct ggagaatcct aatggaaatt ccatcgtggc tcgtgttaag atctacaaga 900
   acggttattc aggatgaaga agaaatcctt tgatttcccc ccccccctct tcctttaaaa 960
   ttcaacataa taaaaaaaaa aaaaaaaaa 989
<210> 45
   <211> 148
   <212> PRT
   <213> Lutzomyia longipalpis
<400> 45
   Met Asn Ser Val Asn Thr Leu Ile Leu Thr Leu Leu Phe Ala Ile Phe
   1 5 10 15
   Leu Leu Val Lys Arg Ser Gln Ala Phe Leu Pro Ser Asp Pro Ser Ile
   20 25 30
   Cys Val Lys Asn Leu Val Leu Asp Thr Gly Arg Thr Cys Glu Glu Ser
   35 40 45
   Glu Tyr Phe Pro Asp Ile Lys Asn Val Lys Asn Gly Lys Arg Val Tyr
   50 55 60
   Ile Val Cys Thr Asp Ser Asp Ala Val Asp Tyr Lys Phe Tyr Ile Cys
   65 70 75 80
   Phe Asp Met Asn Arg Leu Ser Gly Pro Pro Tyr Pro Glu Glu Glu Ile
   85 90 95
   Leu Arg Glu Ser Thr Val Thr Tyr Ala Gln Ile Tyr Glu Leu Met Thr
   100 105 110
   Thr Glu Thr Thr Glu Thr Lys Lys Pro Lys Lys Lys Pro Lys Asn Ser
   115 120 125
   Lys Thr Asp Asp Pro Pro Ala Ile Arg Pro Gly Phe Ser Phe Arg Asn
   130 135 140
   Ser Ile Ser Val
   145
<210> 46
   <211> 826
   <212> DNA
   <213> Lutzomyia longipalpis
<400> 46
   gtcttttcct gagtgtttca ttaacaaaat gaattcagta aacactttaa ttttaactct      60
   tctatttgca atttttttat tagtgaaaag gtctcaggct tttcttccat ctgacccaag     120
   tatctgtgtt aaaaatttag tattggatac aggaaggact tgtgaggaaa gtgaatattt     180
   tccggatatc aagaacgtta aaaatggaaa aagagtttac attgtctgca ctgattcaga     240
   tgcagttgat tataaatttt atatttgttt cgatatgaat cgtctttctg gaccaccgta     300
   tcctgaggaa gaaatccttc gtgaatcaac ggtaacttat gcccaaattt atgagctgat     360
   gactacggaa accactgaaa ccaaaaagcc aaaaaagaaa ccaaagaatt caaaaacgga     420
   cccagaccct ccagcaattc gtccaggatt ttcatttaga aattcaattt ctgtttaatt     480
   ttacaattta ttttgaaaga aaaatgatat ttcgaaatat tctatacaaa aaaacaacag     540
   ttataaaacg aaaattcaat catttcaatg agaaaactta gtcttgagta aggtttattc     600
   accacccgac gccacgctat ggtgaataat tttctttatt caccacatca aaatgacggc     660
   ttataaactt caacaaatag tttggaaaat acatttctaa ctaatgcaat gtttacttaa     720
   aatcacttta caaattcacg catttgagat gcaacaaata tatacaattc aacgatataa     780
   actttccaca aggaaaactt tcaaccaaaa aaaaaaaaaa aaaaaa                    826
<210> 47
   <211> 397
   <212> PRT
   <213> Lutzomyia longipalpis
<400> 47
   Met Lys Leu Phe Phe Phe Leu Tyr Thr Phe Gly Leu Val Gln Thr Ile
   1               5                   10                  15
   Phe Gly Val Glu Ile Lys Gln Gly Phe Lys Trp Asn Lys Ile Leu Tyr
               20                  25                  30
   Glu Gly Asp Thr Ser Glu Asn Phe Asn Pro Asp Asn Asn Ile Leu Thr
           35                  40                  45
   Ala Phe Ala Tyr Asp Pro Glu Ser Gln Lys Leu Phe Leu Thr Val Pro
       50                  55                  60
   Arg Lys Tyr Pro Glu Thr Met Tyr Thr Leu Ala Glu Val Asp Thr Glu
   65                  70                  75                  80
   Lys Asn Ser Phe Glu Ser Gly Asp Thr Ser Pro Leu Leu Gly Lys Phe
                   85                  90                  95
   Ser Gly His Glu Thr Gly Lys Glu Leu Thr Ser Val Tyr Gln Pro Val
               100                 105                 110
   Ile Asp Glu Cys His Arg Leu Trp Val Val Asp Val Gly Ser Val Glu
           115                 120                 125
   Arg Asn Ser Asp Gly Thr Glu Gly Gln Pro Glu His Asn Pro Thr Leu
       130                 135                 140
   Val Ala Tyr Asp Leu Lys Glu Ala Asn Tyr Pro Glu Val Ile Arg Tyr
   145                 150                 155                 160
   Thr Phe Pro Asp Asn Ser Ile Glu Lys Pro Thr Phe Leu Gly Gly Phe
                   165                 170                 175
   Ala Val Asp Val Val Lys Pro Asp Glu Cys Ser Glu Thr Phe Val Tyr
               180                 185                 190
   Ile Thr Asn Phe Leu Thr Asn Ala Leu Ile Val Tyr Asp His Lys Asn
           195                 200                 205
   Lys Asp Ser Trp Thr Val Gln Asp Ser Thr Phe Gly Pro Asp Lys Lys
   210 215 220
   Ser Lys Phe Asp His Asp Gly Gln Gln Tyr Glu Tyr Glu Ala Gly Ile
   225 230 235 240
   Phe Gly Ile Thr Leu Gly Glu Arg Asp Asn Glu Gly Asn Arg Gln Ala
   245 250 255
   Tyr Tyr Leu Val Ala Ser Ser Thr Lys Leu His Ser Ile Asn Thr Lys
   260 265 270
   Glu Leu Lys Gln Lys Gly Ser Lys Val Asn Ala Asn Tyr Leu Gly Asp
   275 280 285
   Arg Gly Glu Ser Thr Asp Ala Ile Gly Leu Val Tyr Asp Pro Lys Thr
   290 295 300
   Lys Thr Ile Phe Phe Val Glu Ser Asn Ser Lys Arg Val Ser Cys Trp
   305 310 315 320
   Asn Thr Gln Glu Thr Leu Asn Lys Asp Lys Ile Asp Val Ile Tyr His
   325 330 335
   Asn Ala Asp Phe Ser Phe Gly Thr Asp Ile Ser Ile Asp Ser Gln Asp
   340 345 350
   Asn Leu Trp Phe Leu Ala Asn Gly Leu Pro Pro Leu Glu Asn Ser Asp
   355 360 365
   Lys Phe Val Phe Thr Lys Pro Arg Tyr Gln Ile Phe Lys Val Asn Ile
   370 375 380
   Gln Glu Ala Ile Ala Gly Thr Lys Cys Glu Lys Asn Leu
   385 390 395
<210> 48
   <211> 1325
   <212> DNA
   <213> Lutzomyia longipalpis
<400> 48
   atcattcaaa aggcagcagc acaatgaagt tatttttctt tctttacact tttggtctag 60
   tccaaacgat ttttggagta gaaattaaac aaggatttaa atggaataaa atcctttatg 120
   agggcgatac atcagaaaac ttcaatccag ataacaacat ccttacggct tttgcgtacg 180
   atcctgagag tcagaaactc ttcctaactg tcccgaggaa atatcccgaa actatgtaca 240
   ctttggcaga agttgatact gagaaaaatt cttttgaatc gggagatact tccccgctcc     300
   ttggaaaatt cagtggtcat gaaactggga aagaacttac atcagtttat cagccagtta     360
   tcgatgaatg tcatcgtctt tgggttgttg atgttggatc agtagaacgt aactcagacg     420
   gcacagaagg tcagccagaa cataatccta cccttgtggc gtacgatctc aaagaagcca     480
   actatcctga agttattcgt tacacgtttc ccgataattc cattgagaag cccacatttc     540
   tgggtggatt tgccgttgat gttgtaaagc cggatgaatg cagtgaaact tttgtctaca     600
   tcacaaactt cctcaccaac gccctcatag tatacgatca taagaataag gactcctgga     660
   cggtacaaga ttcaactttt ggaccagata aaaagtcaaa gtttgaccac gatggacaac     720
   agtatgaata cgaagcagga atcttcggga ttacccttgg agagagagat aacgaaggaa     780
   atcgtcaagc gtactattta gtagcaagta gtaccaaact tcacagcatc aacaccaaag     840
   aactgaagca aaaaggaagc aaagttaatg caaattattt gggagatcgt ggtgaatcca     900
   ccgatgccat aggcttagtt tacgatccaa aaaccaaaac tatcttcttc gttgagtcaa     960
   atagcaaaag agtatcatgc tggaataccc aggaaacact aaacaaggat aaaattgatg    1020
   taatctatca caatgcagac ttttcctttg gaacagatat atcgattgat agtcaggata    1080
   atttgtggtt cctagcaaat ggacttccac ctctggaaaa ttctgataaa tttgtcttta    1140
   caaagccacg ttatcaaata ttcaaagtca acattcaaga agcaattgct ggaactaaat    1200
   gtgaaaagaa tctttaacaa atgaaacttt gtagaaaaat acataatatc tgaataaaaa    1260
   gtcataaatg taccataaaa aaaaaaaaaa aaaaaaaaaa aaaaaaaaaa aaaaaaaaaa    1320
   aaaaa                                                                1325
<210> 49
   <211> 350
   <212> PRT
   <213> Lutzomyia longipalpis
<400> 49
   Met Thr Phe Leu Ile Ile Leu Gly Ala Phe Leu Leu Val Gln Ile Ile
   1               5                   10                  15
   Thr Ala Ser Ala Leu Gly Leu Pro Glu Gln Phe Lys Gly Leu Glu Asp
               20                  25                  30
   Leu Pro Lys Lys Pro Leu Ala Glu Thr Tyr Tyr His Glu Gly Leu Asn
           35                  40                  45
   Asp Gly Lys Thr Asp Glu Met Val Asp Ile Phe Lys Ser Leu Ser Asp
       50                  55                  60
   Glu Phe Lys Phe Ser Asp Glu Asn Leu Asp Val Gly Glu Glu Lys Asn
   65                  70                  75                  80
   Tyr Lys Lys Arg Asp Ile Thr Gln Asn Ser Val Ala Arg Asn Phe Leu
                   85                  90                  95
   Ser Asn Val Lys Gly Ile Pro Ser Met Pro Ser Leu Pro Ser Met Pro
               100                 105                 110
   Ser Met Pro Ser Ile Pro Ser Leu Trp Ser Ser Gln Thr Gln Ala Ala
           115                 120                 125
   Pro Asn Thr Ala Leu Ala Leu Pro Glu Ser Asp Tyr Ser Leu Leu Asp
       130                 135                 140
   Met Pro Asn Ile Val Lys Asn Phe Leu Lys Glu Thr Arg Asp Leu Tyr
   145                 150                 155                 160
   Asn Asp Val Gly Ala Phe Leu Lys Ala Ile Thr Glu Ala Leu Thr Asn
                   165                 170                 175
   Arg Ser Ser Ser Ser Gln Leu Leu Ser Ser Pro Met Val Ser Thr Asn
               180                 185                 190
   Lys Thr Lys Glu Phe Ile Arg Asn Glu Ile Gln Lys Val Arg Lys Val
           195                 200                 205
   Arg Asn Phe Val Gln Glu Thr Leu Gln Lys Ile Arg Asp Ile Ser Ala
       210                 215                 220
   Ala Ile Ala Lys Lys Val Lys Ser Ser Glu Cys Leu Ser Asn Leu Thr
   225                 230                 235                 240
   Asp Ile Lys Gly Leu Val Ser Asp Gly Ile Asn Cys Leu Lys Glu Lys
                   245                 250                 255
   Phe Asn Asp Gly Lys Arg Ile Ile Leu Gln Leu Tyr Asn Asn Leu Leu
               260                 265                 270
   Lys Gly Leu Lys Ile Pro Asn Asp Leu Met Val Glu Leu Lys Lys Cys
           275                 280                 285
   Asp Thr Asn Gln Asn Asn Thr Leu Gly Arg Ile Ile Cys Tyr Phe Leu
       290                 295                 300
   Thr Pro Leu Gln Leu Glu Lys Glu Gln Ile Leu Leu Pro Val Glu Phe
   305                 310                 315                 320
   Ile Lys Arg Ile Leu Glu Leu Thr His Tyr Phe Ser Thr Met Lys Glu
                   325                 330                 335
   Asp Leu Ile Asn Cys Gly Ile Thr Thr Ile Ala Ser Ile Thr
               340                 345                 350
<210> 50
   <211> 1275
   <212> DNA
   <213> Lutzomyia longipalpis
<400> 50
   ctttaaagca aaaattttgt gggaaaggaa gttacccgga gatgacgttt ctaattatac      60
   ttggtgcatt tctccttgtt caaattatta cagcttcagc tttaggattg cctgaacagt     120
   ttaaaggttt agaggattta cctaaaaaac ctttggcaga gacttattat cacgaaggat     180
   tgaatgatgg aaaaacggat gaaatggtgg atatttttaa aagtcttagc gatgaattta     240
   aattcagtga tgaaaattta gatgttggtg aggagaaaaa ttacaagaaa cgtgatataa     300
   cccaaaattc agtggcaagg aacttcctat caaacgtaaa gggaattcct tcaatgccat     360
   cactcccttc aatgccttca atgccatcaa ttccttcact ttggtcaagt cagacacagg     420
   cggcaccaaa taccgcactt gcccttcctg aatctgatta ttcccttcta gatatgccga     480
   atattgtgaa aaatttccta aaggaaacaa gagacctcta taacgatgtt ggagcttttc     540
   ttaaggcaat tacagaagct ttaacaaata gatcttcatc atctcaactt ctttcctccc     600
   caatggtgag cacgaataaa accaaagaat ttattcggaa tgaaatacaa aaagtccgaa     660
   aagtgagaaa tttcgtccag gaaactcttc agaaaatccg agacatttct gctgctattg     720
   ccaaaaaggt aaaatcatca gaatgtctgt ccaatcttac ggacatcaaa ggacttgtat     780
   cagacggaat taattgttta aaggaaaaat tcaatgatgg aaaacgaatt atcctgcaat     840
   tgtacaataa tttactaaaa ggactcaaaa ttccaaatga cctaatggtt gaattgaaga     900
   aatgtgatac aaatcaaaac aatactttgg gaagaataat ctgttatttt ttgacaccat     960
   tgcaactgga aaaagaacaa attcttctac ctgtagaatt tataaagcgc attcttgaat    1020
   taacccacta cttttccaca atgaaagaag atcttatcaa ctgtggcatc acaacgattg    1080
   catccattac gtaaaaaatg gaaaaatgtg ccggtgaaat gcttgaaatc accaaagaaa    1140
   tttcatcgca aataacagtt ccagaataac caaattttaa tgattacttc tcaaggaaaa    1200
   tactaccaaa aggcattaat taaaacgatg ttttttataa acaatgtaag aaaaaaaaaa    1260
   aaaaaaaaaa aaaaa                                                      1275
<210> 51
   <211> 60
   <212> PRT
   <213> Lutzomyia longipalpis
<400> 51
   Met Leu Lys Ile Val Leu Phe Leu Ser Val Leu Ala Val Leu Val Ile
   1               5                   10                  15
   Cys Val Ala Ala Met Pro Gly Ser Asn Val Pro Trp His Ile Ser Arg
               20                  25                  30
   Glu Glu Leu Glu Lys Leu Arg Glu Ala Arg Lys Asn His Lys Ala Leu
           35                  40                  45
   Glu Lys Ala Ile Asp Glu Leu Ile Asp Lys Tyr Leu
       50                  55                  60
<210> 52
   <211> 413
   <212> DNA
   <213> Lutzomyia longipalpis
<400> 52
   agttaatctt ctgtcaagct acaaaaatgc ttaaaatcgt tttatttcta tcagttttgg     60
   ctgtattagt gatttgtgta gcagcaatgc caggatccaa tgttccttgg cacatttcac    120
   gagaagagct tgagaagctt cgtgaagctc gaaagaatca caaggcactc gagaaggcaa    180
   ttgatgaatt aattgacaaa tatctctgat tttgaagagc aaggaagagg aaataaacgg    240
   ccgaggaagg attttcttta gagattcttc tttttattac ttcaaaccta acttcaaaat    300
   cagtctgata tttttttaat ttgaaaaaaa tattgaaaat tttaactatt tgtgaaattt    360
   aaataaataa agaatgtcag aagcaaaaaa aaaaaaaaaa aaaaaaaaaa aaa           413
<210> 53
   <211> 120
   <212> PRT
   <213> Lutzomyia longipalpis
<400> 53
   Met Lys Phe Ser Cys Pro Val Phe Val Ala Ile Phe Leu Leu Cys Gly
   1               5                   10                  15
   Phe Tyr Arg Val Glu Gly Ser Ser Gln Cys Glu Glu Asp Leu Lys Glu
               20                  25                  30
   Glu Ala Glu Ala Phe Phe Lys Asp Cys Asn Glu Ala Lys Ala Asn Pro
           35                  40                  45
   Gly Glu Tyr Glu Asn Leu Thr Lys Glu Glu Met Phe Glu Glu Leu Lys
       50                  55                  60
   Glu Tyr Gly Val Ala Asp Thr Asp Met Glu Thr Val Tyr Lys Leu Val
   65                  70                  75                  80
   Glu Glu Cys Trp Asn Glu Leu Thr Thr Thr Asp Cys Lys Arg Phe Leu
                   85                  90                  95
   Glu Glu Ala Glu Cys Phe Lys Lys Lys Asn Ile Cys Lys Tyr Phe Pro
               100                 105                 110
   Asp Glu Val Lys Leu Lys Lys Lys
           115                 120
<210> 54
   <211> 428
   <212> DNA
   <213> Lutzomyia longipalpis
<400> 54
   aattttcacc atgaagtttt cttgcccagt tttcgttgca attttccttt tgtgcggatt      60
   ttatcgtgtt gaggggtcat cacaatgtga agaagattta aaagaagaag ctgaagcttt     120
   ctttaaggat tgcaatgaag caaaagccaa tcctggtgaa tacgagaatc tcaccaaaga     180
   agaaatgttt gaagaattga aagaatatgg agttgctgac acagacatgg agacagttta     240
   caaacttgtg gaagaatgtt ggaatgaatt aacaacaacg gattgtaaga gatttctcga     300
   agaggctgaa tgcttcaaga agaagaatat ttgtaaatat ttcccagatg aagtgaaatt     360
   gaagaagaaa taaattttta gcttgaaaaa aaaaaaaaaa aaaaaaaaaa aaaaaaaaaa     420
   aaaaaaaa                                                              428
<210> 55
   <211> 572
   <212> PRT
   <213> Lutzomyia longipalpis
<400> 55
   Met Leu Phe Phe Leu Asn Phe Phe Val Leu Val Phe Ser Ile Glu Leu
   1               5                   10                  15
   Ala Leu Leu Thr Ala Ser Ala Ala Ala Glu Asp Gly Ser Tyr Glu Ile
               20                  25                  30
   Ile Ile Leu His Thr Asn Asp Met His Ala Arg Phe Asp Gln Thr Asn
           35                  40                  45
   Ala Gly Ser Asn Lys Cys Gln Glu Lys Asp Lys Ile Ala Ser Lys Cys
       50                  55                  60
   Tyr Gly Gly Phe Ala Arg Val Ser Thr Met Val Lys Lys Phe Arg Glu
   65 70 75 80
   Glu Asn Gly Ser Ser Val Leu Phe Leu Asn Ala Gly Asp Thr Tyr Thr
   85 90 95
   Gly Thr Pro Trp Phe Thr Leu Tyr Lys Glu Thr Ile Ala Thr Glu Met
   100 105 110
   Met Asn Ile Leu Arg Pro Asp Ala Ala Ser Leu Gly Asn His Glu Phe
   115 120 125
   Asp Lys Gly Val Glu Gly Leu Val Pro Phe Leu Asn Gly Val Thr Phe
   130 135 140
   Pro Ile Leu Thr Ala Asn Leu Asp Thr Ser Gln Glu Pro Thr Met Thr
   145 150 155 160
   Asn Ala Lys Asn Leu Lys Arg Ser Met Ile Phe Thr Val Ser Gly His
   165 170 175
   Arg Val Gly Val Ile Gly Tyr Leu Thr Pro Asp Thr Lys Phe Leu Ser
   180 185 190
   Asp Val Gly Lys Val Asn Phe Ile Pro Glu Val Glu Ala Ile Asn Thr
   195 200 205
   Glu Ala Gln Arg Leu Lys Lys Glu Glu Asn Ala Glu Ile Ile Ile Val
   210 215 220
   Val Gly His Ser Gly Leu Ile Lys Asp Arg Glu Ile Ala Glu Lys Cys
   225 230 235 240
   Pro Leu Val Asp Ile Ile Val Gly Gly His Ser His Thr Phe Leu Tyr
   245 250 255
   Thr Gly Ser Gln Pro Asp Arg Glu Val Pro Val Asp Val Tyr Pro Val
   260 265 270
   Val Val Thr Gln Ser Ser Gly Lys Lys Val Pro Ile Val Gln Ala Tyr
   275 280 285
   Cys Phe Thr Lys Tyr Leu Gly Tyr Phe Lys Val Thr Ile Asn Gly Lys
   290 295 300
   Gly Asn Val Val Gly Trp Thr Gly Gln Pro Ile Leu Leu Asn Asn Asn
   305 310 315 320
   Ile Pro Gln Asp Gln Glu Val Leu Thr Ala Leu Glu Lys Tyr Arg Glu
                   325                 330                 335
   Arg Val Glu Asn Tyr Gly Asn Arg Val Ile Gly Val Ser Arg Val Ile
               340                 345                 350
   Leu Asn Gly Gly His Thr Glu Cys Arg Phe His Glu Cys Asn Met Gly
           355                 360                 365
   Asn Leu Ile Thr Asp Ala Phe Val Tyr Ala Asn Val Ile Ser Thr Pro
       370                 375                 380
   Met Ser Thr Asn Ala Trp Thr Asp Ala Ser Val Val Leu Tyr Gln Ser
   385                 390                 395                 400
   Gly Gly Ile Arg Ala Pro Ile Asp Pro Arg Thr Ala Ala Gly Ser Ile
                   405                 410                 415
   Thr Arg Leu Glu Leu Asp Asn Val Leu Pro Phe Gly Asn Ala Leu Tyr
               420                 425                 430
   Val Val Lys Val Pro Gly Asn Val Leu Arg Lys Ala Leu Glu His Ser
           435                 440                 445
   Val His Arg Tyr Ser Asn Thr Ser Gly Trp Gly Glu Phe Pro Gln Val
       450                 455                 460
   Ser Gly Leu Lys Ile Arg Phe Asn Val Asn Glu Glu Ile Gly Lys Arg
   465                 470                 475                 480
   Val Lys Ser Val Lys Val Leu Cys Ser Asn Cys Ser Gln Pro Glu Tyr
                   485                 490                 495
   Gln Pro Leu Arg Asn Lys Lys Thr Tyr Asn Val Ile Met Asp Ser Phe
               500                 505                 510
   Met Lys Asp Gly Gly Asp Gly Tyr Ser Met Phe Lys Pro Leu Lys Ile
           515                 520                 525
   Ile Lys Thr Leu Pro Leu Gly Asp Ile Glu Thr Val Glu Ala Tyr Ile
       530                 535                 540
   Glu Lys Met Gly Pro Ile Phe Pro Ala Val Glu Gly Arg Ile Thr Val
   545                 550                 555                 560
   Leu Gly Gly Leu Gln Lys Ser Asp Glu Asp Trp His
                   565                 570
<210> 56
   <211> 1839
   <212> DNA
   <213> Lutzomyia longipalpis
<400> 56
   agttgcaaga atttcttcat tgcgttaaga tgttgttttt ccttaacttt tttgtgctgg     60
   tgttcagcat agaactggcg ttgttaacag catcagcagc agcagaagac ggcagctatg    120
   agatcataat tcttcacacc aatgatatgc acgcgcgttt tgatcaaacc aatgctggaa    180
   gcaacaaatg ccaagaaaaa gacaagattg cttccaaatg ctacggagga tttgcaagag    240
   tttcaacaat ggtgaaaaaa ttccgagaag aaaatggcag cagtgtcttg ttcttgaatg    300
   ctggtgacac gtatacaggt accccatggt ttaccctcta caaggagacc attgcaacgg    360
   agatgatgaa catccttcgt ccagatgcag cctcactggg aaatcatgaa ttcgacaaag    420
   gagtagaagg actcgtgcca ttcctcaatg gtgtcacctt ccctatttta acagcgaatt    480
   tggacacttc tcaagagcca acaatgacca atgctaaaaa tctcaaacgc tcaatgattt    540
   ttacggtttc cgggcacaga gttggtgtaa ttggctacct aacgcctgat acaaaattcc    600
   tctcggacgt tggtaaagtt aattttattc cggaagttga agccatcaat acggaagcac    660
   agcgtctgaa gaaagaggaa aatgccgaaa taatcatcgt tgttggacat tcagggttga    720
   taaaagatcg agaaattgca gagaaatgcc cactggttga cataattgtt ggaggacatt    780
   cacacacatt cctctacaca ggaagtcagc ctgatcgtga ggttcctgta gacgtttatc    840
   ctgttgttgt gacccaatcc agtgggaaga aagttccaat tgttcaagcc tattgcttta    900
   caaagtattt ggggtacttt aaagtgacga tcaacggaaa aggaaatgtt gtgggatgga    960
   ctgggcagcc aattctcctt aataacaaca ttccccaaga tcaggaagtt ctcactgctc   1020
   ttgaaaagta cagagaacgc gtggaaaact atggaaatcg cgtaattgga gtttcccgtg   1080
   taattctcaa tggggggcat actgaatgtc gtttccatga atgcaatatg ggtaatctca    1140
   tcacggacgc ttttgtgtat gccaatgtaa tcagtacacc aatgagtacg aatgcctgga   1200
   cagatgcaag tgttgttctg tatcaaagtg gtggcattcg tgccccaatt gatcctcgta   1260
   ccgcggcagg gagcatcaca cgcctcgagt tggacaatgt tctaccattt gggaatgcac   1320
   tgtacgtcgt aaaagttcct gggaatgtct tacgcaaagc tttggaacat tcagttcatc   1380
   gatactccaa cacttcggga tggggagaat ttccacaagt ttcggggcta aagattcgtt   1440
   ttaacgtcaa tgaagaaatt ggaaaacgcg taaagtccgt taaagttctc tgtagcaatt   1500
   gctctcaacc tgaataccaa ccactgagaa ataaaaaaac ttacaacgtt atcatggaca   1560
   gttttatgaa ggatggaggt gatgggtata gcatgttcaa gcccttgaag atcatcaaga   1620
   ccctcccact gggagatatt gaaacagtag aagcttatat tgagaaaatg ggccccattt   1680
   tcccagcagt cgagggaagg atcactgttc ttgggggact tcaaaaatca gatgaggatt   1740
   ggcattagaa acatcctgga cgttatggaa agaataaaag aaggatcata gaaaaaaaaa   1800
   aaaaaaaaat aaaaaaaaaa aaaaaaaaaa aaaaaaaaa                          1839
<210> 57
   <211> 86
   <212> PRT
   <213> Lutzomyia longipalpis
<400> 57
   Met Lys Gln Ile Leu Leu Ile Ser Leu Val Val Ile Leu Ala Val Leu
   1               5                   10                  15
   Ala Phe Asn Val Ala Glu Gly Cys Asp Ala Thr Cys Gln Phe Arg Lys
               20                  25                  30
   Ala Ile Glu Asp Cys Lys Lys Lys Ala Asp Asn Ser Asp Val Leu Gln
           35                  40                  45
   Thr Ser Val Gln Thr Thr Ala Thr Phe Thr Ser Met Asp Thr Ser Gln
       50                  55                  60
   Leu Pro Gly Asn Asn Val Phe Lys Ala Cys Met Lys Glu Lys Ala Lys
   65                  70                  75                  80
   Glu Phe Arg Ala Gly Lys
                   85
<210> 58
   <211> 419
   <212> DNA
   <213> Lutzomyia longipalpis
<400> 58
   gtcagtgatc tgataagtta ttaaaatgaa gcaaatcctt ctaatctctt tggtggtgat     60
   tcttgccgtg cttgccttca atgttgctga gggctgtgat gcaacatgcc aatttcgcaa    120
   agccatagaa gactgcaaga agaaggcgga taatagcgat gttttgcaga cttctgtaca    180
   aacaactgca acattcacat caatggatac atcccaacta cctggaaata atgtcttcaa    240
   agcatgcatg aaggagaagg ctaaggaatt tagggcagga aagtaagaga ttgaggaaaa    300
   ttgtagccga agagagaagg aaggaaagtc ccatattttg tttgttaatt gtaacgaatt    360
   ttgcgaaaaa aataaaatat tatgcactcc aaaaaaaaaa aaaaaaaaaa aaaaaaaaa     419
<210> 59
   <211> 84
   <212> **PRT**
   <213> Lutzomyia longipalpis
<400> 59
   Met Asn Val Leu Phe Val Ser Phe Thr Leu Thr Ile Leu Leu Leu Cys
   1 5 10 15
   Val Lys Ala Arg Pro Glu Asp Phe Val Ala Leu Gln Asp Gln Ala Asn
   20 25 30
   Phe Gln Lys Cys Leu Glu Gln Tyr Pro Glu Pro Asn Gln Ser Gly Glu
   35 40 45
   Val Leu Ala Cys Leu Lys Lys Arg Glu Gly Ala Lys Asp Phe Arg Glu
   50 55 60
   Lys Arg Ser Leu Asp Asp Ile Glu Gly Thr Phe Gln Glu Ser Gly Asn
   65 70 75 80
   Leu Trp Gly Ala
<210> 60
   <211> 429
   <212> **DNA**
   <213> Lutzomyia longipalpis
<400> 60
   tatttttaat aattctgtgt aaaatgaacg ttcttttcgt gtctttcacg ctcacaattc 60
   ttcttctctg tgttaaggca cggccagaag atttcgtagc tcttcaggat caagctaatt 120
   tccagaaatg cctcgaacaa tatccagaac caaatcaatc tggagaagtt cttgcgtgcc 180
   tcaagaagcg cgaaggtgcc aaagatttcc gggaaaagag gagcctggat gacatagaag 240
   ggactttcca agagtctgga aatctctggg gtgcatagga agctcagagg acttctaatc 300
   aatctgtgag aagagaaccc aacggctaga gaaaatttaa ggaaaataaa gaaattaatg 360
   aagcattaaa aaaaaaaaaa aaaaaaaaaa aaaaaaaaaa aaaaaaaaaa aaaaaaaaaa 420
   aaaaaaaaa 429
<210> 61
   <211> 626
   <212> PRT
   <213> Lutzomyia longipalpis
<400> 61
   Met Lys Ile Thr Val Ile Leu Phe Thr Gly Phe Thr Ile Ala Leu Val
   1 5 10 15
   Ser Ser Ala Val Leu Lys Lys Asn Gly Glu Thr Ile Glu Glu Glu Glu
   20 25 30
   Val Arg Ala Glu Gln Arg Leu Arg Glu Ile Asn Glu Glu Leu Asp Arg
   35 40 45
   Arg Lys Asn Ile Asn Thr Val Ala Ala Trp Ala Tyr Ala Ser Asn Ile
   50 55 60
   Thr Glu Val Asn Leu Lys Asn Met Asn Asp Val Ser Val Glu Thr Ala
   65 70 75 80
   Lys Tyr Tyr Lys Glu Leu Ala Ser Glu Leu Lys Gly Phe Asn Ala Lys
   85 90 95
   Glu Tyr Lys Ser Glu Asp Leu Lys Arg Gln Ile Lys Lys Leu Ser Lys
   100 105 110
   Leu Gly Tyr Ser Ala Leu Pro Ser Glu Lys Tyr Lys Glu Leu Leu Glu
   115 120 125
   Ala Ile Thr Trp Met Glu Ser Asn Tyr Ala Lys Val Lys Val Cys Ser
   130 135 140
   Tyr Lys Asp Pro Lys Lys Cys Asp Leu Ala Leu Glu Pro Glu Ile Thr
   145 150 155 160
   Glu Ile Leu Ile Lys Ser Arg Asp Pro Glu Glu Leu Lys Tyr Tyr Trp
   165 170 175
   Lys Gln Trp Tyr Asp Lys Ala Gly Thr Pro Thr Arg Glu Ser Phe Asn
   180 185 190
   Lys Tyr Val Gln Leu Asn Arg Glu Ala Ala Lys Leu Asp Gly Phe Tyr
   195 200 205
   Ser Gly Ala Glu Ser Trp Leu Asp Glu Tyr Glu Asp Glu Thr Phe Glu
   210 215 220
   Lys Gln Leu Glu Asp Ile Phe Ala Gln Ile Arg Pro Leu Tyr Glu Gln
   225 230 235 240
   Leu His Ala Tyr Val Arg Phe Lys Leu Arg Glu Lys Tyr Gly Asn Asp
   245 250 255
   Val Val Ser Glu Lys Gly Pro Ile Pro Met His Leu Leu Gly Asn Met
   260 265 270
   Trp Gly Gln Thr Trp Ser Glu Val Ala Pro Ile Leu Val Pro Tyr Pro
   275 280 285
   Glu Lys Lys Leu Leu Asp Val Thr Asp Glu Met Val Lys Gln Gly Tyr
   290 295 300
   Thr Pro Ile Ser Met Phe Glu Lys Gly Asp Glu Phe Phe Gln Ser Leu
   305 310 315 320
   Asn Met Thr Lys Leu Pro Lys Thr Phe Trp Glu Tyr Ser Ile Leu Glu
   325 330 335
   Lys Pro Gln Asp Gly Arg Glu Leu Ile Cys His Ala Ser Ala Trp Asp
   340 345 350
   Phe Tyr Thr Lys Asp Asp Val Arg Lys Gln Cys Thr Arg Val Thr Met
   355 360 365
   Asp Gln Phe Phe Thr Ala His His Glu Leu Gly His Ile Gln Tyr Tyr
   370 375 380
   Leu Gln Tyr Gln His Leu Pro Ser Val Tyr Arg Glu Gly Ala Asn Pro
   385 390 395 400
   Gly Phe His Glu Ala Val Gly Asp Val Leu Ser Leu Ser Val Ser Ser
   405 410 415
   Pro Lys His Leu Glu Lys Val Gly Leu Leu Lys Asp Phe Lys Phe Asp
   420 425 430
   Glu Glu Ser Gln Ile Asn Gln Leu Leu Asn Leu Ala Leu Asp Lys Met
   435 440 445
   Ala Phe Leu Pro Phe Ala Tyr Thr Ile Asp Lys Tyr Arg Trp Gly Val
   450 455 460
   Phe Arg Gly Glu Ile Ser Pro Ser Glu Tyr Asn Cys Lys Phe Trp Glu
   465 470 475 480
   Met Arg Ser Tyr Tyr Gly Gly Ile Glu Pro Pro Ile Ala Arg Ser Glu
   485 490 495
   Ser Asp Phe Asp Pro Pro Ala Lys Tyr His Ile Ser Ser Asp Val Glu
   500 505 510
   Tyr Leu Arg Tyr Leu Val Ser Phe Ile Ile Gln Phe Gln Phe His Gln
   515 520 525
   Ala Val Cys Gln Lys Thr Gly Gln Phe Val Pro Asn Asp Pro Glu Lys
       530                 535                 540
   Thr Leu Leu Asn Cys Asp Ile Tyr Gln Ser Ala Glu Ala Gly Asn Ala
   545                 550                 555                 560
   Phe Lys Glu Met Leu Lys Leu Gly Ser Ser Lys Pro Trp Pro Asp Ala
                   565                 570                 575
   Met Glu Ile Leu Thr Gly Gln Arg Lys Met Asp Ala Ser Ala Leu Ile
               580                 585                 590
   Glu Tyr Phe Arg Pro Leu Ser Glu Trp Leu Gln Lys Lys Asn Lys Glu
           595                 600                 605
   Leu Gly Ala Tyr Val Gly Trp Asp Lys Ser Thr Lys Cys Val Lys Asn
       610                 615                 620
   Val Ser
   625
<210> 62
   <211> 2121
   <212> DNA
   <213> Lutzomyia longipalpis
<400> 62
   gtatatcaag tatcattcaa gtgaatcatt ggctccgtaa tttgtacaaa agaaaaaaaa 60
   agttgataaa atcatgaaaa tcactgtgat tttattcacg ggatttacaa ttgccctcgt 120
   gagtagtgct gtgcttaaga aaaacggtga aactattgaa gaagaagaag taagagctga 180
   gcaacgactt agagagatca atgaggaact tgatcgtagg aagaatatca atactgtagc 240
   cgcttgggct tatgcatcca atattactga ggtcaatctc aagaacatga atgatgtgtc 300
   ggttgaaacc gcgaaatact acaaggaact tgcatctgaa ttgaagggat tcaatgccaa 360
   ggaatacaag agtgaggatc tgaagagaca aattaagaag ctaagcaagt tgggatatag 420
   tgctttacca tctgagaagt ataaggagct tttggaagct atcacatgga tggaatcgaa 480
   ttatgcaaaa gtgaaagttt gctcatacaa ggatccaaag aaatgtgatt tagcacttga 540
   acctgaaatt acggaaatcc ttattaaaag tcgagatcct gaggaactta aatattattg 600
   gaaacaatgg tacgacaaag ctggcacacc aactcgagag agttttaata agtatgtaca 660
   actaaatcgt gaagcagcga aattggatgg attttattcg ggtgcagaat cttggcttga 720
   tgaatatgaa gatgagacat ttgagaaaca acttgaggat atcttcgccc aaattcgccc 780
   actgtacgag caactccatg cttatgttag attcaagctg agggaaaagt atggaaatga 840
   cgttgtttcg gagaaaggtc ccattccaat gcatctcttg gggaacatgt ggggtcaaac     900
   gtggagtgaa gttgccccaa ttttagtccc ataccccgaa aagaagctcc tcgatgttac     960
   cgatgagatg gttaagcagg gatacacacc aatttctatg tttgaaaaag gagacgaatt    1020
   tttccaaagc ttgaatatga cgaaacttcc aaaaaccttc tgggagtaca gtattttgga    1080
   aaaaccccaa gatggtaggg aattgatctg ccatgcaagt gcatgggact tctatacaaa    1140
   ggatgatgta aggattaaac agtgtaccag agttacaatg gatcaattct tcacggctca    1200
   tcatgagctt ggtcacattc aatattattt gcaatatcaa catttgccga gtgtttacag    1260
   agaaggtgcc aatccaggct ttcacgaggc tgttggggat gttctctctc tttcggtatc    1320
   aagtcctaaa catttggaaa aagttggttt gcttaaagac ttcaaatttg atgaagaatc    1380
   ccagataaat caacttctaa atttagctct ggataaaatg gcattcctcc catttgccta    1440
   taccattgat aaatatcgct ggggtgtgtt tcggggtgaa atttcgccgt ctgagtacaa    1500
   ttgcaaattt tgggaaatgc gttcctacta tggtggtata gaaccaccaa ttgcacgttc    1560
   tgagagtgat tttgatccac cagcaaaata tcatatttca tcggatgttg agtacctcag    1620
   gtatttggtt tccttcatta ttcagttcca attccatcaa gctgtgtgcc aaaagactgg    1680
   tcagttcgta ccgaatgatc cggagaagac tcttctaaat tgtgacatct accagagtgc    1740
   tgaggctggt aatgccttca aagaaatgct caaattggga tcctcaaaac catggccaga    1800
   tgcaatggaa attcttacgg ggcaaaggaa aatggatgct tctgcattaa ttgagtactt    1860
   ccgtccactc agtgagtggt tgcagaagaa gaataaggaa ctaggagctt atgttggctg    1920
   ggacaaatct actaagtgtg tcaaaaacgt cagttaattt tttgtgagcc ctaaaaaata    1980
   ttcataacat ttcaatatga caaaatatat gattttcgtg aaaactaagc atgagtaagt    2040
   tttttttgtg aatttttagc agtttcattt cagaataaac gtcaaatttt taaaaaaaaa    2100
   aaaaaaaaaa aaaaaaaaaa a                                              2121
<210> 63
   <211> 42
   <212> **PRT**
   <213> Lutzomyia longipalpis
<400> 63
   Met Lys Thr Phe Ala Leu Ile Phe Leu Ala Leu Ala Val Phe Val Leu
   1               5                   10                  15
   Cys Ile Asp Gly Ala Pro Thr Phe Val Asn Leu Leu Asp Asp Val Gln
               20                  25                  30
   Glu Glu Val Glu Val Asn Thr Tyr Glu Pro
           35                  40
<210> 64
   <211> 463
   <212> DNA
   <213> Lutzomyia longipalpis
<400> 64
   tcagttagtt gactaacaaa ccacaataga gacactaaaa tgaagacatt cgccttaatc      60
   ttcttggctc ttgctgtttt tgtgctctgc attgacggag ctccaacttt tgtgaattta     120
   ctggacgacg tacaggaaga ggtagaagtt aatacgtatg agccttagga agaaaatgtt     180
   tgaggagttt caggcagagg cagagctttc ccagagaggg agcttttgcc ttgctgtaga     240
   tttttaaaaa tgaatcaatt tgattggagc aattacgcta tatttgtggg aatatttttg     300
   aattaaaaac taattatgga aattaatata taattttcag aatttcaata aattcatcaa     360
   aattgtatta attaaaaaat attgtatgaa attcccaata aaagctttca aattaaaaaa     420
   aaaaaaaaaa aaaaaaaaaa aaaaaaaaaa aaaaaaaaaa aaa                       463
<210> 65
   <211> 139
   <212> PRT
   <213> Lutzomyia longipalpis
<400> 65
   Met Asn His Leu Cys Phe Ile Ile Ile Ala Leu Phe Phe Leu Val Gln
   1               5                   10                  15
   Gln Ser Leu Ala Glu His Pro Glu Glu Lys Cys Ile Arg Glu Leu Ala
               20                  25                  30
   Arg Thr Asp Glu Asn Cys Ile Leu His Cys Thr Tyr Ser Tyr Tyr Gly
           35                  40                  45
   Phe Val Asp Lys Asn Phe Arg Ile Ala Lys Lys His Val Gln Lys Phe
       50                  55                  60
   Lys Lys Ile Leu Val Thr Phe Gly Ala Val Pro Lys Lys Glu Lys Lys
   65                  70                  75                  80
   Lys Leu Leu Glu His Ile Glu Ala Cys Ala Asp Ser Ala Asn Ala Asp
                   85                  90                  95
   Gln Pro Gln Thr Lys Asp Glu Lys Cys Thr Lys Ile Asn Lys Tyr Tyr
               100                 105                 110
   Arg Cys Val Val Asp Gly Lys Ile Leu Pro Trp Asn Ser Tyr Ala Asp
           115                 120                 125
   Ala Ile Ile Lys Phe Asp Lys Thr Leu Asn Val
       130                 135
<210> 66
   <211> 579
   <212> DNA
   <213> Lutzomyia longipalpis
<400> 66
   ggccattatg gccggggata gaacttaatt gttgttaaaa tgaatcactt gtgctttatt      60
   attattgctc tattcttttt ggttcaacaa tctttggctg aacatccaga agaaaaatgt     120
   attagagaat tggcgagaac tgatgaaaac tgcattcttc attgtacgta ttcgtactac     180
   ggattcgttg ataaaaattt caggatcgct aaaaaacatg ttcaaaaatt caaaaaaatc     240
   ctagttacat tcggcgctgt tcctaagaaa gaaaaaaaga aacttttaga gcacattgag     300
   gcttgtgcgg attctgcgaa tgctgatcaa cctcaaacta aagatgaaaa atgtacaaaa     360
   ataaataagt actatcgttg tgttgtggat ggaaaaatat taccctggaa tagttatgct     420
   gatgcaatca ttaagtttga taaaaccctt aacgtatgaa gcaaagatat tcgaaaaaaa     480
   aacatcaaga ttatgctgga aagaaaaaaa taaaaaaaaa ttgtgctaat caaattgaat     540
   taacgcttaa tgctatatta aaaaaaaaaa aaaaaaaaa                            579
<210> 67
   <211> 446
   <212> PRT
   <213> Lutzomyia longipalpis
<400> 67
   Met Lys Ile Ile Phe Leu Ala Ala Phe Leu Leu Ala Asp Gly Ile Trp
   1               5                   10                  15
   Ala Ala Glu Glu Pro Ser Val Glu Ile Val Thr Pro Gln Ser Val Arg
               20                  25                  30
   Arg His Ala Thr Pro Lys Ala Gln Asp Ala Arg Val Gly Ser Glu Ser
           35                  40                  45
   Ala Thr Thr Ala Pro Arg Pro Ser Glu Ser Met Asp Tyr Trp Glu Asn
       50                  55                  60
   Asp Asp Phe Val Pro Phe Glu Gly Pro Phe Lys Asp Ile Gly Glu Phe
   65                  70                  75                  80
   Asp Trp Asn Leu Ser Lys Ile Val Phe Glu Glu Asn Lys Gly Asn Ala
                   85                  90                  95
   Ile Leu Ser Pro Leu Ser Val Lys Leu Leu Met Ser Leu Leu Phe Glu
               100                 105                 110
   Ala Ser Ala Ser Gly Thr Leu Thr Gln His Gln Leu Arg Gln Ala Thr
           115                 120                 125
   Pro Thr Ile Val Thr His Tyr Gln Ser Arg Glu Phe Tyr Lys Asn Ile
       130                 135                 140
   Phe Asp Gly Leu Lys Lys Lys Ser Asn Asp Tyr Thr Val His Phe Gly
   145                 150                 155                 160
   Thr Arg Ile Tyr Val Asp Gln Phe Val Thr Pro Arg Gln Arg Tyr Ala
                   165                 170                 175
   Ala Ile Leu Glu Lys His Tyr Leu Thr Asp Leu Lys Val Glu Asp Phe
               180                 185                 190
   Ser Lys Ala Lys Glu Thr Thr Gln Ala Ile Asn Ser Trp Val Ser Asn
           195                 200                 205
   Ile Thr Asn Glu His Ile Lys Asp Leu Val Lys Glu Glu Asp Val Gln
       210                 215                 220
   Asn Ser Val Met Leu Met Leu Asn Ala Val Tyr Phe Arg Gly Leu Trp
   225                 230                 235                 240
   Arg Lys Pro Phe Asn Arg Thr Leu Pro Leu Pro Phe His Val Ser Ala
                   245                 250                 255
   Asp Glu Ser Lys Thr Thr Asp Phe Met Leu Thr Asp Gly Leu Tyr Tyr
               260                 265                 270
   Phe Tyr Glu Ala Lys Glu Leu Asp Ala Lys Ile Leu Arg Ile Pro Tyr
           275                 280                 285
   Lys Gly Lys Gln Tyr Ala Met Thr Val Ile Leu Pro Asn Ser Lys Ser
       290                 295                 300
   Gly Ile Asp Ser Phe Val Arg Gln Ile Asn Thr Val Leu Leu His Arg
   305                 310                 315                 320
   Ile Lys Trp Leu Met Asp Glu Val Glu Cys Arg Val Ile Leu Pro Lys
                   325                 330                 335
   Phe His Phe Asp Met Thr Asn Glu Leu Lys Glu Ser Leu Val Lys Leu
               340                 345                 350
   Gly Ile Ser Gln Ile Phe Thr Ser Glu Ala Ser Leu Pro Ser Leu Ala
           355                 360                 365
   Arg Gly Gln Gly Val Gln Asn Arg Leu Gln Val Ser Asn Val Ile Gln
       370                 375                 380
   Lys Ala Gly Ile Ile Val Asp Glu Lys Gly Ser Thr Ala Tyr Ala Ala
   385                 390                 395                 400
   Ser Glu Val Ser Leu Val Asn Lys Phe Gly Asp Asp Glu Phe Val Met
                   405                 410                 415
   Phe Asn Ala Asn His Pro Phe Leu Phe Thr Ile Glu Asp Glu Thr Thr
               420                 425                 430
   Gly Ala Ile Leu Phe Thr Gly Lys Val Val Asp Pro Thr Gln
           435                 440                 445
<210> 68
   <211> 1651
   <212> DNA
   <213> Lutzomyia longipalpis
<220>
   <221> misc_feature
   <222> (1636)..(1636)
   <223> n is a, c, g, or t
<400> 68
   gtcggagatc gtctgccttg atgatcacat cgtgattgtg agttacaaga gtgaaacttt      60
   ttaagtgtgt gtgtcttagc aaagtgattt ccacaatgaa gattattttt ttagccgctt     120
   ttctactagc ggatggtatt tgggctgctg aagaaccttc agtggaaatt gtaacaccac     180
   aatcagtgcg gagacacgct acgccaaaag cccaggacgc gagggtagga agtgaatccg     240
   caacaacagc accaagacca agtgaatcaa tggattactg ggagaatgat gatttcgtcc     300
   catttgaggg tccattcaag gatattggag aattcgactg gaacctttcg aagatcgttt     360
   ttgaggaaaa caaaggtaat gccatcttgt cgccactctc tgtgaagcta ctaatgagtt     420
   tgctcttcga ggccagtgcg tcaggtacct tgacccagca ccaactcaga caagccactc     480
   ccaccatcgt cacccactat cagtctcgag aattttacaa gaatatcttt gacggtctca     540
   agaaaaagag taacgactac acggttcact ttggtacgag aatctacgtg gatcagtttg     600
   tgacgcctcg ccagagatat gctgccattt tggagaagca ttatctgact gatctcaaag     660
   ttgaggactt ctcgaaggca aaagaaacaa ctcaggcaat caatagttgg gtgtcaaaca     720
   tcacaaatga gcacataaag gatctcgtga aggaggaaga tgttcagaat tcagttatgc     780
   tcatgcttaa tgcagtctac ttccgcggac tctggcgcaa gcctttcaat cgtacactcc     840
   cactgccctt ccacgtgagc gctgatgagt ccaagacgac tgattttatg ctaaccgatg     900
   ggctctacta cttctacgag gcaaaggaat tggatgctaa gatcctcaga attccttaca     960
   aaggtaaaca atacgcaatg actgtgatct taccaaattc caagagtggc attgatagct    1020
   ttgtgcgtca gattaacacg gtcctcctgc acaggattaa gtggttgatg gatgaagtgg    1080
   agtgcagggt tattctaccc aagttccact ttgacatgac gaatgagctg aaggaatcgc    1140
   tcgtaaagtt gggcatcagt cagattttca catcagaggc atctttgcca tcattagcac    1200
   gaggacaggg cgtacagaat cgtctgcagg tgtctaatgt gattcagaag gcgggaataa    1260
   ttgtggatga gaagggcagc acagcctatg ctgcgtcaga agtgagccta gtcaacaagt    1320
   ttggagatga tgagttcgtc atgttcaacg ctaatcatcc attcctcttt acaattgagg    1380
   acgaaaccac cggcgcaatc ctatttacgg gaaaagtcgt cgatcccacg caatagggaa    1440
   tgaaaagcat ttcatcgtat acaacttttt ttttaattaa ttattcctca ttgaaggaca    1500
   ttaatagagc atcttctcag gaaggcactc ctgacttatt tttactaaat gtgatccttg    1560
   gacacataaa aaaaacagct gtactttcta ctttttataa tatacgacca tatttgtgag    1620
   gaaaaaaaaa aaaaanaaaa aaaaaaaaaa a                                   1651
<210> 69
   <211> 166
   <212> PRT
   <213> Lutzomyia longipalpis
<400> 69
   Met Arg Phe Leu Leu Leu Ala Phe Ser Val Ala Leu Val Leu Ser Pro
   1               5                   10                  15
   Thr Phe Ala Lys Pro Gly Leu Trp Asp Ile Val Thr Gly Ile Asn Asp
               20                  25                  30
   Met Val Lys Asn Thr Ala Asn Ala Leu Lys Asn Arg Leu Thr Thr Ser
           35                  40                  45
   Val Thr Leu Phe Thr Asn Thr Ile Thr Glu Ala Ile Lys Asn Ala Asn
       50                  55                  60
   Ser Ser Val Ser Glu Leu Leu Gln Gln Val Asn Glu Thr Leu Thr Asp
   65                  70                  75                  80
   Ile Ile Asn Gly Val Gly Gln Val Gln Ser Ala Phe Val Asn Ser Ala
                   85                  90                  95
   Gly Asn Val Val Val Gln Ile Val Asp Ala Ala Gly Asn Val Leu Glu
               100                 105                 110
   Val Val Val Asp Glu Ala Gly Asn Ile Val Glu Val Ala Gly Thr Ala
           115                 120                 125
   Leu Glu Thr Ile Ile Pro Leu Pro Gly Val Val Ile Gln Lys Ile Ile
       130                 135                 140
   Asp Ala Leu Gln Gly Asn Ala Gly Thr Thr Ser Asp Ser Ala Ser Ser
   145                 150                 155                 160
   Thr Val Pro Gln Gln Ser
                   165
<210> 70
   <211> 739
   <212> DNA
   <213> Lutzomyia longipalpis
<400> 70
   tcagttaagc agattttcaa gctaaagaaa cttaactaag atgcgattcc ttcttttggc      60
   cttctccgtt gctttggtgc tttcaccaac attcgccaaa ccaggtcttt gggacattgt     120
   aactggtatt aatgatatgg taaaaaatac tgcgaatgca ctcaaaaatc gtctaacaac     180
   ttctgtgaca ttattcacaa ataccatcac cgaagctata aaaaatgcaa attcttctgt     240
   ttcggaactc cttcagcaag tcaatgaaac ccttacggat attattaatg gtgtaggaca     300
   agtgcagagt gcctttgtga attcagctgg aaatgttgtt gtgcaaattg ttgatgccgc     360
   tggaaatgtt ttggaagttg ttgttgatga ggctggaaat atcgtggagg tagctggaac     420
   agcattggaa actatcattc cactgcccgg tgtagtgatt cagaagataa ttgatgctct     480
   ccaaggaaat gcagggacta catcggattc agcttcatca actgtgcccc aacaatctta     540
   actacaaccg caatgatgtt gtctttaacg gagaattttt aaatttgaat atcaaaatcc     600
   aagatgaaat attcagattt ttcaatcaat atgatacgaa attttgaaat tatttttccg     660
   actaaagcaa tttgtaaaag gaaaaccaaa taaatatttg aaattgtaaa gaaaaaaaaa     720
   aaaaaaaaaa aaaaaaaaa                                                  739
<210> 71
   <211> 109
   <212> PRT
   <213> Lutzomyia longipalpis
<400> 71
   Met Val Lys Tyr Ser Cys Leu Val Leu Val Ala Ile Phe Leu Leu Ala
   1               5                   10                  15
   Gly Pro Tyr Gly Val Val Gly Ser Cys Glu Asn Asp Leu Thr Glu Ala
               20                  25                  30
   Ala Lys Tyr Leu Gln Asp Glu Cys Asn Ala Gly Glu Ile Ala Asp Glu
           35                  40                  45
   Phe Leu Pro Phe Ser Glu Glu Glu Val Gly Glu Ala Leu Ser Asp Lys
       50                  55                  60
   Pro Glu Asn Val Gln Glu Val Thr Asn Ile Val Arg Gly Cys Phe Glu
   65                  70                  75                  80
   Ala Glu Gln Ala Lys Glu His Gly Lys Cys Glu Arg Phe Ser Ala Leu
                   85                  90                  95
   Ser Gln Cys Tyr Ile Glu Lys Asn Leu Cys Gln Phe Phe
               100                 105
<210> 72
   <211> 447
   <212> DNA
   <213> Lutzomyia longipalpis
<400> 72
   atatcaattt tatcatcatg gtgaagtact cgtgtcttgt tcttgttgca atttttcttc     60
   tggccggacc ctacggcgtt gtaggttctt gtgagaatga cctgacagag gccgccaagt    120
   atcttcaaga tgaatgcaat gcaggtgaaa ttgcagatga atttctaccc ttctctgaag    180
   aagaagtggg tgaagcattg agcgacaaac cagaaaacgt gcaggaagtc accaacatcg    240
   tgagaggatg ctttgaagct gaacaagcca aagagcatgg aaaatgtgaa agattttccg    300
   ctttgagtca atgctacatt gaaaagaatt tatgtcaatt cttctaaaat attttgaaga    360
   aaagttatga atgaaaattt tctgaaattt tgttgcaaaa atatataaat tgcccaatta    420
   aaaaaaaaaa aaaaaaaaaa aaaaaaa                                        447
<210> 73
   <211> 115
   <212> PRT
   <213> Lutzomyia longipalpis
<400> 73
   Met Lys Phe Phe Tyr Leu Ile Phe Ser Ala Ile Phe Phe Leu Ala Asp
   1               5                   10                  15
   Pro Ala Leu Val Lys Cys Ser Glu Asp Cys Glu Asn Ile Phe His Asp
               20                  25                  30
   Asn Ala Tyr Leu Leu Lys Leu Asp Cys Glu Ala Gly Arg Val Asp Pro
           35                  40                  45
   Val Glu Tyr Asp Asp Ile Ser Asp Glu Glu Ile Tyr Glu Ile Thr Val
       50                  55                  60
   Asp Val Gly Val Ser Ser Glu Asp Gln Glu Lys Val Ala Lys Ile Ile
   65                  70                  75                  80
   Arg Glu Cys Ile Ala Gln Val Ser Thr Gln Asp Cys Thr Lys Phe Ser
                   85                  90                  95
   Glu Ile Tyr Asp Cys Tyr Met Lys Lys Lys Ile Cys Asn Tyr Tyr Pro
               100                 105                 110
   Glu Asn Met
           115
<210> 74
   <211> 496
   <212> DNA
   <213> Lutzomyia longipalpis
<400> 74
   agtttaattt tcatcatgaa gttcttctac ttgattttct ctgcaatttt ctttctggct     60
   gatcctgctt tggtcaagtg ttcagaggat tgtgagaata tttttcatga caatgcgtac    120
   ctccttaaat tggattgtga agcaggaagg gttgatcctg ttgaatacga cgatatttcg    180
   gatgaagaaa tatatgaaat aacggtcgat gttggagttt catctgagga ccaggagaaa    240
   gttgcgaaaa taataaggga gtgcattgca caagtttcaa cgcaagattg cacgaaattt    300
   tcagaaattt atgattgtta catgaagaag aaaatctgta attattatcc tgaaaatatg    360
   taaaaaaaaa ttatttattt atataaaaaa atataaggat taaaatctct tattgattgt    420
   aaaaatggcc taatattgaa gcaaaaatta aagcatgaaa caagaccaaa aaaaaaaaaa    480
   aaaaaaaaaa aaaaaa                                                    496
<210> 75
   <211> 409
   <212> PRT
   <213> Lutzomyia longipalpis
<400> 75
   Met His Leu Gln Leu Asn Leu Cys Ala Ile Leu Leu Ser Val Leu Asn
   1               5                   10                  15
   Gly Ile Gln Gly Ala Pro Lys Ser Ile Asn Ser Lys Ser Cys Ala Ile
               20                  25                  30
   Ser Phe Pro Glu Asn Val Thr Ala Lys Lys Glu Pro Val Tyr Leu Lys
   35 40 45
   Pro Ser Asn Asp Gly Ser Leu Ser Thr Pro Leu Gln Pro Ser Gly Pro
   50 55 60
   Phe Val Ser Leu Lys Ile Gly Glu Ser Leu Ala Ile Phe Cys Pro Gly
   65 70 75 80
   Asp Gly Lys Asp Val Glu Thr Ile Thr Cys Asn Thr Asn Phe Asp Leu
   85 90 95
   Ala Ser Tyr Ser Cys Asn Lys Ser Thr Ser Thr Asp Thr Ile Glu Thr
   100 105 110
   Glu Glu Val Cys Gly Gly Ser Gly Lys Val Tyr Lys Val Gly Phe Pro
   115 120 125
   Leu Pro Ser Gly Asn Phe His Ser Ile Tyr Gln Thr Cys Phe Asp Lys
   130 135 140
   Lys Asn Leu Thr Pro Leu Tyr Ser Ile His Ile Leu Asn Gly Gln Ala
   145 150 155 160
   Val Gly Tyr His Leu Lys His Thr Arg Gly Ser Phe Arg Thr Asn Gly
   165 170 175
   Ile Tyr Gly Lys Val Asn Ile Asp Lys Leu Tyr Lys Thr Gln Ile Glu
   180 185 190
   Lys Phe Asn Lys Leu Phe Gly Pro Lys Gln Thr Phe Phe Arg Arg Pro
   195 200 205
   Leu Asn Phe Leu Ser Arg Gly His Leu Ser Pro Glu Val Asp Phe Thr
   210 215 220
   Phe Arg Arg Glu Gln His Ala Thr Glu Met Tyr Ile Asn Thr Ala Pro
   225 230 235 240
   Gln Tyr Gln Ser Ile Asn Gln Gly Asn Trp Leu Arg Val Glu Asn His
   245 250 255
   Val Arg Asp Leu Ala Lys Val Leu Gln Lys Asp Ile Thr Val Val Thr
   260 265 270
   Gly Ile Leu Gly Ile Leu Arg Leu Lys Ser Lys Lys Ile Glu Lys Glu
   275 280 285
   Ile Tyr Leu Gly Asp Asp Val Ile Ala Val Pro Ala Met Phe Trp Lys
       290                 295                 300
   Ala Val Phe Asp Pro Gln Lys Gln Glu Ala Ile Val Phe Val Ser Ser
   305                 310                 315                 320
   Asn Asn Pro His Val Lys Thr Phe Asn Pro Asn Cys Lys Asp Val Cys
                   325                 330                 335
   Ala Gln Ala Gly Phe Gly Asn Asp Asn Leu Glu Tyr Phe Ser Asn Tyr
               340                 345                 350
   Ser Ile Gly Leu Thr Ile Cys Cys Lys Leu Glu Glu Phe Val Lys Arg
           355                 360                 365
   Asn Lys Ile Ile Leu Pro Lys Glu Val Asn Asn Lys Asn Tyr Thr Lys
       370                 375                 380
   Lys Leu Leu Lys Phe Pro Lys Thr Arg Asn Lys Glu Gly Asp Lys Lys
   385                 390                 395                 400
   Val Val Arg Lys Arg Ala Lys Gly Ala
                   405
<210> 76
   <211> 1281
   <212> DNA
   <213> Lutzomyia longipalpis
<400> 76
   tcaatctaac aatgcacctg caattgaatt tgtgcgctat tctcctttcg gtactaaatg 60
   gaattcaggg cgctcccaaa agtattaatt caaaatcctg cgcaatctcc tttccggaga 120
   atgtaacggc taagaaggag ccagtgtact tgaaaccatc aaatgatggc tcattgagta 180
   cccccctaca gccaagtggg ccatttgtaa gtctcaaaat tggagaatct cttgcaatct 240
   tctgtccagg tgatggaaag gacgtagaga caattacgtg caatacaaat ttcgatttag 300
   cttcatattc gtgcaacaag agcacatcaa cggataccat tgaaacggaa gaagtttgcg 360
   gaggaagtgg aaaagtgtac aaagttggtt ttccgctgcc ctctgggaat ttccattcaa 420
   tctaccaaac gtgttttgat aagaaaaatc tcacacctct ctactcaatt cacattctca 480
   atggtcaagc tgttggatat caccttaagc acacaagagg aagctttcgt accaatggta 540
   tctacgggaa agtcaacatt gataaactct acaagacgca aattgagaaa ttcaacaaac 600
   ttttcggccc taaacaaaca tttttccgta gacccctcaa ttttctatca cgtggacact 660
   taagccccga agtggacttt acattccgta gggaacaaca tgcaacggaa atgtacatta 720
   acacagcacc acagtaccaa tcaattaatc aaggaaattg gctacgtgtt gaaaatcacg 780
   tgagggatct cgcaaaagtt ctgcagaagg acataacagt cgttacggga attttgggga 840
   tacttcggtt gaagagtaag aaaatagaga aagaaatcta tttaggagat gacgtaattg 900
   ccgtaccagc aatgttctgg aaggctgttt ttgaccctca aaaacaagaa gcaattgtct 960
   ttgtttcctc aaataatccc cacgtgaaga cctttaatcc caactgcaag gatgtatgcg 1020
   ctcaagctgg atttgggaat gataatcttg aatatttctc caattattct attggtctga 1080
   ctatttgttg caaacttgag gaatttgtta aaagaaataa aataattcta cccaaagaag 1140
   taaataacaa aaactacacc aaaaaactcc ttaagtttcc taaaacaaga aacaaggagg 1200
   gagataagaa ggtggtacgt aagcgcgcca aaggagcata aatattaaac gaaaaaaaaa 1260
   aaaaaaaaaa aaaaaaaaaa a 1281
<210> 77
   <211> 160
   <212> PRT
   <213> Lutzomyia longipalpis
<400> 77
   Met Asn Leu His Leu Ala Ile Ile Leu Phe Val Ser Tyr Phe Thr Leu
   1               5                   10                  15
   Ile Thr Ala Thr Asp Leu Ile Glu Lys Glu Leu Ser Asp Cys Lys Lys
               20                  25                  30
   Ile Phe Ile Ser Lys Ala Glu Leu Thr Trp Phe Gln Ala Leu Asp Phe
           35                  40                  45
   Cys Thr Glu Gln Asn Leu Thr Leu Leu Ser Ile Lys Ser Ala Arg Glu
       50                  55                  60
   Asn Asp Glu Val Thr Lys Ala Val Arg Ala Glu Val His Leu Pro Asp
   65                  70                  75                  80
   Thr Lys Lys Ser His Ile Trp Leu Gly Gly Ile Arg Tyr Asp Gln Asp
                   85                  90                  95
   Lys Asp Phe Arg Trp Ile Ser Asp Gly Thr Thr Val Thr Lys Thr Val
               100                 105                 110
   Tyr Ile Asn Trp Tyr Gln Gly Glu Pro Asn Gly Gly Arg Tyr Gln Lys
           115                 120                 125
   Glu Phe Cys Met Glu Leu Tyr Phe Lys Thr Pro Ala Gly Gln Trp Asn
       130                 135                 140
   Asp Asp Ile Cys Thr Ala Lys His His Phe Ile Cys Gln Glu Lys Lys
   145                 150                 155                 160
<210> 78
   <211> 671
   <212> DNA
   <213> Lutzomyia longipalpis
<400> 78
   gttctacgat aaaattttct tttcaaactt ttcttttaaa gaaaaatctt caaaaagtta      60
   aaatgaattt gcaccttgcg attatcctct ttgtgagtta cttcacactg atcactgcta     120
   cggatctaat tgaaaaggaa ctttctgatt gcaaaaagat cttcatctcc aaggctgagc     180
   taacttggtt ccaagctctc gatttctgta ccgaacaaaa cctaactttg ctctcaatta     240
   aatccgcccg ggaaaatgat gaggtgacta aagcagttcg agctgaggtt catcttccag     300
   acacaaagaa gtctcacatt tggctcggag gtattcgtta tgatcaagac aaggatttcc     360
   gttggataag cgatggaaca actgttacga agacagtcta catcaattgg taccaaggag     420
   aaccaaatgg tgggaggtac caaaaggaat tttgtatgga attgtacttt aaaactccag     480
   ctggtcaatg gaatgatgat atttgtacag caaagcatca ttttatatgt caggagaaaa     540
   aataaattga attgttcatg tgtctttggc ggtgcgaagg tataattcag gttgacgaca     600
   taaattgatt tttctttcat taagaaaata aaggcttgaa tttataaaaa aaaaaaaaaa     660
   aaaaaaaaaa a                                                          671
<210> 79
   <211> 160
   <212> PRT
   <213> Lutzomyia longipalpis
<400> 79
   Met Asn Leu Pro Leu Ala Ile Ile Leu Phe Val Ser Tyr Phe Thr Leu
   1               5                   10                  15
   Ile Thr Ala Ala Asp Leu Thr Glu Lys Glu Leu Ser Asp Gly Lys Lys
               20                  25                  30
   Ile Phe Ile Ser Lys Ala Glu Leu Ser Trp Phe Asp Ala Leu Asp Ala
           35                  40                  45
   Cys Thr Glu Lys Asp Leu Thr Leu Leu Thr Ile Lys Ser Ala Arg Glu
       50                  55                  60
   Asn Glu Glu Val Thr Lys Ala Val Arg Ala Glu Val His Leu Pro Asp
   65                  70                  75                  80
   Thr Lys Lys Ser His Ile Trp Leu Gly Gly Ile Arg Tyr Asp Gln Asp
   85 90 95
   Lys Asp Phe Arg Trp Ile Ser Asp Gly Thr Thr Val Thr Lys Thr Val
   100 105 110
   Tyr Ile Asn Trp Tyr Gln Gly Glu Pro Asn Gly Gly Arg Tyr Gln Lys
   115 120 125
   Glu Phe Cys Met Glu Leu Tyr Phe Lys Thr Pro Ala Gly Gln Trp Asn
   130 135 140
   Asp Asp Ile Cys Thr Ala Lys His His Phe Ile Cys Gln Glu Lys Lys
   145 150 155 160
<210> 80
   <211> 672
   <212> DNA
   <213> Lutzomyia longipalpis
<400> 80
   gttctacgat aaaattttct tttcaaactt ttcttttaaa gaaaaatctt caaaaagtta 60
   aaatgaattt gccccttgcg attatcctct ttgtgagtta cttcacactg atcactgctg 120
   cggatctaac tgaaaaggaa ctttctgatg gcaaaaagat cttcatctcc aaggctgagc 180
   taagttggtt cgatgctctc gatgcctgta ccgaaaaaga cctaactttg ctcacaatta 240
   aatccgcccg ggaaaatgag gaagtgacta aagcagttcg agctgaggtt catcttccag 300
   acacaaagaa gtctcacatt tggctcggag gtattcgtta tgatcaagac aaggatttcc 360
   gttggataag cgatggaaca actgttacga agacagtcta catcaattgg taccaaggag 420
   aaccaaatgg tgggaggtac caaaaggaat tttgtatgga attgtacttt aaaactccag 480
   ctggtcaatg gaatgatgat atttgtacag caaagcatca ttttatatgt caggagaaaa 540
   aataaattga attgttcatg tgtctttggc ggtgcgaagg tataattcag gttgacgaca 600
   taaattgatt tttctttcat taagaaaata aaggcttgaa tttagcaaaa aaaaaaaaaa 660
   aaaaaaaaaa aa 672
<210> 81
   <211> 399
   <212> PRT
   <213> Lutzomyia longipalpis
<400> 81
   Met Lys Val Phe Phe Ser Ile Phe Thr Leu Val Leu Phe Gln Gly Thr
   1               5                   10                  15
   Leu Gly Ala Asp Thr Gln Gly Tyr Lys Trp Lys Gln Leu Leu Tyr Asn
               20                  25                  30
   Asn Val Thr Pro Gly Ser Tyr Asn Pro Asp Asn Met Ile Ser Thr Ala
           35                  40                  45
   Phe Ala Tyr Asp Ala Glu Gly Glu Lys Leu Phe Leu Ala Val Pro Arg
       50                  55                  60
   Lys Leu Pro Arg Val Pro Tyr Thr Leu Ala Glu Val Asp Thr Lys Asn
   65                  70                  75                  80
   Ser Leu Gly Val Lys Gly Lys His Ser Pro Leu Leu Asn Lys Phe Ser
                   85                  90                  95
   Gly His Lys Thr Gly Lys Glu Leu Thr Ser Ile Tyr Gln Pro Val Ile
               100                 105                 110
   Asp Asp Cys Arg Arg Leu Trp Val Val Asp Ile Gly Ser Val Glu Tyr
           115                 120                 125
   Arg Ser Arg Gly Ala Lys Asp Tyr Pro Ser His Arg Pro Ala Ile Val
       130                 135                 140
   Ala Tyr Asp Leu Lys Gln Pro Asn Tyr Pro Glu Val Val Arg Tyr Tyr
   145                 150                 155                 160
   Phe Pro Thr Arg Leu Val Glu Lys Pro Thr Tyr Phe Gly Gly Phe Ala
                   165                 170                 175
   Val Asp Val Ala Asn Pro Lys Gly Asp Cys Ser Glu Thr Phe Val Tyr
               180                 185                 190
   Ile Thr Asn Phe Leu Arg Gly Ala Leu Phe Ile Tyr Asp His Lys Lys
           195                 200                 205
   Gln Asp Ser Trp Asn Val Thr His Pro Thr Phe Lys Ala Glu Arg Pro
       210                 215                 220
   Thr Lys Phe Asp Tyr Gly Gly Lys Glu Tyr Glu Phe Lys Ala Gly Ile
   225                 230                 235                 240
   Phe Gly Ile Thr Leu Gly Asp Arg Asp Ser Glu Gly Asn Arg Pro Ala
                   245                 250                 255
   Tyr Tyr Leu Ala Gly Ser Ala Ile Lys Val Tyr Ser Val Asn Thr Lys
               260                 265                 270
   Glu Leu Lys Gln Lys Gly Gly Lys Leu Asn Pro Glu Leu Leu Gly Asn
               275                 280                 285
   Arg Gly Lys Tyr Asn Asp Ala Ile Ala Leu Ala Tyr Asp Pro Lys Thr
       290                 295                 300
   Lys Val Ile Phe Phe Ala Glu Ala Asn Thr Lys Gln Val Ser Cys Trp
   305                 310                 315                 320
   Asn Thr Gln Lys Met Pro Leu Arg Met Lys Asn Thr Asp Val Val Tyr
                   325                 330                 335
   Thr Ser Ser Arg Phe Val Phe Gly Thr Asp Ile Ser Val Asp Ser Lys
               340                 345                 350
   Gly Gly Leu Trp Phe Met Ser Asn Gly Phe Pro Pro Ile Arg Lys Ser
           355                 360                 365
   Glu Lys Phe Lys Tyr Asp Phe Pro Arg Tyr Arg Leu Met Arg Ile Met
       370                 375                 380
   Asp Thr Gln Glu Ala Ile Ala Gly Thr Ala Cys Asp Met Asn Ala
   385                 390                 395
<210> 82
   <211> 1429
   <212> DNA
   <213> Lutzomyia longipalpis
<400> 82
   ttgaattgaa gcagcagcaa tgaaagtgtt tttctcaatt tttacgctcg tcctcttcca     60
   agggaccctt ggagcggata ctcaaggata taaatggaag caattgctct acaataatgt    120
   tacaccagga tcctacaatc cggataatat gatcagtacg gcttttgcct acgatgctga    180
   gggtgaaaaa ctcttcctag ctgtcccaag gaagttaccc agagttccgt atacattggc    240
   ggaagtggat acaaagaata gtcttggtgt taagggaaaa cattcaccgt tacttaacaa    300
   attcagtggg cacaaaactg ggaaggaact aacatcaatc tatcagccag ttattgatga    360
   ttgtcgtcgc ctttgggtgg ttgatattgg ttccgtggaa tatcgctcaa gaggtgccaa    420
   agactacccg agtcatcgtc ctgcaattgt tgcgtacgac ctaaagcaac caaactaccc    480
   cgaagttgtt cgatactatt tccccacaag attagtggag aagccaacat atttcggtgg    540
   atttgccgtt gatgttgcaa acccaaaggg ggattgtagt gaaacttttg tctacattac    600
   aaacttcctc aggggagctc tctttatata cgatcataag aagcaggatt cgtggaatgt    660
   aactcatccc accttcaaag cagaacgacc cactaaattt gattacggcg gaaaggaata    720
   tgaattcaaa gccggaattt tcggaattac tctcggagat cgagacagtg aaggcaatcg    780
   tccagcttac tacttagccg gaagtgccat caaagtctac agcgtcaaca cgaaagaact    840
   taagcagaag ggtggaaagc tgaatccgga gcttcttgga aaccgcggga agtacaacga    900
   tgccattgcc ctagcttacg atcccaaaac taaagttatc ttctttgctg aggccaacac    960
   aaagcaagta tcctgctgga acacacagaa aatgccactg aggatgaaga ataccgacgt   1020
   agtctacact agttctcgct ttgtctttgg aacggacatt tcggttgata gcaagggcgg   1080
   cctctggttc atgtctaacg gctttccgcc tataaggaaa tcagaaaaat tcaaatatga   1140
   cttcccacgc taccgtctaa tgaggatcat ggacacacag gaagcaattg ccggaactgc   1200
   ttgcgatatg aatgcataaa agttaatttt caacccaaga agaagaccta aagaggcttt   1260
   tccaggcttt gatgcaggag aggtggttat caacgcaaaa tcagctattg ttgtatgagg   1320
   aggagaaatt attgattctg aattctataa aaaaaattta atttgtgaaa tatttggcaa   1380
   taataaatta attgaattac aaaaaaaaaa aaaaaaaaaa aaaaaaaaa               1429
<210> 83
   <211> 170
   <212> PRT
   <213> Lutzomyia longipalpis
<400> 83
   Met Gln Ser Lys Ile Leu Ser Phe Val Leu Phe Thr Leu Ser Leu Gly
   1               5                   10                  15
   Tyr Val Leu Gly Glu Thr Cys Ser Asn Ala Lys Val Lys Gly Ala Thr
               20                  25                  30
   Ser Tyr Ser Thr Thr Asp Ala Thr Ile Val Ser Gln Ile Ala Phe Val
           35                  40                  45
   Thr Glu Phe Ser Leu Glu Cys Ser Asn Pro Gly Ser Glu Lys Ile Ser
       50                  55                  60
   Leu Phe Ala Glu Val Asp Gly Lys Ile Thr Pro Val Ala Met Ile Gly
   65                  70                  75                  80
   Asp Thr Thr Tyr Gln Val Ser Trp Asn Glu Glu Val Asn Lys Ala Arg
                   85                  90                  95
   Ser Gly Asp Tyr Ser Val Lys Leu Tyr Asp Glu Glu Gly Tyr Gly Ala
               100                 105                 110
   Val Arg Lys Ala Gln Arg Ser Gly Glu Glu Asn Lys Val Lys Pro Leu
           115                 120                 125
   Ala Thr Val Val Val Arg His Pro Gly Thr Tyr Thr Gly Pro Trp Phe
       130                 135                 140
   Asn Ser Glu Ile Leu Ala Ala Gly Leu Ile Ala Val Val Ala Tyr Phe
   145                 150                 155                 160
   Ala Phe Ser Thr Arg Ser Lys Ile Leu Ser
                   165                 170
<210> 84
   <211> 712
   <212> DNA
   <213> Lutzomyia longipalpis
<400> 84
   tctctttggt taacattgtg aagttatcgg acgtggccgg tttctatttc ttttgcaaaa     60
   atgcagtcaa aaattctttc tttcgtcctt ttcaccttat ccttgggcta tgttttgggt    120
   gaaacatgct caaatgctaa ggttaaggga gctacctctt attccacaac ggatgccaca    180
   attgtaagcc aaattgcctt tgtgactgaa ttctccttgg aatgctcaaa tcctggatcc    240
   gagaaaatct ccctatttgc tgaagtcgat ggcaaaatta ctcctgttgc catgatcggg    300
   gataccacct accaggtgag ctggaatgaa gaggttaata aggctagaag tggtgactac    360
   agtgtgaagc tgtacgatga agaaggatac ggagcagtac gcaaagctca gagatcaggt    420
   gaagagaaca aggtcaaacc actagcaacc gttgttgttc gacatccagg aacatacact    480
   ggaccatggt tcaattccga aatcctcgca gctggtctca ttgctgttgt tgcctacttt    540
   gctttctcaa cgcgaagcaa aattctttcc taaagagacg cagcatgaaa tttcacaaaa    600
   aaataaaaac aaattcaagt catcaaccat gtctctttgg cactcagact gtttctgtga    660
   aatacaaact attatttaac aaaaaaaaaa aaaaaaaaaa aaaaaaaaaa aa            712
<210> 85
   <211> 73
   <212> PRT
   <213> Lutzomyia longipalpis
<400> 85
   Met Val Ser Ile Leu Leu Ile Ser Leu Ile Leu Asn Leu Leu Val Phe
   1               5                   10                  15
   Tyr Ala Lys Ala Arg Pro Leu Glu Asp Ile Ser Ser Asp Leu Ser Pro
               20                  25                  30
   Asp Tyr Tyr Ile Thr Glu Gly Tyr Asp Gly Val Lys Glu Lys Arg Glu
           35                  40                  45
   Ile Glu Leu Val Pro Val Thr Phe Gly Ile Phe Asn Ile His Thr Thr
       50                  55                  60
   Pro Ala Pro Arg Ile Thr Phe Glu Trp
   65                  70
<210> 86
   <211> 379
   <212> DNA
   <213> Lutzomyia longipalpis
<400> 86
   attcccacaa gaagctgcta aaatggtgtc aattctgtta atctccttga ttcttaattt      60
   gttggttttc tatgctaaag ctagaccact agaagacatc tcgtcagatc tttcccctga     120
   ttattacatc actgaaggct atgacggtgt gaaggagaag agagagatcg aacttgtacc     180
   tgtgacattt ggaatattta atatacatac aacacctgct cccagaatta cctttgaatg     240
   gtaaaaaatc caagaagaat ttatgatttt attcttcctt ccattgggat ggattgtaag     300
   tcagcataaa acgccgttaa aaatgaattt ttaataaaaa aaaattattc caaaaaaaaa     360
   aaaaaaaaaa aaaaaaaaa                                                   379
<210> 87
   <211> 76
   <212> PRT
   <213> Lutzomyia longipalpis
<400> 87
   Met Lys Leu Phe Cys Leu Ile Phe Val Val Phe Val Ala Leu Glu Val
   1               5                   10                  15
   Cys Ile Glu Thr Val Lys Ala Met Glu Ala Thr Glu Glu Ile Ser Val
               20                  25                  30
   Lys Leu Gln Asp Asp Ala Asn Glu Pro Asp Asp Ser Leu Asp Leu Asp
           35                  40                  45
   Glu Gly Leu Pro Asp Ala Phe Asp Glu Asp Tyr Asn Asn Gln Ala Glu
       50                  55                  60
   Tyr Lys Pro Asn Pro Arg Gly Asp Tyr Arg Arg Arg
   65                  70                  75
<210> 88
   <211> 526
   <212> DNA
   <213> Lutzomyia longipalpis
<400> 88
   cactattcat tggaagattt attaacttca agatgaaatt attttgttta atttttgttg      60
   tgtttgttgc tttagaagtc tgtatagaga ccgtgaaagc tatggaagca acggaggaga     120
   tatctgtaaa attgcaagat gatgcgaatg aacctgatga ctctctggat ttagacgaag 180
   gtcttcctga tgcattcgat gaggactata ataatcaggc tgagtacaag ccgaatccta 240
   gaggggacta cagaagacga taattaatat aaattcagga aaacactcta aaaatttcca 300
   attgactcta ctttaaacga tttaatacct acctacacta aataccatat gcaataatta 360
   tgttttaatt atttagtgca agatctacta gtttcagttc atattttggg actttcccgc 420
   ctttctctcg atggaaaaat gattttacgg attcttaatt ttcattgtac agagttaata 480
   aaacaattga aagcaattaa aaaaaaaaaa aaaaaaaaaa aaaaaa 526
<210> 89
   <211> 1021
   <212> DNA
   <213> Lutzomyia longipalpis
<400> 89
   cttctttgga tttattgagt gattaacagg aaattagctg aagaaatgaa ttcgattaat 60
   ttcctatcaa tagttggttt aatcagtttt ggattcattg ttgcagtaaa gtgtgatggt 120
   gatgaatatt tcattggaaa atacaaagaa aaagatgaga cactgttttt tgcaagctac 180
   ggcctaaaga gggatccttg ccaaattgtc ttaggctaca aatgctcaaa caatcaaacc 240
   cactttgtgc ttaattttaa aaccaataag aaatcctgca tatcagcaat taagctgact 300
   tcttacccaa aaatcaatca aaactcggat ttaactaaaa atctctactg ccaaactgga 360
   ggaataggaa cagataactg caaacttgtc ttcaagaaac gtaaaagaca aatagcagct 420
   aatattgaaa tctacggcat tccagcgaag aaatgttcct tcaaggatcg ttacattgga 480
   gctgatccac tccacgtcga ttcctatggg cttccgtatc agtttgatca ggaacatgga 540
   tggaatgtgg aacgatataa cattttcaaa gacacaagat tttccacaga agttttctac 600
   cacaaaaatg gtttatttaa cacccaaata acttatttgg ctgaagaaga ttccttctct 660
   gaagctcgag agattactgc gaaggatatt aagaagaagt tttcaattat tttgcccaat 720
   gaagagtata agaggattag tttcttggac gtttattggt tccaggagac tatgcgaaaa 780
   aagcctaaat atccctacat tcactacaat ggagaatgca gcaatgagaa taaaacttgt 840
   gaacttgtct ttgacaccga tgaactaatg acctacgccc ttgttaaagt ctttactaat 900
   cctgagagtg atggatctag gctcaaagaa gaggatttgg gaagaggata aatcttctta 960
   ataaaaaaaa gttctgtaag aaaatattgt tcaataaatt aaaaaaaaaa aaaaaaaaaa 1020
   a 1021
<210> 90
   <211> 1409
   <212> DNA
   <213> Lutzomyia longipalpis
<400> 90
   agtcagtgtt aatgaagaaa ttgcaattat gaggttcttc tttgttttcc ttgccatcgt     60
   cctttttcaa gggatccacg gagcttatgt ggaaatagga tattctctga gaaatattac    120
   attcgatgga ttggatacag atgactacaa tccaaagttc aacattccaa cgggtttggc    180
   agttgatccc gaaggatata ggctcttcat agccatccca aggagaaagc caaaggttcc    240
   ctacactgtg gctgaactga atatggtcat gaatcccgga tttcccgtcg agagagctcc    300
   gagctttgag aaattcaaaa aattcaatgg cgagggcaaa aaggatcttg ttaatgtgta    360
   tcagccagtc attgatgatt gtcgtcgtct ttgggtgctt gacattggga aggtggaata    420
   caccggtggt gatgctgatc aatatcccaa aggaaagcct accctaattg cctacgacct    480
   caagaaggat catactccgg aaattcatcg atttgaaatt ccagacgatc tctatagctc    540
   acaagttgaa tttggtggat ttgccgttga tgttgttaac acgaaaggag actgtacgga    600
   gtcatttgtc tacctgacca atttcaagga taactctcta attgtctacg atgagacaca    660
   aaagaaagct tggaaattca cagataaaac atttgaagct gataaggaat ccacgttctc    720
   ctactcggga gaggaacaaa tgaagtacaa agtcggtctt tttgggatag ctctgggtga    780
   tagggatgaa atggggcatc gtcctgcctg ctacatcgct gggagtagca ccaaagtcta    840
   cagtgttaac actaaagaac tcaaaacaga gaatggtcag ttaaatcctc agcttcacgg    900
   tgatcgtgga aagtacacag atgcaattgc cctagcctac gatcctgagc ataaagtcct    960
   ctactttgct gaatccgaca gcaggcaggt gtcctgttgg aatgtaaata tggagctaaa   1020
   accagacaat acggatgtga tcttctctag tgcccgtttt acttttggaa cggatatttt   1080
   ggttgatagc aagggaatgc tgtggataat ggctaatgga catccaccag tagaggatca   1140
   agagaagatt tggaagatga gattcgtaaa ccggaagatc cgtattatga aagtggatac   1200
   ggaacgtgtt ttcaaatatt cacgctgcaa tccaaattat aagcccccaa aggaaattga   1260
   agtttgagac acaggaaaaa gctcaatttt caacaagaat ttgatcttaa tctgaatacc   1320
   ctaaagtctg tcaaagaatt tcatattatt tgaaaaccaa taaattgatt aattttccga   1380
   aaaaaaaaaa aaaaaaaaaa aaaaaaaaa                                        1409
<210> 91
   <211> 1239
   <212> DNA
   <213> Lutzomyia longipalpis
<400> 91
   atgcggttct tcttcgtgtt cctggccatc gtgctgttcc agggcatcca cggcgcctac      60
   gtggagatcg gctacagcct gcggaacatc accttcgacg gcctggacac cgacgactac     120
   aaccccaagt tcaacatccc caccggcctg gccgtggacc ccgagggcta ccggctgttc     180
   atcgccatcc ccaggcggaa gcccaaggtg ccctacaccg tggccgagct gaacatggtg     240
   atgaaccccg gcttccccgt ggagagggcc cccagcttcg agaagttcaa gaagtttaac     300
   ggcgagggca agaaagacct ggtgaacgtg taccagcccg tgatcgacga ctgcaggcgg     360
   ctgtgggtgc tggacatcgg caaggtggag tacacaggcg gcgacgccga ccagtacccc     420
   aagggcaagc ccaccctgat cgcctacgac ctgaagaagg accacacccc cgagatccac     480
   cggttcgaga tccccgacga cctgtacagc agccaggtgg agttcggcgg ctttgccgtg     540
   gacgtggtga acaccaaggg cgactgcacc gagagcttcg tgtacctgac caacttcaag     600
   gacaacagcc tgatcgtgta cgacgagacc cagaagaagg cctggaagtt caccgacaag     660
   accttcgagg ccgacaaaga gagcaccttc agctacagcg gcgaggaaca gatgaagtac     720
   aaagtgggcc tgttcggcat cgccctgggc gaccgggacg agatgggcca caggcccgcc     780
   tgctacatcg ccggcagcag caccaaggtg tacagcgtga ataccaaaga gctgaaaacc     840
   gagaacggcc agctgaaccc ccagctgcac ggcgaccggg gcaagtacac cgacgccatt     900
   gccctggcct acgaccccga gcacaaggtg ctgtacttcg ccgagagcga cagccggcag     960
   gtgtcctgct ggaacgtgaa catggaactg aagcccgaca acaccgacgt gatcttcagc    1020
   agcgcccggt tcaccttcgg caccgacatc ctggtggaca gcaagggcat gctgtggatc    1080
   atggccaacg gccacccccc cgtggaggac caggaaaaga tctggaagat gcggttcgtg    1140
   aaccggaaga tccggatcat gaaggtggac accgagcggg tgttcaagta cagccggtgc    1200
   aaccccaact acaagccccc caaagaaatc gaagtgtga                           1239
<210> 92
   <211> 4995
   <212> DNA
   <213> Lutzomyia longipalpis
<400> 92
   tcagatatat tagatgcatt gttagttctg tagatcagta acgtatagca tacgagtata      60
   attatcgtag gtagtaggta tcctaaaata aatctgatac agataataac tttgtaaatc     120
   aattcagcaa tttctctatt atcatgataa tgattaatac acagcgtgtc gttatttttt     180
   gttacgatag tatttctaaa gtaaagagca ggaatcccta gtataataga aataatccat     240
   atgaaaaata tagtaatgta catatttcta atgttaacat atttataggt aaatccagga     300
   agggtaattt ttacatatct atatacgctt attacagtta ttaaaaatat acttgcaaac     360
   atgttagaag taaaaaagaa agaactaatt ttacaaagtg ctttaccaaa atgccaatgg     420
   aaattactta gtatgtatat aatgtataaa ggtatgaata tcacaaacag caaatcggct     480
   attcccaagt tgagaaacgg tataatagat atatttctag ataccattaa taaccttata     540
   agcttgacgt ttcctataat gcctactaag aaaactagaa gatacataca tactaacgcc     600
   atacgagagt aactactcat cgtataacta ctgttgctaa cagtgacact gatgttataa     660
   ctcatctttg atgtggtata aatgtataat aactatatta cactggtatt ttatttcagt 720
   tatatactat atagtattaa aaattatatt tgtataatta tattattata ttcagtgtag 780
   aaagtaaaat actataaata tgtatctctt atttataact tattagtaaa gtatgtacta 840
   ttcagttata ttgttttata aaagctaaat gctactagat tgatataaat gaatatgtaa 900
   taaattagta atgtagtata ctaatattaa ctcacatttg actaattagc tataaaaacc 960
   cctagtcaat aaaaactcga gtcatcacac ttcgatttct ttggggggct tgtagttggg 1020
   gttgcaccgg ctgtacttga acacccgctc ggtgtccacc ttcatgatcc ggatcttccg 1080
   gttcacgaac cgcatcttcc agatcttttc ctggtcctcc acgggggggt ggccgttggc 1140
   catgatccac agcatgccct tgctgtccac caggatgtcg gtgccgaagg tgaaccgggc 1200
   gctgctgaag atcacgtcgg tgttgtcggg cttcagttcc atgttcacgt tccagcagga 1260
   cacctgccgg ctgtcgctct cggcgaagta cagcaccttg tgctcggggt cgtaggccag 1320
   ggcaatggcg tcggtgtact tgccccggtc gccgtgcagc tgggggttca gctggccgtt 1380
   ctcggttttc agctctttgg tattcacgct gtacaccttg gtgctgctgc cggcgatgta 1440
   gcaggcgggc ctgtggccca tctcgtcccg gtcgcccagg gcgatgccga acaggcccac 1500
   tttgtacttc atctgttcct cgccgctgta gctgaaggtg ctctctttgt cggcctcgaa 1560
   ggtcttgtcg gtgaacttcc aggccttctt ctgggtctcg tcgtacacga tcaggctgtt 1620
   gtccttgaag ttggtcaggt acacgaagct ctcggtgcag tcgcccttgg tgttcaccac 1680
   gtccacggca aagccgccga actccacctg gctgctgtac aggtcgtcgg ggatctcgaa 1740
   ccggtggatc tcgggggtgt ggtccttctt caggtcgtag gcgatcaggg tgggcttgcc 1800
   cttggggtac tggtcggcgt cgccgcctgt gtactccacc ttgccgatgt ccagcaccca 1860
   cagccgcctg cagtcgtcga tcacgggctg gtacacgttc accaggtctt tcttgccctc 1920
   gccgttaaac ttcttgaact tctcgaagct gggggccctc tccacgggga agccggggtt 1980
   catcaccatg ttcagctcgg ccacggtgta gggcaccttg ggcttccgcc tggggatggc 2040
   gatgaacagc cggtagccct cggggtccac ggccaggccg gtggggatgt tgaacttggg 2100
   gttgtagtcg tcggtgtcca ggccgtcgaa ggtgatgttc cgcaggctgt agccgatctc 2160
   cacgtaggcg ccgtggatgc cctggaacag cacgatggcc aggaacacga agaagaaccg 2220
   cattacgata caaacttaac ggatatcgcg ataatgaaat aatttatgat tatttctcgc 2280
   tttcaattta acacaaccct caagaacctt tgtatttatt ttcacttttt aagtatagaa 2340
   taaagaagct ctaattaatt aacgagcaga tagtctcgtt ctcgccctgc ctgatgacta 2400
   attaattaac ccctagttaa tcaaataaaa agcatacaag ctattgcttc gctatcgtta 2460
   caaaatggca ggaattttgt gtaaactaag ccacatactt gccaatgaaa aaaatagtag 2520
   aaaggatact attttaatgg gattagatgt taaggttcct tgggattata gtaactgggc 2580
   atctgttaac ttttacgacg ttaggttaga tactgatgtt acagattata ataatgttac 2640
   aataaaatac atgacaggat gtgatatttt tcctcatata actcttggaa tagcaaatat 2700
   ggatcaatgt gatagatttg aaaatttcaa aaagcaaata actgatcaag atttacagac 2760
   tatttctata gtctgtaaag aagagatgtg ttttcctcag agtaacgcct ctaaacagtt 2820
   gggagcgaaa ggatgcgctg tagttatgaa actggaggta tctgatgaac ttagagccct 2880
   aagaaatgtt ctgctgaatg cggtaccctg ttcgaaggac gtgtttggtg atatcacagt 2940
   agataatccg tggaatcctc acataacagt aggatatgtt aaggaggacg atgtcgaaaa 3000
   caagaaacgc ctaatggagt gcatgtccaa gtttaggggg caagaaatac aagttctagg 3060
   atggtattaa taagtatcta agtatttggt ataatttatt aaatagtata attataacaa 3120
   ataataaata acatgataac ggtttttatt agaataaaat agagataata tcataatgat 3180
   atataatact tcattaccag aaatgagtaa tggaagactt ataaatgaac tgcataaagc 3240
   tataaggtat agagatataa atttagtaag gtatatactt aaaaaatgca aatacaataa 3300
   cgtaaatata ctatcaacgt ctttgtattt agccgtaagt atttctgata tagaaatggt 3360
   aaaattatta ctagaacacg gtgccgatat tttaaaatgt aaaaatcctc ctcttcataa 3420
   agctgctagt ttagataata cagaaattgc taaactacta atagattctg gcgctgacat 3480
   agaacagata cattctggaa atagtccgtt atatatttct gtatatagaa acaataagtc 3540
   attaactaga tatttattaa aaaaaggtgt taattgtaat agattctttc taaattatta 3600
   cgatgtactg tatgataaga tatctgatga tatgtataaa atatttatag attttaatat 3660
   tgatcttaat atacaaacta gaaattttga aactccgtta cattacgcta taaagtataa 3720
   gaatatagat ttaattagga tattgttaga taatagtatt aaaatagata aaagtttatt 3780
   tttgcataaa cagtatctca taaaggcact taaaaataat tgtagttacg atataatagc 3840
   gttacttata aatcacggag tgcctataaa cgaacaagat gatttaggta aaaccccatt 3900
   acatcattcg gtaattaata gaagaaaaga tgtaacagca cttctgttaa atctaggagc 3960
   tgatataaac gtaatagatg actgtatggg cagtccctta cattacgctg tttcacgtaa 4020
   cgatatcgaa acaacaaaga cacttttaga aagaggatct aatgttaatg tggttaataa 4080
   tcatatagat accgttctaa atatagctgt tgcatctaaa aacaaaacta tagtaaactt 4140
   attactgaag tacggtactg atacaaagtt ggtaggatta gataaacatg ttattcacat 4200
   agctatagaa atgaaagata ttaatatact gaatgcgatc ttattatatg gttgctatgt 4260
   aaacgtctat aatcataaag gtttcactcc tctatacatg gcagttagtt ctatgaaaac 4320
   agaatttgtt aaactcttac ttgaccacgg tgcttacgta aatgctaaag ctaagttatc 4380
   tggaaatact cctttacata aagctatgtt atctaatagt tttaataata taaaattact 4440
   tttatcttat aacgccgact ataattctct aaataatcac ggtaatacgc ctctaacttg 4500
   tgttagcttt ttagatgaca agatagctat tatgataata tctaaaatga tgttagaaat 4560
   atctaaaaat cctgaaatag ctaattcaga aggttttata gtaaacatgg aacatataaa 4620
   cagtaataaa agactactat ctataaaaga atcatgcgaa aaagaactag atgttataac 4680
   acatataaag ttaaattcta tatattcttt taatatcttt cttgacaata acatagatct 4740
   tatggtaaag ttcgtaacta atcctagagt taataagata cctgcatgta tacgtatata 4800
   tagggaatta atacggaaaa ataaatcatt agcttttcat agacatcagc taatagttaa 4860
   agctgtaaaa gagagtaaga atctaggaat aataggtagg ttacctatag atatcaaaca 4920
   tataataatg gaactattaa gtaataatga tttacattct gttatcacca gctgttgtaa 4980
   cccagtagta taaag 4995
<210> 93
   <211> 4995
   <212> DNA
   <213> Lutzomyia longipalpis
<400> 93
   ctttatacta ctgggttaca acagctggtg ataacagaat gtaaatcatt attacttaat      60
   agttccatta ttatatgttt gatatctata ggtaacctac ctattattcc tagattctta     120
   ctctctttta cagctttaac tattagctga tgtctatgaa aagctaatga tttatttttc     180
   cgtattaatt ccctatatat acgtatacat gcaggtatct tattaactct aggattagtt     240
   acgaacttta ccataagatc tatgttattg tcaagaaaga tattaaaaga atatatagaa     300
   tttaacttta tatgtgttat aacatctagt tctttttcgc atgattcttt tatagatagt     360
   agtcttttat tactgtttat atgttccatg tttactataa aaccttctga attagctatt     420
   tcaggatttt tagatatttc taacatcatt ttagatatta tcataatagc tatcttgtca     480
   tctaaaaagc taacacaagt tagaggcgta ttaccgtgat tatttagaga attatagtcg     540
   gcgttataag ataaaagtaa ttttatatta ttaaaactat tagataacat agctttatgt     600
   aaaggagtat ttccagataa cttagcttta gcatttacgt aagcaccgtg gtcaagtaag     660
   agtttaacaa attctgtttt catagaacta actgccatgt atagaggagt gaaaccttta     720
   tgattataga cgtttacata gcaaccatat aataagatcg cattcagtat attaatatct     780
   ttcatttcta tagctatgtg aataacatgt ttatctaatc ctaccaactt tgtatcagta     840
   ccgtacttca gtaataagtt tactatagtt ttgtttttag atgcaacagc tatatttaga     900
   acggtatcta tatgattatt aaccacatta acattagatc ctctttctaa aagtgtcttt     960
   gttgtttcga tatcgttacg tgaaacagcg taatgtaagg gactgcccat acagtcatct    1020
   attacgttta tatcagctcc tagatttaac agaagtgctg ttacatcttt tcttctatta    1080
   attaccgaat gatgtaatgg ggttttacct aaatcatctt gttcgtttat aggcactccg 1140
   tgatttataa gtaacgctat tatatcgtaa ctacaattat ttttaagtgc ctttatgaga 1200
   tactgtttat gcaaaaataa acttttatct attttaatac tattatctaa caatatccta 1260
   attaaatcta tattcttata ctttatagcg taatgtaacg gagtttcaaa atttctagtt 1320
   tgtatattaa gatcaatatt aaaatctata aatattttat acatatcatc agatatctta 1380
   tcatacagta catcgtaata atttagaaag aatctattac aattaacacc tttttttaat 1440
   aaatatctag ttaatgactt attgtttcta tatacagaaa tatataacgg actatttcca 1500
   gaatgtatct gttctatgtc agcgccagaa tctattagta gtttagcaat ttctgtatta 1560
   tctaaactag cagctttatg aagaggagga tttttacatt ttaaaatatc ggcaccgtgt 1620
   tctagtaata attttaccat ttctatatca gaaatactta cggctaaata caaagacgtt 1680
   gatagtatat ttacgttatt gtatttgcat tttttaagta tataccttac taaatttata 1740
   tctctatacc ttatagcttt atgcagttca tttataagtc ttccattact catttctggt 1800
   aatgaagtat tatatatcat tatgatatta tctctatttt attctaataa aaaccgttat 1860
   catgttattt attatttgtt ataattatac tatttaataa attataccaa atacttagat 1920
   acttattaat accatcctag aacttgtatt tcttgccccc taaacttgga catgcactcc 1980
   attaggcgtt tcttgttttc gacatcgtcc tccttaacat atcctactgt tatgtgagga 2040
   ttccacggat tatctactgt gatatcacca aacacgtcct tcgaacaggg taccgcattc 2100
   agcagaacat ttcttagggc tctaagttca tcagatacct ccagtttcat aactacagcg 2160
   catcctttcg ctcccaactg tttagaggcg ttactctgag gaaaacacat ctcttcttta 2220
   cagactatag aaatagtctg taaatcttga tcagttattt gctttttgaa attttcaaat 2280
   ctatcacatt gatccatatt tgctattcca agagttatat gaggaaaaat atcacatcct 2340
   gtcatgtatt ttattgtaac attattataa tctgtaacat cagtatctaa cctaacgtcg 2400
   taaaagttaa cagatgccca gttactataa tcccaaggaa ccttaacatc taatcccatt 2460
   aaaatagtat cctttctact atttttttca ttggcaagta tgtggcttag tttacacaaa 2520
   attcctgcca ttttgtaacg atagcgaagc aatagcttgt atgcttttta tttgattaac 2580
   taggggttaa ttaattagtc atcaggcagg gcgagaacga gactatctgc tcgttaatta 2640
   attagagctt ctttattcta tacttaaaaa gtgaaaataa atacaaaggt tcttgagggt 2700
   tgtgttaaat tgaaagcgag aaataatcat aaattatttc attatcgcga tatccgttaa 2760
   gtttgtatcg taatgcggtt cttcttcgtg ttcctggcca tcgtgctgtt ccagggcatc 2820
   cacggcgcct acgtggagat cggctacagc ctgcggaaca tcaccttcga cggcctggac 2880
   accgacgact acaaccccaa gttcaacatc cccaccggcc tggccgtgga ccccgagggc 2940
   taccggctgt tcatcgccat ccccaggcgg aagcccaagg tgccctacac cgtggccgag 3000
   ctgaacatgg tgatgaaccc cggcttcccc gtggagaggg cccccagctt cgagaagttc 3060
   aagaagttta acggcgaggg caagaaagac ctggtgaacg tgtaccagcc cgtgatcgac 3120
   gactgcaggc ggctgtgggt gctggacatc ggcaaggtgg agtacacagg cggcgacgcc 3180
   gaccagtacc ccaagggcaa gcccaccctg atcgcctacg acctgaagaa ggaccacacc 3240
   cccgagatcc accggttcga gatccccgac gacctgtaca gcagccaggt ggagttcggc 3300
   ggctttgccg tggacgtggt gaacaccaag ggcgactgca ccgagagctt cgtgtacctg 3360
   accaacttca aggacaacag cctgatcgtg tacgacgaga cccagaagaa ggcctggaag 3420
   ttcaccgaca agaccttcga ggccgacaaa gagagcacct tcagctacag cggcgaggaa 3480
   cagatgaagt acaaagtggg cctgttcggc atcgccctgg gcgaccggga cgagatgggc 3540
   cacaggcccg cctgctacat cgccggcagc agcaccaagg tgtacagcgt gaataccaaa 3600
   gagctgaaaa ccgagaacgg ccagctgaac ccccagctgc acggcgaccg gggcaagtac 3660
   accgacgcca ttgccctggc ctacgacccc gagcacaagg tgctgtactt cgccgagagc 3720
   gacagccggc aggtgtcctg ctggaacgtg aacatggaac tgaagcccga caacaccgac 3780
   gtgatcttca gcagcgcccg gttcaccttc ggcaccgaca tcctggtgga cagcaagggc 3840
   atgctgtgga tcatggccaa cggccacccc cccgtggagg accaggaaaa gatctggaag 3900
   atgcggttcg tgaaccggaa gatccggatc atgaaggtgg acaccgagcg ggtgttcaag 3960
   tacagccggt gcaaccccaa ctacaagccc cccaaagaaa tcgaagtgtg atgactcgag 4020
   tttttattga ctaggggttt ttatagctaa ttagtcaaat gtgagttaat attagtatac 4080
   tacattacta atttattaca tattcattta tatcaatcta gtagcattta gcttttataa 4140
   aacaatataa ctgaatagta catactttac taataagtta taaataagag atacatattt 4200
   atagtatttt actttctaca ctgaatataa taatataatt atacaaatat aatttttaat 4260
   actatatagt atataactga aataaaatac cagtgtaata tagttattat acatttatac 4320
   cacatcaaag atgagttata acatcagtgt cactgttagc aacagtagtt atacgatgag 4380
   tagttactct cgtatggcgt tagtatgtat gtatcttcta gttttcttag taggcattat 4440
   aggaaacgtc aagcttataa ggttattaat ggtatctaga aatatatcta ttataccgtt 4500
   tctcaacttg ggaatagccg atttgctgtt tgtgatattc atacctttat acattatata 4560
   catactaagt aatttccatt ggcattttgg taaagcactt tgtaaaatta gttctttctt 4620
   ttttacttct aacatgtttg caagtatatt tttaataact gtaataagcg tatatagata 4680
   tgtaaaaatt acccttcctg gatttaccta taaatatgtt aacattagaa atatgtacat 4740
   tactatattt ttcatatgga ttatttctat tatactaggg attcctgctc tttactttag 4800
   aaatactatc gtaacaaaaa ataacgacac gctgtgtatt aatcattatc atgataatag 4860
   agaaattgct gaattgattt acaaagttat tatctgtatc agatttattt taggatacct 4920
   actacctacg ataattatac tcgtatgcta tacgttactg atctacagaa ctaacaatgc 4980
   atctaatata tctga 4995
<210> 94
   <211> 5040
   <212> DNA
   <213> Lutzomyia longipalpis
<400> 94
   cgagtccttc taacactgtg gtttattggc tggaataaaa ggataaagac acctatactg      60
   attcattttc atctgtcaac gtttctctaa gagattcata ggtattatta ttacatcgat     120
   ctagaagtct aataactgct aagtatatta ttggatttaa cgcgctataa acgcatccaa     180
   aacctacaaa tataggagaa gcttctctta tgaaacttct taaagcttta ctcttactat     240
   tactactcaa aagagatatt acattaatta tgtgatgagg catccaacat ataaagaaga     300
   ctaaagctgt agaagctgtt atgaagaata tcttatcaga tatattagat gcattgttag     360
   ttctgtagat cagtaacgta tagcatacga gtataattat cgtaggtagt aggtatccta     420
   aaataaatct gatacagata ataactttgt aaatcaattc agcaatttct ctattatcat     480
   gataatgatt aatacacagc gtgtcgttat tttttgttac gatagtattt ctaaagtaaa     540
   gagcaggaat ccctagtata atagaaataa tccatatgaa aaatatagta atgtacatat     600
   ttctaatgtt aacatattta taggtaaatc caggaagggt aatttttaca tatctatata     660
   cgcttattac agttattaaa aatatacttg caaacatgtt agaagtaaaa aagaaagaac     720
   taattttaca aagtgcttta ccaaaatgcc aatggaaatt acttagtatg tatataatgt     780
   ataaaggtat gaatatcaca aacagcaaat cggctattcc caagttgaga aacggtataa     840
   tagatatatt tctagatacc attaataacc ttataagctt gacgtttcct ataatgccta     900
   ctaagaaaac tagaagatac atacatacta acgccatacg agagtaacta ctcatcgtat     960
   aactactgtt gctaacagtg acactgatgt tataactcat ctttgatgtg gtataaatgt    1020
   ataataacta tattacactg gtattttatt tcagttatat actatatagt attaaaaatt    1080
   atatttgtat aattatatta ttatattcag tgtagaaagt aaaatactat aaatatgtat    1140
   ctcttattta taacttatta gtaaagtatg tactattcag ttatattgtt ttataaaagc    1200
   taaatgctac tagattgata taaatgaata tgtaataaat tagtaatgta gtatactaat    1260
   attaactcac atttgactaa ttagctataa aaacccgggt taattaatta gtcatcaggc    1320
   agggcgagaa cgagactatc tgctcgttaa ttaattagag cttctttatt ctatacttaa    1380
   aaagtgaaaa taaatacaaa ggttcttgag ggttgtgtta aattgaaagc gagaaataat    1440
   cataaattat ttcattatcg cgatatccgt taagtttgta tcgtaatgaa cagcatcaac    1500
   tttctgagca tcgtgggcct gatcagcttc ggcttcatcg tggccgtgaa gtgcgacggc 1560
   gacgagtact tcatcggcaa gtacaaagag aaggacgaga ccctgttctt cgccagctac 1620
   ggcctgaagc gggacccctg ccagatcgtg ctgggctaca agtgcagcaa caaccagacc 1680
   cacttcgtgc tgaacttcaa gaccaacaag aagagctgca tcagcgccat caagctgacc 1740
   agctacccca agatcaacca gaacagcgac ctgaccaaga acctgtactg ccagaccggc 1800
   ggcatcggca ccgacaactg caagctggtg ttcaagaagc ggaagcggca gatcgccgcc 1860
   aacatcgaga tctacggcat ccccgccaag aagtgcagct tcaaggaccg gtacatcggc 1920
   gccgaccccc tgcacgtgga ctcctacggc ctgccctacc agttcgacca ggaacacggc 1980
   tggaacgtcg agcggtacaa catcttcaag gacacccggt tcagcaccga ggtgttctac 2040
   cacaagaacg gcctgttcaa cacccagatc acctacctgg ccgaagagga cagcttcagc 2100
   gaggcccggg agatcaccgc caaggacatc aagaagaagt tcagcatcat cctgcccaac 2160
   gaggaataca agcggatcag cttcctggac gtgtactggt tccaggaaac catgcggaag 2220
   aagcccaagt acccctacat ccactacaac ggcgagtgct ccaacgagaa caagacctgc 2280
   gaactggtgt tcgacaccga cgagctgatg acctacgccc tggtgaaggt gttcaccaac 2340
   cccgagagcg acggcagccg gctgaaagaa gaggacctgg gcaggggctg atgactcgag 2400
   tttttattga ctagttaatc aaataaaaag catacaagct attgcttcgc tatcgttaca 2460
   aaatggcagg aattttgtgt aaactaagcc acatacttgc caatgaaaaa aatagtagaa 2520
   aggatactat tttaatggga ttagatgtta aggttccttg ggattatagt aactgggcat 2580
   ctgttaactt ttacgacgtt aggttagata ctgatgttac agattataat aatgttacaa 2640
   taaaatacat gacaggatgt gatatttttc ctcatataac tcttggaata gcaaatatgg 2700
   atcaatgtga tagatttgaa aatttcaaaa agcaaataac tgatcaagat ttacagacta 2760
   tttctatagt ctgtaaagaa gagatgtgtt ttcctcagag taacgcctct aaacagttgg 2820
   gagcgaaagg atgcgctgta gttatgaaac tggaggtatc tgatgaactt agagccctaa 2880
   gaaatgttct gctgaatgcg gtaccctgtt cgaaggacgt gtttggtgat atcacagtag 2940
   ataatccgtg gaatcctcac ataacagtag gatatgttaa ggaggacgat gtcgaaaaca 3000
   agaaacgcct aatggagtgc atgtccaagt ttagggggca agaaatacaa gttctaggat 3060
   ggtattaata agtatctaag tatttggtat aatttattaa atagtataat tataacaaat 3120
   aataaataac atgataacgg tttttattag aataaaatag agataatatc ataatgatat 3180
   ataatacttc attaccagaa atgagtaatg gaagacttat aaatgaactg cataaagcta 3240
   taaggtatag agatataaat ttagtaaggt atatacttaa aaaatgcaaa tacaataacg 3300
   taaatatact atcaacgtct ttgtatttag ccgtaagtat ttctgatata gaaatggtaa 3360
   aattattact agaacacggt gccgatattt taaaatgtaa aaatcctcct cttcataaag     3420
   ctgctagttt agataataca gaaattgcta aactactaat agattctggc gctgacatag     3480
   aacagataca ttctggaaat agtccgttat atatttctgt atatagaaac aataagtcat     3540
   taactagata tttattaaaa aaaggtgtta attgtaatag attctttcta aattattacg     3600
   atgtactgta tgataagata tctgatgata tgtataaaat atttatagat tttaatattg     3660
   atcttaatat acaaactaga aattttgaaa ctccgttaca ttacgctata aagtataaga     3720
   atatagattt aattaggata ttgttagata atagtattaa aatagataaa agtttatttt     3780
   tgcataaaca gtatctcata aaggcactta aaaataattg tagttacgat ataatagcgt     3840
   tacttataaa tcacggagtg cctataaacg aacaagatga tttaggtaaa accccattac     3900
   atcattcggt aattaataga agaaaagatg taacagcact tctgttaaat ctaggagctg     3960
   atataaacgt aatagatgac tgtatgggca gtcccttaca ttacgctgtt tcacgtaacg     4020
   atatcgaaac aacaaagaca cttttagaaa gaggatctaa tgttaatgtg gttaataatc     4080
   atatagatac cgttctaaat atagctgttg catctaaaaa caaaactata gtaaacttat     4140
   tactgaagta cggtactgat acaaagttgg taggattaga taaacatgtt attcacatag     4200
   ctatagaaat gaaagatatt aatatactga atgcgatctt attatatggt tgctatgtaa     4260
   acgtctataa tcataaaggt ttcactcctc tatacatggc agttagttct atgaaaacag     4320
   aatttgttaa actcttactt gaccacggtg cttacgtaaa tgctaaagct aagttatctg     4380
   gaaatactcc tttacataaa gctatgttat ctaatagttt taataatata aaattacttt     4440
   tatcttataa cgccgactat aattctctaa ataatcacgg taatacgcct ctaacttgtg     4500
   ttagcttttt agatgacaag atagctatta tgataatatc taaaatgatg ttagaaatat     4560
   ctaaaaatcc tgaaatagct aattcagaag gttttatagt aaacatggaa catataaaca     4620
   gtaataaaag actactatct ataaaagaat catgcgaaaa agaactagat gttataacac     4680
   atataaagtt aaattctata tattctttta atatctttct tgacaataac atagatctta     4740
   tggtaaagtt cgtaactaat cctagagtta ataagatacc tgcatgtata cgtatatata     4800
   gggaattaat acggaaaaat aaatcattag cttttcatag acatcagcta atagttaaag     4860
   ctgtaaaaga gagtaagaat ctaggaataa taggtaggtt acctatagat atcaaacata     4920
   taataatgga actattaagt aataatgatt tacattctgt tatcaccagc tgttgtaacc     4980
   cagtagtata aagtgatttt attcaattac gaagataaac attaaatttg ttaacagata     5040

## Description

This application claims benefit of US provisional application Serial No. 61/051,635 filed May 8, 2008, and of US provisional application Serial No. 61/101,345 filed September 30, 2008, which make reference to International Application No. PCT/US2003/034453 entitled Lutzomyia Longipalpis Polypeptides and Methods of Use filed October 29, 2003, which claims priority to provisional application No. 60/422,301 filed October 29, 2002.

All documents cited or referenced herein ("herein cited documents"), and all documents cited or referenced in herein cited documents, together with any manufacturer's instructions, descriptions, product specifications, and product sheets for any products mentioned herein may be employed in the practice of the invention.

### FIELD

The present invention relates to formulations for combating Leishmania infections in canines. Specifically, the present description provides vectors that contain and express in *vivo* or *in vitro* sand fly *Lu. longipalpis* salivary antigens that elicit an immune response in animal or human against Leishmania, including compositions comprising said vectors, methods of vaccination against *Leishmania,* and kits for use with such methods and compositions.

### BACKGROUND OF THE INVENTION

Leishmaniasis is a major and severe parasitic disease that affects humans, canines (dogs, wolves, foxes, coyotes, jackals), and to a lesser degree, felines (lions, tigers, domestic cats, wild cats, other big cats, and other felines including cheetahs and lynx).

Leishmania and Viannia subgenera are grouped into complexes of .species and subspecies based upon molecular, biochemical and immunological similarities. There are several forms of the disease named by their clinical presentation including cutaneous, mucocutaneous or visceral leishmaniasis. Each of these forms of disease is caused· by different species of sand flies found in different regions of the world. Cutaneous leishmaniasis of humans is associated with members of L. aethiopica, L. major, and L. tropica complexes in the Old World and L. mexicana and L. braziliensis complexes in the New World. Visceral leishmaniasis is caused by L. donovani and L. infautum in Old World regions while L chagasi is primarily responsible for visceral disease in the New World. Because L. infantum is the primary agent associated with canine leishmaniasis, infections in dogs often are regarded as visceral even though they tend to cause both visceral and cutaneous disease.

The agent of visceral leishmaniasis is a protozoan parasite and belongs to the *leishmania donovani* complex. This parasite is widely distributed in temperate and subtropical countries of Southern Europe, Africa, Asia, South America and Central America (Desjeux P. *et al.*). *Leishmania donovani infantum* (*L. infantum*) is responsible for the feline and canine disease in Southern Europe, Africa, and Asia. In South America and Central America, the agent is *Leishmania donovani chagasi* (*L. chagasi*), which is closely related to *L. infantum.* In humans, the agent is *Leishmania donovani donovani* (*L. donovani*), which is also related to *L. infantum and L. chagasi.*

Sand flies of the genus Phlebotomus (Old World) and Lutzomyia (New World) are the primary vectors responsible for disease transmission. Currently these are the only known vectors capable of spread; fleas, ticks and other arthropods have not been shown to be competent vectors. However, rare cases of leishmaniasis have been contracted through exchange of blood or body fluids, direct contact and at least one case of congenital transmission. The importance of native sand flies is yet undetermined but could be related to infectious dose of the organism. Still, in recent years, and in the absence of known vectors, there has been an overwhelming incidence of leishmaniasis in Foxhound kennels across the United States. It is still not known how transmission of disease occurred or how this disease is maintained in these dogs because infected sand flies have not been reported in the United States. However, certain species of Lutzomyia (L. shannoni), found along the eastern United States and as far north as New Jersey, are considered a potentially competent vector for L. mexicana. *Phlebotomus ariasi* (*P. ariasi*) and *Phlebotomus perniciosus* (*P. perniciosus*), *Phlebotomus neglectus* (*P. neglectus*) are the most common carriers in Southern Europe, Africa, and Asia, whereas *Lutzomyia longipalpis* (*Lu. longipalpis*) is the most common carrier in Southern and Central America.

Leishmaniasis is a slowly progressive disease that can take up to 7 years to become clinically apparent (McConkey SE *et al.;* Slappendel RJ *et al*.). Even then, signs are frequently nonspecific and a diagnosis of Leishmania is seldom considered. Dogs are most commonly infected with L. infantum (L. donovani complex) which is responsible for viscerotropic disease in people. However, up to 90% of infected dogs present with both visceral and cutaneous lesions (Slappendel RJ *et al*.). On the other hand, many dogs appear naturally resistant to this parasite and may remain asymptomatic despite known infection (Grosjean NL *et al.*)*.* It is estimated that only 10% of dogs residing in endemic areas actually develop clinical disease (Lindsay DS *et al.*)*.* This lower incidence of clinical disease is attributed to a genetic predisposition of certain dogs to mount a more protective cell-mediated immune response than a humoral response (Lindsay DS *et al.,* McConkey SE *et al.,* Slappendel RJ, *et al.*)*.* Furthermore, it has been reported that up to 20% of infected dogs may mount an adequate immune response and spontaneously recover from clinical illness (McConkey SE *et al.*)*.* In animals that mount a humoral response, IgG1 appears to correlate with clinical disease while asymptomatic dogs have higher IgG2 antibody levels (Lindsay *et al.*)*.*

Some of the more frequently reported clinical signs of leishmaniasis include listlessness, fatigue and exercise intolerance coupled with anorexia and weight loss that eventually culminate as wasting disease (McConkey SE *et al.*)*.* These signs may or may not be accompanied by fever, local or generalized lymphadenopathy (90%) and/or hepatosplenomegaly (Grosjean NL *et al.,* Lindsay DS *et al.,* McConkey SE *et al.,* Martinez-Subiela S *et al.*)*.* Articular involvement is also fairly common and may present as lameness with swollen joints or simply as a stiff gait. Less common findings include ocular lesions (<5%), chronic diarrhea (30%) and long, deformed brittle nails (20%) referred to as onychogryphosis (Lindsay DS *et al.,* Slappendel RJ *et al*.). Cutaneous lesions are present in up to 89% of infected dogs, with or without overt signs of visceral involvement. Lesions of cutaneous leishmaniasis may occur anywhere on the body but the most common sites are those which are exposed to the environment and are therefore more susceptible to bites from the sand flies. The initial papule rapidly gives rise to an ulcer. Viseral leishmaniasis is invariably fatal if not treated promptly. Viseral leishmaniasis affects the internal body organs, specifically the spleen and the liver.

Dogs are considered the major reservoir of Leishmaniasis. The disease is characterized by chronic evolution of viscero-cutaneous signs occurring in less than 50% of infected animals (Lanotte G. *et al*.). Both asymptomatic and symptomatic dogs with detectable antibodies may be infectious (Molina R. *et al.;* Courtenay O. *et al.*)*.* Cats may also be carriers of the protozoan parasites and are thus considered secondary potential reservoirs.

Due to a number of factors, treatment options for leishmaniasis in dogs and response to therapy are limited at best. For some undefined reason, visceral leishmaniasis is more difficult to treat in dogs than in humans. No treatment option is 100% effective in clearing parasitic infection and clinical disease often reappears with cessation of therapy (Lindsay DS *et al.*)*.* In endemic areas, the most common treatment regimen has been a combination of allopurinol with a pentavalent antimonial such as meglumine antimonite or sodium stibogluconate (Lindsay DS *et al.,* Slappendel RJ *et al*.). However, in recent years this protocol has fallen out of favor due to increasing resistance of the parasite to the drug as well as adverse side effects associated with these compounds (Lindsay DS *et al.*)*.* To further limit treatment options, Pentostam® (sodium stibogluconate) is the only available antimonial in the United States and its distribution is regulated by the Centers for Disease Control and Prevention (CDC) in Atlanta, GA (Lindsay DS *et al.*)*.*

Other protocols have been tried but have proven no more efficacious at clearing parasitic infection or at preventing clinical relapse. In addition, each protocol is associated with potential adverse effects. Amphotericin B binds sterols and disrupts cell membrane permeability but is nephrotoxic (Lindsay DS *et al.*)*.* When given parenterally, Paramomycin acts synergistically with antimonials causing higher levels of the antimonial for longer periods of time but is also nephrotoxic and is not currently recommended for clinical use (Lindsay DS *et al.).* Pentamidine isethionate is effective against leishmaniasis but requires at least 15 intramuscular injections and is quite painful (Lindsay DS *et al.*)*.* Ketaconazole, miconazole, fluconazole and itraconazole are oral drugs that may be useful in containing the disease but are cost prohibitive and carry the risk of drug resistance when treating patients symptomatically. In summary, the various treatment regimens for leishmaniasis in dogs have been investigated but are not 100% efficacious; relapses are the rule rather than the exception. Ultimately, the veterinary practitioner is faced with the dilemma of treating symptomatic outbreaks of leishmaniasis in dogs at the risk of developing drug resistant strains of this parasite within the United States.

Mass detection of seropositive dogs followed by culling and/or drug treatment, or the mass application of deltamethrin-impregnated collars, was shown to have an impact in reducing human and canine Leishmaniasis prevalence in endemic areas of Southern Europe, Africa, and Asia (Maroli M. *et al.* Mazloumi Gavgani A.S. *et al.*)*,* although the efficacy of eliminating seropositive canines has been debated (Dietze R. *et al.;* Moreira Jr. E.D. *et al*.). These control measures are either considered unacceptable, expensive or not effective (Gradoni L. *et al*.).

Mathematical models used to compare the effectiveness of various tools for controlling Leishmaniasis suggest that a canine vaccine may be the most practical and effective method (Dye C.). Therefore, the development of vaccines able to protect canines from Leishmaniasis and/or to prevent disease progression in infected animals is highly desirable for the implementation of Leishmaniasis control programs as well for the veterinary community (Gradoni L. *et al.).*

Previous investigations have sought to identify methods of diagnosing and treating Leishmania through, for example, administration of antigenic polypeptides (see, for example, WO 2004/039958 and US 2006/051364. However, to date, no vaccine is available for the treatment of *Leishmania.* The vectors and vaccine formulations of the present description fulfill this long-felt need in the art.

Citation or identification of any document in this application is not an admission that such document is available as prior art to the present invention.

### SUMMARY OF THE INVENTION

The present invention relates to a prima-administration vaccine and a boost administration vaccine for use in protecting a subject from leishmaniasis and/or preventing disease progression in an infected subject, wherein the subject is a canine,
wherein the vaccines are formulated for administration in a prime-boost administration regimen comprising a prima-administration with the prima-administration vaccine followed by a boost administration with the boost administration vaccine,
wherein said prima-administration vaccine comprises, in a pharmaceutically or veterinary acceptable vehicle, diluent or excipient, an expression vector containing a polynucleotide for expressing, *in vivo,* a salivary *Lu. longipalpis* polypeptide, and
said boost administration vaccine comprises, in a pharmaceutically or veterinary acceptable vehicle or excipient, the same salivary *Lu. longipalpis* polypeptide,
wherein the salivary *Lu. longipalpis* polypeptide is a LJM17 polypeptide,
wherein the polynucleotide encodes a polypeptide having at least 80% sequence identity to a polypeptide having the sequence as set forth in SEQ ID NO: 5, 7, 15, or 17; or the polynucleotide has at least 70% sequence identity to a polynucleotide encoding a polypeptide having the sequence as set forth in SEQ ID NO: 5, 7, 15, or 17; or the polynucleotide has at least 70% sequence identity to a polynucleotide having the sequence as set forth in SEQ ID NO: 6, 8, 16, 18, 21, 90, or 91; and
wherein the *Lu. longipalpis* polypeptide comprises an amino acid sequence having at least 80% sequence identity to a polypeptide having the sequence as set forth in SEQ ID NO: 5, 7, 15, or 17.

An aim of this description can be any one or all of providing recombinant vectors or viruses as well as methods for making such viruses, and providing compositions and/or vaccines as well as methods for treatment and prophylaxis of infection by Leishmania.

Described herein is a recombinant vector, such as a recombinant virus, e.g., a recombinant poxvirus, that contains and expresses at least one exogenous nucleic acid molecule and, the at least one exogenous nucleic acid molecule may comprise a nucleic acid molecule encoding an immunogen or epitope of interest from salivary proteins from sand fly vectors of *Lu. longipalpis.*

Described herein is a recombinant vector, such as a recombinant virus, e.g., a recombinant poxvirus, that contains and expresses at least one exogenous nucleic acid molecule and, the at least one exogenous nucleic acid molecule may comprise salivary *Lu. longipalpis* polypeptides and/or variants or fragments thereof.

These vaccines may prevent diffusion and/or replication of the parasite in a host.

The description further provides immunological (or immunogenic), or vaccine compositions comprising such an expression vector or the expression product(s) of such an expression vector.

The description further provides methods fur inducing an immunological (or immunogenic) or protective response against Leishmania, as well as methods for preventing or treating Leishmania or disease state(s) caused by Leishmania, comprising administering the expression vector or an expression product of the expression vector, or a composition comprising the expression vector, or a composition comprising an expression product of the expression vector.

The description also relates to expression products from the virus as well as antibodies generated from the expression products or the expression thereof in *vivo* and uses for such products and antibodies, e.g., in diagnostic applications.

These and other embodiments are disclosed or are obvious from and encompassed by, the following Detailed Description.

### BRIEF DESCRIPTION OF DRAWINGS

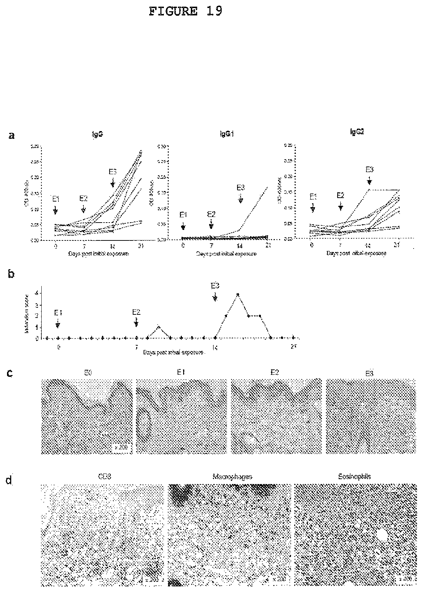

The following detailed description, given by way of example, and which is not intended to limit the invention to specific embodiments described, may be understood in conjunction with the accompanying figures, incorporated herein by reference, in which:
Figure 1 shows the nucleic acid sequence of one strand of the plasmid pVR2001 LJM17 (SEQ ID N0:9), wherein the two BamHI restriction sites are in bold, the sequence encoding the tPA signal peptide is underlined and the sequence encoding the LJM17 is in bold capital letters.
Figure 2 shows the nucleic acid sequence of one strand of the plasmid pVR2001 LJL143 (SEQ ID NO: 10), wherein the two BamHI restriction sites are in bold, the sequence encoding the tPA signal peptide is underlined and the sequence encoding the LJL143 is in bold capital letters.
Figure 3 shows a map of the donor vector, pALVAC C3 H6p-LJM17, having 5737 base pairs.
Figure 4 illustrates the vCP2390 canarypox expression vector, for both forward and reverse complementary strands (SEQ ID NO:93 and SEQ ID NO:92). SEQ ID NO:93 represents the vCP2390 vector strand which contains the codon-optimized LJM17 polynucleotide in the direction that encodes the LJM17 polypeptide (SEQ ID NO:5).
Figure 5 shows a map of the donor vector, pALVAC C3 H6p-LJL143, having 5400 base pairs.
Figure 6 illustrates the vCP2389 canarypox expression vector with its insert encoding LJL143, and provides the nucleotide sequence of one strand of the expression vector (SEQ ID NO:94).
Figure 7 illustrates anti-IgG antibodies to both LJM17 and LJL143, absorbance measured at 405 nm, in vaccinated and control dogs, from one day before V1 administration to two weeks after V5 administration.
Figure 8 illustrates interferon-gamma secretion (pg/mL) from dogs PBMC corresponding to the 5 groups, 2 weeks after the 5^{th} immunization (V5 administration). PBMC were stimulated by SGH (2 pairs), LJL143 (4 µg), LJM17 (4 µg), ConA (4 µg) or non-stimulated by medium (med).
Figure 9 shows a scheme of an *in vitro* killing assay including results, expressed in percentage of infected macrophages (NT: no treatment, LPS: lipopolysaccharide, conA: concavaline A).
Figure 10 shows biopsies of vaccinated and control dogs, at the sand flies bite sites, stained with hematoxylin/eosin (H & E), Luna's, Toludine blue and immunohistochemical procedures for CD3 and macrophage/monocyte markers (Mac).
Figure 11 shows the nucleic acid sequence of one strand of the plasmid pNBO002 (SEQ ID NO: 19), wherein the two BamHI restriction sites are in bold, the sequence encoding the tPA signal peptide is underlined and the sequence encoding the LJM17 is in bold capital letters.
Figure 12 shows a map of the pNBO002 plasmid vector with its insert encoding LJM17, having 6247 base pairs.
Figure 13 shows the nucleic acid sequence of one strand of the plasmid pNBO003 (SEQ ID NO:20), wherein the two BamHI restriction sites are in bold, the sequence encoding the tPA signal peptide is underlined and the sequence encoding the LJL143 is in bold capital letters.
Figure 14 shows a map of the pNBO003 plasmid vector with its insert encoding LJL143, having 5899 base pairs.

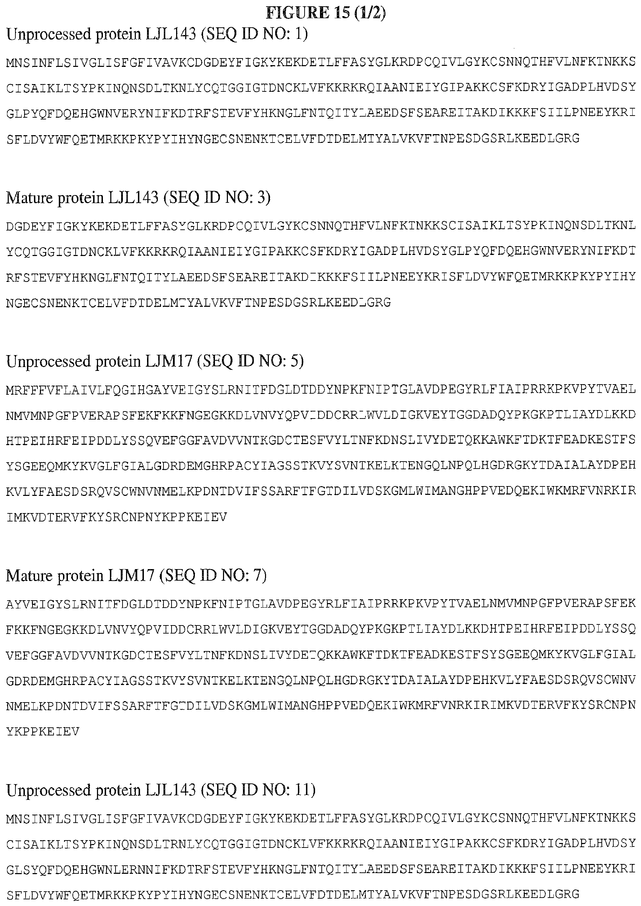

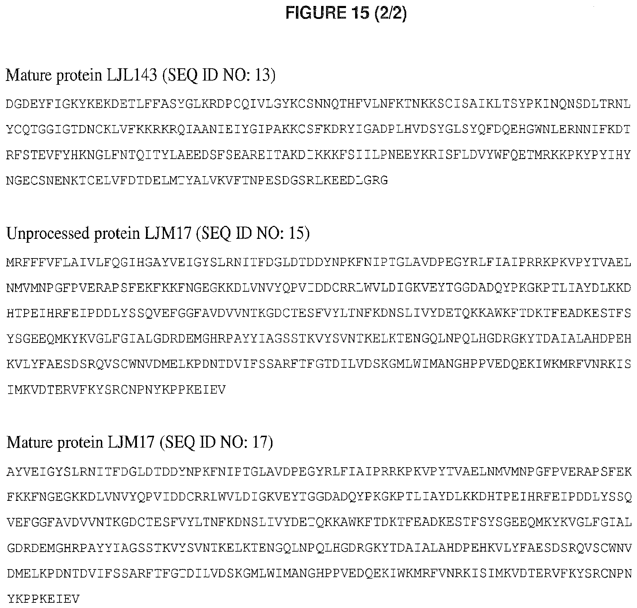

Figure 15 shows the protein sequences of LJL143 and LJM17 from *L. longipalpis.*

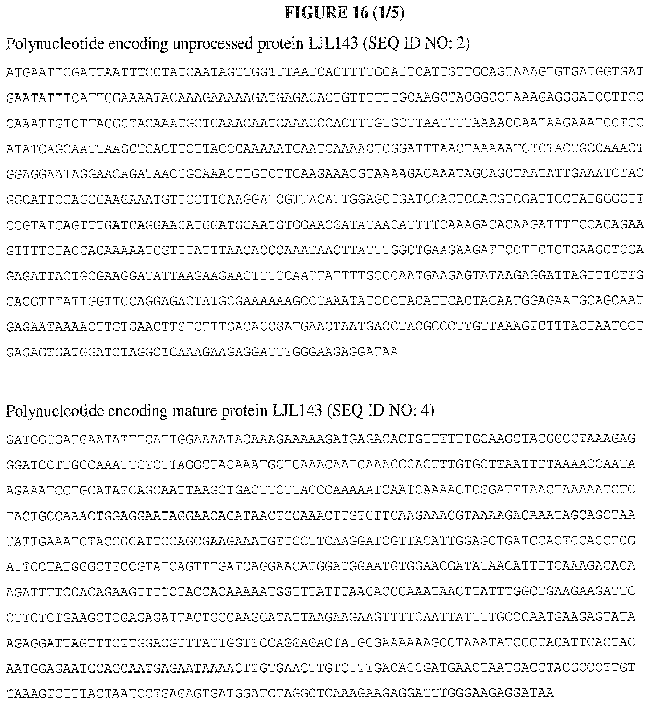

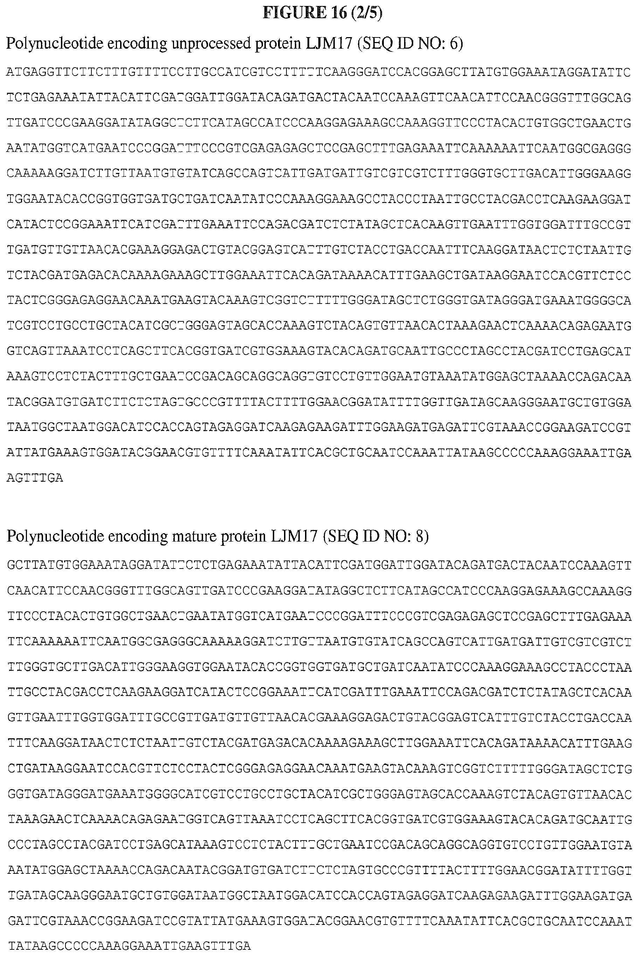

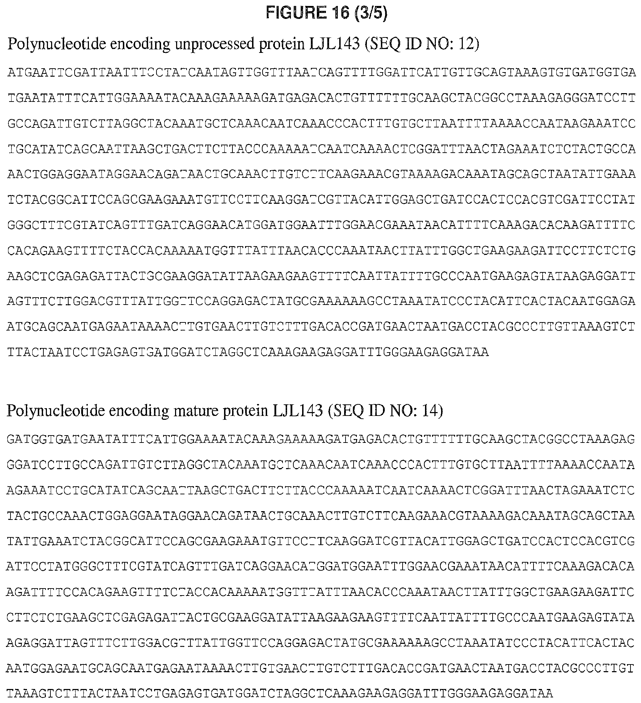

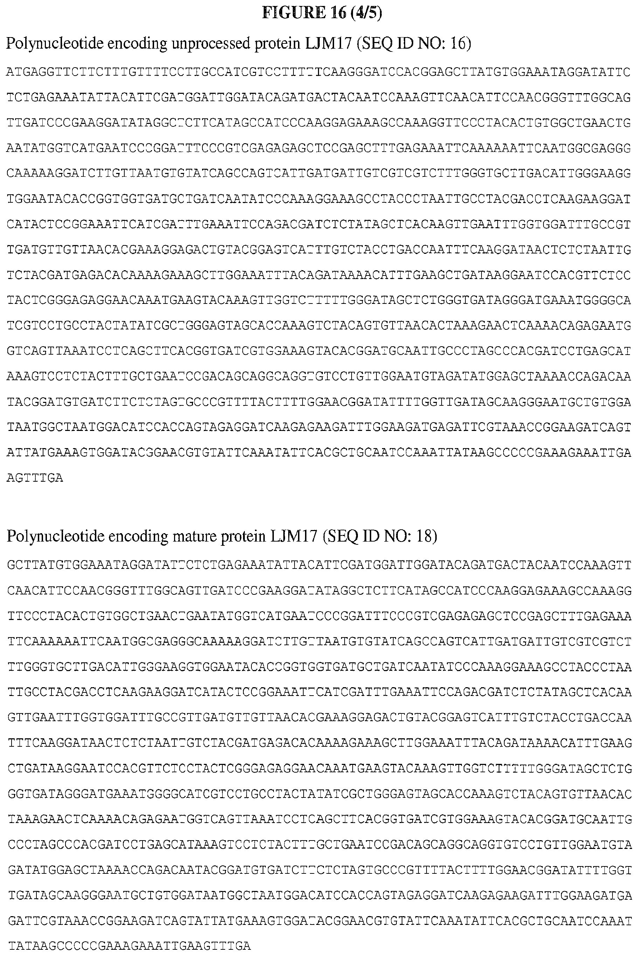

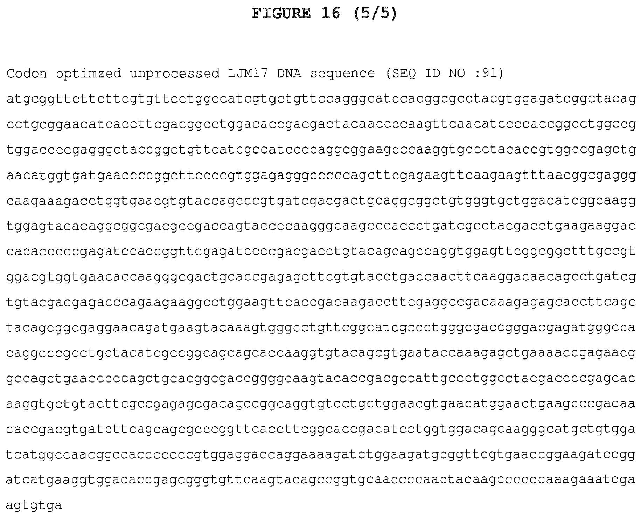

Figure 16 shows the DNA sequences of LJL143 and LJM17 from *L. longipalpis.*

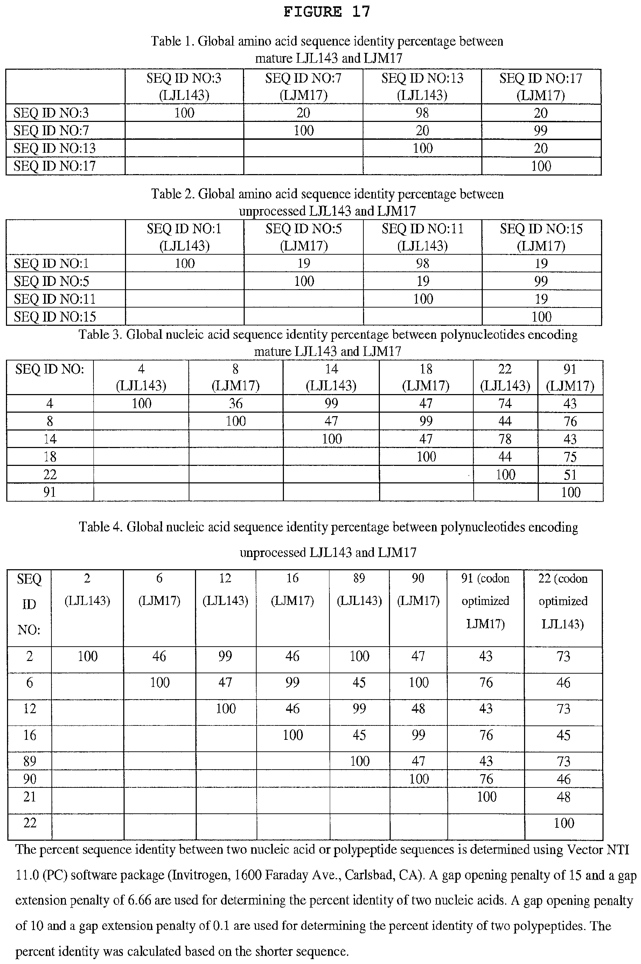

Figure 17 shows the sequence identity tables.

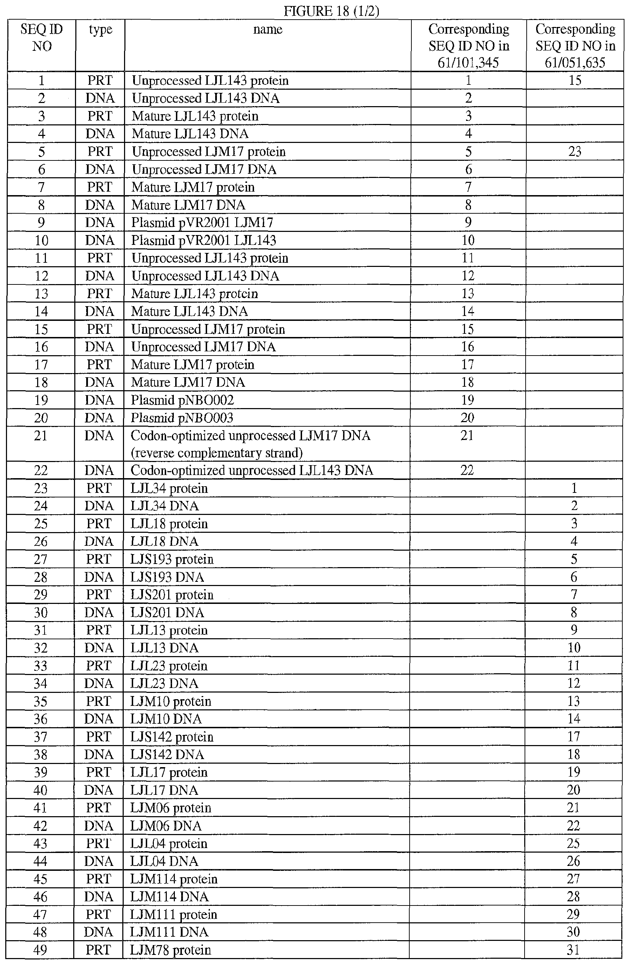

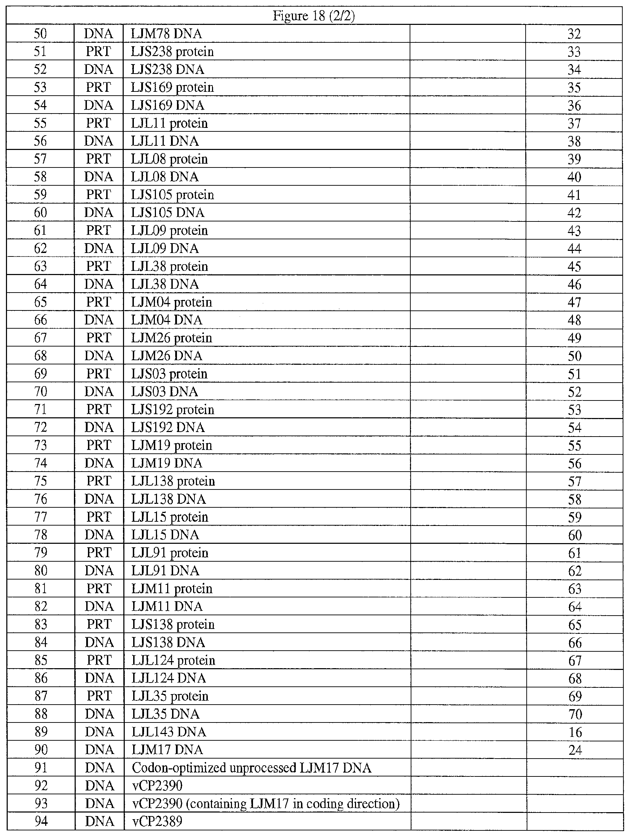

Figure 18 shows the SEQ ID NO assigned to each DNA and polypeptide.
Figuree 19 is a set of graphs and images demonstrating anti-saliva immunity in dogs exposed to the bites of the *Lu. longipalpis* sand fly. FIG. 19A is a set of graphs demonstrating the early kinetics of anti-*Lu*. *longipalpis* IgG, IgG2, and IgG1 antibody titers in exposed dogs. FIG. 19B is a graph of a delayed-type hypersensitivity reaction on a representative dog throughout exposure experiments. FIG. 19C is a set of images of an H/E histological analysis performed on skin punch biopsies before exposure (E0), 47 h after first exposure (E1), 48 h after second exposure (E2) and 48 h after third exposure (E3) to sand fly bites. FIG. 19D is a set of images characterizing the inflammatory population at 48 h after third exposure (E3) to sand fly bites with immunohistochemistry for CD3+ T lymphocytes and macrophages and Luna's stain for eosinophils.

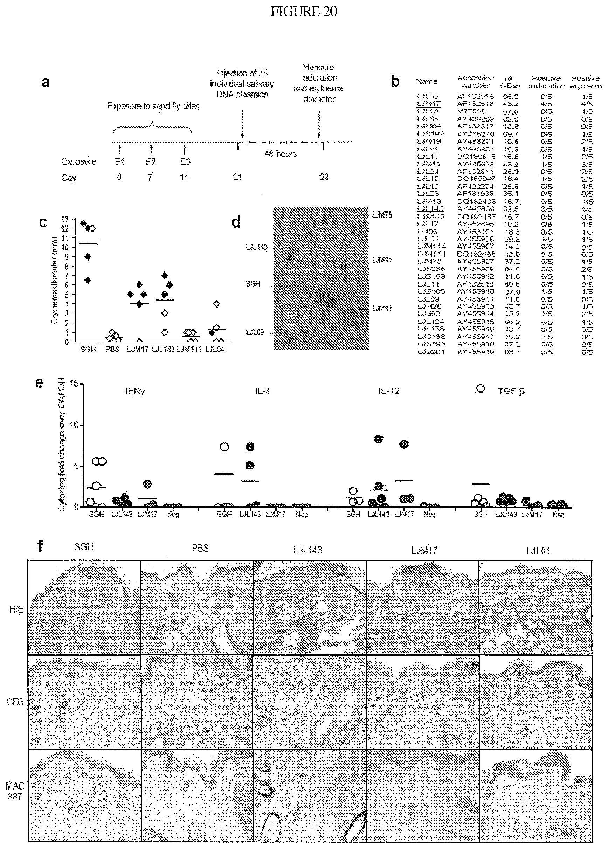

Figure 20 is a set of graphs and images demonstrating a cDNA reverse antigen screening assay in dogs.

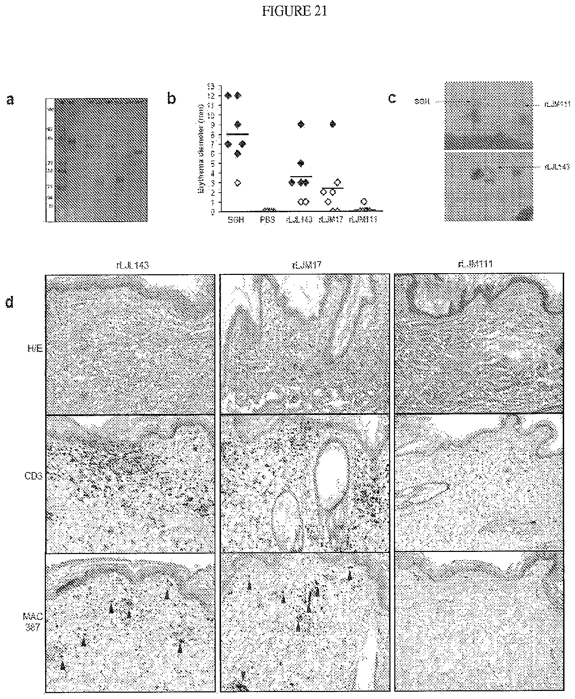

Figure 21 is a set of graphs and images demonstrating a protein reverse antigen screening assay in dogs.

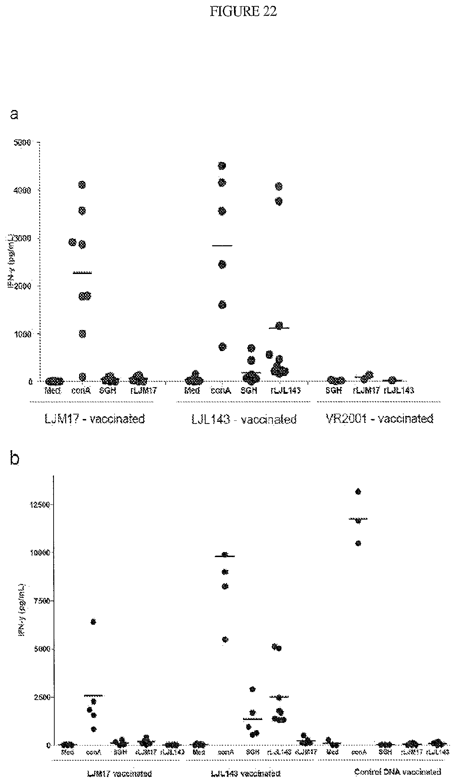

Figure 22 is a set of two graphs demonstrating interferon y production of peripheral blood mononuclear cells (PBMCs) from vaccinated dogs after stimulation with recombinant salivary protein.

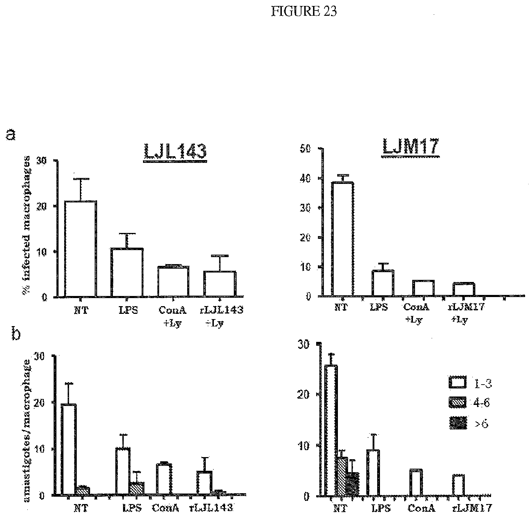

Figure 23 is a set of four bar graphs demonstrating an *in vitro* killing assay for *Leishmania chagasi* by PBMCs from immunized dogs (NT, no treatment; Ly, lymphocytes).

### DETAILED DESCRIPTION

Unless otherwise noted, technical terms are used according to conventional usage. Definitions of common terms in molecular biology may be found in Benjamin Lewin, Genes V. published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8). The singular terms "a," "an," and "the" include plural referents unless context clearly indicates otherwise. Similarly, the word "or" is intended to include "and" unless the context clearly indicate otherwise. The word "or" means any one member of a particular list and also includes any combination of members of that list.

It is noted in this disclosure and the appended claims and/or paragraphs, the term "salivary *Lu. longipalpis* polypeptide", *"Lu. longipalpis* polypeptide", or "salivary polypeptide" is used interchangeably, the term "salivary *Lu. longipalpis* polynucleotide", *"Lu. longipalpis* polynucleotide", or "salivary polynucleotide" is used interchangeably.

The terms "polypeptide" and "protein" are used interchangeably herein to refer to a polymer of consecutive amino acid residues.

The term "nucleic acid", "nucleotide", and "polynucleotide" refers to RNA or DNA and derivatives thereof, such as those containing modified backbones. It should be appreciated that the description provides polynucleotides comprising sequences complementary to those described herein. Polynucleotides according to the description can be prepared in different ways (e.g. by chemical synthesis, by gene cloning etc.) and can take various forms (e.g. linear or branched, single or double stranded, or a hybrid thereof, primers, probes etc.).

The term "gene" is used broadly to refer to any segment of polynucleotide associated with a biological function. Thus, genes or polynucleotides include introns and exons as in genomic sequence, or just the coding sequences as in cDNAs, such as an open reading frame (ORF), starting from the start codon (methionine codon) and ending with a termination signal (stop codon). Genes and polynucleotides can also include regions that regulate their expression, such as transcription initiation, translation and transcription termination. Thus, also included are promoters and ribosome binding regions (in general these regulatory elements lie approximately between 60 and 250 nucleotides upstream of the start codon of the coding sequence or gene; Doree S M *et al.;* Pandher K *et al.;* Chung J Y *et al),* transcription terminators (in general the terminator is located within approximately 50 nucleotides downstream of the stop codon of the coding sequence or gene; Ward CK *et al.).* Gene or polynucleotide also refers to a nucleic acid fragment that expresses mRNA or functional RNA, or encodes a specific protein, and which includes regulatory sequences.

The term "immunogenic polypeptide" or "immunogenic fragment" as used herein refers to a polypeptide or a fragment of a polypeptide which comprises an allele-specific motif, an epitope or other sequence such that the polypeptide or the fragment will bind an MHC molecule and induce a cytotoxic T lymphocyte ("CTL") response, and/or a B cell response (for example, antibody production), and/or T-helper lymphocyte response, and/or a delayed type hypersensitivity (DTH) response against the antigen from which the immunogenic polypeptide or the immunogenic fragment is derived. A DTH response is an immune reaction in which T cell-dependent macrophage activation and inflammation cause tissue injury. A DTH reaction to the subcutaneous injection of antigen is often used as an assay for cell-mediated immunity.

By definition, an epitope is an antigenic determinant that is immunologically active in the sense that once administered to the host, it is able to evoke an immune response of the humoral (B cells) and/or cellular type (T cells). These are particular chemical groups or peptide sequences on a molecule that are antigenic. An antibody specifically binds a particular antigenic epitope on a polypeptide. Specific, non-limiting examples of an epitope include a tetra- to pentapeptide sequence in a polypeptide, a tri- to penta-glycoside sequence in a polysaccharide. In the animal most antigens will present several or even many antigenic determinants simultaneously. Such a polypeptide may also be qualified as an immunogenic polypeptide and the epitope may be identified as described further.

An "isolated" biological component (such as a nucleic acid or protein or organelle) refers to a component that has been substantially separated or purified away from other biological components in the cell of the organism in which the component naturally occurs, for instance, other chromosomal and extra-chromosomal DNA and RNA, proteins, and organelles. Nucleic acids and proteins that have been "isolated" include nucleic acids and proteins purified by standard purification methods. The term also embraces nucleic acids and proteins prepared by recombinant technology as well as chemical synthesis.

The term "purified" as used herein does not require absolute purity; rather, it is intended as a relative term. Thus, for example, a purified polypeptide preparation is one in which the polypeptide is more enriched than the polypeptide is in its natural environment. A polypeptide preparation is substantially purified such that the polypeptide represents several embodiments at least 60%, at least 70%, at least 80%, at least 90%, at least 95%, or at least 98%, of the total polypeptide content of the preparation. The same applies to polynucleotides. The polypeptides disclosed herein can be purified by any of the means known in the art.

A recombinant polynucleotide is one that has a sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, for example, by genetic engineering techniques. In one embodiment, a recombinant polynucleotide encodes a fusion protein.

Described herein are polypeptides from sand fly species *Lu. longipalpis.* Described herein is a polypeptide having a sequence as set forth in SEQ ID NO: 1, 3, 5, 7, 11, 13, 15, 17, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, or 87, and variant or fragment thereof.

As used herein, the term "homologs" includes orthologs, analogs and paralogs. The term "analogs" refers to two polynucleotides or polypeptides that have the same or similar function, but that have evolved separately in unrelated organisms. The term "orthologs" refers to two polynucleotides or polypeptides from different species, but that have evolved from a common ancestral gene by speciation. Normally, orthologs encode polypeptides having the same or similar functions. The term "paralogs" refers to two polynucleotides or polypeptides that are related by duplication within a genome. Paralogs usually have different functions, but these functions may be related. Analogs, orthologs, and paralogs of a wild-type salivary polypeptide can differ from the wild-type salivary polypeptide by post-translational modifications, by amino acid sequence differences, or by both. In particular, homolog for use according to the invention will generally exhibit at least 80-85%, 85-90%, 90-95%, or 95%, 96%, 97%, 98%, 99% sequence identity, with all or part of the wild-type salivary polypeptide or polynucleotide sequences, and will exhibit a similar function.

Described herein is a polypeptide having at least 70%, at least 75%, at least 80%, at least 85%, at least 90%, at least 95%, 96%, 97%, 98% or 99% sequence identity to a polypeptide having a sequence as set forth in SEQ ID NO: 1, 3, 5, 7, 11, 13, 15, 17, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, or 87.

Described herein are .fragments and variants of the *L. longipalpis* polypeptides identified above (SEQ ID NO: 1, 3, 5, 7, 11, 13, 15, 17, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81,
83, 85, or 87) which may readily be prepared by one of skill in the art using well-known molecular biology techniques.

Variants are homologous polypeptides having an amino acid sequence at least 75%, 80%, 85%, 90%, 95%, 96%, 97%, 98%, or 99% identity to the amino acid sequence as set forth in SEQ ID NO: 1, 3, 5, 7, 11, 13, 15, 17, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, or 87.

Variants include allelic variants. The term "allelic variant" refers to a polynucleotide or a polypeptide containing polymorphisms that lead to changes in the amino acid sequences of a protein and that exist within a natural population (e.g., a virus species or variety). Such natural allelic variations can typically result in 1-5% variance in a polynucleotide or a polypeptide. Allelic variants can be identified by sequencing the nucleic acid sequence of interest in a number of different species, which can be readily carried out by using hybridization probes to identify the same gene genetic locus in those species. Any and all such nucleic acid variations and resulting amino acid polymorphisms or variations that are the result of natural allelic variation and that do not alter the functional activity of gene of interest are intended to be within the scope of the description.

A variant is any secreted polypeptide from *Lu. longipalpis* saliva, capable of inducing in animals, such as dogs, vaccinated with this polypeptide a specific cell-based immune response characterized by secretion of interferon gamma (IFN-gamma) upon stimulation by *Lu. longipalpis* salivary gland extract compounds. Such IFN-gamma secretion may be demonstrated using *in vitro* methodology (i.e. Qnantikine® immunoassay from R&D Systems Inc. (catalog number# CAIF00); Djoba Siawaya JF *et al.*)*.*

As used herein, the term "derivative" or "variant" refers to a polypeptide, or a nucleic acid encoding a polypeptide, that has one or more conservative amino acid variations or other minor modifications such that (1) tile corresponding polypeptide has substantially equivalent function when compared to the wild type polypeptide or (2) an antibody raised against the polypeptide is immunoreactive with the wild-type polypeptide. These variants or derivatives include polypeptides having minor modifications of the *Lu. longipalpis* polypeptide primary amino acid sequences that may result in peptides which have substantially equivalent activity as compared to the unmodified counterpart polypeptide. Such modifications may be deliberate, as by site-directed mutagenesis, or may be spontaneous. The term "variant" further contemplates
deletions, additions and substitutions to the sequence, so long as the polypeptide functions to produce an immunological response as defined herein.

The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, i.e., those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cystine, serine, threonine, tyrosine. Phenylalanine, tryptophan, and tyrosine are sometimes classified as aromatic amino acids. Examples of conservative variations include the substitution of one hydrophobic residue such as isoleucine, valine, leucine or methionine for another hydrophobic residue, or the substitution of one polar residue for another polar residue, such as the substitution of arginine for lysine, glutamic acid for aspartic acid, or glutamine for asparagine, and the like; or a similar conservative replacement of an amino acid with a structurally related amino acid that will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide. All of the polypeptides produced by these modifications are included herein. The term "conservative variation" also includes the use of a substituted amino acid in place of an unsubstituted parent amino acid provided that antibodies raised to the substituted polypeptide also immunoreact with the unsubstituted polypeptide.

An immunogenic fragment of a *Lu. longipalpis* polypeptide includes at least 8, 10, 15, or 20 consecutive amino acids, at least 21 amino acids, at least 23 amino acids, at least 25 amino acids, or at least 30 amino acids of a *L. longipalpis* polypeptide having a sequence as set forth in SEQ ID NO: 1, 3, 5, 7, 11, 13, 15, 17, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, or 87, or variants thereof. In another embodiment, a fragment of a *Lu. longipalpis* polypeptide includes a specific antigenic epitope found on a full-length *Lu. longipalpis* polypeptide.

Procedures to determine fragments of polypeptide and epitope such as, generating overlapping peptide libraries (Henner B. *et al.*)*,* Pepscan (Geysen R M *et al.,* 1984; Geysen H. M. *et al.,* 1985; Van der Zee *R.et al.;* Geysen H. M.) and algorithms (De Groot A. *et al.;* Hoop T. *et al.;* Parker K. *et al.),* are known in the art.

Generally, antibodies specifically bind a particular antigenic epitope. Specific, non-limiting examples of epitopes include a tetra- to penta- peptide sequence in a polypeptide, a tri- to penta glycoside sequence in a polysaccharide. In animals most antigens will present several or even many antigenic determinants simultaneously. Preferably wherein the epitope is a protein fragment of a larger molecule it will have substantially the same immunological activity as the total protein.

A polynucleotide for using according to the invention encodes a polypeptide from *Lu. longipalpis* sand fly, such as a polynucleotide encoding a polypeptide having a sequence as set forth in SEQ ID NO: 5, 7, 15 or 17.

In yet another aspect, a polynucleotide for use according to the invention encodes a polypeptide having at least 70%, at least 75%, at least 80%, at least 85%, at least 90%, at least 95%, 96%, 97%, 98% or 99% sequence identity to a polypeptide having a sequence as set forth in SEQ ID NO: 5, 7, 15 or 17.

In another aspect, a polynucleotide for use according to the invention has a nucleotide sequence as set forth in SEQ ID NO: 6, 8, 16, 18, 21, 90, or 91.

In yet another aspect, a polynucleotide for use according to the invention has at least 70%, at least 75%, at least 80%, at least 85%, at least 90%, at least 95%, at least 95%, 96%, 97%, 98% or 99% sequence identity to one of a polynucleotide having a sequence as set forth in SEQ ID NO: 6, 8, 16, 18, 21, 90, or 91.

These polynucleotides may include DNA, cDNA, and RNA sequences that encode a *Lu. longipalpis* polypeptide. It is understood that all polynucleotides encoding a *Lu. longipalpis* polypeptide are also included herein, as long as they encode a polypeptide with the recognized activity, such as the binding to an antibody that recognizes the polypeptide, the induction of an immune response to the polypeptide, or an effect on survival of Leishmania when administered to a subject exposed to the parasite or who undergoes a decrease in a sign or a symptom of Leishmania infection.

The polynucleotides of the disclosure include sequences that are degenerate as a result of the genetic code, e.g., optimized codon usage for a specific host. As used herein, "optimized" refers to a polynucleotide that is genetically engineered to increase its expression in a given species. To provide optimized polynucleotides coding for salivary polypeptides, the DNA sequence of the salivary protein gene can be modified to 1) comprise codons preferred by highly expressed genes in a particular species; 2) comprise an A+T or G+C content in nucleotide base composition to that substantially found in said species; 3) form an initiation sequence of said species; or 4) eliminate sequences that cause destabilization, inappropriate polyadenylation, degradation and termination of RNA, or that form secondary structure hairpins or RNA splice sites. Increased expression of salivary protein in said species can be achieved by utilizing the distribution frequency of codon usage in eukaryotes and prokaryotes, or in a particular species. The term "frequency of preferred codon usage" refers to the preference exhibited by a specific host cell in usage of nucleotide codons to specify a given amino acid. There are 20 natural amino acids, most of which are specified by more than one codon. Therefore, all degenerate nucleotide sequences are included in the disclosure as long as the amino acid sequence of the *Lu. longipalpis* salivary polypeptide encoded by the nucleotide sequence is functionally unchanged.

The sequence identity between two amino acid sequences may be established by the NCBI (National Center for Biotechnology Information) pairwise blast and the blosum62 matrix, using the standard parameters (see, e.g., the BLAST or BLASTX algorithm available on the "National Center for Biotechnology Information" (NCBI, Bethesda, Md., USA) server, as well as in Altschul *et al.;* and thus, this document speaks of using the algorithm or the BLAST or BLASTX and BLOSUM62 matrix by the term "blasts").

Sequence identity between two nucleotide sequences also may be determined using the "Align" program of Myers and Miller, ("Optimal Alignments in Linear Space", CABIOS 4, 11-17, 1988) and available at NCBI, as well as the same or other programs available via the Internet at sites thereon such as the NCBI site.

Alternatively or additionally, the term "identity", for instance, with respect to a nucleotide or amino acid sequence, may indicate a quantitative measure of homology between two sequences. The percent sequence homology may be calculated as: (N*_{ref}* - N*_{dif}*)∗100/N*_{ref}*, wherein N*_{dif}* is the total number of non-identical residues in the two sequences when aligned and wherein N*_{ref}* is the number of residues in one of the sequences. Hence, the DNA sequence AGTCAGTC will have a sequence identity of 75% with the sequence AATCAATC (N*_{ref}* = 8; N*_{dif}*=2).

Alternatively or additionally, "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

The sequence identity or sequence similarity of two amino acid sequences, or the sequence identity between two nucleotide sequences can be determined using Vector NTI software package (Invitrogen, 1600 Faraday Ave., Carlsbad, CA).

Advantageously, sequence identity or homology such as amino acid sequence identity or homology can be determined using the BlastP program (Altschul *et al.*) and available at NCBI, as well as the same or other programs available via the Internet at sites thereon such as the NCBI site.

The following documents provide algorithms for comparing the relative identity or homology of sequences, and additionally or alternatively with respect to the foregoing, the teachings in these references can be used for determining percent homology or identity: Needleman SB and Wunsch CD; Smith TF and Waterman MS; Smith TF, Waterman MS and Sadler JR; Feng DF and Dolittle RF; Higgins DG and Sharp PM; Thompson JD, Higgins DG and Gibson TJ; and, Devereux J, Haeberlie P and Smithies O. And, without undue experimentation, the skilled artisan can consult with many other programs or references for determining percent homology.

The *Lu. longipalpis* polynucleotides may include a recombinant DNA which is incorporated into a vector, into an autonomously replicating plasmid or virus, or into the genomic DNA of a prokaryote or eukaryote, or which exists as a separate molecule (for example, a cDNA) independent of other sequences.

Recombinant vectors disclosed herein may include a polynucleotide encoding a polypeptide, a variant thereof or a fragment thereof. Recombinant vectors may include plasmids and viral vectors and may be used for *in vitro* or *in vivo* expression. Recombinant vectors may include further a signal peptide. Signal peptides are short peptide chain (3-60 amino acids long) that direct the post-translational transport of a protein (which are synthesized in the cytosol) to certain organelles such as the nucleus, mitochondrial matrix, endoplasmic reticulum, chloroplast, apoplast and peroxisome. Typically, the naturally occurring salivary *Lu. longipalpis* polypeptides, such as LJM17 and LJL143 proteins, may be translated as precursors, having an N-terminal signal peptide sequence and a "mature" protein domain. The signal peptide may be cleaved off rapidly upon translation. The signal sequence may be the natural sequence from the salivary protein or a peptide signal from a secreted protein e.g. the signal peptide from the tissue plasminogen activator protein (tPA), in particular the human tPA (S. Friezner Degen *et al.;* R. Rickles *et al.;* D. Berg. *et al.*)*,* or the signal peptide from the Insulin-like growth factor 1 (IGF1), in particular the equine IGF1 (K. Otte *et al.*)*,* the canine IGF1 (P. Delafontaine *et al.*)*,* the feline IGF1 (WO03/022886), the bovine IGF1 (S. Lien *et al.*)*,* the porcine IGF1 (M. Muller *et al.*)*,* the chicken IGF1 (Y. Kajimoto *et al.*)*,* the turkey IGF1 (GenBank accession number AF074980). The signal peptide from IGF1 may be natural or optimized which may be achieved by removing cryptic splice sites and/or by adapting the codon usage. Upon translation, the unprocessed polypeptide may be cleaved at a cleavage site to lead to the mature polypeptide. The cleavage site may be predicted using the method of Von Heijne (1986).

A plasmid may include a DNA transcription unit, for instance a nucleic acid sequence that permits it to replicate in a host cell, such as an origin of replication (prokaryotic or eukaryotic). A plasmid may also include one or more selectable marker genes and other genetic elements known in the art. Circular and linear forms of plasmids are encompassed in the present disclosure.

In a further aspect, the vaccine for use according to the invention comprises an *in vivo* expression vector comprising a polynucleotide sequence, which contains and expresses *in vivo* in a host the salivary *Lu. longipalpis* polypeptides.

An *in vivo* expression vector may include any transcription unit containing a polynucleotide or a gene of interest and those essential elements for its *in vivo* expression. These expression vectors may be plasmids or recombinant viral vectors. For *in vivo* expression, the promoter may be of viral or cellular origin. In one embodiment, the promoter may be the cytomegalovirus (CMV) early promoter (CMV-IE promoter), the SV40 virus early or late promoter or the Rous Sarcoma virus LTR promoter, a promoter of a cytoskeleton gene, such as the desmin promoter (Kwissa M *et al.*)*,* or the actin promoter (Miyazaki J. *et al.*)*.* When several genes are present in the same plasmid, they may be provided in the same transcription unit or in different units.

As used herein, the term "plasmid" may include any DNA transcription unit comprising a polynucleotide according to the invention and the elements necessary for its *in vivo* expression in a cell or cells of the desired host or target; and, in this regard, it is noted that a supercoiled or non-supercoiled, circular plasmid, as well as a linear form, are intended to be within the scope of the invention. The plasmids may also comprise other transcription-regulating elements such as, for example, stabilizing sequences of the intron type. In several embodiments, the plasmids may include the first intron of CMV-IE (WO 89/01036), the intron II of the rabbit beta-globin gene (van Ooyen *et al.),* the signal sequence of the protein encoded by the tissue plasminogen activator (tPA; Montgomery *et al.),* and/or a polyadenylation signal (polyA), in particular the polyA of the bovine growth hormone (bGH) gene (US 5,122,458) or the polyA of the rabbit beta-globin gene or of SV40 virus.

Described herein is a vaccine composition comprising: a) an *in vivo* expression vector, wherein the vector comprises a polynucleotide encoding one or more polypeptide selected from the group consisting of a salivary *Lu. longipalpis* polypeptide, a variant or fragment of the salivary *Lu. longipalpis* polypeptide, and a mixture thereof; and b) a pharmaceutically or veterinary acceptable vehicle, diluent or excipient.

Described herein is a vaccine composition comprising: a) a first *in vivo* expression vector, wherein the vector comprises a polynucleotide encoding one or more polypeptide selected from the group consisting of a salivary *Lu. longipalpis* polypeptide, a variant or fragment of the salivary *Lu. longipalpis* polypeptide, and a mixture thereof; b) a second *in vivo* expression vector, wherein the vector comprises a polynucleotide encoding one or more polypeptide selected from the group consisting of a salivary *Lu. longipalpis* polypeptide, a variant or fragment of the salivary *Lu. longipalpis* polypeptide, and a mixture thereof; and c) a pharmaceutically or veterinary acceptable vehicle, diluent or excipient.

The term "vaccine composition" or "vaccine" comprises any composition, once it has been injected to a host, including canines, felines and humans, that protects the host from cutaneous leishmaniasis and/or visceral leishmaniasis, and/or which may prevent implantation of the parasite, and/or which may prevent disease progression in infected subjects, and/or which may limit the diffusion of runaway parasites to internal organs, and/or which may avoid or limit parasite uptake by a sand fly during a blood meal on a vaccinated dog. This may be accomplished upon vaccination according to the present invention through the induction of cytokine secretion, notably IFN-gamma secretion (as example of a method of measurement of IFN-gamma secretion, the Quantikine® immunoassay from R&D Systems Inc. (catalog number# CAIF00) could be used (Djoba Siawaya JF *et al.)).*

The pharmaceutically acceptable vehicles or excipients of use are conventional. Remington's Pharmaceutical Sciences, by E. W. Martin, Mack Publishing Co., Easton, P A, 15th Edition (1975), describes compositions and formulations suitable for pharmaceutical delivery of the polypeptides, plasmids, viral vectors herein disclosed. In general, the nature of the vehicle or excipient will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (for example, freeze dried pastille, powder, pill, tablet, or capsule forms), conventional non-toxic solid vehicles or excipients can include, for example, pharmaceutical grades of mannitol, lactose, starch, or magnesium stearate. In addition to biologically neutral vehicles or excipients, immunogenic compositions to be administered can contain minor amounts of non-toxic auxiliary substances, such as wetting or emulsifying agents, preservatives, and pH buffering agents and the like, for example sodium acetate or sorbitan monolaurate.

The vaccines according to the instant invention may include vectors encoding any polynucleotide according to the present invention as described above.

Multiple insertions may be done in the same vector using different insertion sites or using the same insertion site. When the same insertion site is used, each polynucleotide insert, which may be any polynucleotide of the present invention aforementioned, may be inserted under the control of the same and/or different promoters. The insertion can be done tail-to-tail, head-to-head, tail-to-head, or head-to-tail. IRES elements (Internal Ribosome Entry Site, see EP 0803573) can also be used to separate and to express multiple inserts operably linked to the same and/or different promoters.

In one embodiment, an expression vector for use according to the invention comprises a polynucleotide aforementioned. The expression vector may be an *in vivo* expression vector, or an *in vitro* expression vector.

In vivo expression vectors for use according to the invention include any plasmid (EP-A2-1001025; Chaudhuri P) containing and expressing *in vivo* in a host the polynucleotide or gene of *Lu. longipalpis* salivary polypeptide, variant thereof or fragment thereof and elements necessary for its *in vivo* expression.

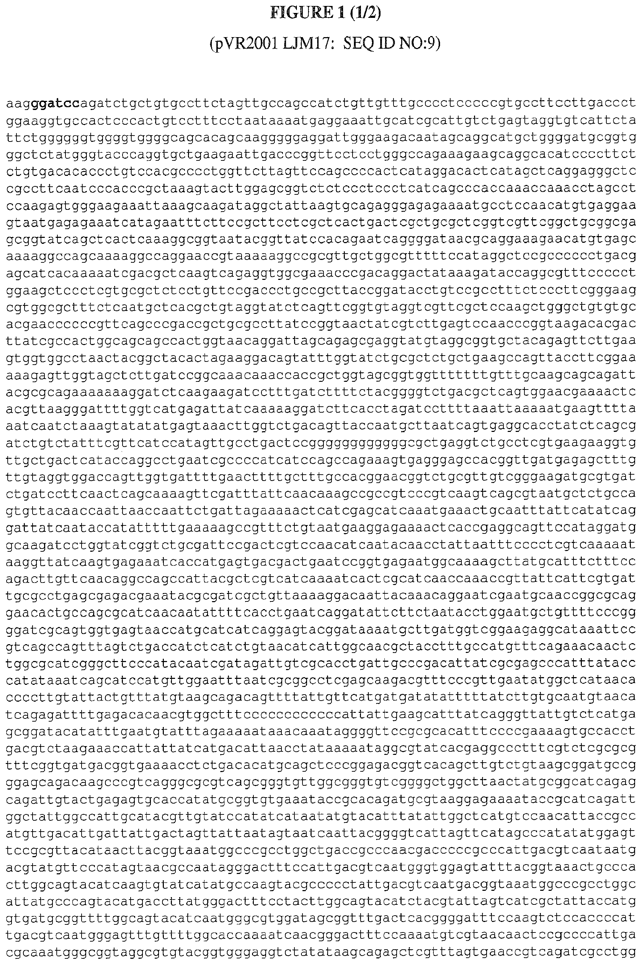

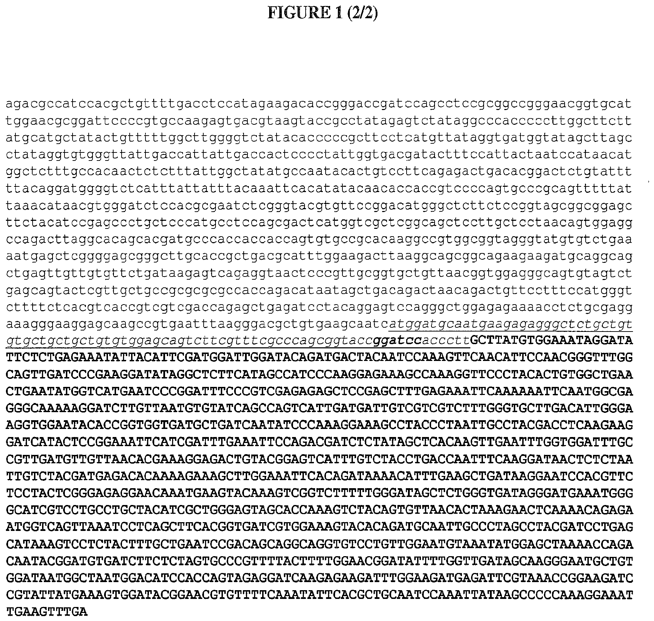

In a specific, non-limiting example, the pVR1020 or pVR1012 plasmid (VI CAL Inc.; Luke C. *et al.;* Hartikka J. *et al.), pVR2001-TOPA* (or pVR2001-TOPO) (Oliveira F. *et al.)* or pAB110 (US 6,852,705) can be utilized as a vector for the insertion of a polynucleotide sequence. The pVR1020 plasmid is derived from pVR1012 and contains the human tPA signal sequence. The pVR1020 is a plasmid backbone available from Vical, Inc., (San Diego, CA) which has been previously used, see, e.g., US Patent Nos. 6,451,769 and 7,078,507. As described in Oliveira *et al.,* plasmid pVR2001-TOPO (or pVR2001-TOPA) is pVR1020 modified by the addition of topoisomerases flanking the cloning site and containing coding for and expressing a signal secretory peptide, for example, tissue plasminogen activator signal peptide (tPA), that increases the likelihood of producing a secreted protein, (see Figure 1 in Oliveira F. *et al.*)*.*

Each plasmid may comprise or contain or consist essentially of, a polynucleotide aforementioned, operably linked to a promoter or under the control of a promoter or dependent upon a promoter, wherein the promoter may be advantageously adjacent
to the polynucleotide for which expression is desired. In general, it is advantageous to employ a strong promoter that is functional in eukaryotic cells. One example of a useful promoter may be the immediate early cytomegalovirus promoter (CMV-IE) of human or murine origin, or it may optionally have another origin such as from rat or guinea pig. The CMV-IE promoter may comprise the actual promoter part, which may or may not be associated with the enhancer part. Reference can be made to EP 260 148, EP 323 597, US 5,168,062, 5,385,839, and 4,968,615, as well as to WO 87/03905. The CMV-IE promoter may advantageously be a human CMV-IE (Boshart M. *et al.*) or murine CMV-IE. In more general terms, the promoter may have either a viral or a cellular origin. A strong viral promoter other than CMV-IE that may be usefully employed in the practice of the invention is the early/late promoter of the SV40 virus or the LTR promoter of the Rous sarcoma virus. A strong cellular promoter that may be usefully employed in the practice of the invention is the promoter of a gene of the cytoskeleton, such as the desmin promoter (Kwissa M. *et al.*)*,* or the actin promoter (Miyazaki J. *et al.*)*.* Functional sub fragments of these promoters, i.e., portions of these promoters that maintain adequate promoter activity, are included within the present invention, e.g. truncated CMV-IE promoters according to WO 98/00166 or US 6,156,567 and may be used in the practice of the invention. A promoter useful in the practice of the invention consequently may include derivatives and/or sub fragments of a full-length promoter that maintain adequate promoter activity and hence function as a promoter, and which may advantageously have promoter activity that is substantially similar to that of the actual or full-length promoter from which the derivative or sub fragment is derived, e.g., akin to the activity of the truncated CMV-IE promoters of US 6,156,567 in comparison to the activity of full-length CMV-IE promoters. Thus, a CMV-IE promoter in the practice of the invention may comprise or consist essentially of or consist of the promoter portion of the full-length promoter and/or the enhancer portion of the full-length promoter, as well as derivatives and/or sub fragments thereof.

Advantageously, the plasmids comprise or consist essentially of other expression control elements. It is especially advantageous to incorporate stabilizing sequence(s), e.g., intron sequence(s), for example, the first intron of the hCMV-IE (WO 89/01036), the intron II of the rabbit β-globin gene (van Ooyen *et al.*)*.* As to the polyadenylation signal (polyA) for the plasmids and viral vectors other than poxviruses, use can be made of the poly(A) signal of the bovine growth hormone (bGH) gene (*see* US 5,122,458), or the poly(A) signal of the rabbit β-globin gene or the poly(A) signal of the SV40 virus,

In one embodiment of the present invention, the plasmid vector for use according to the invention is pVR2001 comprising LJM17 polynucleotide, as described in example 1 herein.

Described herein the plasmid vector is pR2001 comprising LJL143 polynucleotide, as described in example 2 herein.

In another embodiment of the present invention, the plasmid vector for use according to the invention is pNBO002, as described in example 10 herein .

Described herein the plasmid vector pNBO003, as described in example 10 herein.

More generally, the present description encompasses *in vivo* expression vectors including any recombinant viral vector containing a polynucleotide or gene encoding one or more salivary *Lu. longipalpis* immunogens and/or variants or fragments thereof, including any elements necessary for its *in vivo* expression.

Said recombinant viral vectors could be selected from, for example, the poxviruses, especially avipox viruses, such as fowl pox viruses or canarypox viruses. In one embodiment, the fowlpox virus is a TROVAC (see WO 96/40241). In another embodiment, the canarypox vector is an ALVAC. The use of these recombinant viral vectors and the insertion of polynucleotides or genes of interest are fully described in US 5,174,993; US 5,505,941 and US 5,766,599 for fowlpox, and in US 5,756,103 for canarypox. More than one insertion site inside the viral genome could be used for the insertion of multiple genes of interest.

In one embodiment the viral vector is an adenovirus, such as a human adenovirus (HAV) or a canine adenovirus (CAV).

In another embodiment the viral vector is a human adenovirus, specifically a serotype 5 adenovirus, rendered incompetent for replication by a deletion in the E1 region of the viral genome, especially from about nucleotide 459 to about nucleotide 3510 by reference to the sequence of the hAd5 disclosed in Genbank under the accession number M73260 and in the referenced publication Chroboczek et al, 1992. The deleted adenovirus is propagated in El-expressing 293 (Graham *et al.,* 1977) or PER cells, especially PER.C6 (Falloux *et al.,* 1998). The human adenovirus can additionally or alternatively be deleted in the E3 region, especially from about nucleotide 28592 to about nucleotide 30470. The deletion in the El region can be done in combination with a deletion in the E3 region (see, e.g. Shriver *et al.;* Graham *et al.;* Ilan *et al.;* U.S. Patent Nos. 6,133,028 and 6,692,956; Tripathy *et al.;* Tapnell; Danthinne *et al.;* Berkner; Berkner *et al.;* Chavier *et al*.). The insertion sites can be the E1 and/or E3 loci (region) eventually after a partial or complete deletion of the E1 and/or E3 regions. Advantageously, when the expression vector is an adenovirus, the polynucleotide to be expressed is inserted under the control of a promoter functional in eukaryotic cells, such as a strong promoter, advantageously a cytomegalovirus immediate-early gene promoter (CMV-IE promoter), especially the enhancer / promoter region from about nucleotide -734 to about nucleotide +7 in Boshart *et al.,* or the enhancer / promoter region from the pCI vector from Promega Corp. The CMV-IE promoter is advantageously of murine or human origin. The promoter of the elongation factor 1α can also be used. A muscle specific promoter can also be used (Li *et al.*)*.* Strong promoters are also discussed herein in relation to plasmid vectors. In one embodiment, a splicing sequence can be located downstream of the enhancer / promoter region. For example, the intron 1 isolated from the CMV-IE gene (Stenberg *et al.*)*,* the intron isolated from the rabbit or human β-globin gene, especially the intron 2 from the β-globin gene, the intron isolated from the immunoglobulin gene, a splicing sequence from the SV40 early gene or the chimeric intron sequence isolated from the pCI vector from Promege Corp. A poly(A) sequence and terminator sequence can be inserted downstream the polynucleotide to be expressed, e.g. a bovine growth hormone gene, especially from about nucleotide 2339 to about nucleotide 2550 in Genbank under the accession number BOVGHRH, a rabbit β-globin gene or a SV40 late gene polyadenylation signal.

In another embodiment the viral vector is a canine adenovirus, especially a CAV-2 (see, e.g. Fischer *et al.;* U.S. Patent Nos. 5,529,780 and 5,688,920; WO 95/14102). For CAV, the insertion sites can be in the E3 region and /or in the region located between the E4 region and the right ITR region (see U.S. Patent Nos. 6,090,393 and 6,156,567). In one embodiment the insert is under the control of a promoter, such as a cytomegalovirus immediate-early gene promoter (CMV-IE promoter) or a promoter already described for a human adenovirus vector. A poly(A) sequence and terminator sequence can be inserted downstream the polynucleotide to be expressed, e.g. a bovine growth hormone gene or a rabbit β-globin gene polyadenylation signal.

In another embodiment, the viral vector is a herpesvirus such as a canine herpesvirus (CHV) or a feline herpesvirus (FHV). For CHV, the insertion sites may be in the thymidine kinase gene, in the ORF3, or in the UL43 ORF (see US 6,159,477). In one embodiment the polynucleotide to be expressed is inserted under the control of a promoter functional in eukaryotic cells, advantageously a CMV-IE promoter (murine or human). A poly(A) sequence and terminator sequence can be inserted downstream the polynucleotide to be expressed, e.g. bovine growth hormone or a rabbit β-globin gene polyadenylation signal.

For recombinant vectors based on a poxvirus vector, a vaccinia virus or an attenuated vaccinia virus, (for instance, MVA, a modified Ankara strain obtained after more than 570 passages of the Ankara vaccine strain on chicken embryo fibroblasts; *see* Stickl & Hochstein-Mintzel; Sutter *et al.;* available as ATCC VR-1508; or NYVAC, *see* US 5,494,807, and U.S. Patent No. 5,494,807 which discuss the construction of NYVAC, as well as variations of NYVAC with additional ORFs deleted from the Copenhagen strain vaccinia virus genome, as well as the insertion of heterologous coding nucleic acid molecules into sites of this recombinant, and also, the use of matched promoters; see also WO 96/40241), an avipox virus or an attenuated avipox virus (e.g., canarypox, fowlpox, dovepox, pigeonpox, quailpox, ALVAC or TROVAC; see, e.g., U.S. Patent Nos. 5,505,941, 5,494,807) can be used. Attenuated canarypox viruses are described in US 5,756,103 (ALVAC) and WO 01/05934. Reference is also made to US5,766,599 which pertains to the attenuated fowlpox strain TROVAC. Reference is made to the canarypox available from the ATCC under access number VR-111. Numerous fowlpox virus vaccination strains are also available, e.g. the DIFTOSEC CT strain marketed by MERIAL and the NOBILIS VARIOLE vaccine marketed by INTERVET. For information on the method used to generate recombinants thereof and how to administer recombinants thereof, the skilled artisan can refer documents cited herein and to WO 90/12882, e.g., as to vaccinia virus, mention is made of U.S. Patents Nos. 4,769,330, 4,722,848, 4,603,112, 5,110,587, 5,494,807, and 5,762,938 *inter alia*; as to fowlpox, mention is made of U.S. Patents Nos. 5,174,993, 5,505,941 and 5,766,599 *inter alia;* as to canarypox, mention is made of U.S. Patent No. 5,756,103 *inter alia.* When the expression vector is a vaccinia virus, insertion site or sites for the polynucleotide or polynucleotides to be expressed are advantageously at the thymidine kinase (TK) gene or insertion site, the hemagglutinin (HA) gene or insertion site, the region encoding the inclusion body of the A type (ATI); see also documents cited herein, especially those pertaining to vaccinia virus. In the case of canarypox, advantageously the insertion site or sites are ORF(s) C3, C5 and/or C6; see also documents cited herein, especially those pertaining to canarypox virus. In the case of fowlpox, advantageously the insertion site or sites are ORFs F7 and/or F8; see also documents cited herein, especially those pertaining to fowlpox virus. The insertion site or sites for MVA virus are advantageously as in various publications, including Carroll M. W. *et al.;* Stittelaar K. J. *et al.;* Sutter G. *et al.;* and, in this regard it is also noted that the complete MVA genome is described in Antoine G., Virology, which enables the skilled artisan to use other insertion sites or other promoters. Advantageously, the polynucleotide to be expressed is inserted under the control of a specific poxvirus promoter, e.g., the vaccinia promoter 7,5 kDa (Cochran *et al),* the vaccinia promoter I3L (Riviere *et al.),* the vaccinia promoter HA (Shida), the cowpox promoter ATI (Funahashi *et al.),* the vaccinia promoter H6 (Taylor J. *et al.;* Guo P. *et al.* J.; Perkus M. *et al.*)*, inter alia.*

In a further embodiment, the recombinant viral vector for use according to the invention is the recombinant ALVAC canarypox virus vCP2390-SEQ ID NO:6, expressing the *Lu, longipalpis* salivary LJM17 polypeptide, as described in example 3.

In a further embodiment, the recombinant viral vector for use according to the invention is the recombinant MVA virus MVA-LJL17, expressing the *Lu. longipalpis* salivary LJM17 polypeptide, as described in example 5.

Described herein the recombinant viral vector is the recombinant ALVAC canarypox virus vCP2389-SEQ ID NO:2, expressing the *Lu. longipalpis* salivary LJL143 polypeptide, as described in example 4.

Described herein the recombinant viral vector is the recombinant ALVAC canarypox virus vCP2389, expressing the *Lu, longipalpis* salivary LJL143 polypeptide, as described in the example 12.

In a further embodiment, the recombinant viral vector for use according to the invention is the recombinant ALVAC canarypox virus vCP2390, expressing the *Lu, longipalpis* salivary LJM17 polypeptide, as described in example 11.

Described herein the recombinant viral vector is the recombinant MVA virus MVA-LJL143, expressing the *Lu. longipalpis* salivary LJL143 polypeptide, as described in the example 6.

Any of the polynucleotides disclosed here may be expressed *in vitro* by DNA transfer or expression vectors into a suitable host cell. The host cell may be prokaryotic or eukaryotic. The term "host cell" also includes any progeny of the subject host cell. Methods of stable transfer, meaning that the foreign polynucleotide is continuously maintained in the host cell, are known in the art. Host cells may include bacteria (for example, *Escherichia coli*), yeast, insect cells, and vertebrate cells. Methods of expressing DNA sequences in eukaryotic cells are well known in the art. As a method for *in vitro* expression, recombinant Baculovirus vectors (for example, Autographa California Nuclear Polyhedrosis Virus (AcNPV)) may be used with the nucleic acids disclosed herein. For example, polyhedrin promoters may be utilized with insect cells (for example, *Spodoptera frugiperda* cells, like Sf9 cells available at the ATCC under the Accession number CRL 1711, or Sf21 cells) (see for example, Smith *et al.;* Pennock *et al.;* Vialard *et al.;* Verne A.; O'Reilly *et al.;* Kidd I. M. & Emery V.C.; EP 0370573; EP 0265785; US 4,745,051). For expression, the BaculoGold Starter Package (Cat # 21001K) from Pharmingen (Becton Dickinson) may be used. As a method for *in vitro* expression, recombinant *E. coli* may be used with a vector. For example, when cloning in bacterial systems, inducible promoters such as arabinose promoter, pL of bacteriophage lambda, plac, ptrp, ptac (ptrp-lac hybrid promoter), and the like may be used. Transformation of a host cell with recombinant DNA may be carried out by conventional techniques are well known to those skilled in the art. Where the host is prokaryotic, such as *E. coli,* competent cells which are capable of DNA uptake can be prepared from cells harvested after exponential growth phase and subsequently treated by the CaCl2 method using procedures well known in the art. Alternatively, MgCl2 or RbCl can be used. Transformation can also be performed by electroporation. When the host is a eukaryote, such methods of transduction of DNA as calcium phosphate coprecipitates, conventional mechanical procedures such as microinjection, electroporation, insertion of a plasmid encased in liposomes, or virus vectors may be used. Eukaryotic cells may also be cotransformed with *L. Iongipalpis* polynucleotide sequences, and a second foreign DNA molecule encoding a selectable phenotype, such as the herpes simplex thymidine kinase gene. Another method is to use a eukaryotic viral vector (see above), such as a herpes virus or adenovirus (for example, canine adenovirus 2), to transiently transduce eukaryotic cells and express the protein (Gluzman EA). In addition, a transfection agent can be utilized, such as dioleoyl-phosphatidyl-ethanolamme (DOPE).

Isolation and purification of recombinantly expressed polypeptide may be carried out by conventional means including preparative chromatography (for example, size exclusion, ion exchange, affinity), selective precipitation and ultra-filtration. Examples of state of the art techniques that can be used, but not limited to, may be found in "Protein Purification Applications", Second Edition, Edited by Simon Roe and available at Oxford University Press. Such a recombinantly expressed polypeptide is part of the present disclosure. The methods for production of any polypeptide according to the present, description as described above include the use of a recombinant expression vector comprising a polynucleotide according to the disclosure and of a host cell.

The vaccines containing recombinant viral vectors for use according to the invention may be freeze-dried, advantageously with a stabilizer. Freeze-drying can be done according to well-known standard freeze-drying procedures. The pharmaceutically or veterinary acceptable stabilizers may be carbohydrates (e.g. sorbitol, mannitol, lactose, sucrose, glucose, dextran, trehalose), sodium glutamate (Tsvetkov T *et al.;* Israeli E *et* al.), proteins such as peptone, albumin, lactalbumin or casein, protein containing agents such as skimmed milk (Mills C K *et al.;* Wolff E *et al.*)*,* and buffers (e.g. phosphate buffer, alkaline metal phosphate buffer). An adjuvant may be used to make soluble the freeze-dried preparations.

Any vaccine composition for use according to the invention can also advantageously contain one or more adjuvant.

The plasmid-based vaccines may be formulated with cationic lipids, advantageously with DMRIE (N-(2-hydroxyethyl)-N,N-dimethyl-2,3-bis(tetradecyloxy)-1-propanammonium; WO96/34109), and advantageously in association with a neutral lipid, for example DOPE (dioleoyl-phosphatidyl-ethanolamine; Behr J. P.), in order to form DMRIE-DOPE. In one embodiment, the mixture is made extemporaneously, and before its administration it is advantageous to wait about 10 min to about 60 min, for example, about 30 min, for the appropriate complexation of the mixture. When DOPE is used, the molar ratio of DMRIE/DOPE can be from 95/5 to 5/95 and is advantageously 1/1. The weight ratio plasmid/DMRIE or DMRIE-DOPE adjuvant is, for example, from 50/1 to 1/10, from 10/1 to 1/5 or from 1/1 to 1/2.

Optionally a cytokine may be added to the composition, especially GM-CSF or cytokines inducing Th1 (e.g. IL12). These cytokines can be added to the composition as a plasmid encoding the cytokine protein. In one embodiment, the cytokines are from canine origin, e.g. canine GM-CSF which gene sequence has been deposited at the GenBank database (accession number 84973 8). This sequence can be used to create said plasmid in a manner similar to what was made in WO 00/77210.

The recombinant viral vector-based vaccine may be combined with fMLP (N-formyl-methionyl-leucyl-phenylalanine; US 6,017,537) and/or Carbomer adjuvant (Phameuropa Vol. 8, No. 2, June 1996). Persons skilled in the art can also refer to US 2,909,462, which describes such acrylic polymers cross-linked with a polyhydroxylated compound having at least 3 hydroxyl groups, advantageously not more than 8, the hydrogen atoms of at least three hydroxyls being replaced by unsaturated aliphatic radicals having at least 2 carbon atoms. For example, the radicals are those containing from 2 to 4 carbon atoms, e.g. vinyls, allyls and other ethylenically unsaturated groups. The unsaturated radicals may themselves contain other substituents, such as methyl. The products sold under the name Carbopol® (BF Goodrich, Ohio, USA) are appropriate. The products are cross-linked with an allyl sucrose or with allyl pentaerythritol. Among them, there may be advantageously mentioned Carbopol® 974P, 934P and 971P.

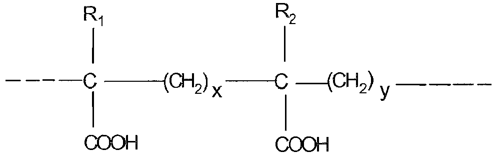

Among the copolymers of maleic anhydride and alkenyl derivative, the copolymers EMA® (Monsanto) which are copolymers of maleic anhydride and ethylene, linear or cross-linked, for example cross-linked with divinyl ether, are advantageous. Reference may be made to J. Fields *et al..*

The polymers of acrylic or methacrylic acid and the copolymers EMA® are formed, for example, of basic units of the following formula in which:
- R₁ and R₂, which are identical or different, represent H or CH₃
- x = 0 or 1, preferably x = 1
- y = 1 or 2, with x + y = 2

For the copolymers EMA®, x = 0 and y = 2. For the carbomers, x = y =1.

The dissolution of these polymers in water leads to an acid solution, which is neutralized, advantageously to physiological pH, in order to provide the adjuvant solution into which the vaccine itself is incorporated. The carboxyl groups of the polymer are then partly in COO⁻ form. In one embodiment, a solution of adjuvant, especially of carbomer (Pharmeuropa, vol. 8, No.2, June 1996), is prepared in distilled water, advantageously in the presence of sodium chloride, the solution obtained being at an acidic pH. This stock solution is diluted by adding it to the desired quantity (for obtaining the desired final concentration), or a substantial part thereof, of water charged with NaCl, advantageously physiological saline (NaCl 9 g/1) all at once in several portions with concomitant or subsequent neutralization (pH 7.3 to 7.4), advantageously with NaOH. This solution at physiological pH is used for mixing with the vaccine, which may be especially stored in freeze-dried, liquid or frozen form.

The polymer concentration in the final vaccine composition can be from 0.01% to 2% w/v, from 0.06 to 1% w/v, or from 0.1 to 0.6% w/v.

The sub-unit vaccine may be combined with adjuvants, like oil-in-water, water-in-oil-in water emulsions based on mineral oil and/or vegetable oil and non ionic surfactants such as block copolymers, Tween®, Span®. Such emulsions are notably those described in page 147 of "Vaccine Design -The Subunit and Adjuvant Approach", Pharmaceutical Biotechnology, 1995, or TS emulsions, notably the TS6 emulsion, and LF emulsions, notably LF2 emulsion (for both TS and LF emulsions, see WO 04/024027). Other suitable adjuvants are for example vitamin E, saponins, and Carbopol® (Noveon; see WO 99/51269; WO 99/44633), aluminium hydroxide or aluminium phosphate ("Vaccine Design, The subunit and adjuvant approach", Pharmaceutical Biotechnology, vol. 6, 1995), biological adjuvants (i.e. C4b, notably murine C4b (Ogata R T *et al.)* or equine C4b, GM-CSF, notably equine GM-CSF (US 6,645,740)), toxins (i.e. cholera toxins CTA or CTB, *Escherichia coli* heat-labile toxins LTA or LTB (Olsen C W *et al.;* Fingerut E *et al.;* Zurbriggen R *et al.* Peppoloni S *et al.*)*,* and CpG (i.e. CpG#2395 (see Jurk M *et al.*)*,* CpG #2142 (see SEQ. ID. NO: 890 in EP 1,221,955)).

The vaccine may also contain or comprise one or more *Leishmania* antigens, for example, kinetoplastid membrane protein 11 (KMP11).

*Leishmania* KMP11 antigens are derived from, for example, *L. infantum* or *L. chagasi.* KMP11 is a highly conserved surface membrane protein present in all members of the family Kinetoplastidae, and is differentially expressed both in amastigote and promastigote forms of *Leishmania* (Jardim A. *et al.;* Jardim A. *et al.;* Berberich C. *et al.).* The nucleic acid sequence of the gene and the amino acid sequence of the protein KMP11 of *Leishmania* are available in public databases, notably as *L. infantum* in the GenBank database under the accession numbers X95627 and X95626. The nucleic acid sequence of *L. donovani* is also available from the GenBank database, notably under the accession number S77039.

The state of the art regarding KMP11, vectors expressing KMP11 and vaccines are best summarized in patent application WO 08/064181. A plasmid-based vaccine comprising pVR1020 KMP11 is described in example 1 and the canarypox virus vector-based vaccine comprising vCP2350 is described in example 3. WO 08/064181 also gives information regarding adjuvants, formulation, doses and route of administration.

KMP11 polypeptides and variants or fragments thereof may be produced, isolated and purified in the same manner set forth for *in vitro* expression of sand fly salivary polypeptides.

In one embodiment, the vaccine for use according to the present invention additionally comprises vectors comprising me KMP11 polynucleotide encoding the KMP11 polypeptide and/or fragments or variants thereof from *Leishmania.* Said vaccine contains a *Lu. longipalpis* salivary polypeptide including; a polypeptide having at least 70%, at least 75%, at least 80%, at least 85%, at least 90%, at least 95%, 96%, 97%, 98% or 99% sequence identity to a polypeptide having a sequence as set forth in SEQ ID NO: 5, 7, 15, or 17; or a polynucleotide encoding a *Lu. longipalpis* salivary polypeptide having at least 70%, at least 75%, at least 80%, at least 85%, at least 90%, at least 95%, 96%, 97%, 98% or 99% sequence identity to a polypeptide having a sequence as set forth in SEQ ID NO: 5,7, 15, or 17; or a polynucleotide having at least 70%, at least 75%, at least 80%, at least 85%, at least 90%, at least 95%, 96%, 97%, 98% or 99% to a polynucleotide having a sequence as set forth in SEQ ID NO: 6, 8, 16, 18, 21, 90 or 91.

The vaccine for use according to the invention may comprise *Lu. longipalpis* salivary LJMI7 polypeptides and/or variants thereof as described herein, and/or vectors comprising the polynucleotide encoding the *Lu. longipalpis* LJM17 polypeptide and/or variants thereof as described herein, and/or
vectors comprising the KMP11 polynucleotide encoding the KMP11 polypeptide and/or fragments or variants thereof from *Leishmania.* For example, the vectors for LJM17 may be selected from the group consisting of pVR2001 LJM17, pNBO002, vCP2390, vCP2390-SEQ ID N0:6 and MVA-LJM17. The vectors fur KMP11 may be selected from the group consisting of pVR1020 KMP11 and vCP2350. In one embodiment, the vaccine comprises plasmids pVR2001 LJM17 and pVR1020 KMP11. In another embodiment, the vaccine comprises vectors vCP2390 and vCP2350. In yet another embodiment, the vaccine comprises plasmids pNBO002 and pVR1020 KMP11. In yet another embodiment, the vaccine comprises vCP2390-SEQ ID N0:6 and vCP2350.

Described herein the vaccine comprises *Lu. longipalpis* salivary LJL143 polypeptides and/or variants or fragments thereof, and/or vectors comprising the polynucleotide encoding the *Lu. longipalpis* LJL143 polypeptide and/or variants or fragments thereof, and/or vectors comprising the polynucleotide encoding *Leishmania* KMP11 polypeptides and/or variants or fragments thereof. For example, the vectors for LJL143 may be selected from the group consisting of pVR2001 LJL143, pNBO003, vCP2389, vCP2389-SEQ ID NO:2 and MVA-LJL143. The vectors for KMP11 may be selected from the group consisting of pVR1020 KMP11 and vCP2350. In one embodiment, the vaccine comprises plasmids pVR2001 LJL143 and pVR1020 KMP11. In another embodiment, the vaccine comprises vectors vCP2389 and vCP2350. In yet another embodiment, the vaccine comprises plasmids pNBO003 and pVR1020 KMP11. In yet another embodiment, the vaccine comprises vectors vCP2389-SEQ ID NO:2 and vCP2350.

In another particular embodiment, the vaccine for use according to the invention comprises at least one of *Lu. longipalpis* salivary LJM17 polypeptides, variants thereof as described herein, or vectors comprising the polynucleotide encoding the *Lu. longipalpis* LJM17 polypeptide and thereof as described herein, and may additionally comprise *Lu. longipalpis* salivary LJL143, polypeptides and/or variants or fragments thereof, and/or vectors comprising tile LJLI43 polynucleotide encoding the *Lu. Longipalpis* LJL143 polypeptide and/or variants or fragments thereof, and/or *Leishmania* KMP11 polypeptides and/or variants or fragments thereof, and/or vectors comprising the KMP11 polynucleotide or gene. For example, the vectors for LJL143 may be selected from the group consisting of pVR2001 LJL143, pNBO003, vCP2389, vCP2389-SEQ ID N0:2 and MVA-LJL143. The vectors for L1MJ7 may be selected from the group consisting of pVR2001 LJM17, pNBO002, vCP2390, vCP2390-SEQ ID NO:6 and MVA-LJM17. The vectors for KMP11 may be selected from the group consisting of pVR1020 KMP11 and vCP2350. In one embodiment, the vaccine comprises plasmids pVR2001 LJL143, pVR2001 LJM17 and pVR1020 KMP11. In another embodiment, the vaccine comprises vectors vCP2389, vCP2390 and vCP2350. In yet another embodiment, the vaccine comprises plasmids pNBO003, pNBO002 and pVR1020 KMP11. In another embodiment, the vaccine comprises vCP2389-SEQ ID NO:2, vCP2390-SEQ ID NO:6 and vCP2350 vectors.

The vaccine may also be associated with at least one *Leishmania* antigen, for example inactivated *Leishmania.*

In a particular embodiment, the *Leishmania* strain may be *Leishmania infantum,* and/or *Leishmania braziliensis.* In a preferred embodiment, the *Leishmania* strain may be *Leishmania braziliensis.*

These strains of *Leishmania* may be inactivated by chemical or physical methods. The chemical methods are notably BPL, formaldehyde. The physical methods may notably be sonication. One method for inactivating Leishmania for use in a vaccine is described in R. Cordeiro Giunchetti et al., Vaccine, 2007. The promastigotes are cultivated in NNN/LIT medium for 6 to 14 days, but more preferably 10 days, until the differentiation between promastigote procyclic form into promastigote metacyclic form is achieved on the basis of a microscopic observation. The culture can then be harvested by centrifugation (2000Xg, 20minutes, 4°C). When applicable, the supernatant is discarded and the biomass is washed three times in saline buffer. Whether the culture is clarified or not, the promastigote suspension (i.e. crude culture or promastigote resuspended in saline buffer after centrifugation) is subsequently disrupted by ultrasound treatment using a power from 10 to 375W, but more preferably 40W, for 1 minute, at 0°C. The batch volume for this treatment is between 5 and 150 mL, preferably 30 mL. After treatment the lysate can be stored at -80°C.

The vaccine formulation may be prepared from the protein concentrate that is obtained following cell lysis. The cell lysate protein quantity that may be used for vaccination of a canine is from about 50 µg to about 2000 µg, preferably from about 50 µg to about 600 µg. Protein concentration is determined according to the method of Lowry.

The inactivated *Leishmania* vaccine may be combined with adjuvants, like those described previously for sub-unit vaccines.

In one embodiment, the vaccine for use according to the invention comprises *Lu. longipalpis* salivary polypeptides and/or variants or fragments thereof, and/or vectors comprising the *Lu. longipalpis* salivary polynucleotide and/or variants or fragments thereof, and or inactivated *Leishmania.* Said vaccine contains the *Lu. longipalpis* salivary polypeptide including a polypeptide having at least 70%, at least 75%, at least 80%, at least 85%, at least 90%, at least 95%, 96%, 97%, 98% or 99% sequence identity to a polypeptide having a sequence as set forth in SEQ ID NO: 5, 7, 15, or 17; or the polynucleotide encodes a *Lu. longipalpis* salivary polypeptide having at least 70%, at least 75%, at least 80%, at least 85%, at least 90%, at least 95%, 96%, 97%, 98% or 99% sequence identity to a polypeptide having a sequence as set forth in SEQ ID NO: 5, 7, 15, or 17; or the polynucleotide has at least 70%, at least 75%, at least 80%, at least 85%, at least 90%, at least 95%, 96%, 97%, 98% or 99% to a polynucleotide having a sequence as set forth in SEQ ID NO: 6, 8, 16, 18, 21, 90, or 91.

In particular embodiment, the vaccine for use according to the invention comprises *Lu. longipalpis* salivary LJM17 polypeptides and/or variants or fragments thereof, and/or vectors comprising the LJM17 polynucleotide and/or variants or fragments thereof, and/or inactivated *Leishmania.* For example, the vectors for LJM17 may be selected among the group consisting of pVR2001 LJM17, pNBO002, vCP2390, vCP2390-SEQ ID NO:6 and MVA-LJM17, and may be combined with inactivated *Leishmania* selected from the group consisting of sonicated inactivated *Leishmania infantum,* and/or *Leishmania braziliensis.* For example, in one embodiment, the vaccine comprises the vCP2390 vector and sonicated inactivated *Leishmania braziliensis.* In another embodiment, the vaccine comprises the vCP2390-SEQ ID NO:6 vector and sonicated inactivated *Leishmania braziliensis.*

Described herein the vaccine comprises *Lu. longipalpis* salivary LJL143 polypeptides and/or variants or fragments thereof, and/or vectors comprising the LJL143 polynucleotide and/or variants or fragments thereof, and/or inactivated *Leishmania.* For example,
the vectors for LJL143 may be selected from the group consisting of pVR2001 LJL143, pNBO003, vCP2389, vCP2389-SEQ ID NO:2 and MVA-LJL143. The inactivated *Leishmania* may be selected from the group consisting of sonicated inactivated *Leishmania infantum,* and/or *Leishmania braziliensis.* For example, in one embodiment, the vaccine comprises the vCP2389 vector and sonicated inactivated *Leishmania braziliensis* . In another embodiment, the vaccine comprises the vCP2389-SEQ ID NO:2 vector and sonicated inactivated *Leishmania braziliensis.* In another particular embodiment, the vaccine for use according to the invention comprises at least one of *Lu. longipalpis* salivary LJM17 polypeptides and/or variants thereof as described herein and/or vectors comprising the LJM17 polynucleotide and/or variants thereof as described herein, and may additionally comprise *Lu. longipalpis* salivary LJL143 polypeptides and/or variants or fragments thereof, and/or vectors comprising the LJL143 polynucleotide and/or variants or fragments thereof, and/or inactivated *Leishmania.* For example, the vectors could be selected for LJL143 from the group consisting of pVR2001 LJL143, pNBO003, vCP2389, vCP2389-8EQ ID NO:2 and MVA-LJL143. For LJM17, the vectors may be selected from the group consisting of p VR2001 LJM17, pNBO002, vCP2390, vCP2390-SEQ 15 ID NO:6 and MVA-LJM17. The inactivated *Leishmania* may be selected from the group consisting of sonicated inactivated *Leishmania infantum,* and/or *Leishmania braziliensis.* In one embodiment, the vaccine comprises vCP2389 and vCP2390 vectors and sonicated inactivated *Leishmania braziliensis.* In another embodiment, the vaccine comprises vCP2389-SEQ ID NO:2 and vCP2390-SEQ ID NO: 6 vectors and sonicated inactivated *Leishmania braziliensis.*

The present invention relates to vaccine compositions described herein for use in vaccinating a canine against *Leishmania.*

The host is a canine (for example, dogs, bitchs, puppies, foxes, jackals, and wolves).

The routes of administration may be, for example, intramuscular (IM) or intradermal (ID) or transdermal (TD) or subcutaneous (SC). The means of administration may be, for example, a syringe with a needle, or needle free apparatus, or a syringe with a needle coupled to electrotransfer (ET) treatment, or needle free apparatus coupled to ET treatment.

Another aspect of the description relates to the use of a plasmid-based vaccine according to the present description for administration to *Leishmania,* a host, wherein this administration is coupled to ET treatment. The administration of a plasmid-based vaccine is advantageously intramuscular. The means of administration is, for example, a syringe and a needle. One or several injections may be administered successively. In the case of several injections, they may be carried out 2 to 6 weeks apart, for example, about 3 weeks apart. In one embodiment, a semi-annual booster or an annual booster is further administered.

For plasmid-based vaccines, advantageous routes of administration may be ID or IM. This administration may be through use of a syringe with a needle or with a needle free apparatus like Dermojet or Biojector (Bioject, Oregon, USA) or Vetjet™ (Merial) or Vitajet™ (Bioject Inc.), see US 2006/0034867. The dosage may be from 50 µg to 500 µg per plasmid. When DMRIE-DOPE is added, 100 µg per plasmid may be utilized. When canine GM-CSF or other cytokines are used, the plasmid encoding this protein may be present at a dosage of from about 200 µg to about 500 µg and may advantageously be 200 µg. The volume of doses can be between 0.01 ml and 0.5 ml, for example, 0.25 ml. Administration may be provided with multiple points of injection.

Alternatively, plasmid-based vaccines may be administered via the IM route coupled to electrotransfer (ET) treatment. The ET treatment may be performed using an apparatus for electrotransfer and the specifications of the manufacturer (i.e. Sphergen G250 generator (Sphergen SARL, Evry Genopole, France); MedPulser® DNA electroporation system (Innovio Biomedical Corporation, San Diego, California, USA)). In one embodiment, the apparatus for electrotransfer has a unipolar field. The field intensity may be from about 50 to about 250 V/cm, from about 50 to about 200 V/cm, or from about 50 to about 175 V/cm. The pulse duration may be from about 1 to about 50 msec, or from about 15 to about 25 msec. The frequency may be from about 1 to about 50 Hz, or from about 5 to about 15 Hz. The interpulse interval may be from about 1 to 1000 msec, or from about 1 to about 200 msec. The number of pulses may be from 1 to 20, or from 5 to 10. The intra tissular intensity may advantageously be up to about 2 A. The distance between electrodes may be from about 0.2 to about 1 cm, or from about 0.2 to about 0.5 cm.

For recombinant viral vector-based vaccines, the routes of administration may advantageously be SC or IM or TD or ID. This administration may be made by a syringe with a needle or with a needle free apparatus like Dermojet or Biojector (Bioject, Oregon, USA) or Vetjet™ (Merial) or Vitajet™ (Bioject Inc.). The dosage may be from about 10³ pfu to about 10⁹ pfu per recombinant poxvirus vector. When the vector is a canarypox virus, the dosage may be, for example, from about 10⁵ pfu to about 10⁹ pfu, or from about 10⁶ pfu to about 10⁸ pfu. The volume of doses may be from about 0.01 ml to 0.2 ml, and is advantageously 0.1 ml. Administration may comprise multiple points of injection.

For the IM route the volume of the vaccine provided may be from 0.2 to 2 ml, in particular from about 0.5 to 1 ml. The same dosages are utilized for any of the vectors for use according to the present invention.

For sub-unit vaccines, the route of administration may advantageously be via SC or IM or TD or ID. This administration may be made by a syringe with a needle or with a needle free apparatus like Dermojet or Biojector (Bioject, Oregon, USA) or Veljet™ (Merial) or Vitajet™ (Bioject Inc.). The dosage may be from about 50 to about 500 µg, in particular from about 50 to about 150 µg, and more particularly from about 50 to about 100 µg. The volume of the sub-unit vaccine provided is from 0.2 to 2 ml, in particular from about 0.5 to 1 ml.

Described herein is a vaccine strategy, which is based on a prime-boost administration regimen, where the prima-administration and the boost administration(s) utilize a composition comprising a pharmaceutically or veterinary acceptable excipient, diluent or vehicle and an *in vivo* expression vector comprising a polynucleotide sequence, that contains and expresses the *Lu. longipalpis* salivary polypeptide and/or variants or fragments thereof.

Described herein is the use of *in vivo* expression vectors in a prime-boost administration regimen, comprising a prima-administration of a vaccine comprising a pharmaceutically or veterinary acceptable vehicle, diluent or excipient, an *in vivo* expression vector containing a polynucleotide sequence for expressing, *in vivo, Lu. longipalpis* salivary polypeptides and/or variants or fragments thereof, followed by a boost administration of a vaccine comprising a pharmaceutically or veterinary acceptable vehicle or excipient, an *in vivo* expression vector containing a polynucleotide sequence for expressing, *in vivo,* sand fly *Lu. longipalpis* polypeptides and/or variants or fragments thereof as described above, to protect a host from leishmaniasis and/or to prevent disease progression in infected hosts.

A prime-boost regimen comprises at least one prima-administration and at least one boost administration using at least one common polypeptide and/or variants or fragments thereof. The vaccine used in prima-administration may be different in nature from those used as a later booster vaccine. The prime-administration may comprise one or more administrations. Similarly, the boost administration may comprise one or more administrations.

The routes of administration, doses and volumes are as previously disclosed herein.

The prime-boost administrations may be advantageously carried out 2 to 6 weeks apart, for example, about 3 weeks apart. According to one embodiment, a semi-annual booster or an annual booster, advantageously using the viral vector-based vaccine, is also envisaged. The animals are advantageously at least 6 to 8 weeks old at the time of the first administration.

Described herein the prime-boost administration regimen comprises at least one prima-administration of a plasmid-based vaccine according to the present description and at least one boost-administration of a recombinant viral vector-based vaccine according to the present description.

In a particular embodiment, the prime-boost administration regimen comprises at least one prima-administration of a recombinant viral vector-based for use according to the present invention and at least one boost-ad1ninistration of a sub-unit vaccine for use according to the present invention.

Described herein the prime-boost administration regimen comprises at least on prima-administration of a recombinant viral vector-based vaccine according to the present description and at least one boost-administration of a plasmid-based vaccine according to the present description.

Described herein the present description relates to a method of vaccinating a subject susceptible to *Leishmania* comprising a prime-boost administration regimen wherein said regiment comprises a prima-administration of a vaccine comprising, in a pharmaceutically or veterinary acceptable vehicle, diluent or excipient, a plasmid containing a polynucleotide for expressing, *in vivo,* a salivary *Lu. longipalpis* polypeptide, a variant or fragment of the salivary *Lu. longlpalpis* polypeptide, or a mixture thereof, followed by a boost administration of a vaccine comprising, in a pharmaceutically or veterinary acceptable vehicle or excipient, a recombinant viral vector comprising a polynucleotide for expressing, *in vivo,* the same salivary *Lu. longlpalpis* polypeptide(s), variant thereof, fragment thereof, to protect the subject from leishmaniasis and/or to prevent disease progression in infected subject.

Described herein the present description relates to a method vaccinating a subject susceptible to *Leishmania* comprising a prime-boost administration regimen wherein said regiment comprises a prima-administration of a vaccine comprising, in a pharmaceutically or veterinary acceptable vehicle, diluent or excipient, a recombinant viral vector comprising a polynucleotide for expressing, *in vivo,* a salivary *Lu. longipalpis* polypeptide, a variant or fragment of the salivary *Lu. longipalpis* polypeptide, or a mixture thereof, followed by a boost administration of a vaccine comprising, in a pharmaceutically or veterinary acceptable vehicle or excipient, a plasmid containing a polynucleotide for expressing, *in vivo,* the same salivary *Lu. longipalpis* polypeptide(s), variant thereof, fragment thereof, to protect the subject from leishmaniasis and/or to prevent disease progression in infected subject.

In one embodiment, the present invention relates to a method of vaccinating a canine susceptible to *Leishmania* comprising a prime-boost administration regimen wherein said regiment comprises a prima-administration of a vaccine comprising, in a pharmaceutically or veterinary acceptable vehicle, diluent or excipient, a recombinant viral vector comprising a polynucleotide for expressing, *in vivo,* a salivary *Lu. longipalpis* polypeptide, a variant of the salivary *Lu. longipalpis* polypeptide, or a mixture thereof, followed by a boost administration of a vaccine comprising, in a pharmaceutically or veterinary acceptable vehicle or excipient, the same salivary *Lu. longipalpis* polypeptide(s), variant thereof, to protect the canine from leishmaniasis and/or to prevent disease progression in infected canine.

In another embodiment, the prime-boost administration regimen for use according to the invention may additionally comprise at least one boost-administration of a vCP2390 or vCP2390-SEQ ID NO:6 vector-based vaccine.

In another embodiment, the prime-boost administration regimen for use according to the invention may additionally comprise at least one boost-administration of a vCP2389 or vCP2389-SEQ ID NO:2 vector-based vaccine.

In another embodiment, the prime-boost administration regimen for use according to the invention comprises at least one prima-administration of a pVR2001 LJL143 and pVR2001 LJM17 plasmid-based vaccine, and may additionally comprise at least one boost-administration of a vCP2389 or vCP2390 vector-based vaccine.

In another embodiment, the prime-boost administration regimen for use according to the invention comprises at least one prima-administration of a pNBO003 and pNBO002 plasmid-based vaccine, and may additionally comprise at least one boost-administration of a vCP2389-SEQ ID NO:2 and vCP2390-SEQ ID NO:6 vector-based vaccine.

In yet another embodiment, the prime-boost administration regimen for use according to the invention may additionally comprise at least one boost-administration of a MVA-LJM17 vector-based vaccine.

In another embodiment, the prime-boost administration regimen for use according to the invention may additionally comprise at least one boost-administration of a MVA-LJL143 vector-based vaccine.

In another embodiment, the prime-boost administration regimen for use according to the invention comprises at least one primo-administration of a pVR2001 LJL143 and pVR2001 LJM17 plasmid-based vaccine, and at least one boost-administration of a MVA-LJLI43 and MVA-LJM17 vector-based vaccine.

In yet another embodiment, the prime-boost administration regimen for use according to the invention comprises at least one primo-administration of a pNBO003 and pNBO002 plasmid-based vaccine, and may additionally comprise at least one boost-administration of a MVA-LJL143 and MVA-LJM17 vector-based vaccine.

In another embodiment, the prime-boost administration regimen for use according to the invention comprises at least one primo-administration of a vCP2390 or vCP2390-SEQ ID NO:6 vector-based vaccine, and at least one boost-administration of a LJM17 polypeptide sub-unit vaccine.

In another embodiment, the prime-boost administration regimen for use according to the invention may additionally comprise at least one boost-administration of a LJL143 polypeptide sub-unit vaccine.

In another embodiment, the prime-boost administration regimen for use according to the invention comprises at least one prima-administration of a vCP2389 or vCP2389-SEQ ID NO:2 and vCP2390 or vCP2390-SEQ ID NO:6 vector-based vaccine, and at least one boost-administration of a LJLJ 43 polypeptide and LJM17 polypeptide sub-unit vaccine.

In yet another embodiment, the prime-boost administration regimen for use according to the invention comprises at least one prima-administration of a MVA-LJM17 vector-based, and at least one boost-administration of a LJM17 polypeptide sub-unit vaccine.

Described herein the prime-boost administration regimen comprises at least one prima-administration of a MVA-LJL143 vector-based vaccine, and at least one boost administration of a LJL143 polypeptide sub-unit vaccine.

In another embodiment, the prime-boost administration regimen for use according to the invention comprises at least one prima-administration of a MVA-LJLJ43 and MVA-LJM17 vector-based vaccine, and at least one boost-administration of a LJL143 polypeptide and LJM17 polypeptide sub-unit vaccine.

In another embodiment, the prime-boost administration regimen for use according to the invention may additionally comprise at least one boost-administration of a pVR2001 LJM17 or pNBO002 plasmid-based vaccine.

In another embodiment, the prime-boost administration regimen, for use according to the invention may additionally comprise at least one boost-administration of a pVR2001 LJL143 or pNBO003 plasmid-based vaccine.

In another embodiment, the prime-boost administration regimen for use according to the invention comprises at least one prima-administration of a vCP2389 and vCP2390 vector-based vaccine, and may additionally comprise at least one boost-administration of a pVR2001 LJLI43 and pVR2001 LJMI7 plasmid-based vaccine.

In another embodiment, the prime-boost administration regimen for use according to the invention comprises at least one prima-administration of a vCP2389-SEQ ID NO:2 and vCP2390-SEQ ID NO:6 vector-based vaccine, and may additionally comprise at least one boost-administration of a pNBO003 and pNBO002 plasmid-based vaccine.

Described herein the prime-boost administration regimen comprises at least one prima-administration of a MVA-LJM17 vector-based vaccine, and at least one boost administration of a pVR2001 LJM17 or pNBO002 plasmid-based vaccine.

Described herein the prime-boost administration regimen comprises at least one prima-administration of a MVA-LJL143 vector-based vaccine, and at least one boost administration of a pVR2001 LJL143 or pNBO003 plasmid-based vaccine.

In another embodiment, the prime-boost administration regimen for use according to the invention comprises at least one prima-administration of a MVA-LJL143 and MVA-LJM17 vector-based vaccine, and may additionally comprise at least one boost-administration of a pNBO003 and pNBO002 plasmid-based vaccine.

Another aspect of the present invention relates to a kit for prime-boost vaccination according to the present description. The kit may comprise at least two vials: a first vial containing a vaccine for the prima-vaccination according to the present description and a second vial containing a vaccine for the boost-vaccination according to the present description. The kit may advantageously contain additional first or second vials for additional prima-vaccinations or additional boost-vaccinations.

In one embodiment, the kit may comprise two vials, one containing a plasmid-based vaccine for the prima-vaccination according to the present description the other vial containing a recombinant viral vector-based vaccine for the boost-vaccination according to the present description.

Described herein the kit may comprise two vials, one containing a recombinant viral vector-based vaccine for the prima-vaccination according to the present description, the other vial containing a sub-unit vaccine for the boost-vaccination according to the present description.

In another embodiment, the kit may comprise two vials, one containing a recombinant viral vector-based vaccine for the prima-vaccination according to the present description, the other vial containing a plasmid-based vaccine for the boost-vaccination according to the present description.

It is disclosed herein that individuals who experience an *anti-Leishmania* DTH response conversion also have an increase in antibodies against *Lu. longipalpis* salivary proteins. Thus, the presence or absence of antibodies to *Lu. longipalpis* salivary proteins can be used to ascertain if a subject has a *Leishmania* infection.

A method is disclosed herein for diagnosing infection with *Leishmania* by detecting the presence of antibodies that specifically bind one or more polypeptides having an amino acid sequence as set forth in SEQ ID NO: 1, 3, 5, 7, 11, 13, 15, 17, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, or 87, or a polypeptide having at least 80%, at least 90%, at least 95%, or at least 99% homologous to one of these polypeptides, a conservative variant, a homolog or an immunogenic fragment of one of these polypeptides. The method can utilize a single *Lu. longipalpis* polypeptide or a combination of these polypeptides. In certain examples, the method of diagnosis detects antibodies that specifically bind at least 3, 6, or 10 of these polypeptides, or immunogenic fragments thereof.

Described herein one or more *Lu. longipalpis* polypeptide can be bound to a solid substrate. For example, the *Lu. longipalpis* polypeptide having an amino acid sequence as set forth in SEQ ID NO: 1, 3, 5, 7, 11, 13, 15, 17, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, or 87 can be bound to the substrate. One of more of these polypeptides can be bound to the substrate, for example at least 3, 6, or 10 of these polypeptides, or an immunogenic fragment thereof. In one example, one or more polypeptides having a sequence as set forth in SEQ ID NO: 1, 3, 5, 7, 11, 13, 15, 17, 25, 33, 39, 47, or 77 can be bound to the substrate. In another example, one or more *Lu. longipalpis* polypeptides having a sequence as set forth in SEQ ID NO: 1, 3, 5, 7, 11, 13, 15, 17, or 57 can be bound to the substrate. In one specific, non-limiting example, at least six *Lu. longipalpis* polypeptides are bound to a solid substrate, wherein each of the polypeptides comprises an amino acid sequence as set forth in SEQ ID NO: 25, SEQ ID NO: 1 (or 3, or 11, or 13), SEQ ID NO: 5 (or 7, or 15, or 17), SEQ ID NO: 47, SEQ ID NO: 73, or SEQ ID NO: 77, or an immunogenic fragment thereof. In another specific, non-limiting example, at least three *Lu. Longipalpis* polypeptides are bound to a solid substrate, wherein each of the polypeptides comprises an amino acid sequence as set forth in SEQ ID NO: 1 (or 3, or 11, or 13), SEQ ID NO: 5 (or 7, or 15, or 17), or SEQ ID NO: 57, or an immunogenic fragment thereof.

Described herein two or more (for example at least 3, 6, or 10) *Lu. longipalpis* polypeptides (or immunogenic fragments thereof) are applied to a solid substrate, for example as a series of "dots," such as in a "dot blot" assay. Described herein two or more *Lu. longipalpis* polypeptides are applied to a substrate such as in a linear array. Described herein *Lu. longipalpis* polypeptides are applied to a membrane in a two-dimensional array. In this manner, the presence of antibodies to more than one *Lu. longipalpis* polypeptide is assessed. Each *Lu. longipalpis* polypeptide can be applied directly to the surface of a membrane in a single location or in a combination of locations.

The solid substrate can be a polystyrene bead, a membrane, a chip or a plate. A plastic or glass substrate can be utilized. Described herein a membrane is utilized that is composed of porous materials such as nylon, nitrocellulose, cellulose acetate, glass fibers, and other porous polymers. The surface of a solid support may be activated by chemical processes that cause covalent linkage of polypeptide to the support. However, any other suitable method may be used for immobilizing a polypeptide to a solid support including, without limitation, ionic interactions, hydrophobic interactions, covalent interactions and the like. Once the polypeptide is applied to the substrate, the substrate can be contacted with a substance, such as protein-containing solution, which non-specifically saturates the binding sites thereon. Specific, non-limiting examples of a protein-containing solution include a solution made from powdered milk or serum albumin, such as bovine serum albumin.

A specimen (for example, sera, blood, plasma, urine, semen, saliva, sputum, lacrimal fluid, lymph fluid) is then added to the substrate, and the combined specimen and substrate are incubated for a sufficient time to allow specific binding. Specific binding of antibodies to the *Lu. longipalpis* polypeptides disclosed herein, are then detected using any means known to one of skill in the art. Described herein a labeled secondary antibody is used to detect the antibodies that specifically bind the *Lu. longipalpis* polypeptides. The label can be a radio label (for example, ¹²⁵I), an enzymatic label (for example, alkaline phosphatase or horseradish peroxidase), or a fluorescent label (for example, fluoroscein isothiocyanate). Detection systems for these labels are known to one of skill in the art. Binding of the specimen, or a component of the specimen, to the *Lu. longipalpis* polypeptide, as indicated by the presence of the marker, indicates infection with *Leishmania.*

Described herein the specimen is adsorbed onto a solid substrate containing binding sites for polypeptides, such as antibody molecules. Described herein the solid substrate is a polystyrene bead, a chip, a membrane or a plate. The substrate is thereafter contacted with a substance, such as a protein-containing solution that non-specifically saturates the binding sites thereon. The substrate is then washed with a buffer. A solution of one or more *Lu. longipalpis* polypeptides is then added to the bound specimens. Described herein the *Lu. longipalpis* polypeptide is directly labeled. The labeling of the *Lu. longipalpis* polypeptide can be brought about by use of any marker, such as by incorporation of a radioactive isotope or group, or by coupling this component to an enzyme, a dyestuff, for example a chromophotic moiety or a fluorescent group. The enzymes of use are those which can be colorimetrically, spectrophotometrically, or fluorimetrically determined. Non-limiting examples of enzymes for use in the present description include enzymes from the group of oxidoreductases, such as catalase, peroxidase, glucose oxidase, beta-glucuronidase, beta-D-glucosidase, beta-D-galactosidase, urease and galactose oxidase. After the labeled *Lu. longipalpis* polypeptide is incubated with the solid substrate, any unbound labeled *Lu. longipalpis* polypeptide is removed by washing. Bound labeled *Lu. longipalpis* polypeptide is then detected by an appropriate assay. Binding of the labeled *Lu. longipalpis* polypeptide to the specimen, or to a component of the specimen, is indicative of infection with *Leishmania.*

In general, the incubation steps utilized in carrying out the procedures can be performed in a known manner, such as by incubating at temperatures between about 4°C and about 25°C, for about 30 minutes to about 48 hours. Washings can be included with an aqueous solution such as a buffer, wherein the buffer is from about pH 6 to about pH 8, such as by using an isotonic saline solution of a pH of about 7.

Competitive binding assays are also of use in detecting infection with *Leishmania.* One of skill in the art, given the *Lu. longipalpis* polypeptides disclosed herein, will readily be able to design additional assays, such as competitive binding assays, of use in detecting *Leishmania* infection.

Described herein the *Lu. longipalpis* polypeptides disclosed herein can be included in a diagnostic test kit. For example, a diagnostic test kit for detecting a *Leishmania* infection includes a solid substrate having applied thereon one or more *Lu. longipalpis* polypeptide disclosed herein. Described herein the kit includes written instructions and/or a container including a specified amount of labeled antibodies to immunoglobulins, such as IgG or IgM, or labeled secondary antibodies that bind antibodies from a species of interest. For example labeled antibodies can be provided that specifically detect dog or human immunoglobulins. The labeled antibodies can be fluorescently labeled, enzymatically labeled, or radio labeled. Labeled antibodies used in the above-described test kits can be packaged in either solution or lyophilized form suitable for reconstitution.

Described herein the test kit includes a specified amount of one or more *Lu. longipalpis* polypeptide described herein in a container, and written instructions. In one example, the *Lu. longipalpis* polypeptide is directly labeled. In another example, the one or more *Lu. longipalpis* polypeptide is unlabeled. If the *Lu. longipalpis* polypeptide is unlabeled, a container can also be included with a detection reagent that specifically binds the *Lu. longipalpis* polypeptide, such as a labeled monoclonal antibody. The kit can also optionally include a solid substrate for binding the specimen.

The above described process and test kit for detection of antibodies to the *Lu. longipalpis* polypeptides disclosed herein can be utilized in many applications, including, but not limited to detecting *Leishmania* infection in a subject using the methods disclosed herein. The tests and kits disclosed herein can be used to detect the efficacy of a therapeutic treatment to a subject.

Described herein the tests and kits disclosed herein can also be used to assess a primary infection with *Leishmania* or to predict recovery from *Leishmania* infection by taking a body fluid from an infected subject, for example at various times following infection, and applying the above described detection procedures.

The invention will now be further described by way of the following non-limiting examples.

### EXAMPLES

Without further elaboration, it is believed that one skilled in the art can, using the preceding descriptions, practice the present invention to its fullest extent. The following detailed examples are to be construed as merely illustrative, and not limitations of the preceding disclosure in any way whatsoever, Those skilled in the art will promptly recognize appropriate variations from the procedures both as to reactants and as to reaction conditions and techniques.

Construction of DNA inserts, plasmids and recombinant viral vectors was carried out using the standard molecular biology techniques described by J. Sambrook et al. (Molecular Cloning: A Laboratory Manual, 2nd Edition, Cold Spring Harbor Laboratory. Cold Spring Harbor, New York, 1989). All the restriction fragments used for the present description were isolated using the "Geneclean" kit (BIO 101 Inc., La Jolla, Calif.).

### EXAMPLE 1: Construction of the pVR2001 LJM17 plasmid expressing the Lu. longipalpis salivary LJM17 polypeptide

The polynucleotide encoding the *Lu. longipalpis* LJM17 polypeptide is synthesized and has the sequence described in SEQ ID NO: 8 which contains poly(A) tail. The LJM17 fragment is amplified by PCR and cloned into the TOPO cloning site of the pVR2001-TOPA donor plasmid (also referred to as pVR2001-TOPO). The resultant plasmid, pVR2001 LJM17 therefore contains and expresses a nucleotide encoding a promoter capable of driving expression in a mammalian cell, a leader peptide for facilitating secretion/release of a prokaryotic protein sequence from a mammalian cell, LJM17, topoisomerases flanking the DNA encoding LJM17, as well as a termination sequence.

The nucleic acid sequence of one strand of the plasmid pVR2001 LJM17 is described in SEQ ID NO: 9 and in Figure 1, wherein BamHI sites are in positions [4-9] and [5051-5056], the nucleotide sequence encoding the tPA signal peptide is in position [4976-5062] and the nucleotide sequence encoding LJM17 is in position [5063-6247].

### EXAMPLE 2: Construction of the pVR2001 LJL143 plasmid expressing the Lu. longipalpis salivary LJL143 polypeptide

The polynucleotide encoding the *Lu. longipalpis* LJL143 polypeptide is synthesized and has the sequence described in SEQ ID NO: 4 which contains poly(A) tail. The LJL143 fragment is amplified by PCR and cloned into the TOPO cloning site of the pVR2001-TOPA plasmid as described in example 1, to generate the plasmid pVR2001 LJL143.

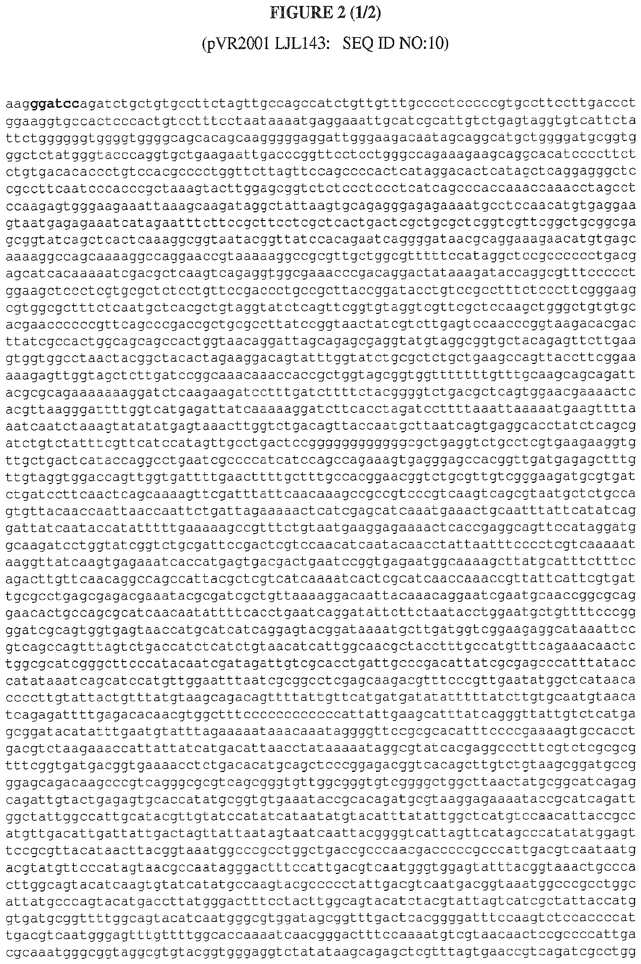

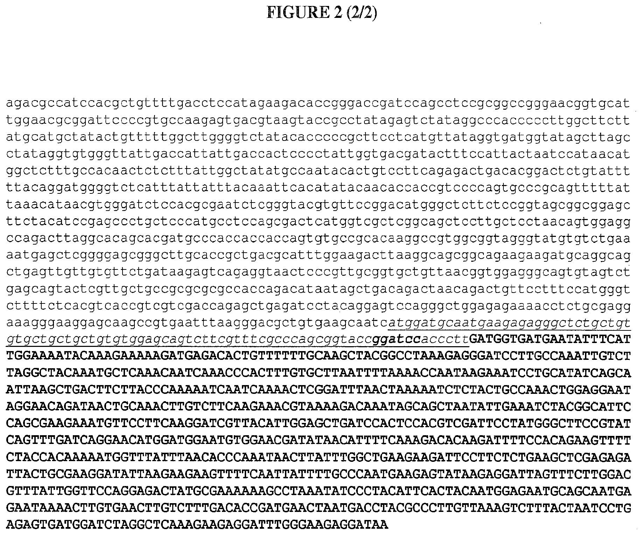

The nucleic acid sequence of one strand of the plasmid pVR2001 LJL143 is described in SEQ ID NO: 10 and in Figure 2, wherein BamHI sites are in positions [4-9] and [5051-5056], the nucleotide sequence encoding the tPA signal peptide is in position [4976-5062] and the nucleotide sequence encoding LJL143 is in position [5063-5899].

### EXAMPLE 3: Construction of an ALVAC canarypox virus vector expressing the Lu. longipalpis salivary LJM17 polypeptide

For a discussion and examples of the plasmid pALVAC, and the C3 locus, see e.g., U.S. Patent Nos. 5,756,103; 5,833,975; and 6,780,407. The sequence of the vaccinia virus H6 promoter has been previously described (see e.g., Taylor *et al.;* Taylor *et al.;* Guo *et al.).*

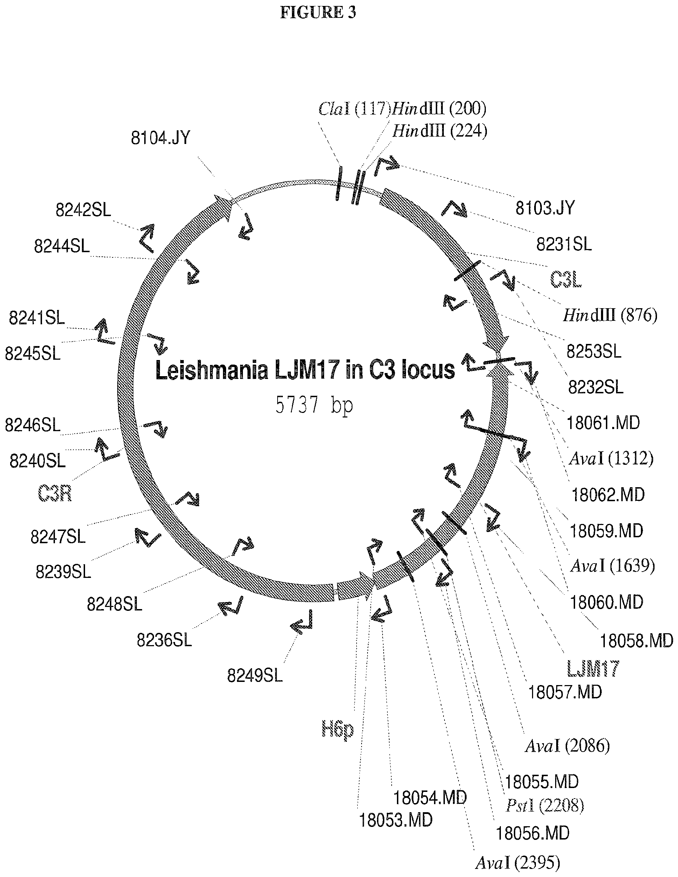

The polynucleotide encoding the *Lu. longipalpis* LJM17 polypeptide is synthesized and has the sequence described in SEQ ID NO:6. This polynucleotide is then ligated to the pALVAC C3 H6p donor plasmid resulting in pALVAC C3 H6p-LJM17 containing 5737 base pairs (Figure 3).

To generate vCP2390-SEQ ID NO:6, the pALVAC C3 H6p-LJM17 plasmid is linearized with NotI restriction enzyme. The linearized fragments were individually transfected into ALVAC-infected primary CEF cells using the calcium phosphate precipitation method (see, Panicali *et al.;* Piccini *et al.*)*.* After 24h, the transfected cells are harvested, sonicated and used for recombinant virus screening.

Recombinant plaques are screened based on the plaque lift hybridization method using a *Lu. longipalpis* LJM17-specific probe which is labeled with horse radish peroxidase according to the manufacturer's protocol (Amersham Cat# RPN-3001). After three sequential rounds of plaque purification, the recombinants are generated by hybridization confirmation as 100% positive for the *Lu. longipalpis* LJM17 insert and 100% negative for the C3 ORF.

A single plaque is selected from the third round of plaque purification and expanded to obtain P1 (60 mm), P2 (T75 flasks), and P3 (roller bottles) stocks to amplify vCP2390-SEQ ID NO:6. The infected cell culture fluid from the roller bottles is harvested and concentrated to produce virus stock.

The construct is sequenced to confirm the sequences of the *Lutzomyia longipalpis* LJM17 insert and the C3 left and right arms around the *Lutzomyia longipalpis* LJM17 insert in vCP2390-SEQ ID NO:6.

### EXAMPLE 4: Construction of an ALVAC canarypox virus vector expressing the Lu. longipalpis salivary LJL143 polypeptide

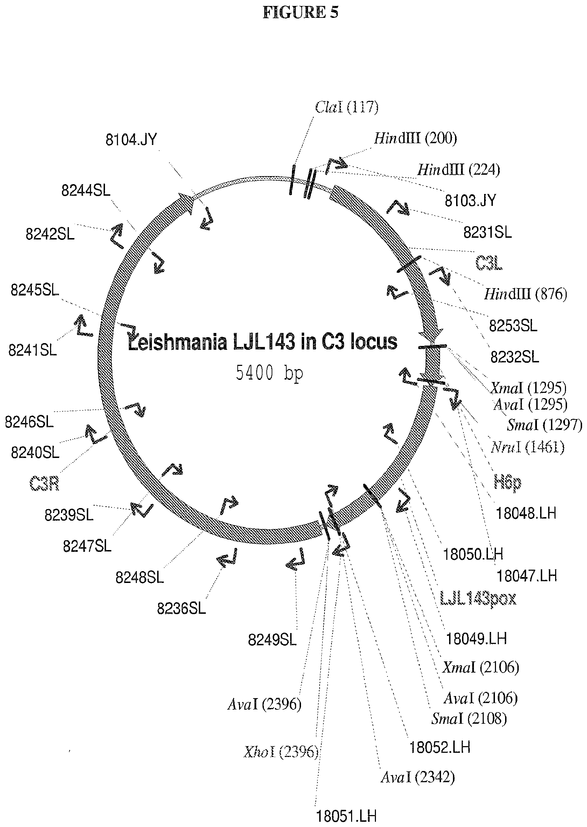

The polynucleotide encoding the *Lu. longipalpis* LJL143 polypeptide is synthesized and has the sequence described in SEQ ID NO:2. This sequence is then ligated to a pALVAC C3 H6p donor plasmid. The resulting plasmid, pALVAC C3 H6p-LJL143 comprises 5400 base pairs (Figure 5), and is sequenced to confirm the nucleic acid sequence (SEQ ID NO: 2) of the LJL143 gene.

To generate vCP2389-SEQ ID NO:2, the pALVAC C3 H6p-LJL143 plasmid is linearized with NotI restriction enzyme. The linearized fragments are individually transfected into ALVAC-infected primary CEF cells by using the calcium phosphate precipitation method (see, Panicali *et al.;* Piccini *et al.*)*.* After 24h, the transfected cells are harvested, sonicated and used for recombinant virus screening.

Recombinant plaques are screened based on the plaque lift hybridization method using a *Lu. longipalpis* LJL143-specific probe which is labeled with horse radish peroxidase according to the manufacturer's protocol (Amersham Cat# RPN-3001). After three sequential rounds of plaque purification, the recombinants are generated by hybridization confirmation as 100% positive for the *Lu. longipalpis* LJL143 insert and 100% negative for the C3 ORF.

A single plaque is selected from the third round of plaque purification and expanded to obtain P1 (60 mm), P2 (T75 flasks), P3 (roller bottles) stocks to amplify vCP2389-SEQ ID NO:2. The infected cell culture fluid from the roller bottles are harvested and concentrated to produce virus stock.

The construct is sequenced to confirm the sequences of the *Lutzomyia longipalpis* LJL143 insert and the C3 left and right arms around the *Lutzomyia longipalpis* LJL143 insert in vCP2389-SEQ ID NO:2.

### EXAMPLE 5: Construction of a MVA vector expressing the Lu. longipalpis salivary LJM17 polypeptide

MVA is a modified Ankara strain obtained after more than 570 passages of the Ankara vaccine strain on chicken embryo fibroblasts (see Stickl & Hochstein-Mintzel; Sutter *et al.*) available as ATCC VR-1508. Its adaptation to these cells caused the excision of 6 regions which are nonessential for its development and its infectious cycle on this type of cells (disappearance of about 15% of the viral genome; Meyer *et al.*). Exogenous genetic material may be inserted into any of these excision regions. In the context of the present description foreign genetic material is inserted into excisions II and III which are located using the HindIII restriction fragments N and A respectively (Altenburger *et al.).*

Engineering of the recombinant MVA virus expressing LJM17 is performed as previously described in Staib C. *et al.,* with the exception that the 723-base pairs DNA fragment containing the gfp ORF is replaced by the 1239 base pairs DNA fragment encoding LJM17 antigen (SEQ ID NO: 6), generating MVA-LJM17.

### EXAMPLE 6: Construction of a MVA vector expressing the Lu. longipalpis salivary LJL143 polypeptide

Construction of the recombinant MVA virus expressing LJL143 is performed as previously described in Staib C. *et al.,* with the exception that the 723-base pairs DNA fragment containing the gfp ORF is replaced by the 906 base pairs DNA fragment encoding LJL143 antigen (SEQ ID NO: 2), generating MVA-LJL143.

### EXAMPLE 7: Expression of Lu. longipalpis salivary protein in vitro

Expression plasmids containing His-tagged *Lu. longipalpis* salivary antigens encoded by cDNA derived from the plasmids pVR2001 LJM17 (see example 1) and pVR2001 LJL143 (see example 2) are constructed and transfected in HEK-293F cells. Supernatants collected at 72 h are analyzed by HPLC chromatography using Nickel column and imidazole gradient HPLC fractions positive for the recombinant salivary protein with 6x His motif at their C-terminus are tested with polyclonal sera of mice previously immunized with the corresponding original cDNA plasmids. Recombinant proteins are tested by SDS-PAGE and Western blotting, aliquoted in PBS and stored at -70°C.

The sub-unit vaccine comprising LJM17 is referred to herein as rLJM17. The vaccine is prepared by combining 100 µg of purified recombinant protein LJM17 with 300 µg of the adjuvant CpG #2142 in 20% Emulsigen® (MVP laboratories) (for CpG #2142, see SEQ. ID. NO: 890 in EP-B1-1,221,955).

The sub-unit vaccine comprising LJL143 is referred to herein as rLJL143. The vaccine is prepared by combining 100 µg of purified recombinant protein LJL143 with 300 µg of the adjuvant CpG #2142 in 20% Emulsigen®.

### EXAMPLE 8: Vaccination of dogs against leishmaniasis

25 dogs (1-2 years old female beagles) are randomly divided into 5 groups of 5 dogs each. All dogs from groups 1 to 4 are vaccinated at D0 (V1) by the intradermal (ID) route into the ear pinnea using a syringe and a needle with 500 µg of purified pVR2001 LJM17 plasmid (example 1) expressing LJM17 (groups 1 and 2) or pVR2001 LJL143 plasmid (example 2) expressing LJL143 (groups 3 and 4).

Dogs from group 1 and group 3 are boosted at D14 (V2) and D28 (V3) with 500 µg of the same plasmids used for V1 by the transdermal (TD) route in the inner upper part of both hind legs using the VetJet™ (Merial) needle-free delivery device. Dogs from group 1 and group 3 were further boosted at D42 (V4) with 500 µg of the same plasmids by the intramuscular route coupled to electroporation (ET/IM) in the external side of both thighs using the Sphergen devise and technology (parameters: 88V, T1=20, T2=80, N=10).

Dogs from groups 2 and 4 are boosted at D14 (V2) and D28 (V3) with 500 µg of the same plasmids used for V1 by the IM route coupled to electroporation (ET/IM) as described above. Dogs from groups 2 and 4 are further boosted at D42 (V4) by the ID route into the ear pinnea as described above with sub-unit vaccines (see example 7) rLJM17 (group 2) or rLJL143 (group 4), respectively.

All dogs from groups 1 and 2 receive a final vaccine booster at D192 (V5) by the IM route in the left quadriceps using 10⁸ pfu of a recombinant canarypox virus vCP2390 (example 3) expressing LJM17. All dogs from groups 3 and 4 receive a final vaccine booster at D192 (V5) by the IM route in the left quadriceps using 10⁸ pfu of a recombinant canarypox virus vCP2389 (example 4) expressing LJL143.

Dogs from group 5 are vaccinated with 500 µg of the purified parental plasmid pVR2001 which expresses no antigen at D0 by ID, D14 by TD, D28 by TD and D42 by ET/IM and receive a final booster at D192 by the IM route using 10⁸ pfu of a control recombinant canarypox virus (Purevax™).

### EXAMPLE 9: Construction of the pNBO002 plasmid expressing the Lu. longipalpis salivary LJM17 polypeptide

The nucleic acid sequence encoding the *Lu. longipalpis* LJM17 polypeptide was synthesized and has the sequence described in SEQ ID NO: 18 which contains poly(A) tail. The LJM17 fragment was amplified by PCR and cloned into the TOPO cloning site of the pVR2001-TOPA donor plasmid as described in example 1, to generate the plasmid pNBO002.

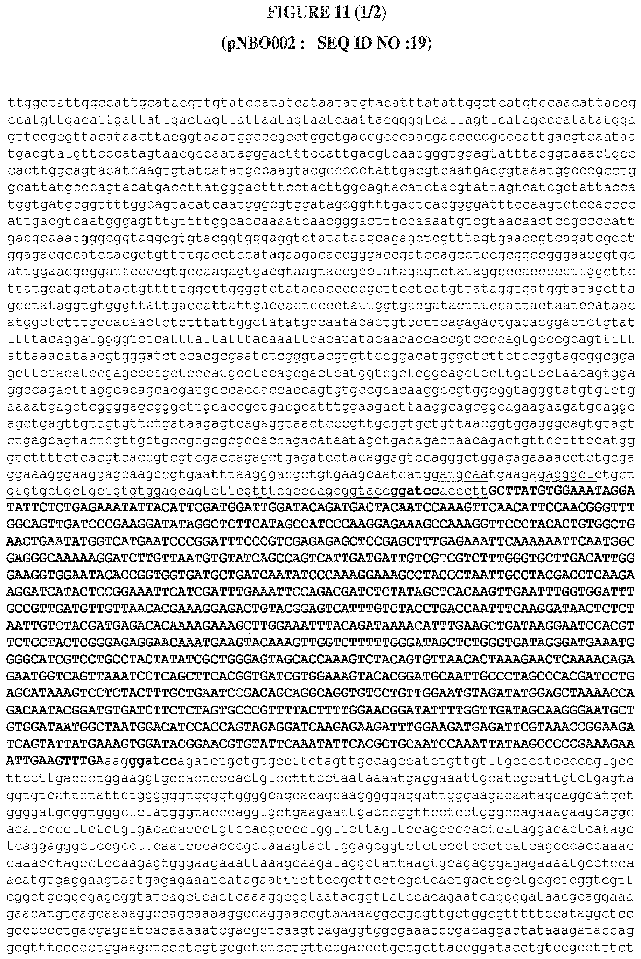

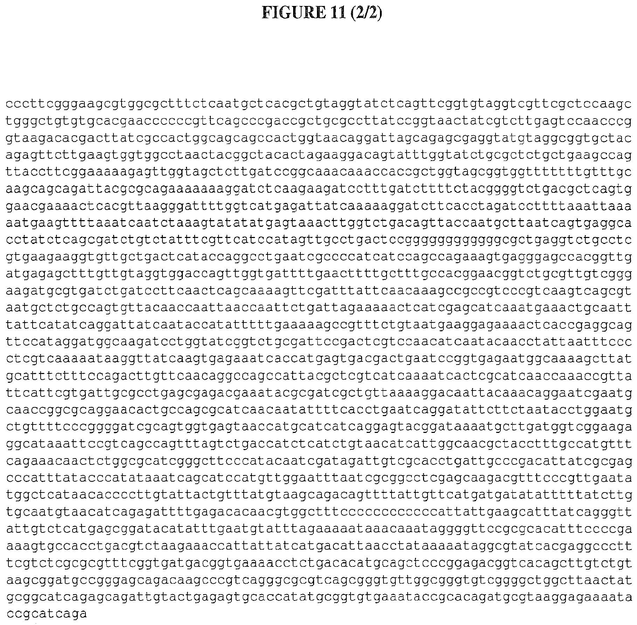

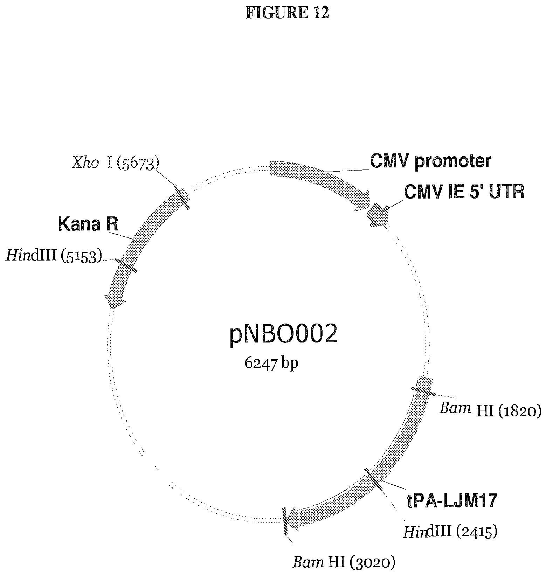

The nucleic acid sequence of one strand of the plasmid pNBO002 is described in SEQ ID NO: 19 and in Figure 11, wherein BamHI sites are in positions [1819-1824] and [3019-3024], the nucleotide sequence encoding the tPA signal peptide is in position [1744-1830] and the nucleotide sequence encoding LJM17 is in position [1831-3015]. The map of the pNBO002 plasmid is shown in Figure 12.

pNBO002 is analogous to pVR2001 LJM17. In this case, this construct and the construct of example 1 are identifiable by the nucleic acid sequence of their inserts, SEQ ID NO: 18 and SEQ ID NO: 8, respectively.

### EXAMPLE 10: Construction of the pNBO003 plasmid expressing the Lu. longipalpis salivary LJL143 polypeptide

The nucleic acid sequence encoding the *Lu. longipalpis* LJL143 was synthesized and has the sequence described in SEQ ID NO: 14, which contains poly(A) tail. The LJL143 fragment was amplified by PCR and cloned into the TOPO cloning site of the pVR2001-TOPA plasmid, as described in example 1, to generate the plasmid pNBO003.

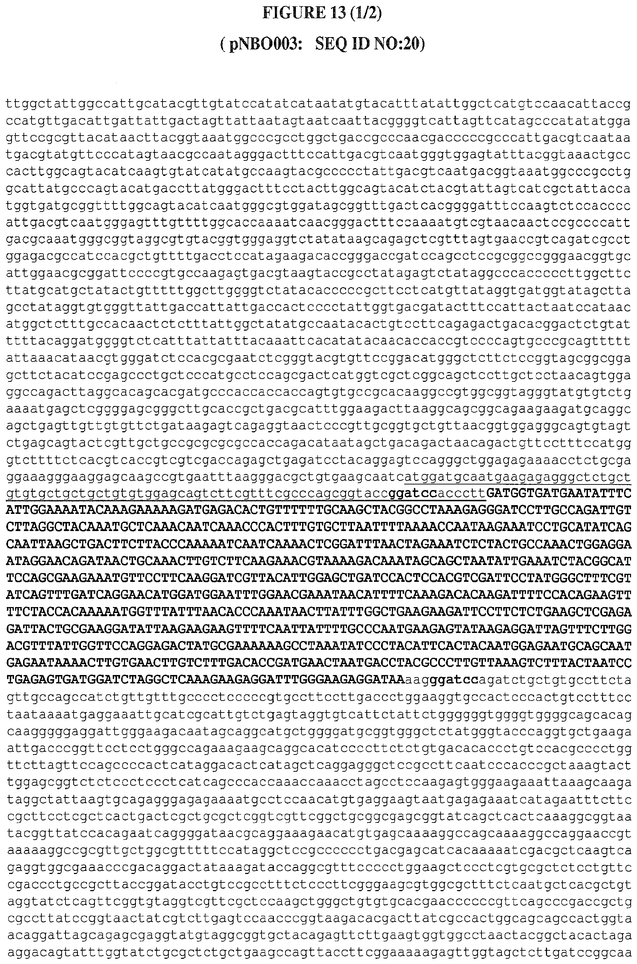

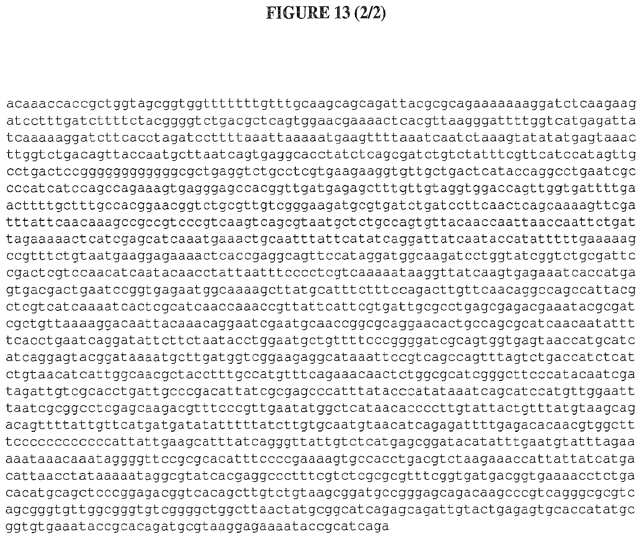

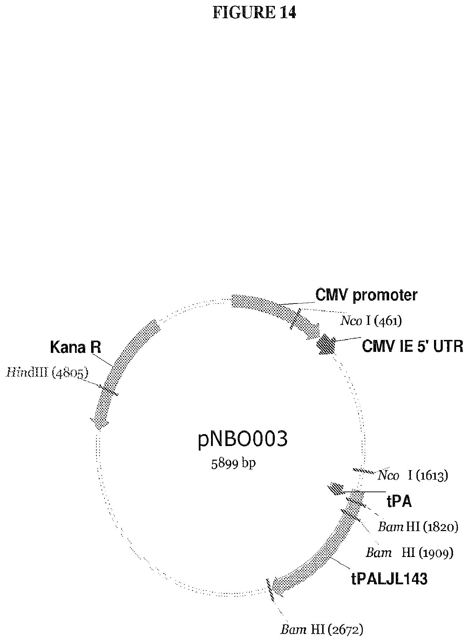

The nucleic acid sequence of one strand of the plasmid pNBO003 is described in SEQ ID NO: 20 and in Figure 13, wherein BamHI sites are in positions [1819-1824] and [2671-2676], the nucleotide sequence encoding the tPA signal peptide is in position [1744-1830] and the nucleotide sequence encoding LJL143 is in position [1831-2667]. The map of the pNBO003 plasmid is shown in Figure 14.

pNBO003 is analogous to pVR2001 LJL143. In this case, this construct and the construct of example 2 are identifiable by the nucleic acid sequence of their inserts, SEQ ID NO: 14 and SEQ ID NO: 4, respectively.

### EXAMPLE 11: Construction of an ALVAC canarypox virus vector expressing the Lu. longipalpis salivary LJM17 polypeptide

For discussion and examples of the plasmid pALVAC and the C3 locus, see e.g., U.S. Patent Nos. 5,756,103; 5,833,975; and 6,780,407. The sequence of the vaccinia virus H6 promoter has been previously described (see e.g., Taylor *et al.;* Taylor *et al.;* Guo *et al.).*

The nucleic acid sequence encoding the *Lu. longipalpis* LJM17 polypeptide was synthesized and has the sequence described in SEQ ID NO:6. This sequence was codon-optimized for mammalian expression by Geneart GmbH (Regensburg, Germany), resulting in the sequence described in SEQ ID NO:91, which encodes the LJM17 polypeptide (SEQ ID NO:5).

This codon-optimized sequence was then ligated to the pALVAC C3 H6p donor plasmid to generate pALVAC C3 H6p-LJM17 containing 5737 base pairs (Figure 3). The resulting plasmid, pALVAC C3 H6p-LJM17, was sequenced and confirmed to contain the nucleic acid sequence (SEQ ID NO:91) of the LJM17 gene.

To generate vCP2390, the pALVAC C3 H6p-LJM17 plasmid was linearized with NotI restriction enzyme. The linearized fragments were individually transfected into ALVAC-infected primary CEF cells by using the calcium phosphate precipitation method described previously (Panicali *et al.;* Piccini *et al.*)*.* After 24h, the transfected cells were harvested, sonicated and used for recombinant virus screening.

Recombinant plaques were screened based on the plaque lift hybridization method using a *Lu. longipalpis* synthetic LJM17-specific probe which was labeled with horse radish peroxidase according to the manufacturer's protocol (Amersham Cat# RPN-3001). After three sequential rounds of plaque purification, the recombinants were generated and confirmed by hybridization as 100% positive for the synthetic LJM17 insert and 100% negative for the C3 ORF.

A single plaque was selected from the third round of plaque purification and expanded to obtain P1 (60 mm), P2 (T75 flasks), P3 (roller bottles) stocks to amplify vCP2390. The infected cell culture fluid from the roller bottles was harvested and concentrated to produce virus stock.

The construct was sequenced to confirm the sequences of the condon-optimized LJM17 insert and the C3 left and right arms around the codon-optimized LJM17 insert in vCP2390.

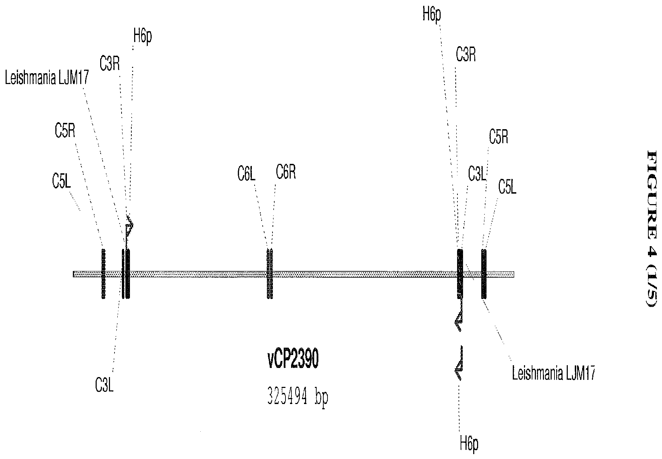

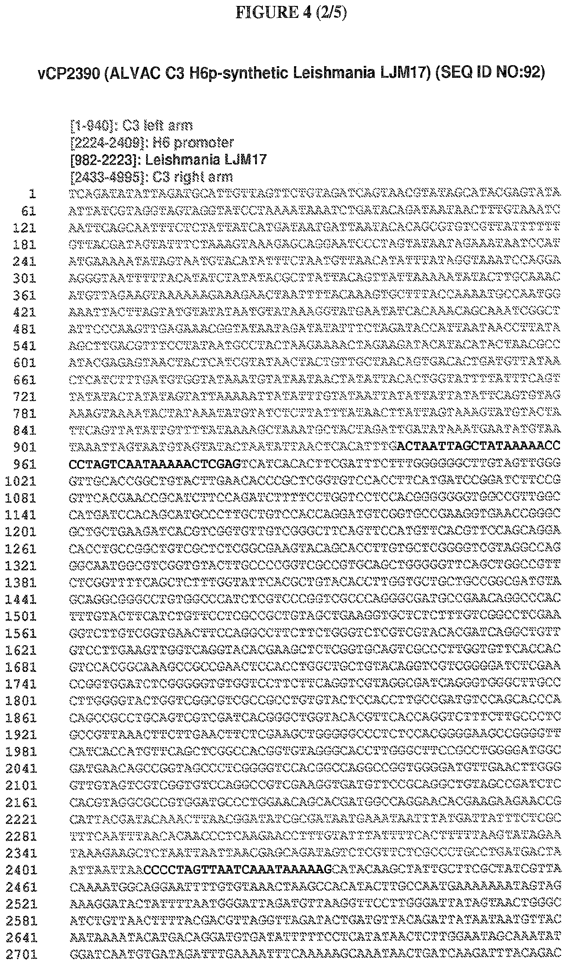

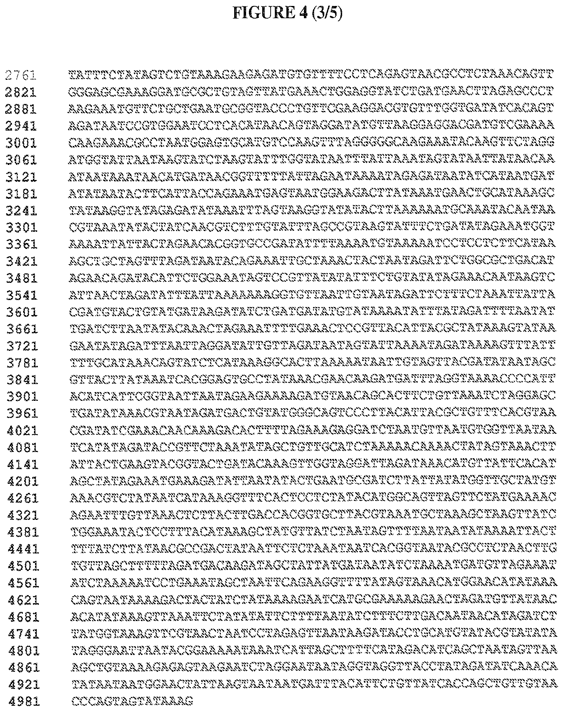

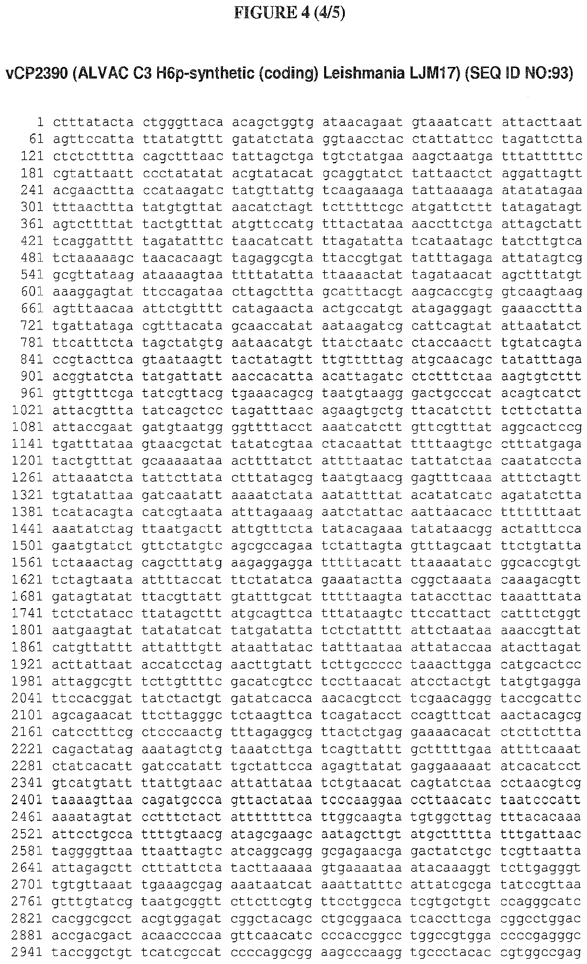

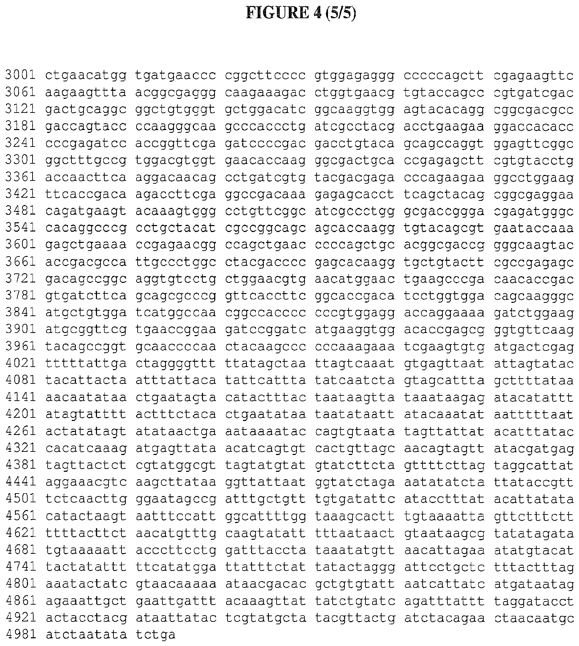

The vCP2390 vector is illustrated in Figure 4. The nucleic acid sequences for both vCP2390 strands are described in Figure 4.

### EXAMPLE 12: Construction of an ALVAC canarypox virus vector expressing the Lu. longipalpis salivary LJL143 polypeptide

The nucleic acid sequence encoding the *Lu. longipalpis* LJL143 was synthesized and has the sequence described in SEQ ID NO:2. This sequence was codon-optimized for mammalian expression by Geneart GmbH (Regensburg, Germany), resulting in the sequence described in SEQ ID NO:22, which encodes the LJL143 protein (SEQ ID NO:1).

This codon-optimized sequence was then ligated to the pALVAC C3 H6p donor plasmid resulting in pALVAC C3 H6p-LJL143 containing 5400 base pairs (Figure 5), which was sequenced and confirmed to contain the nucleic acid sequence (SEQ ID NO:22) of the LJL143 gene.

To generate vCP2389, the plasmid pALVAC C3 H6p-LJL143 plasmid was linearized with NotI restriction enzyme. The linearized fragments were individually transfected into ALVAC-infected primary CEF cells by using the calcium phosphate precipitation method (see, Panicali *et al.*; Piccini *et al.*)*.* After 24h, the transfected cells were harvested, sonicated and used for recombinant virus screening.

Recombinant plaques were screened based on the plaque lift hybridization method using a *Lu. longipalpis* synthetic LJL143-specific probe which was labeled with horse radish peroxidase according to the manufacturer's protocol (Amersham Cat# RPN-3001). After three sequential rounds of plaque purification, the recombinants were generated and confirmed by hybridization as 100% positive for the synthetic LJL143 insert and 100% negative for the C3 ORF.

A single plaque was selected from the third round of plaque purification and expanded to obtain P1 (60 mm), P2 (T75 flasks), P3 (roller bottles) stocks to amplify vCP2389. The infected cell culture fluid from the roller bottles was harvested and concentrated to produce virus stock.

After sequencing, the results showed that the sequence of the synthetic LJL143 insert and the C3 left and right arms around the synthetic LJL143 insert in vCP2389 were correct.

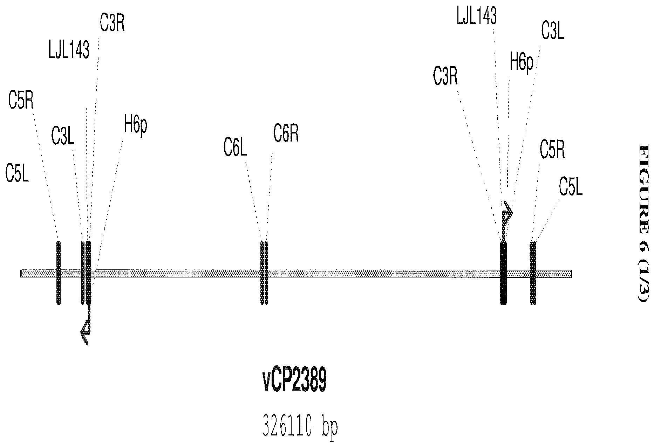

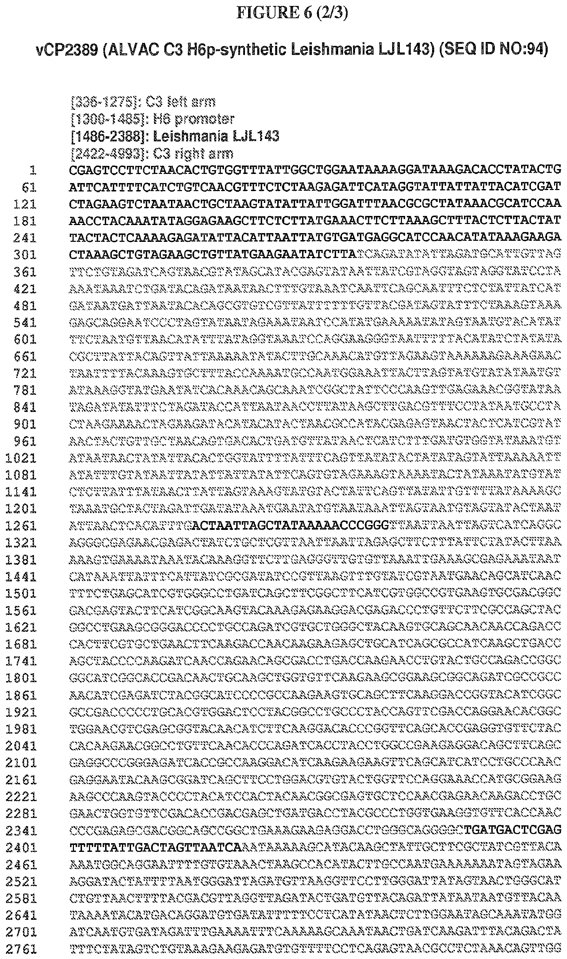

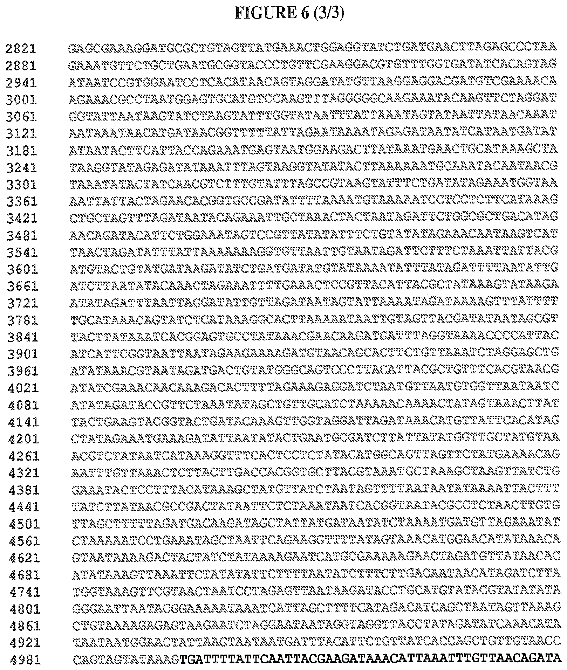

The vCP2389 vector is illustrated in Figure 6.

### EXAMPLE 13: Expression of Lu. longipalpis salivary protein in vitro

Expression plasmids containing His-tagged *Lu. longipalpis* salivary antigens encoded by cDNA derived from plasmids pNBO002 (see example 9) and pNBO003 (see example 10) were constructed and transfected into HEK-293F cells. Supernatants collected at 72 h were analyzed by HPLC chromatography using Nickel column and imidazole gradient. HPLC fractions positive for the recombinant salivary protein with 6x His motif at their C-terminus were tested with polyclonal sera of mice previously immunized with the corresponding original cDNA plasmids. Recombinant proteins were tested by SDS-PAGE and Western blotting, aliquoted in PBS and stored at -70°C.

The sub-unit vaccine comprising LJM17 is referred to herein as rLJM17. The vaccine is prepared by combining 100 µg of purified recombinant protein LJM17 with 300 µg of the adjuvant CpG #2142 in 20% Emulsigen® (MVP laboratories) (for CpG #2142, see SEQ. ID. NO: 890 in EP 1,221,955).

The sub-unit vaccine comprising LJL143 is referred to herein as rLJL143. The vaccine is prepared by combining 100 µg of purified recombinant protein LJL143 with 300 µg of the adjuvant CpG #2142 in 20% Emulsigen®.

### EXAMPLE 14: Vaccination of dogs against leishmaniasis

25 dogs (1-2 years old female beagles) were randomly divided into 5 groups of 5 dogs each. All dogs from groups 1 to 4 were vaccinated at D0 (V1) by the intradermal (ID) route into the ear pinnea using a syringe and a needle with 500 µg of purified pNBO002 plasmid (example 9) expressing LJM17 (groups 1 and 2) or pNBO003 plasmid (example 10) expressing LJL143 (groups 3 and 4) antigens, respectively.

Dogs from group 1 and group 3 were boosted at D14 (V2) and D28 (V3) with 500 µg of the same plasmids used for V1 by the transdermal (TD) route in the inner upper part of both hind legs using the VetJet™ (Merial) needle-free delivery device. Dogs from group 1 and group 3 were further boosted at D42 (V4) with 500 µg of the same plasmids by the intramuscular route coupled to electroporation (ET/IM) in the external side of both thighs using the Sphergen devise and technology (parameters: 88V, T1=20, T2=80, N=10).

Dogs from groups 2 and 4 were boosted at D14 (V2) and D28 (V3) with 500 µg of the same plasmids used for V1 by the IM route coupled to electroporation (ET/IM) as described above. Dogs from groups 2 and 4 were further boosted at D42 (V4) by the ID route into the ear pinnea as described above with sub-unit vaccines (see example 13) rLJM17 (group 2) or rLJL143 (group 4), respectively.

All dogs from groups 1 and 2 received a final vaccine booster at D192 (V5) by the IM route in the left quadriceps using 10⁸ pfu of a recombinant canarypox virus vCP2390 (example 11, having SEQ ID NO: 21 as insert) expressing LJM17. All dogs from groups 3 and 4 received a final vaccine booster at D192 (V5) by the IM route in the left quadriceps using 10⁸ pfu of a recombinant canarypox virus vCP2389 (example 12, having SEQ ID NO: 22 as insert) expressing LJL143.

Dogs from group 5 were vaccinated with 500 µg of the purified parental plasmid VR2001 which expresses no antigen at D0 by ID, D14 by TD, D28 by TD and D42 by ET/IM and received a final booster at D192 by the IM route using 10⁸ pfu of a control recombinant canarypox virus (Purevax™).

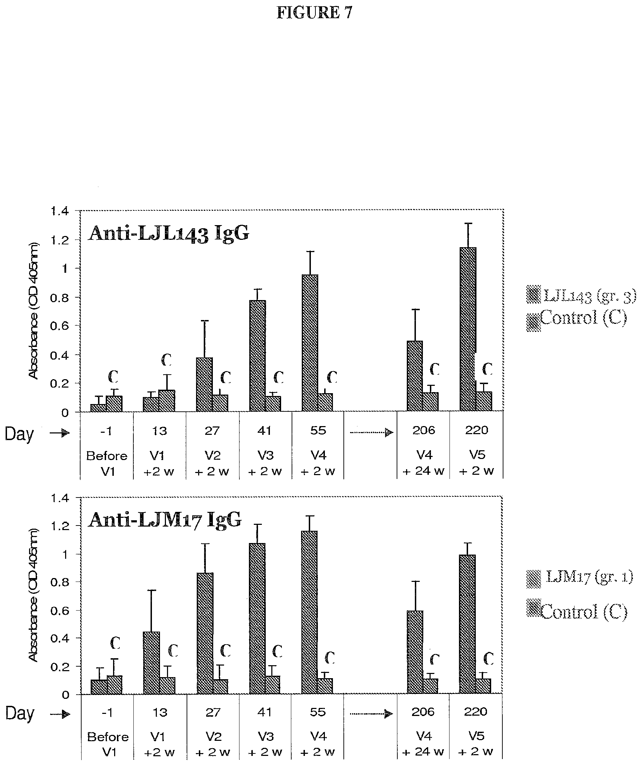

Specific and significant humoral immunity to both LJM17 and LJL143 was evidenced by ELISA in vaccinated dogs (see Figure 7). 96-well plates (Maxisorp™, Nunc) were coated overnight at 4° C with rLJM17 protein (2 µg/mL) or rLJL143 (2 µg/mL). Dog serum was successively added to the plates in triplicate at a dilution of 1/50 with alkaline phosphatase-conjugated AffiniPure rabbit anti-dog IgG (Jackson ImmunoResearch) at 1/5000 and p-nitrophenylphosphate (Sigma). Absorbance was measured at 405 nm using a Spectramax Plus (Molecular Devices).

Significant antibody titers persisted up to 6 months after V4 administration. The vCP booster (V5) recalled specific antibody responses in vaccinated dogs efficiently. Post vCP anamnestic responses established the expression of LJL143 and LJM17 from vCP2389 and vCP2390 respectively, and the ability of the vectors to boost humoral immune responses *in vivo.*

PBMC from dogs taken 2 weeks after the V5 administration were stimulated by 2 pairs of salivary gland homogenate (SGH), LJL143 (4 µg), and LJM17 (4 µg), or by ConA (4 µg). PBMCs that were non-stimulated by medium (med) served as controls. IFN-gamma production was evaluated by measuring the levels of IFN-gamma secretion in the medium at 72 hours (Quantikine ELISA; R&D Systems).

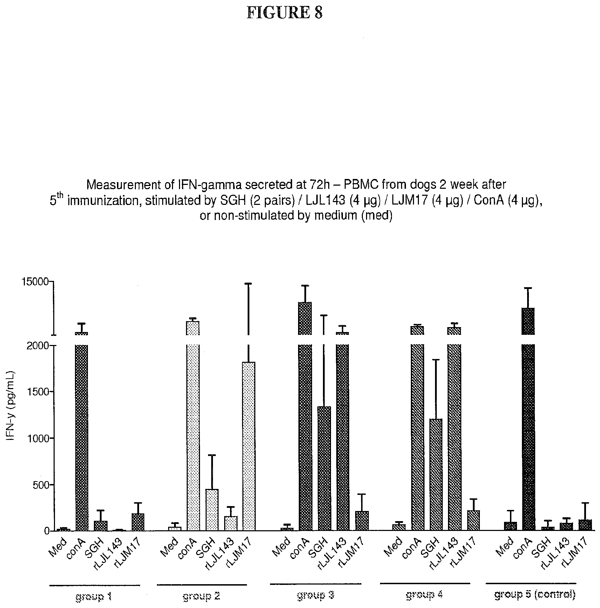

The results are illustrated in Figure 8. Addition of purified recombinant LJM17 or LJL143 proteins caused PBMCs from LJM17- or LJL143-vaccinated dogs to increase secretion of IFN-gamma. No increased secretion of IFN-gamma was evidenced when PBMCs were in the presence of control medium (med).

The addition of ConA or SGH to PBMCs from LJM17- or LJL143-vaccinated dogs also caused an increased secretion of IFN-gamma. Notably, the LJM17 antigen caused very strong INF-gamma responses in animals of group 2 (1800 pg/mL), and also caused a strong response in animals of group 1 (200 pg/mL). The LJL143 antigen also caused very strong INF-gamma responses in animals of groups 3 and 4, with more than 2000 pg/mL, which were comparable to results seen when PBMCs were stimulated by ConA.

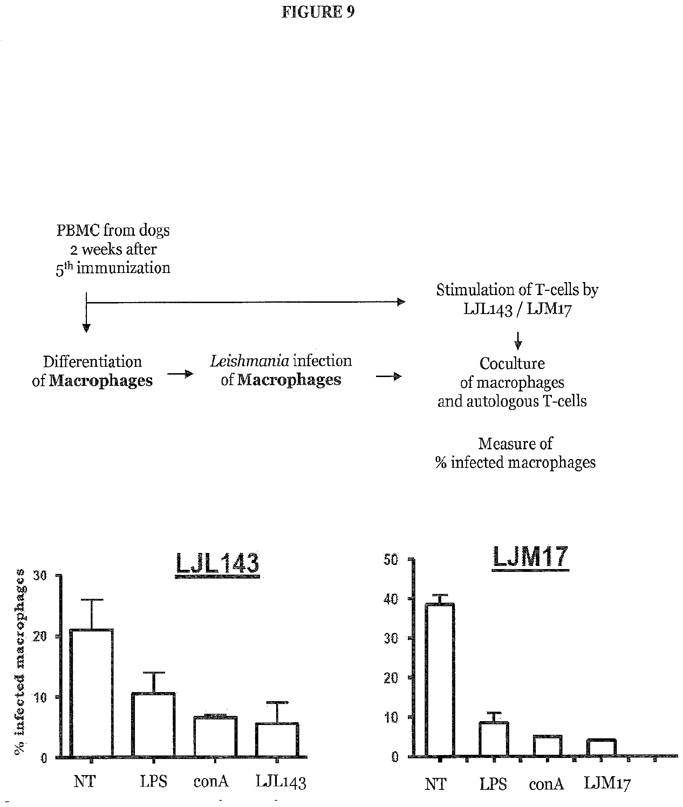

2 weeks after the V5 administration, PBMCs were isolated from two dogs that had been vaccinated with LJM17 and LJL143 . Autologous T cells lymphocytes (5.10⁶ cells) were stimulated with either 25 µg of recombinant LJM17, 25 µg of recombinant LJL143, or 4 µg of ConA. Cells were then incubated in the presence of macrophages infected by *Leishmania chagasi* amastigotes (infected at a 5:1 ratio). Lipopolysaccharides (LPS) and non-stimulated T cells (NT, no treatment) served as controls. Cells were evaluated for efficiency of killing which was measured by a significant reduction in the percent of infected macrophages (Figure 9).

Lymphocytes stimulated with recombinant LJM17 or recombinant LJL143 were as cytototic to macrophages as lymphocytes that had been stimulated by the non-specific mitogen ConA.

LJL143- and LJM17-vaccinated and control dogs were fitted with a Velcro collar device. The Velcro collar device was prepared by disposing twenty uninfected 6 day old female *Lu. longipalpis* were in a 10 mm-thin Plexiglas device which contained a secured screw and a nylon mesh on one of its sides so that sand flies were able to probe through the mesh. The device was kept at 25° C before exposure to limit condensation and was then firmly attached to a Velcro collar for 10 minutes on the shaved belly of LJL143- and LJM17-vaccinated and control dogs. Dogs were unrestrained throughout the time of exposure. 4 mm or 6 mm-skin punches biopsies (Acuderm) were taken from the belly of anesthetized dogs 48 hours after the sand fly bites.

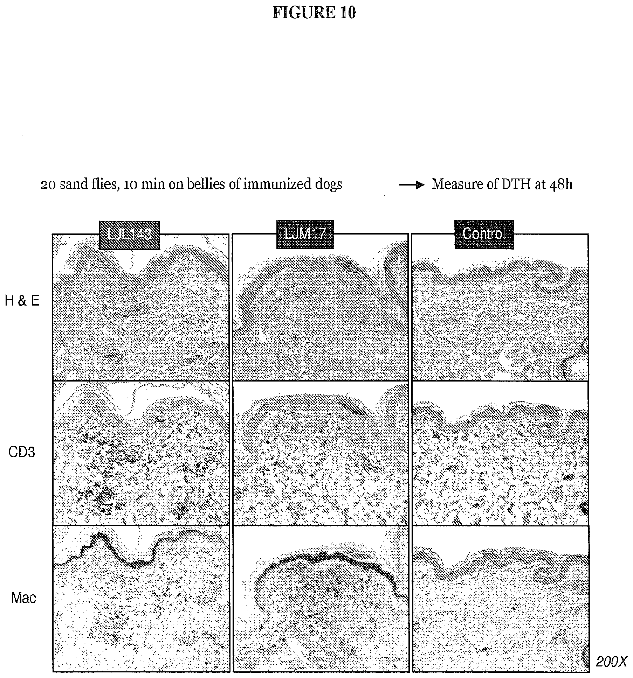

Biopsies were stored in neutral-buffered formaldehyde (10% formalin) and were then routinely processed for staining with hematoxylin/eosin (H & E), Luna's, Toludine blue and immunohistochemical procedures for CD3 and macrophage/monocyte markers (Mac). Figure 10 provides the immunohistochemical results of sand fly bite sites. Specific immune cellular infiltration (darker spots on the pictures) is observed on vaccinated dogs whereas no infiltration was observed on control dogs.

### EXAMPLE 15: Vaccination of dogs with LJL143 and LJM17 salivary proteins produce a protective immune response that killed Leishmania chagasi amastigotes in vitro.

In this study, *Lu. longipalpis* salivary proteins were tested to determine if they are immunogenic in dogs. In addition, *Lu. longipalpis* salivary proteins were identified that can produce a protective cellular immune response and kill *Leishmania infantum* in an *in vitro* assay.

To identify these salivary components, an *in vivo* high-throughput DNA-based reverse antigen-screening assay was developed. This screening identified two strong DTH-inducing antigens out of the 35 most abundant proteins in the salivary glands *of Lu. longipalpis.* These data were further validated with recombinant proteins, resulting in the confirmation of DTH-inducing potential for the *Lu. longipalpis* salivary proteins LJM17 (SEQ ID NO:5) and LJL143 (SEQ ID NO: 1). Furthermore, peripheral blood mononuclear cells of dogs vaccinated with LJL143 and LJM17 produced interferon (IFN)-γ upon stimulation with the salivary recombinant proteins and, more importantly, stimulation of these cells with the recombinant proteins resulted in the killing of *Leishmania infantum in vitro.* These molecules represent therefore strong candidates to be used as a vaccine to control *Leishmania infantum* in dogs.

### Material and Methods

*Dogs:* 1-2 year old female beagles (Marshall Farms) were housed at the animal facility of NIH, Bethesda, USA, following the Animal Care and User Committee guidelines. They were well-fed animals under constant scrutiny for health problems by a veterinarian and had all received routine vaccinations. No ectoparasitic treatments were administrated during the last four months before sand fly exposure experiments.

*Sand flies*: *Lutzomyia longipalpis,* Jacobina strain, were reared using as larval food a mixture of fermented rabbit feces and rabbit food. Adult sand flies were offered a cotton swab containing 20% sucrose but were starved from sugar 24 hours before exposure experiments. Some females were used for dissection of salivary glands at 4-7 days following emergence and salivary gland homogenates (SGH) were prepared as described above.

*Exposure to sand fly bites:* Six day old female *Lu. longipalpis* were placed in a 10 mm-thin Plexiglas device. The device contained a secured screw and a nylon mesh on one of its sides for sand fly probing through it. The device was kept at 25°C before exposure to limit condensation and was firmly attached with a Velcro collar on the shaved neck of dogs for 20 minutes. Dogs were unrestrained throughout the time of exposure.

*Reverse Antigen-screening (RAS)*: Dogs pre-exposed to sand fly bites were anesthetized and injected intradermally with DNA plasmids or recombinant proteins. Injection sites were separated from each other by 15mm. For DNA plasmids, the molecules were coded and randomly regrouped for each dog prior to injection on the belly. The code was broken only after induration and erythema measurements were completed. For DNA-RAS, 38 samples were injected in a total volume of 40 µL, including PBS, 1 pair of *Lu. longipalpis* SGH diluted in PBS, and 20 µg of control vector and the 35 recombinant DNA plasmids, diluted in PBS, encoding *Lu. longipalpis* individual salivary proteins. For protein-RAS, 5 samples were injected in duplicates (40 µL) including PBS, 1 pair of *Lu. longipalpis* SGH diluted in PBS, and 3 *Lu. longipalpis* salivary recombinant proteins (300 ng). Measure of induration and erythema diameters were performed 48 hours after intradermal injection of the samples.

*Histology and real-time PCR on skin punch biopsies:* 4mm or 6mm-skin punches biopsies (Acuderm) were taken from the neck or belly of anesthetized dogs and split into two equal halves. One half was stored in neutral-buffered formaldehyde (10% formalin) then routinely processed for staining with hematoxylin/eosin, Luna's, Toludine blue and immunohistochemical procedures for CD3 and macrophage/monocyte markers. The other half, stored in RNAlater (Sigma), was used for RNA extraction (Agencourt® RNAdvance™ Tissue, Beckman Coulter). RNA were reverse-transcribed (Transcriptor first strand cDNA synthesis, Roche) and used for Real-time PCR using the LightCycler 480 (Roche), primer set (0.2µM final concentration) and FAM/TAMRA dual-labeled probes to a total of 15 µl per reaction in triplicates. Primers and probes for canine IL4, IL12, TGF-β, IFN-γ and GAPDH were described previously (Breathnach *et al.*). Amplification conditions, acquisition, melting curve analysis and standard curve were performed as described previously (Breathnach *et al.*)*.* Expression levels of the interested gene were normalized to endogenous GAPDH levels to control for RNA quantity.

*Recombinant cDNA:* From a cDNA library (see above), the 35 most abundant molecules from *Lu. longipalpis* salivary glands were selected and their cDNA cloned in the pVR2001-TOPO vector by standard cloning techniques. Plasmids were prepared using GenElute™ endotoxin-Free Plasmid Megaprep (Sigma), cleaned with ultrapure water using Centricon® Plus-20 (Millipore) and eluted in PBS. Quality control of the 35 purified plasmids included endotoxin measurements, restriction profile analyses and sequencing. Purified salivary DNA plasmids were sterilely filtered and stored at -70°C.

*Recombinant proteins:* Expression plasmids containing His-tagged *Lu. longipalpis* salivary antigens encoding cDNA derived from the parental salivary DNA plasmids were constructed as described (Oliveira et al.) and transfected in HEK-293F cells. Supernatants collected at 72 h were submitted to HPLC chromatography using Nickel column and imidazole gradient. HPLC fractions positive for the recombinant salivary protein with 6x His motif at their C-terminus were tested with polyclonal sera of mice previously immunized with the corresponding original cDNA plasmids. Recombinant proteins were tested by SDS-PAGE and Western blotting, aliquoted in PBS and stored at -70°C.

*Recombinant canarypox viruses*: Two canarypox viruses derived from ALVAC vectors expressing respectively the LJL143 (vCP2389) or LJM17 (vCP2390) antigens were generated using standard methods and validated by sequencing of viral DNA, RT-PCR on mRNA of infected cells and Western Blotting of supernatants of infected cells. Purevax™ ferret distemper vaccine (Merial) was used as control of canarypox virus injections.

*Immunization of dogs with salivary vaccines:* 25 dogs were randomly divided in 5 groups of 5 dogs each. All dogs from groups 1 to 4 were vaccinated at D0 (V1) by the intradermal (ID) route into the ear pinnea using a syringe and a needle with 500µg of purified plasmid expressing LJM17 (groups 1 and 2) or LJL143 (groups 3 and 4) antigens, respectively. Dogs from group 1 and group 3 were boosted at D14 (V2) and D28 (V3) with 500µg of the same plasmids used for V1 by the transdermal (TD) route in the inner upper part of both hind legs using the VetJet™ (Merial) needle-free delivery device. Dogs from group 1 and group 3 were further boosted at D42 (V4) with 500µg of the same plasmids by the intramuscular (IM) route coupled to electroporation in the external side of both thighs using the Sphergen devise and technology (parameters: 88V, T1=20, T2=80, N=10). Dogs from groups 2 and 4 were boosted at D14 (V2) and D28 (V3) with 500µg of the same plasmids used for V1 by the IM route coupled to electroporation as described above. Dogs from groups 2 and 4 were further boosted at D42 (V4) with 100µg of aforementioned purified recombinant protein LJM17 (rLJM17) (group 2) or LJL143 (rLJL143) (group 4) in association with 300 µg CpG ODN in 20% Emulsigen® (MVP laboratories) by the ID route into the ear pinnea as described above. All dogs from groups 1 to 4 received a final vaccine booster at D192 (V5) by the IM route in the left quadriceps using 10⁸ pfu of a recombinant canarypoxvirus vCP2390 and vCP2389 expressing respectively LJM17 (groups 1 and 2) or LJL143 (groups 3 and 4) antigens. Dogs from group 5 were vaccinated at D0, D14, D28 and D42 with 500µg of the purified parental plasmid VR2001 which expresses no antigen and received a final booster at D192 by the IM route using 10⁸ pfu of a control recombinant canarypoxvirus (Purevax™).

*ELISA:* 96-well plates (Maxisorp™, Nunc) were coated overnight at 4 °C with *Lu. longipalpis* SGH (5 pairs/ml), rLJM17 protein (2µg/mL) or rLJL143 (2µg/mL). Dog serum in triplicate at 1/50 dilution, alkaline phosphatase-conjugated AffiniPure rabbit anti-dog IgG (Jackson ImmunoResearch) at 1/5000 and p-nitrophenylphosphate (Sigma) were successively added to the plates. 405nm absorbance was measured using a Spectramax Plus (Molecular Devices). IFN-γ production in cell supernatants was measured after 72 h (Quantikine ELISA; R&D Systems) after stimulation with SGH (1 or 2 pairs), conA (4 µg), rLJM17 (2 or 10 µg) or rLJL143 (2 or 10 µg).

*Anti-leishmanial activity:* Canine monocyte-derived macrophages were prepared as using standard methods. Autologous T cells (5x10⁶ cells) were taken from the culture after 1 week, stimulated with *Lu. longipalpis* SGH (2 pairs), conA (4 µg), rLJM17 (25 µg) or rLJL143 (25 µg), and put back in presence of macrophages infected by *L. infantum* infected at a 5:1 ratio. Anti-leishmanial activity was assessed by changes in the percentages of infected cells and number of amastigotes per macrophage after microscopic examination of Giemsa-stained preparations.

### Bites of Lutzomyia longipalpis sand flies induce a strong delayed type hypersensitivity response in dogs

In rodent models, cellular immunity characterized by a Th1 delayed type hypersensitivity (DTH) response to sand fly salivary proteins, protect animals from cutaneous and visceral leishmaniasis. There is no information pertaining to the presence and nature of cellular immunity to sand fly saliva in dogs, the main reservoirs of visceral leishmaniasis caused by *Leishmania infantum* (*chagasi*) in Europe and Latin America. Thus, the early kinetics of anti-saliva immunity in dogs following exposure to bites of *Lutzomyia longipalpis,* the vector of *Leishmania infantum chagasi* in Latin America, was investigated. Seven of nine beagles showed specific anti-saliva antibodies one week after the third exposure to bites (FIG. 19A). Apart from a single dog, these animals showed a strong IgG2 antibody response in the absence of IgG1 (FIG. 19A). One dog showed a mixed IgG2/IgG1 antibody response. To investigate whether dogs exposed to sand fly bites develop a DTH response, the skin induration at the bite site up to 96 hours following each exposure was measured. Following the second exposure to sand fly bites, a small induration was observed in the 7 dogs that produced significant levels of *Lu. longipalpis* IgG antibodies (FIG. 19B). This was characterized by a localized erythema, swelling and eventually thickening of the skin. The intensity and duration of the observed induration was significantly increased following the third exposure lasting up to 96 hours following sand fly bites (FIG. 19B). This induration was not observed after the first exposure in naive animals (FIG. 19B). Histological analyses of the induration site show minimal inflammation characterized by scattered perivascular lymphocytes and rare neutrophils within the superficial dermis 48 hours following the first and second exposure (FIG. 19C). A dramatic increase in the cellular infiltrate was noted 48 hours following the third exposure. This was characterized by a prominent thickening of the epidermis and the presence of multifocal infiltrates of inflammatory cells consisting of lymphocytes, macrophages and eosinophils (FIG. 19D). Based on the timing of the reaction as well as the nature of the infiltrate, it was concluded that sand fly saliva induces a delayed-type hypersensitivity reaction in the skin of dogs after repeated exposures.

### Lutzomyia longipalpis salivary proteins that induce a DTH in dogs

Salivary molecules of *Lu. longipalpis* which are responsible for the generation of a DTH response in dogs was investigated. Transcripts coding for the 35 most abundant secreted proteins from the salivary glands of this species were identified above. Identifying candidates capable of inducing a cellular immune response from a large pool of antigens in large animals such as dogs is prohibitive in terms of cost and space. To overcome this obstacle, a reverse antigen screening approach was developed that consisted of exposing a minimal number of dogs (five) to sand fly bites and injecting each animal with up to 38 samples (35 DNA plasmids encoding for salivary proteins and three controls) (FIG. 20A). Out of the 35 injected DNA plasmids only 4 (LJM17, LJM11, LJL143 and LJL138) induced a significant erythema in more than 3 dogs, 48 hours after challenge (FIG. 20B) and only 2 DNA plasmids (LJL143 and LJM17) produced a strong induration in 3 dogs, 48 hours after challenge (FIG. 20B). The majority of the injected plasmids did not produce a significant erythema or induration (FIG. 20B) and the injection of PBS and empty vector control induced minimal erythema at the site of injection compared to LJM17 and LJL143 (FIG. 20C). The specificity and reactivity of injected DNA plasmids is shown in FIG. 20D. Based upon these results LJM17 and LJL143 were chosen for further analysis. LJL143 and LJM17 induced the production of cytokines indicative of a Th1 environment at the injection site, including IL-12 and IFN-γ (FIG. 20E). Histological sections taken 48 hours after challenge show that LJL143 and LJM17 recruit lymphocytes and macrophages at the site of injection indicative of a classical delayed type hypersensitivity (DTH) response (FIG. 20F). To validate the specificity of this approach soluble highly purified LJL143, LJM17 and LJM111, among several other salivary recombinant proteins, was prepared (FIG. 21A). LJL143 and LJM17 recombinant proteins reproduced the DTH response observed upon injection of their respective DNA plasmids (FIG. 21B and 21C) including the recruitment of lymphocytes and macrophages to the site of injection (FIG. 21D).

### Dogs immunized with LJL143 and LJM17 produce IFN-γ specific to recombinant salivary proteins.

Dogs (5 per group) were immunized with DNA plasmids coding for LJL143, LJM17 or control DNA plasmid, a prime boost with the respective recombinant salivary proteins and a final immunization with canarypox virus expressing the respective salivary proteins (Table 1). PBMCs from immunized dogs were stimulated with up to 4 ug of their respective recombinant protein, ConA and 1 pair of salivary gland homogenate (SGH). LJL143 vaccinated dogs produced significant levels of IFN-γ five weeks post-fourth vaccination and before canarypox injection (FIG. 22A). More importantly, the native LJL143 protein present in 1 pair of salivary gland homogenate was able to generate a similar response in LJL143 vaccinated dogs (FIG. 22A). LJM17 vaccinated dogs produced a considerable lower levels of IFN-γ compared to LJL143 vaccinated dogs (FIG. 22A). A similar profile was observed two weeks after canarypox vaccination, where PBMCs from dogs vaccinated with LJL143 produced two fold higher IFN-γ compared to their pre-pox status (FIG. 22B).

**Table 1**

| Group | Antigen | D0 (V1) | D14 (V2) | D28 (V3) | D42 (V4) | D192 (V5) |
|---|---|---|---|---|---|---|
| 1 | LJM17 | ID-cDNA | TD-cDNA | TD-cDNA | IM/ET-cDNA | IM-vCP |
| 2 | LJM17 | ID-cDNA | IM/ET-cDNA | IM/ET -cDNA | ID-protein | IM-vCP |
| 3 | LJL143 | ID-cDNA | TD-cDNA | TD-cDNA | IM/ET-cDNA | IM-vCP |
| 4 | LJL143 | ID-cDNA | IM/ET-cDNA | IM/ET-cDNA | ID-protein | IM-vCP |
| 5 | Control | ID-cDNA | TD-cDNA | TD-cDNA | IM/ET-cDNA | IM-vCP |

### Vaccination with LJL143 and LJM17 generates a protective immune response that kills Leishmania chagasi amastigotes in vitro.

Infected macrophages from PBMCs of two dogs vaccinated with LJM17 and LJL143 efficiently killed *Leishmania chagasi* amastigotes following the addition of autologous lymphocytes stimulated with LJM17 and LJL143 recombinant proteins respectively (FIG. 23). The efficiency of killing was measured by a significant reduction in the percent of infected macrophages (FIG. 23A) as well as in the number of amastigotes per macrophages (FIG. 23B). This killing effect was comparable to that observed upon the addition of the non-specific mitogen ConA (FIG. 23).

### EXAMPLE 16: Production of an Immune Response in Dogs

Twelve dogs approximately three years old with natural immunity against Leishmaniasis are injected via an intradermal route (ID) in the back after shaving, with 100 µg of each individual plasmid suspended in 100 µl of PBS. Each plasmid is injected at a different point. The points are separated by at least 3 cm to avoid interference between DTH responses. The negative control (100 µl of buffer) is also inoculated by ID route.

The DTH response is assessed 72 hours after injection by measuring the larger diameter of the skin tumefaction area. The results are expressed as the mean value of the tumefaction area for all the dogs and as a percentage of dogs having a positive DTH response. A positive DTH is a tumefaction area diameter greater than or equal to 4 mm at 72 hours after injection.

In a second study, 10 naive dogs 4 to 6 months old are immunized by ID injection in 10 points (100 µl per point) in the right ear with a pool of the plasmids encoding a *Lu. longipalpis* polypeptide, 100 µg for each one suspended in 1000 µl of PBS. On day 21, dogs are injected in 10 points (100 µl per point) in the left ear and in 10 points (100 µl per point) in the belly with a pool of the plasmids, 100 µg for each one suspended in 2000 µl of PBS. All dogs are challenged on day 35 by inoculation by ID route in the back (after shaving), with 100 µg of each individual plasmid suspended in 100 µl of PBS. Each plasmid is injected at a different point. The points are separated by at least 3 cm to avoid interference. As a negative control, 100 µl of buffer is inoculated intradermally. The DTH response is assessed 72 hours after challenge, by measuring the larger diameter of the skin tumefaction area. The results are expressed as the mean value of the tumefaction area for all the dogs and as a percentage of dogs having a positive DTH response. A positive DTH is a tumefaction area diameter higher or equal of 4 mm at 72 hours after injection.

The results of this study show that plasmids can induce a cellular immunity in dogs after injection, a cellular immunity reveled by a DTH response. The variation of the DTH response level can be by the variation of the expression of the insert.

### References

1. Adler and Theodor, Ann. Trop. Med. Parasitol. 20:109, 1926
2. Altenburger et al., 1989, Arch. Virol. 105, 15-27
3. Altschul et al. J. Mol. Biol. 1990. 215. 403-410
4. Altschul et al., Nucl. Acids Res. 25, 3389-3402
5. Antoine G., Virology, 1998, 244, 365-396
6. Bairoch, Nucleic Acids Res. 19 (Suppl.):2241,1991
7. Barral et al., Am J Trop Med Hyg 62:740-5, 200
8. Behr J. P., Bioconjugate Chemistry, 1994: 5: 382-389
9. Berberich C. et al., Biochim. Biophys. Acta, 1998, 1442: 230-7
10. Boshart M. et al., Cell, 1985, 41, 521-530
11. Boshart M. et al., Cen 41:521-530, 1985
12. Breathnach et al., Vet Dermatol 2006, 17:313-21
13. Carroll M. W. et al., Vaccine, 1997, 15 (4), 387-394
14. Charlab et al., Proc Natl Acad Sci USA 96:15155-60, 1999
15. Chaudhuri P Res. Vet. Sci. 2001, 70(3), 255-6
16. Chung J Y et al., FEMS Microbiol letters 1998, 166: 289-296
17. Cochran et al., J. Virology, 1985, 54, 30-35
18. D. Berg. et al Biochem. Biophys. Res. Commun. 1991, 179, 1289-1296
19. De Groot A. et al., Nature Biotechnology, 1999, 17, 533-561
20. Desjeux P., Trans. R. Soc. Trop. Med. Hyg., 2001, 95: 239-43
21. Devereux J, Haeberlie P and Smithies O, "A comprehensive set of sequence analysis program for the VAX," Nucl. Acids Res., 12: 387-395 (1984)
22. Dietze R. et al., Clin. Infect. Dis., 1997, 25: 1240-2
23. Djoba Siawaya JF et al., PLoS ONE, 2008, 3(7), e2535
24. Doree S M et al., J. Bacteriol. 2001, 183(6): 1983-9
25. Dye C., Am. J. Trop. Med. Hyg., 1996, 55: 125-30
26. Feng DF and Dolittle RF, "Progressive sequence alignment as a prerequisite to correct phylogenetic trees," J. of Molec. Evol., 25:351-360 (1987)
27. Fingerut E et al., Vaccine, 2005, 23(38): 4685-4696
28. Funahashi et al., J. Gen. Virol., 1988, 69, 35-47
29. Geysen H. M. et al., Proc. Nat. Acad. Sci. USA, 1984, 81 (13), 3998-4002
30. Geysen H. M. et al., Proc. Nat. Acad. Sci. USA, 1985, 82 (1), 178-182
31. Geysen H. M., Southeast Asian J. Trop. Med. Public Health, 1990, 21 (4), 523-533
32. Gradoni L. et al., Vaccine, 2005, 23: 5245-51
33. Grosjean NL et al., Lindsay DS et al., McConkey SE et al., Martinez-Subiela S, Tecles F, Eckersall PD, Cerón JJ: Serum concentrations of acute phase proteins in dogs with leishmaniasis. Vet Rec 150:241-244, 2002
34. Grosjean NL, Vrable RA, Murphy AJ, Mansfield LS: Seroprevalence of antibodies against Leishmania spp among dogs in the United States. J Am Vet Med Assoc 222:603-606, 2003
35. Guo P. et al. J. Virol., 1989, 63, 4189-4198
36. Hartikka J. et al., Human Gene Therapy, 1996, 7, 1205-1217
37. Hemmer B. et al., Immunology Today, 1998, 19 (4), 163-168
38. Henikoff et al., Bioinformatics 15:471, 1999
39. Higgins DG and Sharp PM, "Fast and sensitive multiple sequence alignment on a microcomputer," CABIOS, 5: 151-153 (1989)
40. Hoop T. et al., Mol. Immunol. 1983, 20(4), 483-489
41. Immonogenicity of a killed Leishmania vaccine with Saponin adjuvant in dogs", R. Cordeiro Giunchetti et al., Vaccine, 2007, 25: 7674-7686
42. Israeli E et al., Cryobiology 1993, 30(5): 519-23
43. J. Fields et al., Nature, 186: 778-780, 4 June 1960
44. J. Mol. Biol. 48:444-453 (1970)
45. J. Sambrook et al. (Molecular Cloning: A Laboratory Manual, 2nd Edition, Cold Spring Harbor Laboratory, Cold Spring Harbor, New York, 1989
46. Jardim A. et al., Biochem. J., 1995, 305: 307-13
47. Jardim A. et al., Biochem. J., 1995, 305: 315-20
48. Jeanmougin et al., Trends Biochem. Sci. 23:403, 1998
49. Jurk M et al., Immunobiology 2004, 209(1-2): 141-154
50. K. Otte et al. Gen. Comp. Endocrinol. 1996, 102(1), 11-15
51. Kidd I. M. & Emery V.C., "The use of baculoviruses as expression; vectors," Applied Biochemistry and Biotechnology 42:37-159, 1993
52. Kwissa M. et al., Vaccine, 2000, 18, 2337-2344
53. Lanotte G. et al., Ann. Parasitol. Hum. Comp., 1979, 54: 277-95
54. Lindsay DS, Zajac AM, Barr SC: Leishmaniasis in American Foxhounds: An Emerging Zoonosis? Compend Cont Educ Pract Vet 24:304-312, 2002
55. Luke C. et al., Journal of Infectious Diseases, 1997, 175, 91-97
56. Muller M. et al. Nucleic Acids Res. 1990, 18(2), 364
57. Maniatis et al., Molecular Cloning: a Laboratory Manuel, Cold Spring Harbor Laboratory, 1982
58. Maroli M. et al., Med. Vet. Entomol., 2001, 15: 358-63
59. Martínez-Subiela S, Tecles F, Eckersall PD, Cerón JJ: Serum concentrations of acute phase proteins in dogs with leishmaniasis. Vet Rec 150:241-244, 2002
60. Mazloumi Gavgani A.S. et al., Lancet, 2002, 360: 374-9
61. McConkey SE, López A, Shaw D, Calder J: Leishmanial polyarthritis in a dog. Canine Vet J 43:607-609, 2002
62. Melanby, Nature. 158, 554-555.13, 1946
63. Meyer et al., 1991, J. Gen. Virol. 72, 1031-1038
64. Mills C K et al., Cryobiology 1988, 25(2): 148-52
65. Miyazaki J. et al., Gene, 1989, 79, 269-277
66. Molina R. et al., Trans. R. Soc. Trop. Med. Hyg., 1994, 88: 491-3; Courtenay O. et al., J. Infect. Dis., 2002, 186: 1314-20
67. Montgomery et al., Cell. Mol. Biol. 43:285-292, 1997
68. Moreira Jr. E.D. et al., Vet. Parasitol., 2004, 122: 245-52
69. Nielsen et al., Protein Eng. 10:1, 1997
70. Ogata R T et al., J. Biol. Chem. 1989, 264(28): 16565-16572
71. Oliveira F. et al. Vaccine (2006) 24: 374-90
72. Olsen C W et al., Vaccine, 1997, 15(10): 1149-1156
73. Optimal Alignments in Linear Space", CABIOS 4, 11-17, 1988
74. O'Reilly et al., "Baculovirus expression vectors, A laboratory manual," New York Oxford, Oxford University Press, 1994
75. P. Delafontaine et al. Gene 1993, 130, 305-306
76. Pandher K et al., Infect. Imm. 1998, 66(12): 5613-9
77. Panicali et al., Proc. Natl Acad Sci USA, 1982, 79: 4927-4931
78. Parker K. et al., Immunol. Res. 1995, 14(1), 34-57
79. Piccini et al., Methods Enzymol., 1987, 153: 545-563
80. Pasleau et al., Gene 38:227-232, 1985
81. Pennock et al, Mol. Cell Biol. 4: 399- 406, 1994
82. Peppoloni S et al., Expert Rev Vaccines, 2003, 2(2): 285-293
83. Perkus M. et al., J. Virol., 1989, 63, 3829-3836
84. Phameuropa Vol. 8, No. 2, June 1996
85. Pharmaceutical Biotechnology, 1995, volume 6, edited by Michael F. Powell and Mark J. Newman, Plenum Press, New York and London
86. Piccini et al., Methods Enzymol., 1987, 153: 545-563
87. Ribeiro et al., Insect Biochem. 19:409-412, 1989
88. Rickles R. et al J. Biol. Chem. 1988, 263, 1563-1569
89. Riviere et al., J. Virology, 1992, 66, 3424-3434
90. S. Friezner Degen et al J. Biol. Chem. 1996, 261, 6972-6985
91. S. Lien et al. Mamm. Genome 2000, 11(10), 877-882
92. Shida, Virology, 1986, 150, 451-457
93. Slappendel RJ, Ferrer L. In: Greene CE: Infectious Diseases of the Dog and Cat. WB Saunders Co, Philadelphia, 1998, pp. 450-458
94. Smith et al., Mol. Cell Biol. 3:2156-2165, 1983
95. Smith TF and Waterman MS, "Comparison of Bio-sequences," Advances in Applied Mathematics 2:482-489 (1981)
96. Smith TF, Waterman MS and Sadler JR, "Statistical characterization of nucleic acid sequence functional domains," Nucleic Acids Res., 11:2205-2220 (1983)
97. Soares et al., J. Immunol. 160:1811-6, 1998
98. Staib C. et al., Biotechniques, 2000, 28(6): 1137-42, 1144-6, 1148
99. Stickl & Hochstein-Mintzel, Munch. Med. Wschr., 1971, 113, 1149-1153
100. Stittelaar K. J. et al., J. Virol., 2000, 74 (9), 4236-4243;
101. Sutter et al., Proc. Natl. Acad. Sci. U.S.A., 1992, 89, 10847-10851
102. Sutter G. et al., 1994, Vaccine, 12 (11), 1032-1040
103. Taylor et al. Vaccine. 6: 497-503, 1988a
104. Taylor et al. Vaccine. 6: 504-508, 1988b
105. Taylor J. et al., Vaccine, 1988, 6, 504-508
106. Thompson JD, Higgins DG and Gibson TJ, "ClusterW: improving the sensitivity of progressive multiple sequence alignment through sequence weighing, positions-specific gap penalties and weight matrix choice," Nucleic Acid Res., 22:4673-480, 1994
107. Titus and Ribeiro, Parasitol Today 6:157-159, 1990
108. Tsvetkov T et al., Cryobiology 1983, 20(3): 318-23
109. Vaccine Design, The subunit and adjuvant approach", Pharmaceutical Biotechnology, vol. 6, Edited by Michael F. Powell and Mark J. Newman, 1995, Plenum Press New York
110. Valenzuela et al., J. Exp. Med. 194:331-42, 2001
111. Van der Zee R. et al., Eur. J. Immunol., 1989, 19 (1), 43-47
112. van Ooyen et al., Science, 1979, 206, 337-344
113. Verne A., Virology- 167:56 71, 1988
114. Vialard et al., J. Virol. 64:37-50, 1990
115. VICAL Inc.; Luke C. et al., Journal of Infectious Diseases, 1997, 175, 91-97
116. Von Heijne (1986), Nucleic Acid Research, 14; 4683-4691
117. Ward C K et al., Infect. Imm. 1998, 66(7): 3326-36
118. Wilbur and Lipman, 1983 PNAS USA 80:726
119. Wolff E et al., Cryobiology 1990, 27(5): 569-75
120. Y. Kajimoto et al. Mol. Endocrinol. 1989, 3(12), 1907-1913
121. Zurbriggen R et al., Expert Rev Vaccines, 2003, 2(2): 295-304

## Claims

1. A primo-administration vaccine and a boost administration vaccine for use in protecting a subject from leishmaniasis and/or preventing disease progression in an infected subject, wherein the subject is a canine,
wherein the vaccines are formulated for administration in a prime-boost administration regimen comprising a primo-administration with the primo-administration vaccine followed by a boost administration with the boost administration vaccine,
wherein said primo-administration vaccine comprises, in a pharmaceutically or veterinary acceptable vehicle, diluent or excipient, an expression vector containing a polynucleotide for expressing, *in vivo,* a salivary *Lu. longipalpis* polypeptide, and
said boost administration vaccine comprises, in a pharmaceutically or veterinary acceptable vehicle or excipient, the same salivary *Lu. longipalpis* polypeptide,
wherein the salivary *Lu. longipalpis* polypeptide is a LJM17 polypeptide,
wherein the polynucleotide encodes a polypeptide having at least 80% sequence identity to a polypeptide having the sequence as set forth in SEQ ID NO: 5, 7, 15, or 17; or the polynucleotide has at least 70% sequence identity to a polynucleotide encoding a polypeptide having the sequence as set forth in SEQ ID NO: 5, 7, 15, or 17; or the polynucleotide has at least 70% sequence identity to a polynucleotide having the sequence as set forth in SEQ ID NO: 6, 8, 16, 18, 21, 90, or 91; and
wherein the *Lu. longipalpis* polypeptide comprises an amino acid sequence having at least 80% sequence identity to a polypeptide having the sequence as set forth in SEQ ID NO: 5, 7, 15, or 17.

2. The primo-administration vaccine and the boost administration vaccine for use according to claim 1, wherein the expression vector is a recombinant viral vector or a plasmid.

3. The primo-administration vaccine and the boost administration vaccine for use according to claim 1, wherein the expression vector is selected from the group consisting of pVR2001-TOPO, pVR2001-TOPA, pVR1020, pVR1012, pAB110, ALVAC, TROVAC, MVA, and baculovirus vector.

4. The primo-administration vaccine and the boost administration vaccine for use according to claim 1, wherein the vector is selected from the group consisting of pVR2001 LJM17 shown as SEQ ID NO:9, vCP2390 shown as SEQ ID NO:93, MVA-LJM17, pNBO002 shown as SEQ ID NO:19, vCP2390-SEQ ID NO:6, and mixtures thereof.

5. The primo-administration vaccine and the boost administration vaccine for use according to claim 1, wherein the expression vector is an ALVAC vector comprising SEQ ID NO: 21 or 91.

6. The primo-administration vaccine and the boost administration vaccine for use according to claim 1, wherein the polynucleotide sequence is codon optimized for the host so that the amino acid sequence of the *Lu. longipalpis* polypeptide encoded by the polynucleotide sequence is functionally unchanged.

7. The primo-administration vaccine and the boost administration vaccine for use according to any preceding claim, wherein the canine is a dog.

## Patentansprüche

1. Impfstoff für die Erstverabreichung und Impfstoff für eine Booster-Verabreichung zur Verwendung beim Schutz eines Individuums vor Leishmaniose und/oder beim Verhindern des Fortschreitens der Erkrankung in einem infizierten Individuum, wobei das Individuum hundeartig ist,
wobei die Impfstoffe für die Verabreichung in einem Dosierungsschema für eine Prime-Booster-Verabreichung formuliert sind, umfassend eine Erstverabreichung mit dem Impfstoff für die Erstverabreichung gefolgt von einer Booster-Verabreichung mit dem Impfstoff für die Booster-Verabreichung, wobei der Impfstoff für die Erstverabreichung in einem Trägerstoff, Verdünnungsmittel oder Exzipienten, der/das/der pharmazeutisch oder veterinärmedizinisch verträglich ist, einen Expressionsvektor umfasst, der ein Polynucleotid für die *in vivo*-Expression des *Lu. longipalpis-*Speichelpolypeptids enthält, und
wobei der Impfstoff für die Booster-Verabreichung in einem Trägerstoff oder Exzipienten, der pharmazeutisch oder veterinärmedizinisch verträglich ist, das gleiche Lu. longipalpis-Speichelpolypeptid umfasst,
wobei das *Lu. longipalpis*-Speichelpolypeptid ein LJM17-Polypeptid ist,
wobei das Polynucleotid ein Polypeptid codiert, das mindestens 80% Sequenzidentität mit einem Polypeptid hat, das die in SEQ ID NO:5, 7, 15 oder 17 dargestellte Sequenz hat; oder das Polynucleotid mindestens 70% Sequenzidentität mit einem Polynucleotid hat, das ein Polypeptid codiert, das die in SEQ ID NO:5, 7, 15 oder 17 dargestellte Sequenz hat; oder das Polynucleotid mindestens 70% Sequenzidentität mit einem Polynucleotid hat, das die in SEQ ID NO:6, 8, 16, 18, 21, 90 oder 91 dargestellte Sequenz hat; und
wobei das *Lu. longipalpis*-Polypeptid eine Aminosäuresequenz umfasst, die mindestens 80% Sequenzidentität mit einem Polypeptid hat, das die in SEQ ID NO:5, 7, 15 oder 17 dargestellte Sequenz hat.

2. Impfstoff für die Erstverabreichung und Impfstoff für die Booster-Verabreichung zur Verwendung nach Anspruch 1, wobei der Expressionsvektor ein rekombinanter viraler Vektor oder ein Plasmid ist.

3. Impfstoff für die Erstverabreichung und Impfstoff für die Booster-Verabreichung zur Verwendung nach Anspruch 1, wobei der Expressionsvektor ausgewählt ist aus der Gruppe bestehend aus pVR2001-TOPO, pVR2001-TOPA, pVR1020, pVR1012, pAB110, ALVAC, TROVAC, MVA und Baculovirus-Vektor.

4. Impfstoff für die Erstverabreichung und Impfstoff für die Booster-Verabreichung zur Verwendung nach Anspruch 1, wobei der Vektor ausgewählt ist aus der Gruppe bestehend aus pVR2001 LJM17, dargestellt als SEQ ID NO:9, vCP2390, dargestellt als SEQ ID NO:93, MVA-LJM17, pNBO002, dargestellt als SEQ ID NO:19, vCP2390-SEQ ID NO:6 und Gemischen davon.

5. Impfstoff für die Erstverabreichung und Impfstoff für die Booster-Verabreichung zur Verwendung nach Anspruch 1, wobei der Expressionsvektor ein ALVAC-Vektor ist, der SEQ ID NO:21 oder 91 umfasst.

6. Impfstoff für die Erstverabreichung und Impfstoff für die Booster-Verabreichung zur Verwendung nach Anspruch 1, wobei die Polynucleotidsequenz für den Wirt Codon-optimiert ist, so dass die Aminosäuresequenz des *Lu. longipalpis*-Polypeptids*,* das von der Polynucleotidsequenz codiert wird, funktionell unverändert ist.

7. Impfstoff für die Erstverabreichung und Impfstoff für die Booster-Verabreichung zur Verwendung nach einem der vorangehenden Ansprüche, wobei der Hundeartige ein Hund ist.

## Revendications

1. Vaccin de primo-administration et vaccin d'administration rappel pour une utilisation dans la protection d'un sujet contre la leishmaniose et/ou la prévention de la progression de la maladie chez un sujet infecté, dans lequel le sujet est un canidé, dans lequel les vaccins sont formulés pour une administration dans un régime d'administration de sensibilisation-rappel comprenant une primo-administration avec le vaccin de primo-administration suivie d'une administration de rappel avec le vaccin d'administration rappel,
dans lequel ledit vaccin de primo-administration comprend, dans un véhicule, un diluant ou un excipient vétérinaire ou pharmaceutiquement acceptable, un vecteur d'expression contenant un polynucléotide pour l'expression, *in vivo,* d'un polypeptide salivaire de *Lu. longipalpis,* et
ledit vaccin d'administration rappel comprend, dans un véhicule ou un excipient vétérinaire ou pharmaceutiquement acceptable, le même polypeptide salivaire de *Lu. longipalpis,*
dans lequel le polypeptide salivaire de *Lu. longipalpis* est un polypeptide LJM17, dans lequel ledit polynucléotide code pour un polypeptide présentant au moins 80 % d'identité de séquence par rapport à un polypeptide ayant la séquence telle que représentée par SEQ ID NO : 5, 7, 15 ou 17 ; ou le polynucléotide présente au moins 70 % d'identité de séquence par rapport à un polynucléotide codant pour un polypeptide ayant la séquence telle que représentée par SEQ ID NO : 5, 7, 15, ou 17 ; ou le polynucléotide présente au moins 70 % d'identité de séquence par rapport à un polynucléotide ayant la séquence telle que représentée par SEQ ID NO : 6, 8, 16, 18, 21, 90, ou 91 ; et
dans lequel le polypeptide de *Lu. longipalpis* comprend une séquence d'acides aminés présentant au moins 80 % d'identité de séquence par rapport à un polypeptide ayant la séquence telle que représentée par SEQ ID NO: 5, 7, 15 ou 17.

2. Vaccin de primo-administration et vaccin d'administration rappel pour une utilisation selon la revendication 1, dans lequel le vecteur d'expression est un vecteur viral recombinant ou un plasmide.

3. Vaccin de primo-administration et vaccin d'administration rappel pour une utilisation selon la revendication 1, dans lequel le vecteur d'expression est sélectionné parmi le groupe constitué de pVR2001-TOPO, pVR2001-TOPA, pVR1020, pVR1012, pAB110, ALVAC, TROVAC, MVA, et un vecteur de baculovirus.

4. Vaccin de primo-administration et vaccin d'administration rappel pour une utilisation selon la revendication 1, dans lequel le vecteur est sélectionné parmi le groupe constitué de pVR2001 LJM17 ayant la séquence représentée par SEQ ID NO : 9, vCP2390 ayant la séquence représentée par SEQ ID NO : 93, MVA-LJM17, pNBO002 ayant la séquence représentée par SEQ ID NO : 19, vCP2390-SEQ ID NO : 6, et des mélanges de ceux-ci.

5. Vaccin de primo-administration et vaccin d'administration rappel pour une utilisation selon la revendication 1, dans lequel le vecteur d'expression est un vecteur ALVAC comprenant SEQ ID NO : 21 ou 91.

6. Vaccin de primo-administration et vaccin d'administration rappel pour une utilisation selon la revendication 1, dans lequel la séquence polynucléotidique est optimisée au niveau des codons pour l'hôte de telle manière que la séquence d'acides aminés du polypeptide de *Lu. longipalpis* codée par la séquence polynucléotidique est fonctionnellement inchangée.

7. Vaccin de primo-administration et vaccin d'administration rappel pour une utilisation selon l'une quelconque des revendications précédentes, dans lequel le canidé est un chien.
